# EUROPEAN PATENT APPLICATION

(11) **EP 4 289 851 A1**
(43) Date of publication of application: **13.12.2023**
(21) Application number: 22749041.4
(22) Date of filing: 27.01.2022
(51) Int. Cl.: C07D 491/147, C07D 491/16, C07D 491/22, A61K 31/437, A61P 35/00

(54) **CAMPTOTHECIN COMPOUND, PREPARATION METHOD THEREFOR, AND APPLICATION THEREOF**

(30) Priority: 05.02.2021 CN 202110159956; 17.05.2021 CN 202110533304; 28.06.2021 CN 202110718245; 16.08.2021 CN 202110936768; 16.11.2021 CN 202111355330
(71) Applicant: Sichuan Kelun-Biotech Biopharmaceutical Co., Ltd., Chengdu, Sichuan 611138 (CN)
(72) Inventor: TIAN, Qiang, Chengdu, Sichuan 611138 (CN); ZHANG, Yitao, Chengdu, Sichuan 611138 (CN); MIAO, Yu, Chengdu, Sichuan 611138 (CN); WANG, Bo, Chengdu, Sichuan 611138 (CN); YE, Jian, Chengdu, Sichuan 611138 (CN); WANG, Xiaobei, Chengdu, Sichuan 611138 (CN); LI, Deliang, Chengdu, Sichuan 611138 (CN); LI, Fen, Chengdu, Sichuan 611138 (CN); SONG, Hongmei, Chengdu, Sichuan 611138 (CN)
(74) Representative: Cabinet Nony
(86) International application number: PCT/CN2022/074328
(87) International publication number: WO 2022/166762

(57) **Abstract**

The present invention relates to a Camptothecin compound having anti-tumor activity, a preparation method therefor, and an application thereof. Specifically, the present invention relates to a compound as shown below or a pharmaceutically acceptable form thereof, a pharmaceutical composition thereof, a preparation method therefor, and a use thereof. The compound can be used as a drug for treating diseases in abnormal cell proliferation,

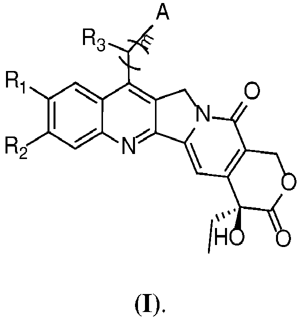

## Description

This application is based on the application with CN application number 202110159956.6 and application date of February 5, 2021, the application with CN application number 202110533304.4 and application date of May 17, 2021, the application with CN application number 202110718245.8 and application date of June 28, 2021, the application with CN application number 202110936768.X and application date of August 16, 2021, and the application with CN application number 202111355330.9 and application date of November 16, 2021, and claims their priorities, and the disclosure contents of the aforementioned CN applications are hereby incorporated in the present application in their entirety.

### Technical Field

The present invention relates to camptothecin compounds with anti-tumor activity and conjugates thereof, as well as their preparation methods and application in medical field.

### Background Art

Camptothecin (CPT, Formula 1) is a pentacyclic quinoline compound, isolated from Camptotheca acuminata of the Nyssaceae family, and consists of quinoline ring AB, pyrrole ring C, pyridone ring D, and α-hydroxylactone ring E, in which the 20-position is in S configuration (see, the structural formula below). In the early 1970s, for its excellent anticancer activity, it was used in clinical practice, but clinical trials were terminated later due to serious side effects such as diarrhea and hemorrhagic cystitis.

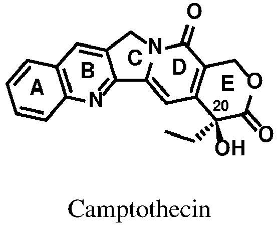

Research data show that camptothecin can form a ternary complex with cellular DNA topoisomerase I, thereby inhibiting the unwinding of DNA, resulting in the blockage of DNA replication and cell death (Cancer Res. 1989, 49, 6365)*.* Camptothecin and its derivatives have potent anti-tumor activity in animal models with lung cancer, breast cancer, colorectal cancer, ovarian cancer, etc. (Nature Review Cancer. 2006, 6, 789)*.*

At present, several camptothecin drugs have been approved for marketing for tumor treatment *(*Med. Res. Rev. 2015, 35, 753). Irinotecan is used for the treatment of colorectal cancer; Topotecan is used for the treatment of ovarian cancer; and Belotecan is used for the treatment of ovarian cancer and small cell lung cancer. Camptothecin derivatives further include Exatecan, Rubitecan, Karenitecan, Diflomotecan, Lurtotecan, Gimatecan, Namitecan, Simmitecan, Silatecan, Chimmitecan, Elomotecan, etc.

Camptothecin drugs or derivatives thereof often have myelosuppression-induced blood toxicity, such as neutropenia, leukopenia, thrombocytopenia, anemia, etc., as well as gastrointestinal side effects, such as nausea, vomiting, and diarrhea. Clinical studies have found that measures to improve the safety and effectiveness of camptothecin compounds include improving their pharmacokinetic properties, regulating activity, reducing dosage, or using their conjugates and antibodies to form antibody-conjugated drugs, etc. Therefore, there is still a high clinical demand and application value for developing camptothecin compounds and their conjugates with novel structures, improved efficacy and improved safety.

### Contents of the present invention

The present invention provides a novel camptothecin compound and a conjugate thereof. The camptothecin compound has good antitumor activity and is expected to be used in the treatment of tumor diseases; the conjugate thereof has a wide application prospect in ADC drugs.

The first aspect of the present invention provides a compound or a pharmaceutically acceptable salt, ester, stereoisomer, polymorph, solvate, nitrogen oxide, isotope-labeled product, metabolite or prodrug thereof, wherein the compound has the structure shown below: 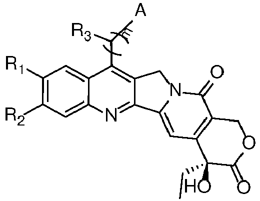 wherein,
R₁ and R₂ are each independently selected from the group consisting of hydrogen, halogen, C₁₋₆ alkyl, C₁₋₆ alkoxyl, C₁₋₆ haloalkyl, hydroxyl, cyano and C₃₋₆ cycloalkyl; or, R₁ and R₂ are connected with adjacent carbon atoms to form a 5- to 6-membered oxygen-containing heterocyclic ring;
R₃ is hydrogen or is connected with an ortho-carbon atom of R₁ to form a 6-membered carbocyclic ring;
A is selected from one of 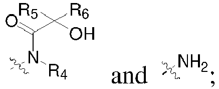
R₄ is selected from the group consisting of hydrogen, C₁₋₆ alkyl, C₁₋₆ haloalkyl, C₁₋₆ alkoxyalkyl, C₃₋₆ cycloalkyl and 3- to 6-membered heterocyclyl;
R₅ and R₆ are each independently selected from the group consisting of hydrogen, C₁₋₆ alkyl, C₁₋₆ haloalkyl, C₁₋₆ alkylaminoalkyl, C₁₋₆ alkoxyalkyl, C₂₋₆ alkenyl, C₂₋₆ alkynyl, C₃₋₆ cycloalkyl, 3- to 6-membered heterocyclyl, 3- to 6-membered heterocyclyl alkyl, aryl and heteroaryl; or R₅ and R₆ are connected with adjacent carbon atoms to form a 3- to 6-membered carbocyclic or heterocyclic ring;
m=1 or 2.

In some embodiments, the compound has the structure of Formula (I): 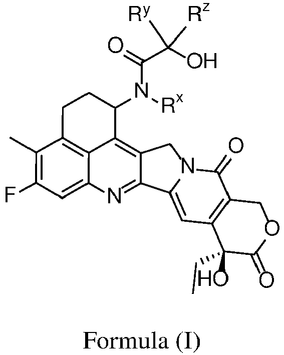 in Formula (I), R^{x} is selected from the group consisting of hydrogen, C₁₋₆ alkyl, C₁₋₆ haloalkyl, C₁₋₆ alkoxyalkyl, C₃₋₆ cycloalkyl and 3- to 6-membered heterocyclyl;
R^{y} and R^{z} are not hydrogen at the same time, and are independently selected from the group consisting of hydrogen, C₁₋₆ alkyl, C₁₋₆ haloalkyl, C₂₋₆ alkenyl, C₂₋₆ alkynyl, C₁₋₆ alkylaminoalkyl, C₁₋₆ alkoxyalkyl, 3- to 6-membered heterocyclylalkyl and 3- to 6-membered heterocyclyl.

In some embodiments, R^{x} is selected from the group consisting of hydrogen, C₁₋₆ alkyl, C₁₋₆ haloalkyl, C₁₋₆ alkoxyalkyl, C₃₋₆ cycloalkyl and 3- to 6-membered heterocyclyl;
R^{y} and R^{z} are not hydrogen at the same time, and are independently selected from the group consisting of hydrogen, C₂₋₆ alkenyl, C₂₋₆ alkynyl, C₁₋₆ alkylaminoalkyl, C₁₋₆ alkoxyalkyl, 3- to 6-membered heterocyclylalkyl and 3- to 6-membered heterocyclyl.

In some embodiments, in Formula (I), R^{x} is selected from the group consisting of hydrogen, C₁₋₆ alkyl, C₁₋₆ haloalkyl, C₁₋₆ alkoxyalkyl, C₃₋₆ cycloalkyl, 3- to 6-membered heterocyclyl;
R^{y} and R^{z} are not hydrogen at the same time, and are independently selected from the group consisting of hydrogen, C₂₋₆ alkenyl, and C₂₋₆ alkynyl.

In some embodiments, R^{x} is selected from the group consisting of hydrogen or C₁₋₆ alkyl.

In some embodiments, R^{x} is hydrogen.

In some embodiments, R^{y} and R^{z} are not hydrogen at the same time, and are independently selected from the group consisting of hydrogen, 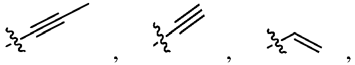 dimethylaminomethylene, morpholinomethylene, and methoxymethylene.

In some embodiments, R^{y} and R^{z} are not hydrogen at the same time, and are independently selected from the group consisting of hydrogen, dimethylaminomethylene, and methoxymethylene.

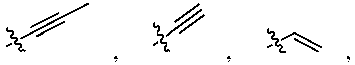

In some embodiments, R^{y} is hydrogen, and R^{z} is selected from the group consisting of 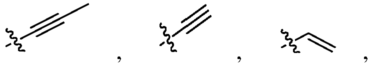 dimethylaminomethylene, morpholinomethylene, and methoxymethylene.

In some embodiments, R^{y} is hydrogen, and R^{z} is selected from the group consisting of 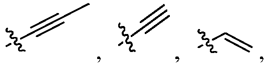 dimethylaminomethylene, and methoxymethylene.

In some embodiments, R^{x} is hydrogen, R^{y} is hydrogen, and R^{z} is selected from the group consisting of 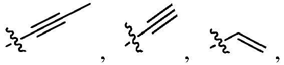 dimethylaminomethylene, morpholinomethylene, and methoxymethylene.

In some embodiments, R^{x} is hydrogen, R^{y} is hydrogen, and R^{z} is selected from the group consisting of 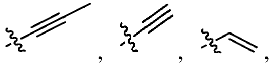 dimethylaminomethylene, and methoxymethylene.

In some embodiments, R^{x} is hydrogen, R^{y} is hydrogen, and R^{z} is selected from the group consisting of 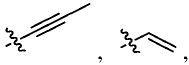 and dimethylaminomethylene.

In some embodiments, in Formula (I), 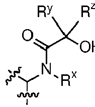 has the configuration of

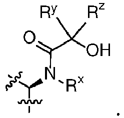

In some embodiments, in Formula (I), 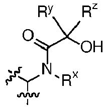 has the configuration of

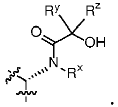

In some embodiments, in Formula (I), 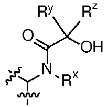 has the configuration of

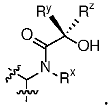

In some embodiments, in Formula (I), 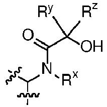 has the configuration of

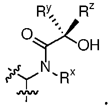

In some embodiments, in Formula (I), 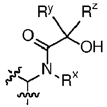 has the configuration of

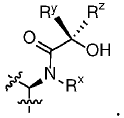

In some embodiments, in Formula (I), 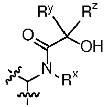 has the configuration of

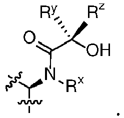

In some embodiments, in Formula (I), 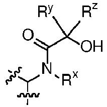 has the configuration of

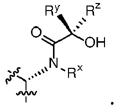

In some embodiments, in Formula (I), 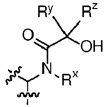 has the configuration of

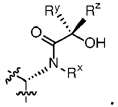

In some embodiments, the compound has the structure of Formula (II):

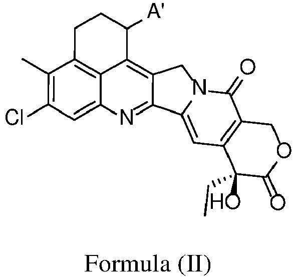

in Formula (II), A' is selected from one of 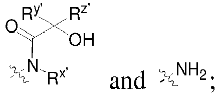
R^{x'} is selected from the group consisting of hydrogen, C₁₋₆ alkyl, C₁₋₆ haloalkyl, C₁₋₆ alkoxyalkyl, C₃₋₆ cycloalkyl and 3- to 6-membered heterocyclyl;
R^{y'} and R^{z'} are independently selected from the group consisting of hydrogen, C₁₋₆ alkyl, C₁₋₆ haloalkyl, C₁₋₆ alkoxyalkyl, C₁₋₆ alkylaminoalkyl, C₃₋₆ cycloalkyl, 3- to 6-membered heterocyclyl, 3- to 6-membered heterocyclylalkyl, C₂₋₆ alkenyl, C₂₋₆ alkynyl, aryl and heteroaryl, or R^{y'} and R^{z'} are connected with adjacent carbon atoms to form a 3- to 6-membered ring.

In some embodiments, the structure of Formula (II) is as shown in the following Formula (II)-1:

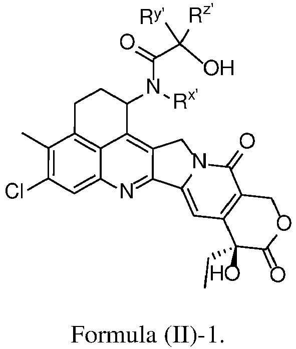

In some embodiments, R^{x'} is selected from the group consisting of hydrogen, C₁₋₆ alkyl, C₁₋₆ haloalkyl, C₁₋₆ alkoxyalkyl, C₃₋₆ cycloalkyl, 3- to 6-membered heterocyclyl;
R^{y'} and R^{z'} are independently selected from the group consisting of hydrogen, C₁₋₆ alkyl, C₁₋₆ haloalkyl, C₁₋₆ alkoxyalkyl, C₃₋₆ cycloalkyl, 3- to 6-membered heterocyclyl, C₂₋₆ alkenyl, C₂₋₆ alkynyl, aryl, heteroaryl, or R^{y'} and R^{z'} are connected with adjacent carbon atoms to form a 3- to 6-membered ring.

In some embodiments, the 3- to 6-membered ring is a 3- to 6-membered carbocyclic ring or a 3- to 6-membered heterocyclic ring.

In some embodiments, R^{x'} is selected from the group consisting of hydrogen and C₁₋₆ alkyl.

In some embodiments, R^{x'} is selected from the group consisting of hydrogen and methyl.

In some embodiments, R^{y'} and R^{z'} are independently selected from the group consisting of hydrogen, C₁₋₆ alkyl, C₁₋₆ alkoxyalkyl, C₁₋₆ alkylaminoalkyl, C₃₋₆ cycloalkyl and C₂₋₆ alkenyl, or R^{y'} and R^{z'} are connected with adjacent carbon atoms to form a 3- to 6-membered cycloalkyl.

In some embodiments, R^{y'} is selected from the group consisting of hydrogen and C₁₋₆ alkyl, R^{z'} is selected from the group consisting of hydrogen, C₁₋₆ alkyl and C₃₋₆ cycloalkyl, or R^{y'} and R^{z'} are connected with adjacent carbon atoms to form a 3- to 6-membered ring.

In some embodiments, R^{y'} is selected from the group consisting of hydrogen and methyl, R^{z'} is selected from the group consisting of hydrogen, methyl and cyclopropyl, or R^{y'} and R^{z'} are connected with adjacent carbon atoms to form a 3-membered carbocyclic ring.

In some embodiments, R^{x'} is selected from the group consisting of hydrogen and methyl, R^{y'} is selected from the group consisting of hydrogen and methyl, R^{z'} is selected from the group consisting of hydrogen, methyl and cyclopropyl, or R^{y'} and R^{z'} are connected with adjacent carbon atoms to form a 3-membered carbocyclic ring.

In some embodiments, R^{x'} is hydrogen, R^{y'} is selected from the group consisting of hydrogen and methyl, R^{z'} is selected from the group consisting of hydrogen, methyl and cyclopropyl, or R^{y'} and R^{z'} are connected with adjacent carbon atoms to form a 3-membered carbocyclic ring.

In some embodiments, A' in Formula (II) is

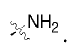

In some embodiments, in Formula (II)-1, 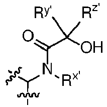 has the configuration of

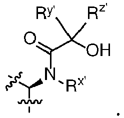

In some embodiments, in Formula (II)-1, 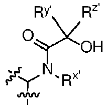 has the configuration of

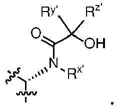

In some embodiments, in Formula (II)-1, 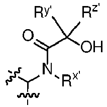 has the configuration of

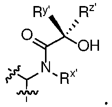

In some embodiments, in Formula (II)-1, 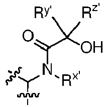 has the configuration of

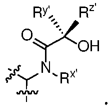

In some embodiments, in Formula (II)-1, 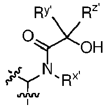 has the configuration of

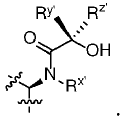

In some embodiments, in Formula (II)-1, 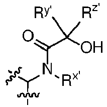 has the configuration of

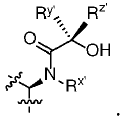

In some embodiments, in Formula (II)-1, 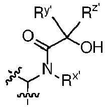 has the configuration of

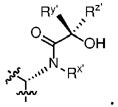

In some embodiments, in Formula (II)-1, 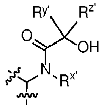 has the configuration of

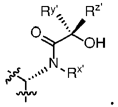

In some embodiments, the compound has the structure of Formula (III): 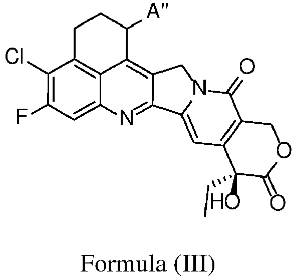 in Formula (III), A" is selected from one of 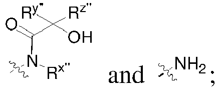
R^{x"} is selected from the group consisting of hydrogen, C₁₋₆ alkyl, C₁₋₆ haloalkyl, C₁₋₆ alkoxyalkyl, C₃₋₆ cycloalkyl and 3- to 6-membered heterocyclyl;
R^{y"} and R^{z"} are independently selected from the group consisting of hydrogen, C₁₋₆ alkyl, C₁₋₆ haloalkyl, C₁₋₆ alkoxyalkyl, C₁₋₆ alkylaminoalkyl, C₃₋₆ cycloalkyl, 3- to 6-membered heterocyclyl, 3- to 6-membered heterocyclylalkyl, 4- to 6-membered heterocyclyl, C₂₋₆ alkenyl, C₂₋₆ alkynyl, aryl and heteroaryl, or R^{y"} and R^{z"} are connected with adjacent carbon atoms to form a 3- to 6-membered ring.

In some embodiments, the structure of the compound of Formula (III) is as shown in the following Formula (III)-1:

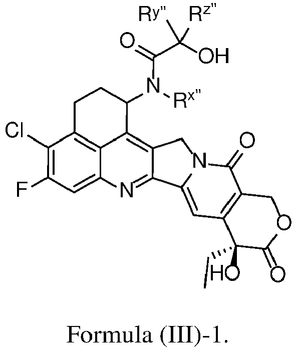

In some embodiments, R^{x"} is selected from the group consisting of hydrogen, C₁₋₆ alkyl, C₁₋₆ haloalkyl, C₁₋₆ alkoxyalkyl, C₃₋₆ cycloalkyl, 3- to 6-membered heterocyclyl;
R^{y"} and R^{z"} are independently selected from the group consisting of hydrogen, C₁₋₆ alkyl, C₁₋₆ haloalkyl, C₁₋₆ alkoxyalkyl, C₃₋₆ cycloalkyl, 4- to 6-membered heterocyclyl, C₂₋₆ alkenyl, C₂₋₆ alkynyl, aryl, heteroaryl, or R^{y"} and R^{z"} are connected with adjacent carbon atoms to form a 3- to 6-membered ring.

In some embodiments, R^{x"} is selected from the group consisting of hydrogen and C₁₋₆ alkyl.

In some embodiments, R^{x"} is hydrogen.

In some embodiments, the 3- to 6-membered ring is a 3- to 6-membered carbocyclic ring or a 3- to 6-membered heterocyclic ring.

In some embodiments, R^{y"} and R^{z"} are each independently selected from the group consisting of hydrogen, C₁₋₆ alkyl, C₁₋₆ haloalkyl, C₁₋₆ alkoxyalkyl, C₁₋₆ alkylaminoalkyl, C₃₋₆ cycloalkyl and vinyl, or R^{y"} and R^{z"} are connected with adjacent carbon atoms to form a 3- to 6-membered ring.

In some embodiments, R^{y"} is hydrogen, R^{z"} is selected from the group consisting of hydrogen, C₁₋₆ alkyl, C₃₋₆ cycloalkyl and vinyl, or R^{y"} and R^{z"} are connected with adjacent carbon atoms to form a 3- to 6-membered carbocyclic ring.

In some embodiments, R^{y"} is hydrogen, and R^{z"} is selected from the group consisting of hydrogen, methyl, cyclopropyl, and vinyl, or R^{y"} and R^{z"} are connected with adjacent carbon atoms to form a 3-membered carbocyclic ring.

In some embodiments, R^{x"} is hydrogen, R^{y"} is hydrogen, R^{z"} is selected from the group consisting of hydrogen, methyl, cyclopropyl, and vinyl, or R^{y"} and R^{z"} are connected with adjacent carbon atoms to form a 3-membered carbocyclic ring.

In some embodiments, A" in Formula (III) is

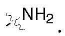

In some embodiments, in Formula (III)-1, 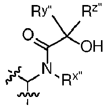 has the configuration of

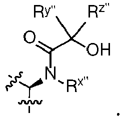

In some embodiments, in Formula (III)-1, 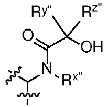 has the configuration of

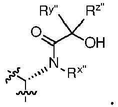

In some embodiments, in Formula (III)-1, 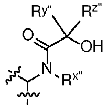 has the configuration of

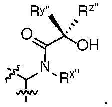

In some embodiments, in Formula (III)-1, 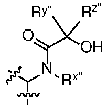 has the configuration of

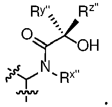

In some embodiments, in Formula (III)-1, 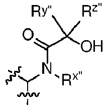 has the configuration of

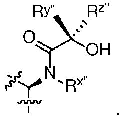

In some embodiments, in Formula (III)-1, 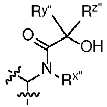 has the configuration of

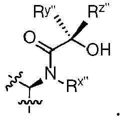

In some embodiments, in Formula (III)-1, 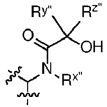 has the configuration of

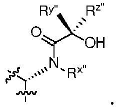

In some embodiments, in Formula (III)-1, 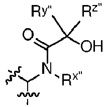 has the configuration of

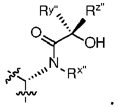

In some embodiments, the compound has the structure of Formula (IV): 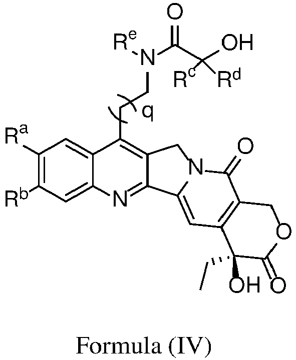 in Formula (IV),
R^{a} and R^{b} are independently selected from the group consisting of hydrogen, halogen, C₁₋₆ alkyl, C₁₋₆ hydroxyalkyl, C₁₋₆ alkoxyalkyl, C₁₋₆ alkoxy, C₁₋₆ haloalkyl, hydroxyl and cyano; or R^{a} and R^{b} are connected with adjacent carbon atoms to form a 5- to 6-membered oxygen-containing heterocyclic ring;
R^{c} and R^{d} are independently selected from the group consisting of hydrogen, C₁₋₆ alkyl, C₁₋₆ haloalkyl, C₁₋₆ alkoxyalkyl, C₁₋₆ alkylaminoalkyl, C₃₋₆ cycloalkyl, 3- to 6-membered heterocyclyl, 3- to 6-membered heterocyclylalkyl, C₂₋₆ alkenyl and C₂₋₆ alkynyl, or R^{c} and R^{d} are connected with adjacent carbon atoms to form a 3- to 6-membered carbocyclic or heterocyclic ring;
R^{e} is selected from the group consisting of hydrogen, C₁₋₆ alkyl, C₃₋₆ cycloalkyl, C₁₋₆ haloalkyl, C₁₋₆ alkoxyalkyl and C₂-C₅ heterocyclyl;
q=0 or 1;
when q=0, R^{c} and R^{d} are not hydrogen at the same time.

In some embodiments, R^{a} and R^{b} are independently selected from the group consisting of hydrogen, halogen, C₁₋₆ alkyl, C₁₋₆ alkoxy, C₁₋₆ haloalkyl, hydroxyl, and cyano; or R^{a} and R^{b} are connected with adjacent carbon atoms to form a 5- to 6-membered oxygen-containing heterocyclic ring;
R^{c} and R^{d} are independently selected from the group consisting of hydrogen, C₁₋₆ alkyl, C₁₋₆ haloalkyl, C₁₋₆ alkoxyalkyl, C₁₋₆ alkylaminoalkyl, C₃₋₆ cycloalkyl, 3- to 6-membered heterocyclyl, 3- to 6-membered heterocyclylalkyl, C₂₋₆ alkenyl and C₂₋₆ alkynyl, or R^{c} and R^{d} are connected with adjacent carbon atoms to form a 3- to 6-membered carbocyclic or heterocyclic ring;
R^{e} is selected from the group consisting of hydrogen, C₁₋₆ alkyl, C₃₋₆ cycloalkyl, C₁₋₆ haloalkyl, C₁₋₆ alkoxyalkyl and 4- to 6-membered heterocyclyl;
q=0 or 1;
when q=0, R^{c} and R^{d} are not hydrogen at the same time.

In some embodiments, in Formula (IV), R^{a} and R^{b} are independently selected from the group consisting of hydrogen, halogen, C₁₋₆ alkyl, C₁₋₆ alkoxy, C₁₋₆ haloalkyl, hydroxyl, and cyano; or R^{a} and R^{b} are connected with adjacent carbon atoms to form a 5- to 6-membered oxygen-containing heterocyclic ring;
R^{c} and R^{d} are independently selected from the group consisting of hydrogen, C₁₋₆ alkyl, C₃₋₆ cycloalkyl, C₁₋₆ haloalkyl, C₁₋₆ alkoxyalkyl, 4- to 6-membered heterocyclyl, C₂₋₆ alkenyl, C₂₋₆ alkynyl, or R^{c} and R^{d} are connected with adjacent carbon atoms to form a 3- to 6-membered carbocyclic or heterocyclic ring;
R^{e} is selected from the group consisting of hydrogen, C₁₋₆ alkyl, C₃₋₆ cycloalkyl, C₁₋₆ haloalkyl, C₁₋₆ alkoxyalkyl, or 4- to 6-membered heterocyclyl;
q=0 or 1;
when q=0, R^{c} and R^{d} are not hydrogen at the same time.

In some embodiments, R^{a} and R^{b} are independently selected from the group consisting of hydrogen, halogen and C₁₋₆ alkyl, or R^{a} and R^{b} are connected with adjacent carbon atoms to form a 5- to 6-membered oxygen-containing heterocyclic ring.

In some embodiments, R^{a} and R^{b} are independently selected from the group consisting of hydrogen, fluorine, chlorine, and methyl, or R^{a} and R^{b} together with a benzene ring attached thereto form 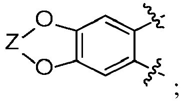 wherein Z is selected from the group consisting of -CH₂-, -CD₂-, -CH₂CH₂- and -CF₂-.

In some embodiments, R^{a} is methyl and R^{b} is fluorine, or R^{a} and R^{b} together with a benzene ring attached thereto form

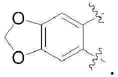

In some embodiments, R^{c} and R^{d} are independently selected from the group consisting of hydrogen, 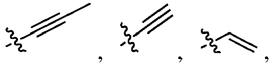 C₁₋₆ alkoxyalkyl and C₁₋₆ alkylaminoalkyl, or R^{c} and R^{d} are connected with adjacent carbon atoms to form a 3-6 member carbocyclic ring.

In some embodiments, R^{c} is hydrogen, R^{d} is selected from the group consisting of hydrogen, 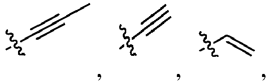 methoxyethyl and cyclopropyl, or R^{c} and R^{d} are connected with adjacent carbon atoms to form a 3- to 6-membered carbocyclic ring.

In some embodiments, R^{e} is selected from the group consisting of hydrogen and C₁₋₆ alkyl.

In some embodiments, R^{e} is selected from the group consisting of hydrogen and isopropyl.

In some embodiments, R^{a} is methyl, R^{b} is fluorine, or R^{a} and R^{b} together with a benzene ring attached thereto form 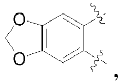 R^{e} is selected from the group consisting of hydrogen and isopropyl, R^{c} is hydrogen, R^{d} is selected from the group consisting of hydrogen, 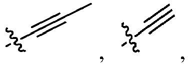 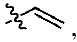 methoxyethyl and cyclopropyl, or R^{c} and R^{d} are connected with adjacent carbon atoms to form a 3-membered carbocyclic ring.

In some embodiments, R^{a} is methyl, R^{b} is fluorine, or R^{a} and R^{b} together with a benzene ring attached thereto form 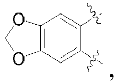 R^{e} is selected from the group consisting of hydrogen and isopropyl, R^{c} is hydrogen, R^{d} is selected from the group consisting of hydrogen, methoxyethyl and cyclopropyl, or R^{c} and R^{d} are connected with adjacent carbon atoms to form a 3-membered carbocyclic ring.

In some embodiments, in Formula (IV), 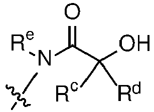 has the configuration of

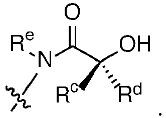

In some embodiments, in Formula (IV), 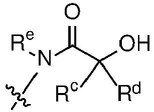 has the configuration of

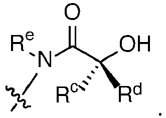

In some embodiments, the compound has the structure of Formula (V): 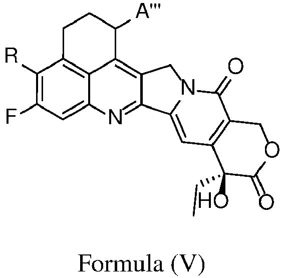 in Formula (V), R is selected from the group consisting of C₃₋₆ cycloalkyl and C₁₋₆ alkoxy;
A‴ is selected from one of 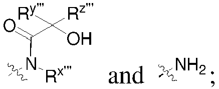
R^{x‴} is selected from the group consisting of hydrogen, C₁₋₆ alkyl, C₁₋₆ haloalkyl, C₁₋₆ alkoxyalkyl, C₃₋₆ cycloalkyl and 3- to 6-membered heterocyclyl;
R^{y‴} and R^{z‴} are independently selected from the group consisting of hydrogen, C₁₋₆ alkyl, C₁₋₆ haloalkyl, C₁₋₆ alkoxyalkyl, C₁₋₆ alkylaminoalkyl, C₃₋₆ cycloalkyl, 3- to 6-membered heterocyclyl, 3- to 6-membered heterocyclylalkyl, C₂₋₆ alkenyl, C₂₋₆ alkynyl, aryl and heteroaryl, or R^{y‴} and R^{z‴} are connected with adjacent carbon atoms to form a 3- to 6-membered ring.

In some embodiments, R is selected from the group consisting of methoxy and cyclopropyl.

In some embodiments, the structure of the compound of Formula (V) is as shown in the following Formula (V)-1:

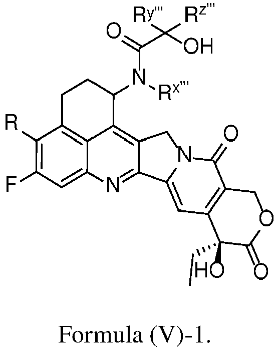

In some embodiments, the 3- to 6-membered ring is a 3- to 6-membered carbocyclic ring or a 3- to 6-membered heterocyclic ring.

In some embodiments, R^{y‴} and R^{z‴} are each independently selected from the group consisting of hydrogen, C₁₋₆ alkyl, C₁₋₆ haloalkyl, C₁₋₆ alkoxyalkyl, C₁₋₆ alkylaminoalkyl, C₃₋₆ cycloalkyl and vinyl, or R^{y‴} and R^{z‴} are connected with adjacent carbon atoms to form a 3- to 6-membered carbocyclic ring.

In some embodiments, both R^{y‴} and R^{z‴} are hydrogen, or R^{y‴} and R^{z‴} are connected with adjacent carbon atoms to form a 3- to 6-membered carbocyclic ring.

In some embodiments, R^{x‴} is selected from the group consisting of hydrogen and C₁₋₆ alkyl.

In some embodiments, R^{x‴} is hydrogen.

In some embodiments, A‴ is

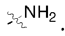

In some embodiments, R is selected from the group consisting of methoxy and cyclopropyl, R^{x‴} is hydrogen, both R^{y‴} and R^{z‴} are hydrogen, or R^{y‴} and R^{z‴} are connected with adjacent carbon atoms to form a 3-membered carbocyclic ring.

In some embodiments, in Formula (V)-1, 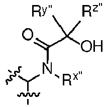 has the configuration of

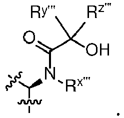

In some embodiments, in Formula (V)-1, 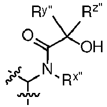 has the configuration of

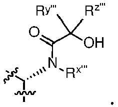

In some embodiments, in Formula (V)-1, 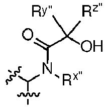 has the configuration of

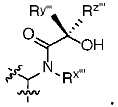

In some embodiments, in Formula (V)-1, 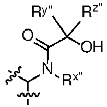 has the configuration of

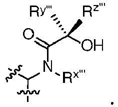

In some embodiments, in Formula (V)-1, 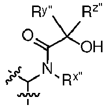 has the configuration of

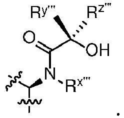

In some embodiments, in Formula (V)-1, 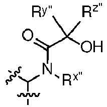 has the configuration of

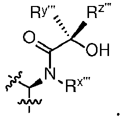

In some embodiments, in Formula (V)-1, 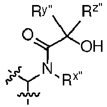 has the configuration of

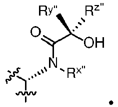

In some embodiments, in Formula (V)-1, 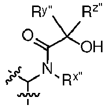 has the configuration of

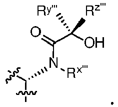

In some embodiments, the present invention provides the following compounds:

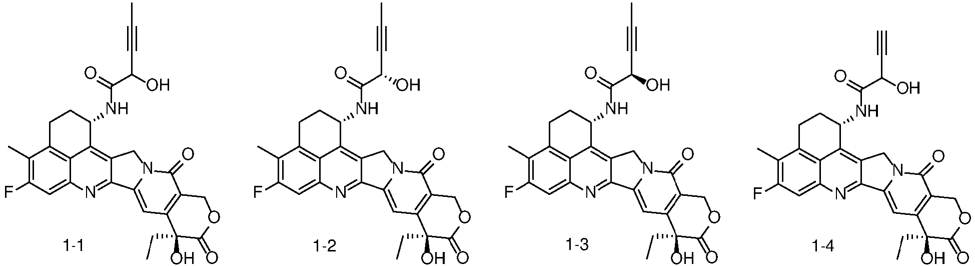

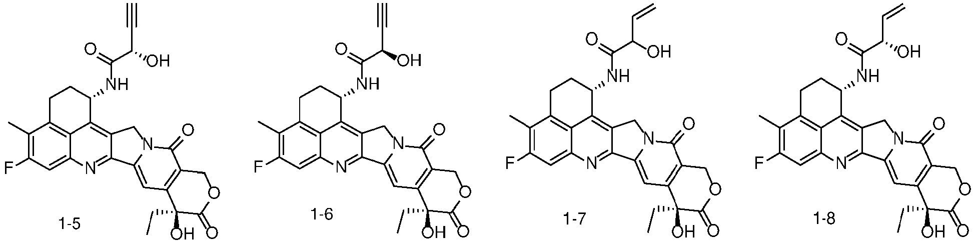

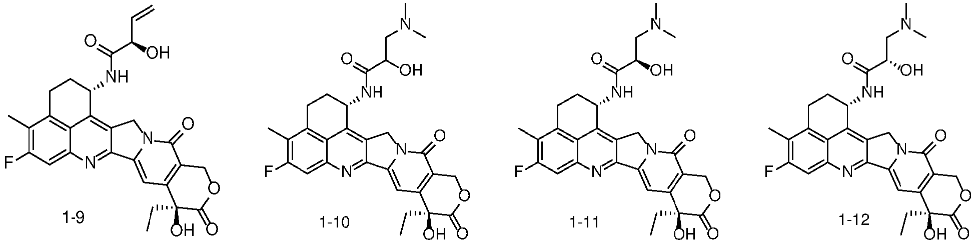

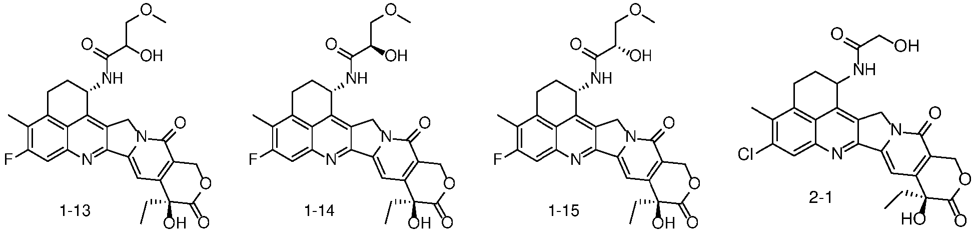

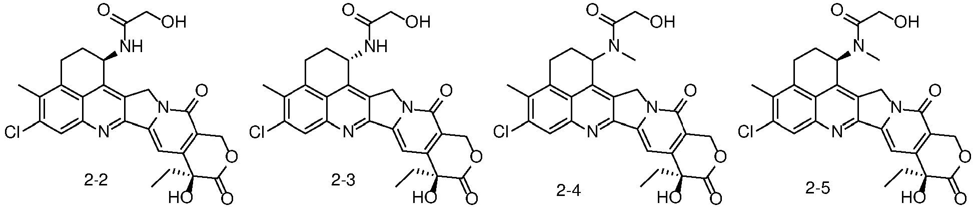

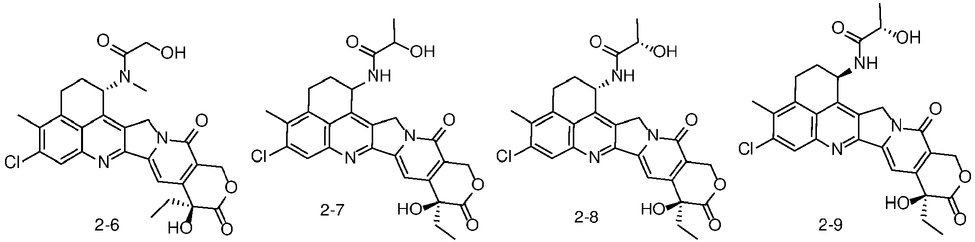

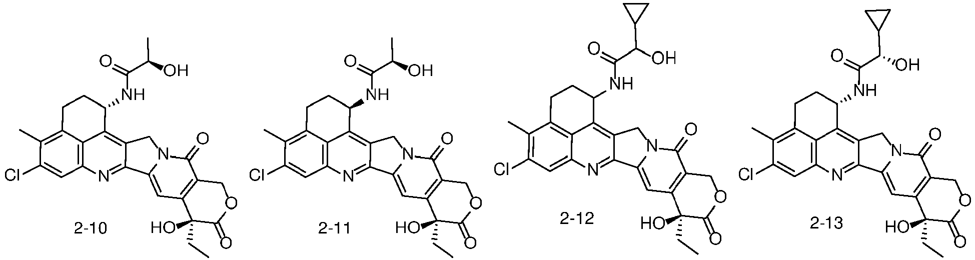

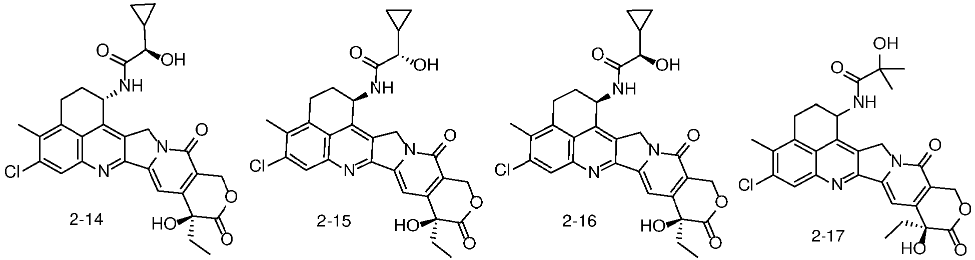

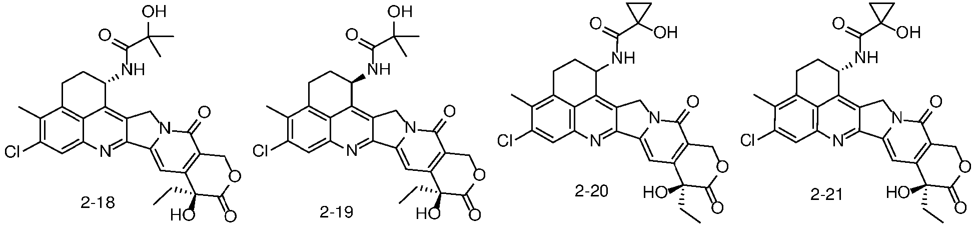

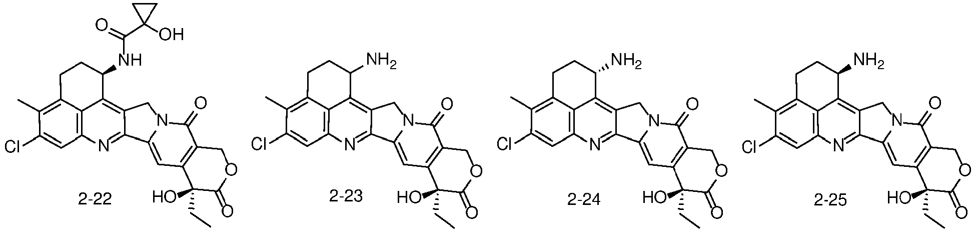

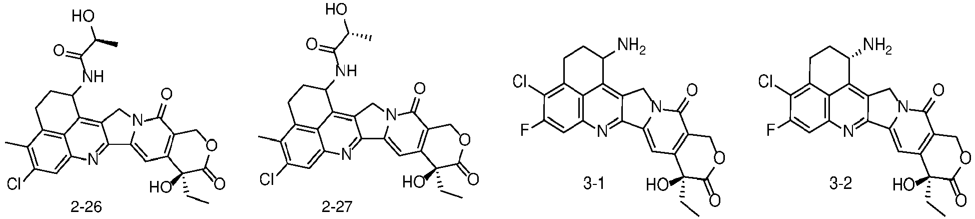

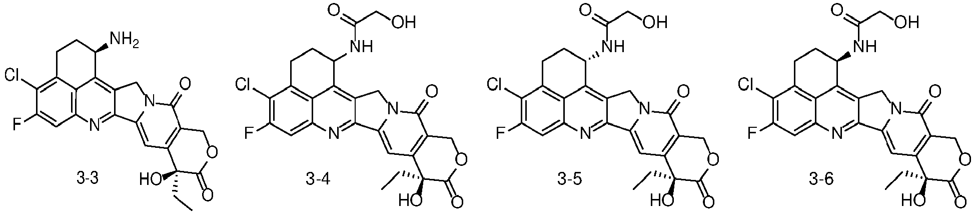

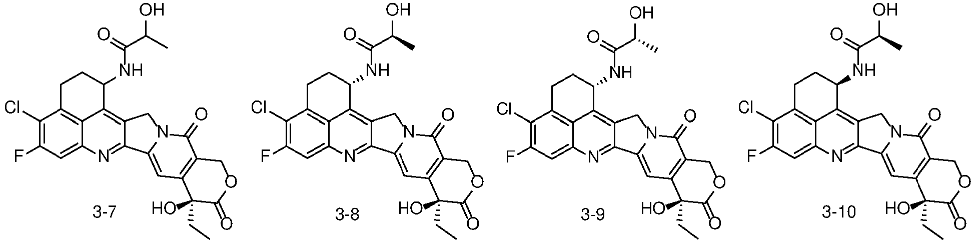

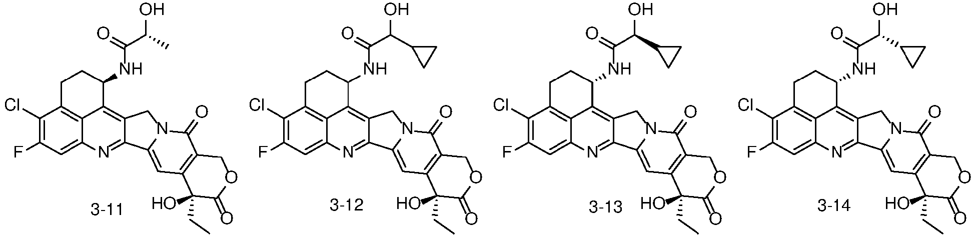

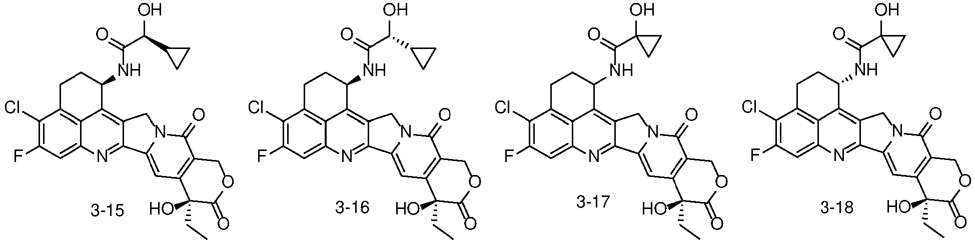

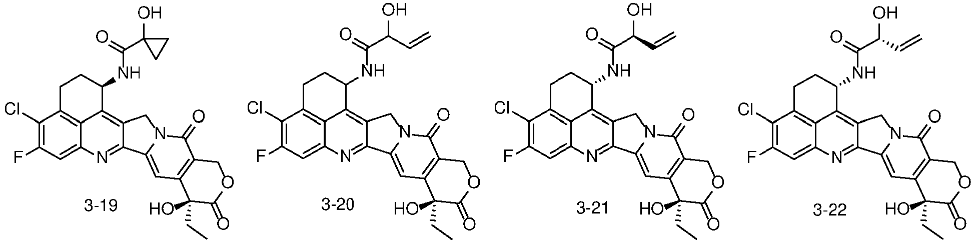

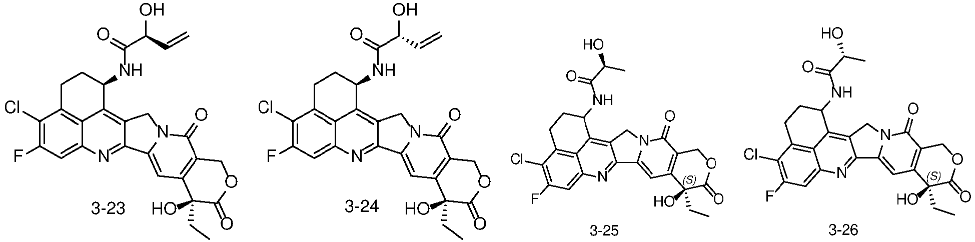

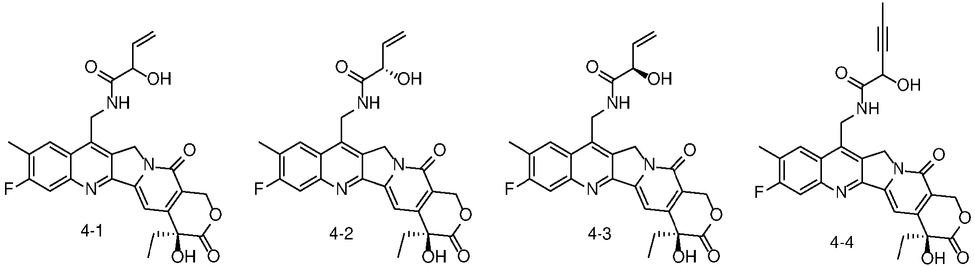

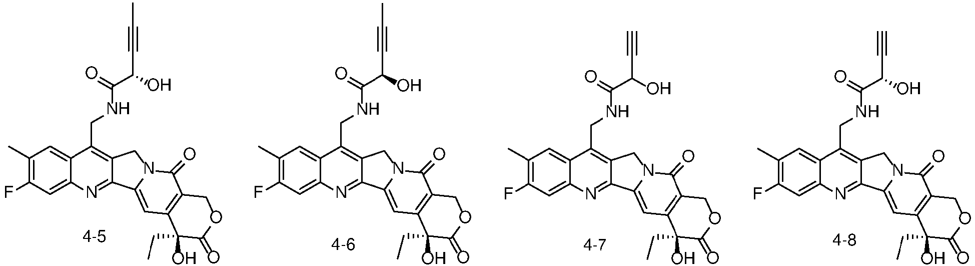

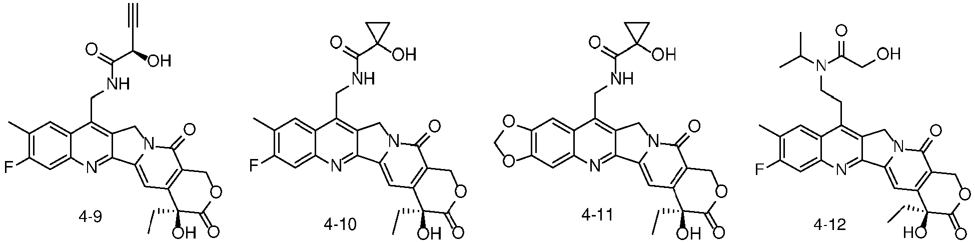

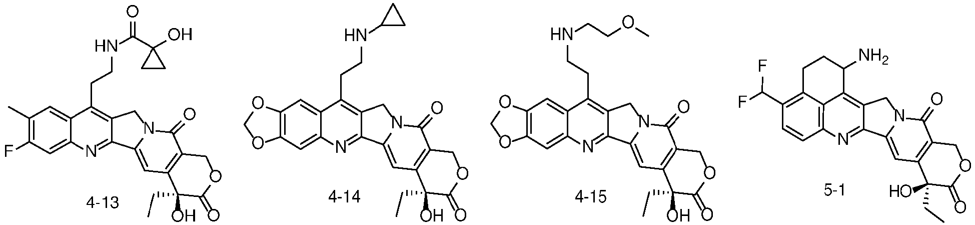

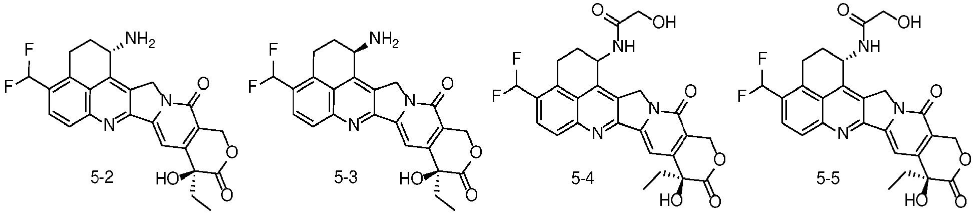

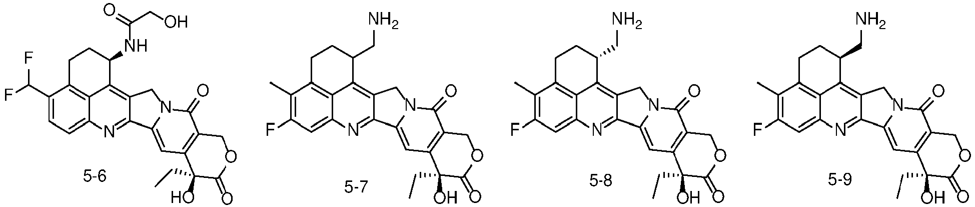

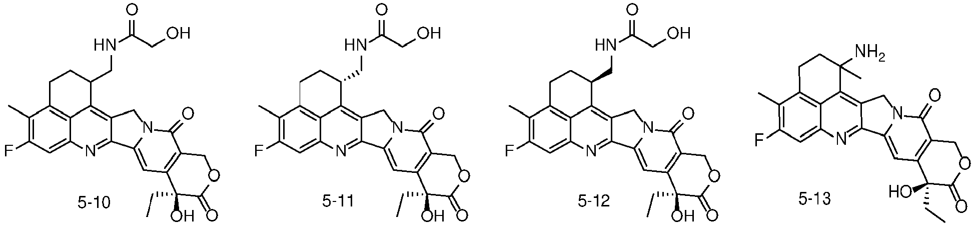

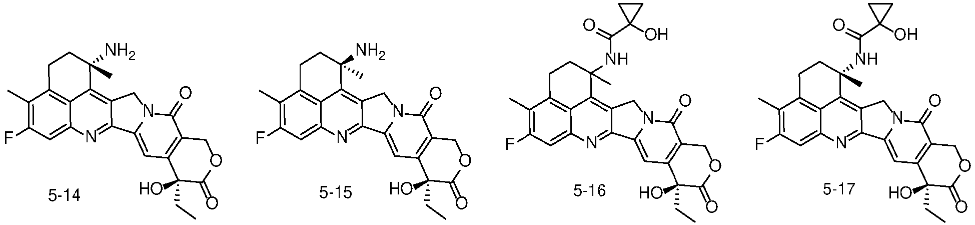

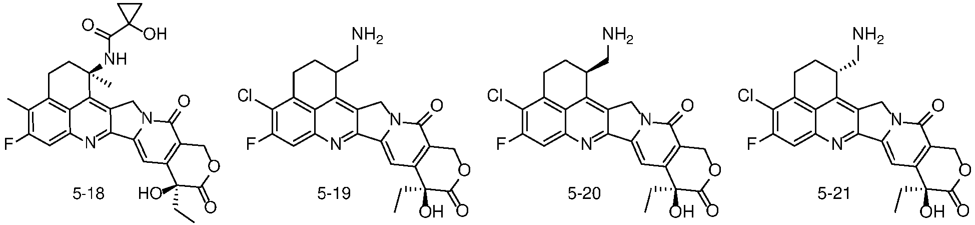

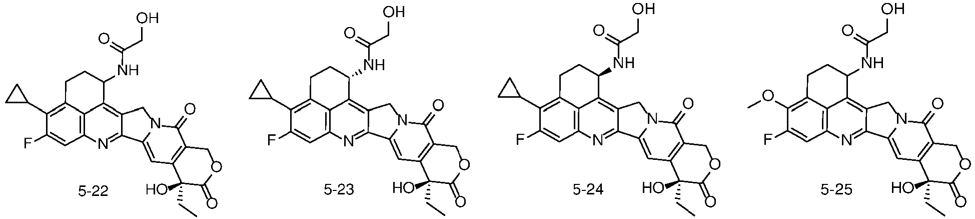

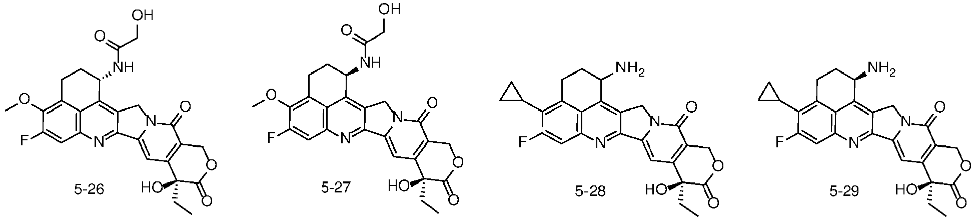

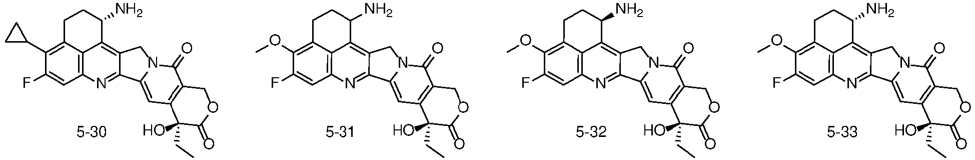

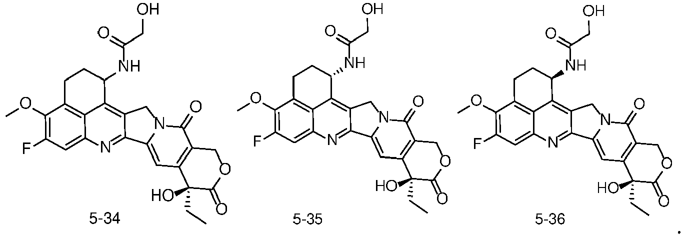

On the other hand, the present invention also provides a compound of Formula (VI) or a pharmaceutically acceptable salt, ester, stereoisomer, polymorph, solvate, nitrogen oxide, isotope-labeled product, metabolite or prodrug thereof:

M-L-E-D Formula (VI)

wherein,
M is a linker moiety of an antibody or antigen-binding fragment thereof;
L is a linker connecting the linker moiety M and E;
E is a structural fragment connecting L and D;
D is a structural fragment of a cytotoxic drug.

In some embodiments, M is selected from the group consisting of the following structures:

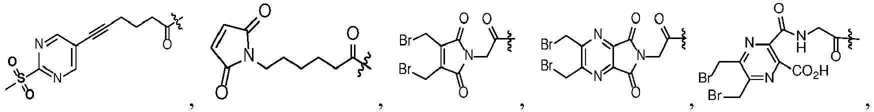

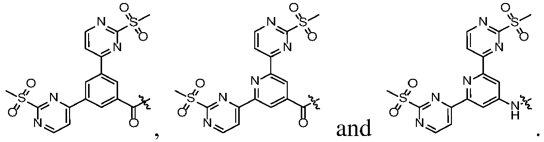

In some embodiments, M is selected from the group consisting of the following structures:

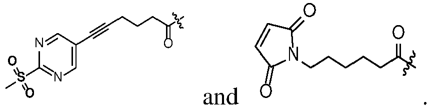

In some embodiments, L is a divalent structure constituted of one or more selected from the group consisting of: C₁₋₆ alkylene, -N(R')-, carbonyl, -O-, Val, Cit, Phe, Lys, D-Val, Leu, Gly, Ala, Asn, Val-Cit, Val-Ala, Val-Lys, Val-Lys(Ac), Phe-Lys, Phe-Lys(Ac), D-Val-Leu-Lys, Gly-Gly-Arg, Ala-Ala-Asn, Ala-Ala-Ala, Val-Lys-Ala, Gly-Gly-Gly, Gly-Gly-Phe-Gly, Gly-Gly-Gly-Gly-Gly, 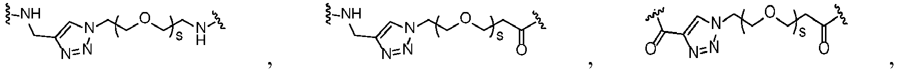 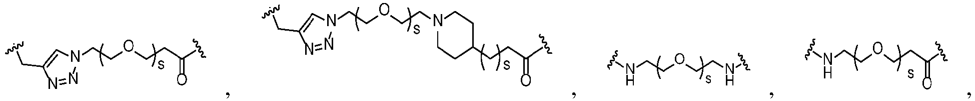 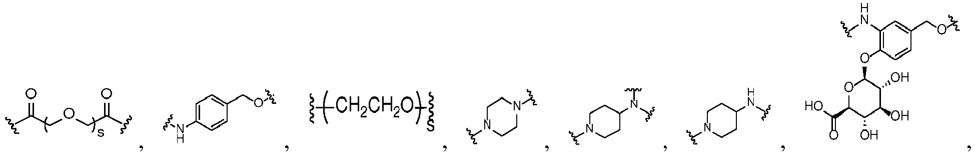 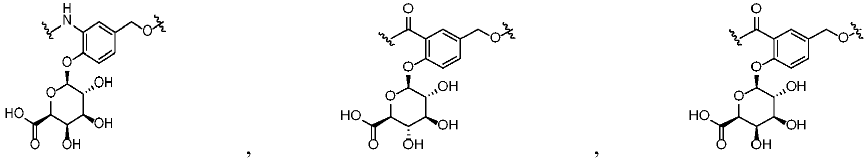 and 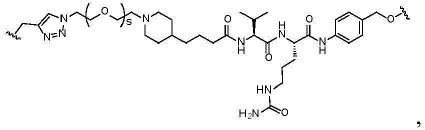 wherein R' represents hydrogen, C₁₋₆ alkyl, or alkyl containing -(CH₂CH₂O)ᵣ-; r is an integer selected from 1 to 10; s is an integer selected from 1 to 10.

In some embodiments, L is selected from the following structures: 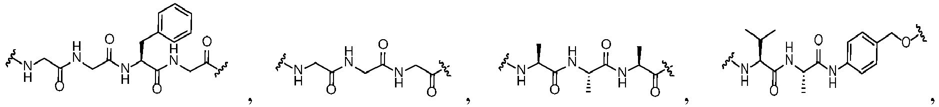 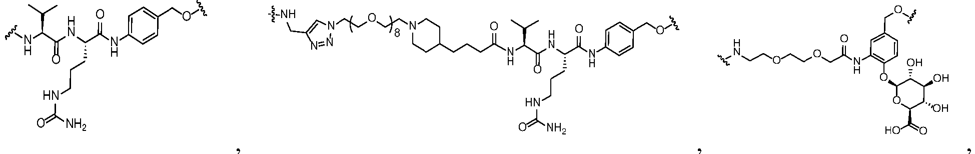 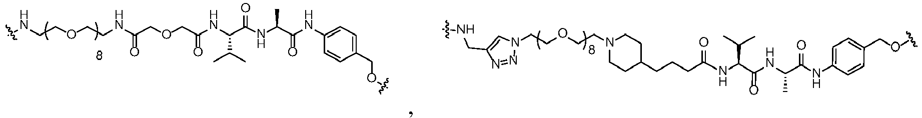 and

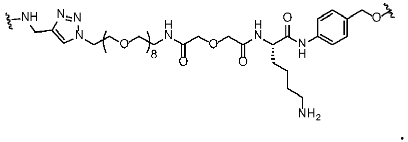

In some embodiments, L is selected from the following structure:

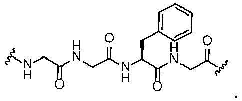

In some embodiments, E is selected from the group consisting of single bond, -NH-CH₂-,

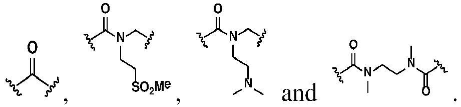

In some embodiments, E is -NH-CH₂-.

In some embodiments, the cytotoxic drug is selected from the compound according to any one of the items of the first aspect of the present invention.

In some embodiments, the cytotoxic drug is selected from the group consisting of the compounds 1-1 to 1-15; 2-1 to 2-27; 3-1 to 3-26; 4-1 to 4-15; or 5-1 to 5-36 of the present invention.

In some embodiments, D is selected from the structure formed by removing a hydrogen atom from the compound of the present invention.

In some embodiments, D is selected from the structure formed by removing a hydrogen atom from the compound 1-1 to 1-15; 2-1 to 2-27; 3-1 to 3-26; 4-1 to 4-15; or 5-1 to 5-36 of the present invention.

In some embodiments, D is selected from the group consisting of the following structures:

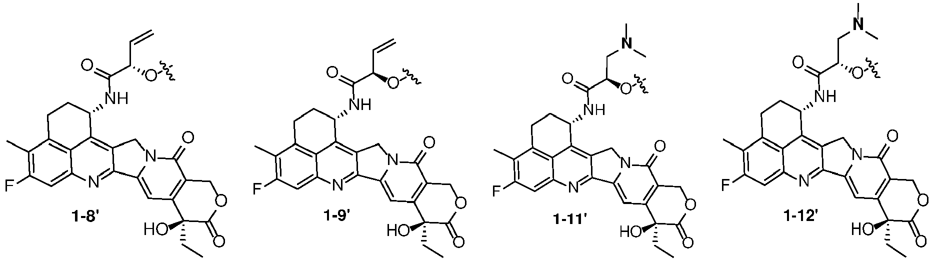

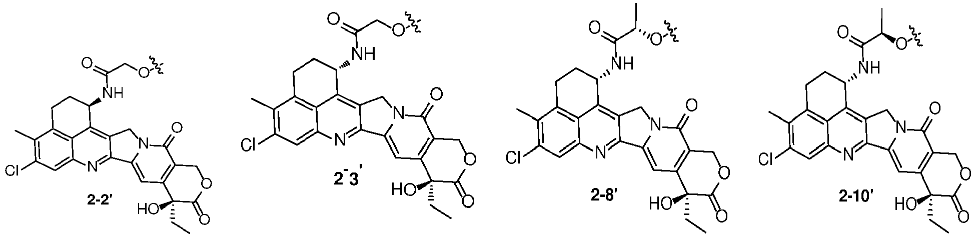

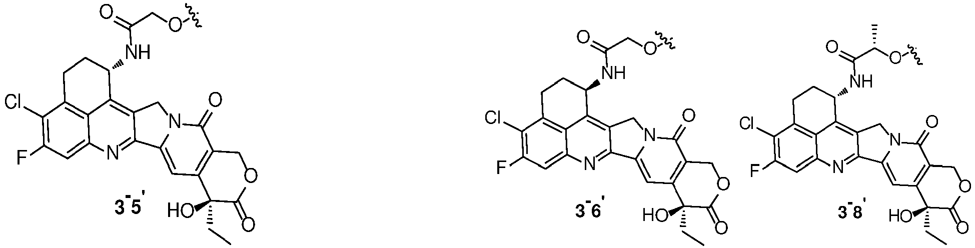

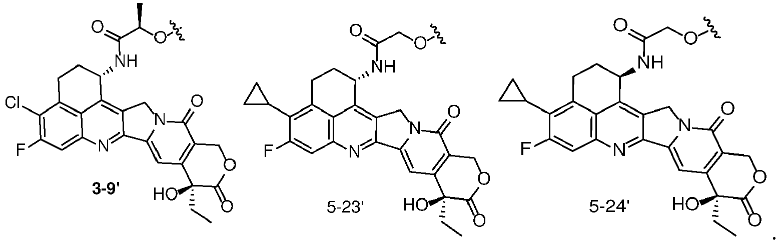

In some embodiments, D is selected from the group consisting of the following structures:

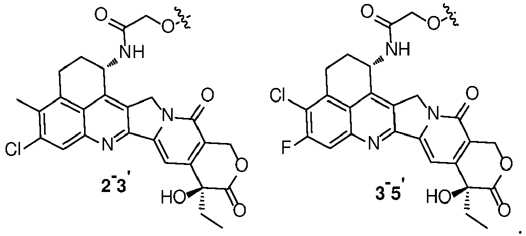

In some embodiments, M-L-E-D is selected from the group consisting of the following compounds:

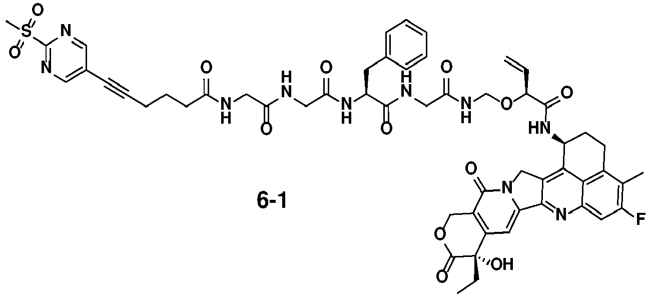

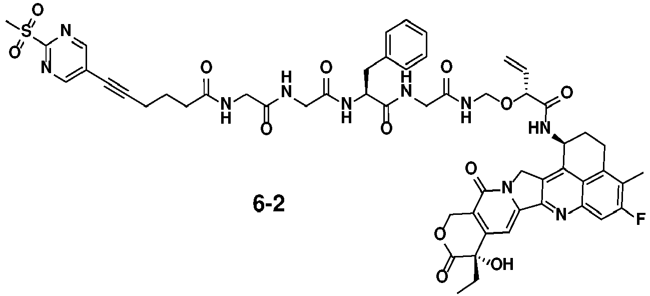

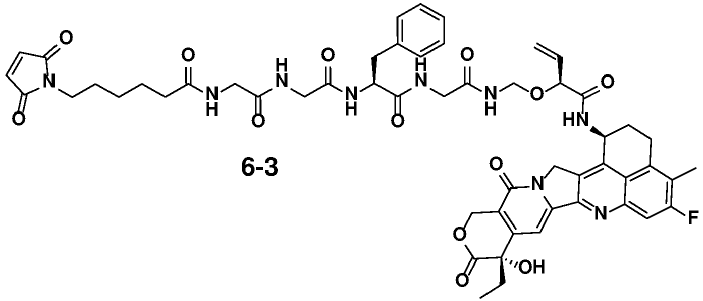

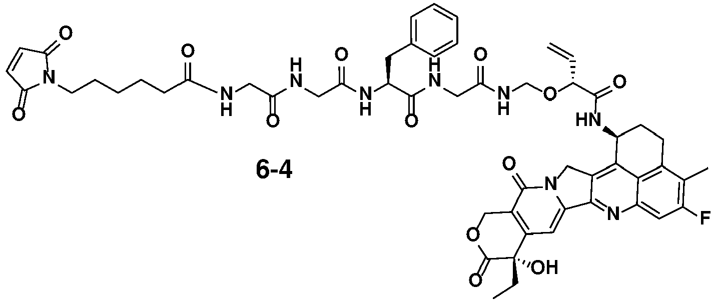

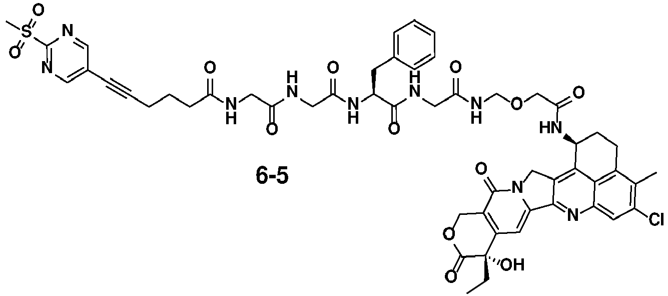

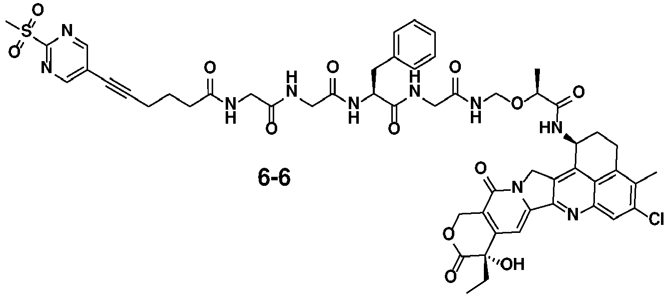

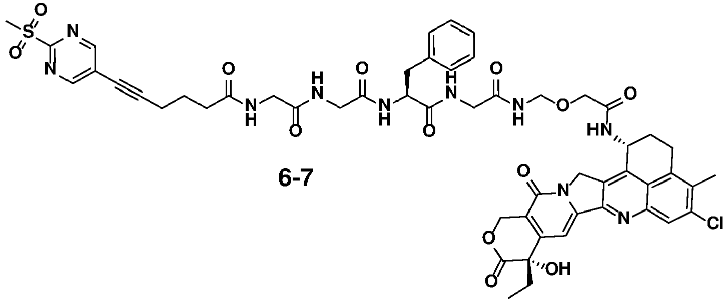

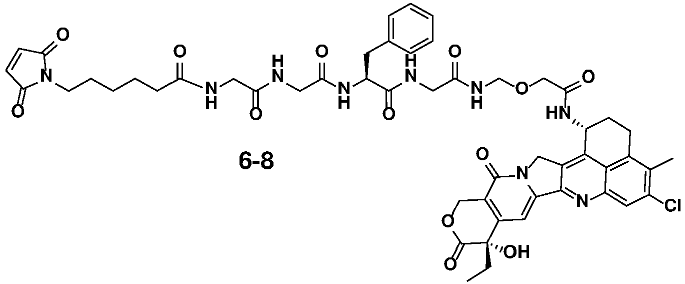

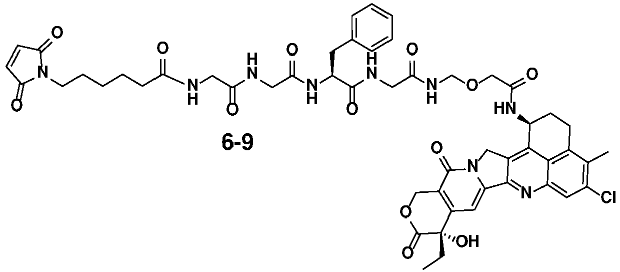

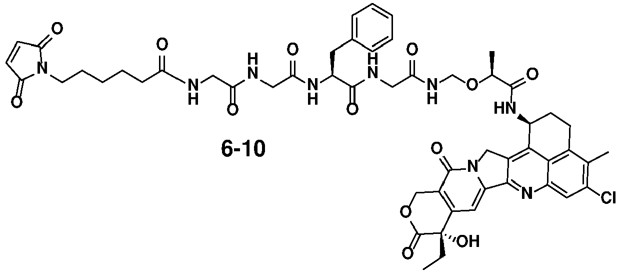

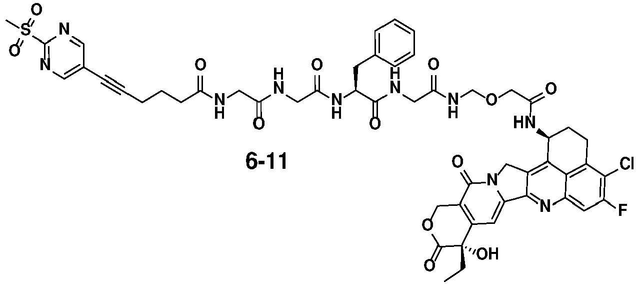

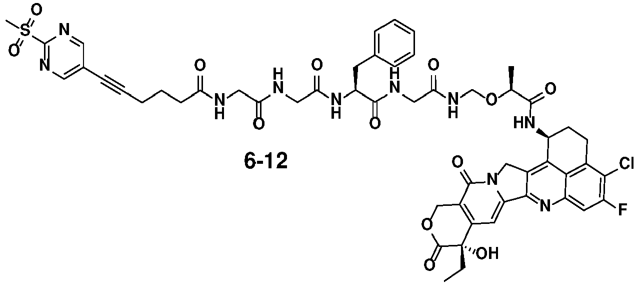

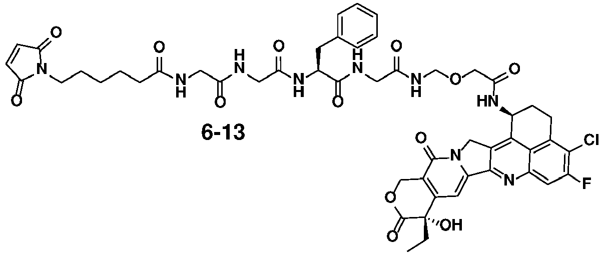

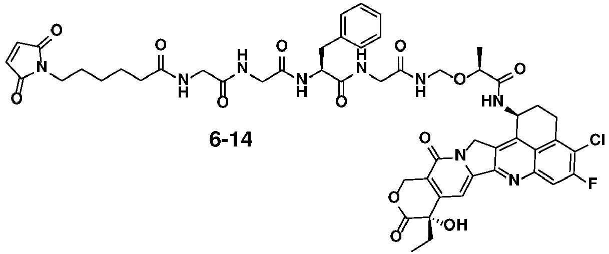

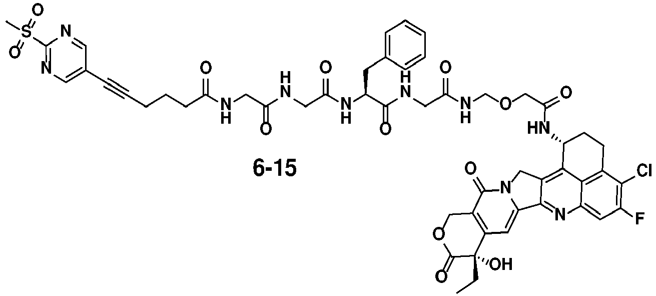

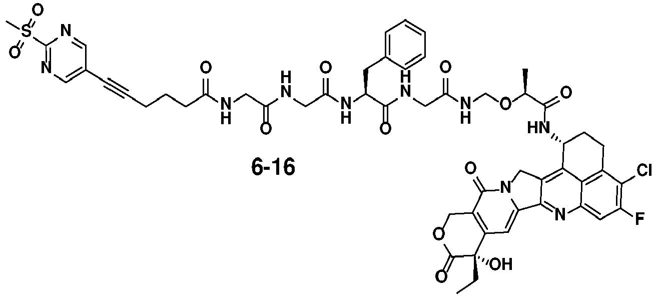

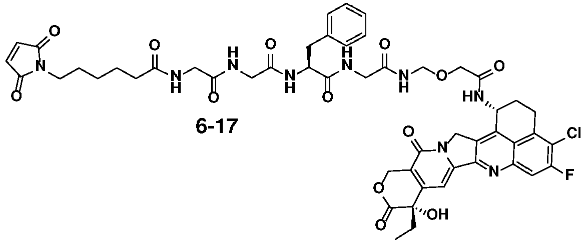

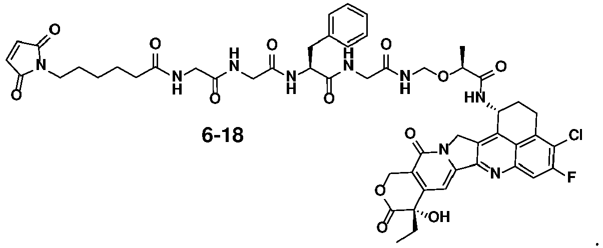

In some embodiments, M-L-E-D is selected from the group consisting of the following compounds:

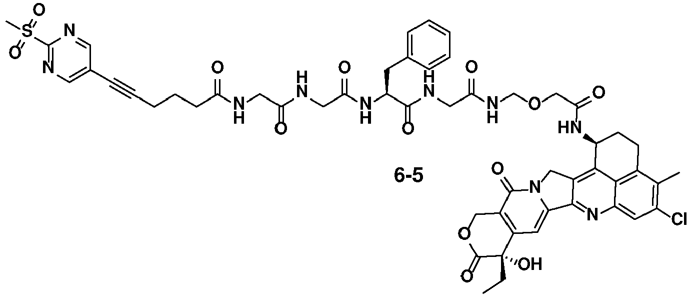

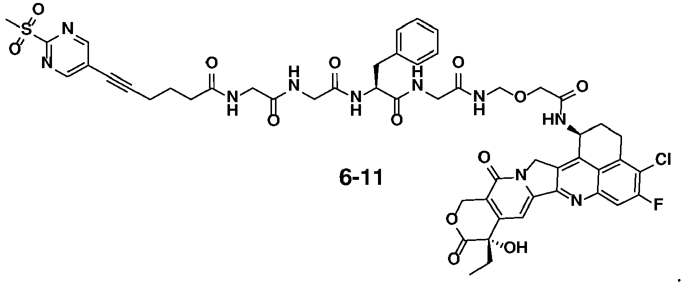

### Definition

Unless defined otherwise hereinafter, all technical and scientific terms used herein have the same meaning as commonly understood by one of ordinary skill in the art. References to techniques used herein are intended to refer to techniques commonly understood in the art, including those variations of the techniques or substitutions of equivalent techniques that are obvious to those skilled in the art. While the following terms are believed to be well understood by those skilled in the art, the following definitions are set forth to better explain the present invention.

The terms "comprising", "including", "having", "containing" or "involving" and other variations thereof herein are inclusive or open-ended and do not exclude other unlisted elements or method steps.

As used herein, the "*" marked in the structural formula of compounds indicates that the marked carbon atom is a chiral carbon atom, and the present invention comprises a pair of enantiomers formed by the chiral carbon atom. If a compound contains two different chiral carbon atoms, the present invention comprises 4 optical isomers formed by the chiral carbon atoms.

As used herein, " 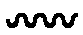 " denotes a bond which may be stereospecific ((R) or (S)) or non-stereospecific.

The term "alkyl" is defined as a saturated straight or branched aliphatic hydrocarbon group. In some embodiments, the alkyl has 1 to 12, for example, 1 to 6 carbon atoms. For example, as used herein, the term "C₁₋₆ alkyl" refers to a straight or branched alkyl group having 1 to 6 carbon atoms (e.g., methyl, ethyl, n-propyl, isopropyl, n-butyl, isobutyl, sec-butyl, tert-butyl, n-pentyl and n-hexyl), which is optionally substituted with one or more (e.g., 1, 2 or 3) suitable substituents.

The term "alkenyl" refers to a straight or branched hydrocarbon group containing at least one carbon-carbon double bond, including, for example, "C₂₋₆ alkenyl", "C₂₋₄ alkenyl" and the like. Examples thereof include, but are not limited to: vinyl, 1-propenyl, 2-propenyl, 1-butenyl, 2-butenyl, 1,3-butadienyl, 1-pentenyl, 2-pentenyl, 3-pentenyl, 1,3-pentadienyl, 1,4-pentadienyl, 1-hexenyl, 2-hexenyl, 3-hexenyl, 1,4-hexdienyl, etc.

The term "alkynyl" refers to a straight or branched hydrocarbon group containing at least one carbon-carbon triple bond, including, for example, "C₂₋₆ alkynyl", "C₄₋₆ alkynyl" and the like. Examples thereof include, but are not limited to: ethynyl, 1-propynyl, 2-propynyl, 1-butynyl, 2-butynyl, 1,3-butadiynyl, 1-pentynyl, 2-pentynyl, 3-pentynyl, 1,3-pentadiynyl, 1,4-pentadiynyl, 1-hexynyl, 2-hexynyl, 3-hexynyl, 1,4-hexadiynyl and the like.

The term "cycloalkyl" refers to a saturated cyclic hydrocarbon group, including but not limited to, monocycloalkyl and bicycloalkyl (e.g., spiro-cycloalkyl, fused cycloalkyl and bridged cycloalkyl). The term "C₃₋₆ cycloalkyl" refers to a cycloalkyl having 3 to 6 ring-forming carbon atoms, such as cyclopropyl, cyclobutyl, cyclopentyl, cyclohexyl, etc., which may be optionally substituted with one or more (e.g., 1, 2 or 3) suitable substituents, for example methyl-substituted cyclopropyl.

The term "carbocyclic ring" or "carbocyclyl" refers to a saturated or partially unsaturated hydrocarbon group with non-aromatic monocyclic or polycyclic structure, it is connected to other part of a compound through a ring-forming carbon atom. Examples thereof include, but are not limited to, cyclopropyl, cyclobutyl, cyclopentyl, cyclopentenyl, cyclohexyl, cyclohexenyl, cycloheptyl, and cyclooctyl.

The term "carbocyclic ring" refers to a saturated or unsaturated non-aromatic monocyclic or polycyclic (e.g., bicyclic) hydrocarbon ring (e.g. monocyclic ring, such as cyclopropane ring, cyclobutane ring, cyclopentane ring, cyclohexane ring, cycloheptane ring, cyclooctane ring, cyclononane ring, or bicyclic ring, including spiro ring, fused or bridged ring system (e.g., bicyclo[1.1.1]pentane ring, bicyclo[2.2.1]heptane ring, bicyclo[3.2.1]octane ring or bicyclo[5.2.0]nonane ring, decahydronaphthalene ring, etc.), which may optionally be substituted with one or more (e.g., 1, 2 or 3) suitable substituents. The term "3- to 6-membered carbocyclic ring" refers to a carbocyclic ring containing 3, 4, 5 or 6 ring-forming carbon atoms.

The term "heterocyclyl" or "heterocyclic ring" refers to a saturated or partially saturated, monocyclic or polycyclic (e.g., bicyclic) non-aromatic ring structure, of which the ring-forming atoms consist of carbon atoms and at least one (e.g., 1, 2 or 3) heteroatoms selected from the group consisting of nitrogen, oxygen and sulfur. The heterocyclyl can be connected to the rest of the molecule through any ring atom, provided that the valence requirements are met. The heterocyclyl of the present invention is preferably a 3- to 6-membered heterocyclyl. The term "3- to 6-membered heterocyclyl" herein refers to a heterocyclic group with 3 to 6 ring atoms, including 3-membered heterocyclyl, 4-membered heterocyclyl, 5-membered heterocyclyl and 6-membered heterocyclyl, including nitrogen-containing heterocyclyl and oxygen-containing heterocyclyl, such as 4- to 6-membered heterocyclyl, such as 4- to 6-membered nitrogen-containing heterocyclyl, and 4- to 6-membered oxygen-containing heterocyclyl. Common heterocyclic groups include, but are not limited to, azetidinyl, oxetanyl, tetrahydrofuryl, pyrrolidinyl, pyrrolidinonyl, imidazolidinyl, pyrazolidinyl, tetrahydropyranyl, piperidinyl, piperazinyl, morpholinyl. The heterocyclyl in the present invention can be optionally substituted with one or more substituents described herein. The heterocyclyl in the present invention is optionally fused with one or more aromatic rings or non-aromatic rings.

The term "oxygen-containing heterocyclic ring" refers to a heterocyclic ring as above-described in which one or more (e.g., 1, 2 or 3) ring atoms are oxygen atoms, such as 5- to 6-membered oxygen-containing heterocyclic ring, and specific examples include but not limited to oxirane ring, tetrahydrofuran ring, furan ring, tetrahydropyran ring, pyran ring, and the like. The term "nitrogen-containing heterocyclic ring" in the present invention refers to a heterocyclic ring as above-mentioned in which one or more (e.g., 1, 2 or 3) ring atoms are nitrogen atoms.

The term "haloalkyl" refers to an alkyl group substituted by one or more (e.g., 1, 2 or 3) identical or different halogen atoms, wherein alkyl is as defined above. For example, the term "C₁₋₆ haloalkyl" used in the present invention refers to a haloalkyl having 1 to 6 carbon atoms. Common haloalkyl includes, but is not limited to, -CH₂F, -CHF₂, -CF₃, -CH₂CF₃, -CF₂CF₃, -CH₂CH₂CF₃, - CH₂Cl, and the like. The haloalkyl of the present invention can be optionally substituted with one or more substituents described herein.

The term "aryl" refers to a group obtained by removing a hydrogen atom from a carbon atom of the aromatic nucleus of an aromatic hydrocarbon molecule, such as 6- to 14-membered aryl, and specific examples include but not limited to phenyl, naphthyl, anthracenyl and the like.

The term "heteroaryl" refers to an aromatic ring group containing at least one ring member selected from the group consisting of N, O and S. Specific examples include, but are not limited to, 5- to 6-membered heteroaryl, 5- to 6-membered nitrogen-containing heteroaryl, 5- to 6-membered oxygen-containing heteroaryl, such as furyl, thienyl, pyrrolyl, thiazolyl, isothiazolyl, thiadiazolyl, oxazolyl, isoxazolyl, oxadiazolyl, imidazolyl, pyrazolyl, 1,2,3-triazolyl, 1,2,4-triazolyl, 1,2,3-oxadiazolyl, 1,2,4-oxadiazolyl, 1,2,5-oxadiazolyl, 1,3,4-oxadiazolyl, pyridyl, pyrimidinyl, pyridazinyl, pyrazinyl, 1,2,3-triazinyl, 1,3,5-triazinyl, 1,2,4,5-tetrazinyl, etc.

The term "alkoxy" refers to a group having the structure "alkyl-O-", wherein alkyl is as defined above. Examples include C₁₋₆ alkoxy, C₁₋₄ alkoxy, C₁₋₃ alkoxy or C₁₋₂ alkoxy and the like. Common alkoxy includes (but is not limited to) methoxy, ethoxy, n-propoxy, isopropoxy, n-butoxy, isobutoxy, tert-butoxy, pentyloxy, hexyloxy, etc. The alkoxy in the present invention is optionally substituted with one or more substituents described herein.

The term "alkoxyalkyl" refers to an alkyl substituted by one or more (e.g. 1, 2, 3 or 4) alkoxy groups, wherein alkoxy and alkyl are as defined above. For example, the term "C₁₋₆ alkoxyalkyl" used in the present invention refers to an alkyl having 1-6 carbon atoms and being substituted by one or more (e.g., 1, 2, 3 or 4) alkoxy groups. Common alkoxyalkyl includes (but is not limited to) CH₃O-CH₂-, C₂H₅-O-CH₂-, C₂H₅-O-CH₂CH₂-, etc.

The term "halo" or "halogen" group is defined to include F, Cl, Br or I.

The term "nitrogen oxide" refers to an oxide (e.g., mono- or di-oxide) of at least one nitrogen atom in the structure of the compound of the present application. A mono-oxide of a nitrogen may exist as a single positional isomer or as a mixture of positional isomers.

The term "substituted" means that one or more (e.g., one, two, three or four) hydrogens on a designated atom are selectively replaced by a denoted group, provided that the normal valence of the designated atom under the current circumstances is not exceeded and the substitution results in a stable compound. Combination of substituents and/or variables is permissible only if such combination results in a stable compound.

If a substituent is described as "optionally substituted," the substituent may be (1) unsubstituted or (2) substituted. If a carbon atom of a substituent is described as being optionally substituted with one or more substituents from a list of substituents, one or more hydrogens on the carbon atom (to the extent of any hydrogen present) may be individually and/or simultaneously replaced by any independently selected substituent. If a nitrogen atom of a substituent is described as being optionally substituted with one or more of listed substituents, one or more hydrogens on the nitrogen atom (to the extent of any hydrogen present) may each be independently replaced by any selected substituent.

If substituents are described as being "independently selected from" a group, each substituent is selected independently to the other. Accordingly, each substituent may be identical to or different from another (other) substituent.

As used herein, the term "one or more" means 1 or more than 1, such as 2, 3, 4, 5 or 10, under reasonable conditions.

As used herein, unless otherwise indicated, the site of attachment of a substituent may be any suitable position of the substituent.

The term "stereoisomer" refers to an isomer formed as a result of at least one asymmetric center. In a compound with one or more (e.g., one, two, three or four) asymmetric centers, there may generate racemic mixtures, single enantiomers, diastereomeric mixtures and individual diastereoisomers. Certain individual molecules may also exist as geometric isomers (cis/trans). Similarly, the compound of the present invention may exist as a mixture of two or more structurally distinct forms (commonly referred to as tautomers) in rapid equilibrium. Representative examples of tautomers include keto-enol tautomers, phenol-keto tautomers, nitroso-oxime tautomers, imine-enamine tautomers, etc. It is to be understood that the scope of the present invention encompasses all such isomers or mixtures thereof in any proportion (e.g., 60%, 65%, 70%, 75%, 80%, 85%, 90%, 95%, 96%, 97%, 98% or 99%).

Carbon-carbon bonds of the compound of the present invention may be depicted herein using solid lines ( 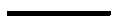 ), solid wedges ( 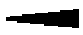 ), or dashed wedges ( 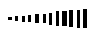 ). A solid line used in delineating a bond to an asymmetric carbon atom is intended to indicate that all possible stereoisomers of that carbon atom are included (e.g., specific enantiomers, racemic mixtures, etc.). A solid line or dashed wedge used in delineating a bond to an asymmetric carbon atom is intended to indicate the stereoisomer as shown. When delineating a racemic mixture, a solid line or dashed wedge is intended to define relative stereochemistry, rather than absolute stereochemistry. Unless otherwise indicated, the compound of the present invention may present in the form of stereoisomers (which includes cis- and trans-isomers, optical isomers (e.g., R and S enantiomers), diastereomers, geometric isomers, rotamers, conformational isomers, atropisomers and mixtures thereof). The compound of the present invention may exhibit more than one type of isomerism and consist of mixtures thereof, such as racemic mixtures and pairs of diastereoisomers.

The present invention covers all possible crystalline forms or polymorphs of the compound of the present invention, which may be a single polymorph or a mixture of more than one polymorph in any proportion.

It will also be understood that certain compounds of the present invention may exist in free form for therapeutic use, or, where appropriate, exist in a pharmaceutically acceptable derivative thereof. In the present invention, the pharmaceutically acceptable derivative includes, but is not limited to, pharmaceutically acceptable salt, ester, solvate, metabolite or prodrug, which can directly or indirectly provide the compound of the present invention or metabolite or residue thereof after being administrated to a patient. Therefore, when a "compound of the present invention" is referred to herein, it is also intended to cover the above-mentioned various derivative forms of the compound.

The pharmaceutically acceptable salt of the compound of the present invention includes acid addition salt and base addition salt thereof.

A suitable acid addition salt can be formed from an acid capable of forming a pharmaceutically acceptable salt, including aspartate, fumarate, glucoheptonate, gluconate, glucuronate, hexafluorophosphate, and the like.

A suitable base addition salt can be formed from a base capable of forming a pharmaceutically acceptable salt, including aluminum salt, arginine salt, choline salt, diethylamine salt, and the like.

For a review of suitable salts, see Stahl and Wermuth, "Handbook of Pharmaceutical Salts: Properties, Selection, and Use" (Wiley-VCH, 2002). Methods for preparing pharmaceutically acceptable salts of the compound of the present invention are known to those skilled in the art.

The term "ester" refers to an ester derived from each of the compounds of the formulas herein, including physiologically hydrolyzable ester (hydrolyzable under physiological conditions to release the compound of the present invention in the form of free acid or alcohol). The compound of the present invention itself may also be an ester.

The compound of the present invention may exist in the form of a solvate, preferably a hydrate, wherein the compound of the present invention comprises a polar solvent (e.g., water, methanol or ethanol in particular) as a structural element of the crystal lattice of the compound. The amount of polar solvent, especially water, may be present in stoichiometric or non-stoichiometric ratio.

The scope of the present invention further comprise a metabolite of the compound of the present invention, that is, a substance formed in vivo when the compound of the present invention is administered. Such substance may be generated, for example, from oxidation, reduction, hydrolysis, amidation, deamidation, esterification, delipidation, enzymatic hydrolysis, etc., of the administered compound. Accordingly, the present invention comprises a metabolite of the compound of the present invention, including a compound produced by contacting the compound of the present invention with a mammal for a time sufficient to produce a metabolite thereof.

The scope of the present invention can further comprise a prodrug of the compound of the present invention. Typically, such prodrug will be a functional group derivative of the compound, which is readily converted in vivo into the desired therapeutically active compound. Therefore, in these cases, the term "administration" used in the therapeutic method of the present invention shall comprise using one or more prodrugs of the compound of the present invention to treat various diseases or conditions, but the prodrug is converted in vivo to the aforementioned compound after administration to a subject. For example, in "Design of Prodrug", ed. H. Bundgaard, Elsevier, 1985, general methods for selecting and preparing suitable prodrug derivatives are described.

The scope of the present invention further comprises an isotope-labeled product of the compound of the present invention, which is identical to the compound of the present invention except that at least one atom thereof is replaced by an atom with the same atomic number but with an atomic mass or mass number different from the atomic mass or mass number of the predominate atom in nature.

The present invention also encompasses a compound of the present invention which contains a protecting group. During any process for preparing the compound of the present invention, it may be necessary and/or desirable to protect a sensitive or reactive group on any molecule involved, thereby forming a chemically protected form of the compound of the present invention. This can be achieved by conventional protecting groups, for example, those protecting groups described in Protective Groups in Organic Chemistry, ed. J.F.W. McOmie, Plenum Press, 1973; and T.W. Greene & P.G.M. Wuts, Protective Groups in Organic Synthesis, John Wiley & Sons, 1991. These references are incorporated herein by reference. The protecting group may be removed at appropriate subsequent stage using a method known in the art.

### Pharmaceutical composition

In a third aspect, the present invention provides a pharmaceutical composition comprising the compound or pharmaceutically acceptable salt, ester, stereoisomer, polymorph, solvate, nitrogen oxide, isotope-labeled product, metabolite or prodrug thereof described in the first aspect or the second aspect of the present invention, and one or more pharmaceutically acceptable carriers.

The term "pharmaceutical composition" refers to a composition that can be used as a medicament, which comprises a pharmaceutically active ingredient (API) (or therapeutic agent) and optionally one or more pharmaceutically acceptable carriers. The term "pharmaceutically acceptable carrier" refers to an excipient that is administered with a therapeutic agent and that is suitable for contacting a human and/or other animal tissue without inducing undue toxicity, irritation, anaphylaxis or resulting in other problems or complications with a reasonable benefit/risk ratio within the scope of sound medical judgment.

The above-mentioned pharmaceutical composition can act systemically and/or locally, which can be achieved by a suitable dosage form. The dosage form includes but is not limited to tablets, capsules, lozenges, hard troches, powders, sprays, creams, ointments, suppositories, gels, pastes, lotions, ointments, aqueous suspensions, injectable solutions, elixirs, syrups.

The above-mentioned pharmaceutical composition may comprise 0.01 mg to 1000 mg of at least one compound or pharmaceutically acceptable salt, ester, stereoisomer, polymorph, solvate, nitrogen oxide, isotope-labeled product, metabolite or prodrug thereof the present invention.

The present invention also provides a method for preparing the above-mentioned pharmaceutical composition or its corresponding dosage form, which comprises combining at least one compound or pharmaceutically acceptable salt, ester, stereoisomer, polymorph, solvate, nitrogen oxide, isotope-labeled product, metabolite or prodrug thereof the present invention with one or more pharmaceutically acceptable carriers.

### Kit product

In a fourth aspect, the present invention provides a kit product, which comprises:
a) at least one compound or pharmaceutically acceptable salt, ester, stereoisomer, polymorph, solvate, nitrogen oxide, isotope-labeled product, metabolite or prodrug thereof described in the first aspect or the second aspect of the present invention as a first therapeutic agent, or the pharmaceutical composition described in the third aspect as a first pharmaceutical composition;
b) optionally, at least one additional therapeutic agent as a second therapeutic agent, or a pharmaceutical composition comprising an additional therapeutic agent as a second pharmaceutical composition; and
c) optionally, a packaging and/or instruction for use.

The above kit product may comprise 0.01 mg to 1000 mg of at least one compound or pharmaceutically acceptable salt, ester, stereoisomer, polymorph, solvate, nitrogen oxide, isotope-labeled product, metabolite or prodrug thereof the present invention.

The present invention also provides a method for preparing the above-mentioned kit, which comprises combining the at least one compound or pharmaceutically acceptable salt, ester, stereoisomer, polymorph, solvate, nitrogen oxide, isotope-labeled product, metabolite or prodrug thereof the present invention or the aforementioned pharmaceutical composition with the optional at least one additional therapeutic agent or pharmaceutical composition comprising an additional therapeutic agent, and the optional packaging and/or instruction for use.

### Medical use

The compound of the present invention can exhibit a potent effect on inhibiting abnormal cell proliferation.

Therefore, the present application provides that the compound or pharmaceutically acceptable salt, ester, stereoisomer, polymorph, solvate, nitrogen oxide, isotope-labeled product, metabolite or prodrug thereof the present invention or the aforementioned pharmaceutical composition, for use in treating a disease related to abnormal cell proliferation.

In addition, the present application also provides use of the compound or pharmaceutically acceptable salt, ester, stereoisomer, polymorph, solvate, nitrogen oxide, isotope-labeled product, metabolite or prodrug thereof the present invention or the aforementioned pharmaceutical composition in the manufacture of a medicament for treating a disease related to abnormal cell proliferation.

In some embodiments, the disease related to abnormal cell proliferation includes, but is not limited to, tumor, such as an advanced solid tumor.

The present application also provides use of the compound or pharmaceutically acceptable salt, ester, stereoisomer, polymorph, solvate, nitrogen oxide, isotope-labeled product, metabolite or prodrug thereof the present invention or the aforementioned pharmaceutical composition in the manufacture of a preparation, and the preparation is used for inhibiting the proliferation of a tumor cell. In certain embodiments, the preparation is used in vivo or in vitro. For example, the preparation may be administered to a subject to inhibit the proliferation of a tumor cell in a subject; alternatively, the preparation may be applied to a cell (e.g., a cell line or cell from a subject) in vitro to inhibit an in vitro tumor cell proliferation.

The tumor of the present invention includes (but is not limited to): brain tumor, lung cancer, squamous cell carcinoma, bladder cancer, gastric cancer, ovarian cancer, peritoneal cancer, pancreatic cancer, breast cancer, head and neck cancer, cervical cancer, endometrium cancer, colorectal cancer, liver cancer, kidney cancer, esophageal adenocarcinoma, esophageal squamous cell carcinoma, prostate cancer, female reproductive tract cancer, carcinoma in situ, lymphoma, neurofibroma, thyroid cancer, bone cancer, skin cancer, brain cancer, colon cancer, testicular cancer, gastrointestinal stromal tumor, prostate tumor, mast cell tumor, multiple myeloma, melanoma, glioma, or sarcoma.

### Therapeutic method

In another aspect, the present invention provides a method for treating a disease related to abnormal cell proliferation, comprising a step of administering to an individual in need thereof a therapeutically effective amount of the compound or pharmaceutically acceptable salt, ester, stereoisomer, polymorph, solvate, nitrogen oxide, isotope-labeled product, metabolite or prodrug thereof the present invention or the aforementioned pharmaceutical composition.

The term "effective amount" refers to a dose capable of inducing a biological or medical response in a cell, tissue, organ or organism (e.g., individual) and sufficient to achieve a desired preventive and/or therapeutic effect.

Dosage regimens may be adjusted to provide the optimum desired response. For example, it can be administered as a single dose or as divided doses over time, or the dose can be proportionally reduced or increased according to the actual situation. It will be appreciated that, for any particular individual, the specific dosage regimen will be adjusted according to the needs and professional judgment of the person administering the composition or supervising the administration of the composition.

The administered amount of the compound of the present invention will depend on the individual condition, the severity of the disease or condition, the rate of administration, the disposition of the compound, and the judgment of the physician for prescription. Generally, the effective amount is about 0.001-10000 mg/kg body weight of subject/day. Where appropriate, the effective amount is about 0.01-1000 mg/kg body weight of subject/day. About 0.01-1000 mg/kg body weight of subject, usually about 0.1-500 mg/kg body weight of subject can be administered every day, every two days or every three days. Exemplary dosage regimens are one or more times per day, or one or more times per week, or one or more times per month. For multiple administrations, the intervals between single doses can generally be daily, weekly, monthly or yearly. Alternatively, it can be administered as a sustained release preparation, in which case less frequent of administration will be required. Dosage and frequency of administration may vary depending on the half-life of the drug in the subject, and whether the use is prophylactic or therapeutic. In prophylactic use, relatively low doses are administered in the long term at relatively infrequent intervals; in therapeutic use, it is sometimes necessary to administer relatively high doses at shorter intervals until the progression of disease is delayed or stopped, preferably until the individual shows partial or complete relieved in disease symptoms, after which a prophylactic use may be employed.

The term "treating" means alleviating or eliminating a targeted disease or condition. If a subject receives a therapeutic amount of the compound or pharmaceutically acceptable form thereof the present invention or the pharmaceutical composition of the present invention, at least one indicator and symptom of the subject exhibits an observable and/or detectable remission and/or improvement, it indicates that the subject has been successfully "treated". It is understood that treatment includes not only complete cure, but also not complete cure but achievement of some biologically or medically relevant result.

The term "administrate/administrating/administration" refers to a process of applying a pharmaceutical active ingredient (e.g., the compound of the present invention) or a pharmaceutical composition (e.g., the pharmaceutical composition of the present invention) comprising the pharmaceutical active ingredient to an individual or its cells, tissues, organs, biological fluids, etc., so that the active pharmaceutical ingredient or pharmaceutical composition contacts with the individual or its cells, tissues, organs, biological fluids, etc. Common modes of administration include, but are not limited to, oral administration, subcutaneous administration, intramuscular administration, subperitoneal administration, ophthalmic administration, nasal administration, sublingual administration, rectal administration, vaginal administration, and the like.

The term "in need thereof" refers to a physician's or other caregiver's judgment that an individual needs or will benefit from a prophylactic and/or therapeutic procedure based on the physician's or other caregiver's various factors in their areas of expertise.

The term "individual" (or subject) refers to a human or non-human animal. The individual of the present invention includes an individual (patient) with a disease and/or condition and a normal individual. The non-human animals of the present invention include all vertebrates, such as non-mammals, such as birds, amphibians, reptiles, etc., and mammals, such as non-human primates, livestock and/or domesticated animals (e.g., sheep, dogs, cats, cows, pigs, etc.).

### Preparation method

The fourth aspect of the present invention provides a synthesis method of the compound.

The compound of Formula (I) in the present invention can be synthesized by the following synthetic route. 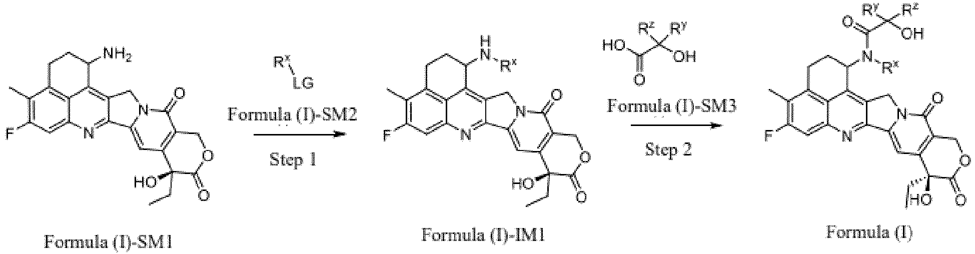 wherein, R^{x}, R^{y} and R^{z} have the meanings as described above; LG is a leaving group selected from the group consisting of methylsulfonyl, trifluoromethylsulfonyloxy and halogen, preferably trifluoromethylsulfonyloxy or iodine;

### Step 1:

The compound of Formula (I)-IM1 is obtained through a substitution reaction between the compound of Formula (I)-SM1 and the compound of Formula (I)-SM2.

In some embodiments, this step is carried out at a suitable temperature, and the temperature is 20°C, 25°C, 40°C, 50°C, 60°C, 100°C, 140°C, preferably 50°C;

In some embodiments, this step is carried out in a suitable organic solvent selected from the group consisting of halogenated hydrocarbons (e.g., dichloromethane (DCM), chloroform (TCM), 1,2-dichloroethane (1,2-DCE), etc.), nitriles (e.g., acetonitrile (AN), etc.), N-methylpyrrolidone (NMP), N,N-dimethylformamide (DMF), N,N-dimethylacetamide (DMA), tetrahydrofuran (THF), 1,4-dioxane (Dioxane), dimethyl sulfoxide (DMSO) and any combination thereof, preferably acetonitrile.

In some embodiments, this step is carried out in the presence of a suitable base, the base includes an organic base or an inorganic base, and the organic base may be selected from the group consisting of N,N-diisopropylethylamine (DIPEA), triethylamine (TEA), potassium tert-butoxide (t-BuOK) and pyridine (Py), and the inorganic base may be selected from the group consisting of potassium phosphate (K₃PO₄), sodium hydride (NaH), potassium carbonate (K₂CO₃), sodium carbonate (Na₂CO₃), sodium bicarbonate (NaHCO₃), cesium carbonate (Cs₂CO₃) and NaOH, preferably Na₂CO₃ or NaHCO₃;

### Step 2:

The compound of Formula (I) is obtained through a condensation reaction of the compound of Formula (I)-IM1 and the compound of Formula (I)-SM3;

In some embodiments, this step is carried out in the presence of a suitable condensation reagent selected from the group consisting of HATU, HBTU, EDCI, DCC and HOBT, preferably HATU;

In some embodiments, this step is carried out at a suitable temperature, and the temperature is 20°C, 25°C, 40°C, 50°C, 60°C, 100°C, preferably 25°C;

In some embodiments, this step is carried out in a suitable organic solvent selected from the group consisting of methanol, tetrahydrofuran, dichloromethane, N,N-dimethylformamide, N-methylpyrrolidone, dimethyl sulfoxide, n-heptane, n-hexane, ethyl acetate, preferably N,N-dimethylformamide.

In some embodiments, this step is carried out in the presence of a suitable base, the base includes an organic base or an inorganic base, the organic base may be selected from the group consisting of DIPEA, TEA, t-BuOK and Py, and the inorganic base may be selected from the group consisting of K₃PO₄, NaH, K₂CO₃, Na₂CO₃, Cs₂CO₃ and NaOH, preferably DIPEA.

The compound of Formula (II)-1 in the present invention may be synthesized via the following synthetic route. 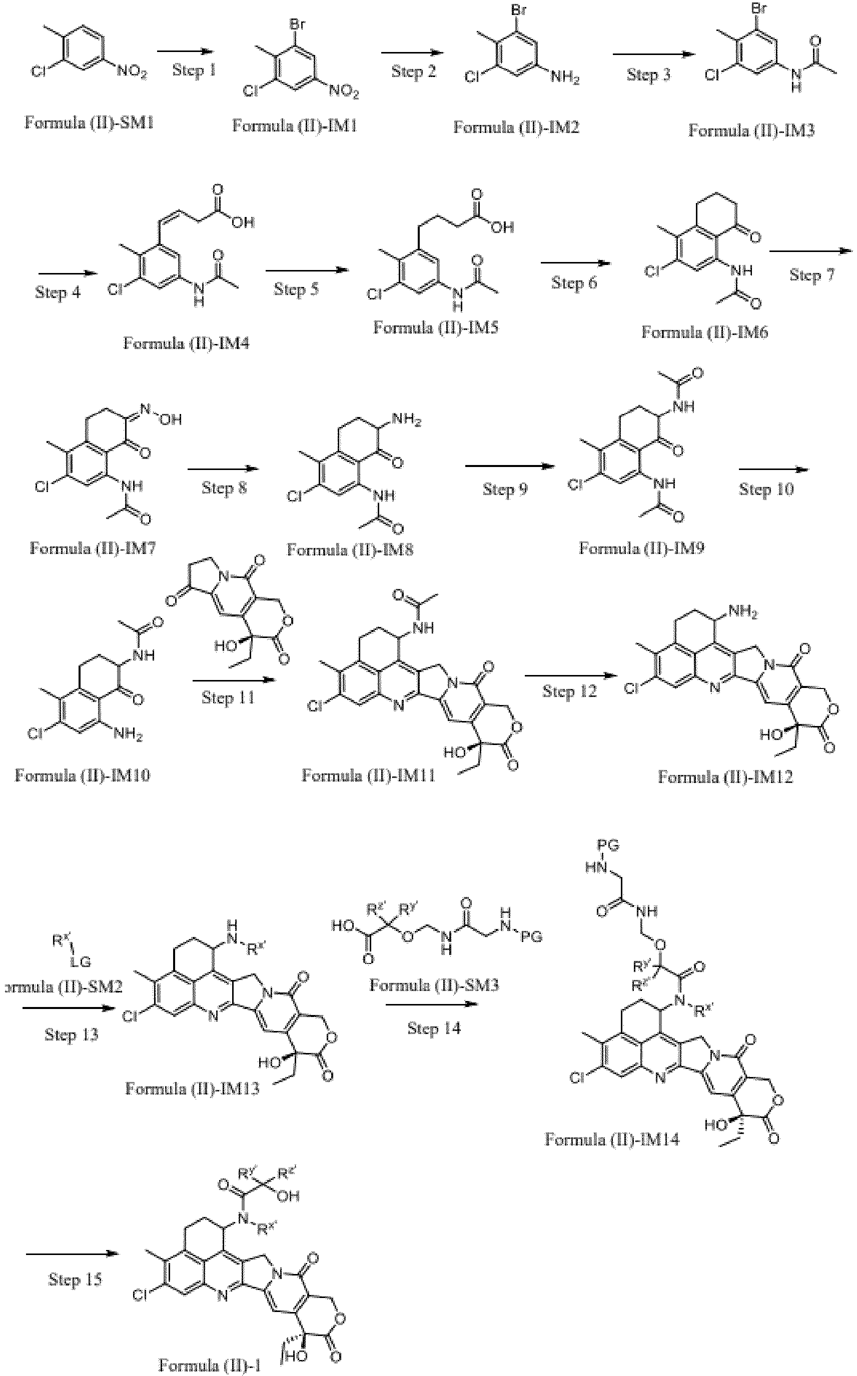 wherein, the meanings of R^{x'}, R^{y'} and R^{z'} are as described above; LG is a leaving group selected from the group consisting of methylsulfonyl, trifluoromethylsulfonyloxy and halogen, preferably trifluoromethylsulfonyloxy or iodine; PG is a protecting group selected from the group consisting of

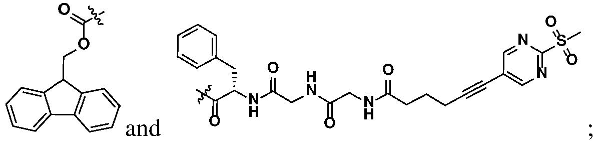

### Step 1

The compound of Formula (II)-IM1 is obtained through a substitution reaction of the compound of Formula (II)-SM1.

In some embodiments, this step is carried out at a suitable temperature, and the temperature is 20°C, 25°C, 50°C, 60°C, 100°C, preferably 50°C;

In some embodiments, this step is carried out in a suitable organic solvent, which may be selected from the group consisting of tetrahydrofuran, dichloromethane, N,N-dimethylformamide, N-methylpyrrolidone, dimethyl sulfoxide, n-heptane, n-hexane, ethyl acetate, preferably n-heptane.

### Step 2

The compound of Formula (II)-IM2 is obtained through a reduction reaction of the compound of Formula (II)-IM1;

In some embodiments, this step is carried out in the presence of a suitable reducing agent, which may be selected from the group consisting of palladium catalyst, platinum catalyst, rhodium catalyst, preferably platinum catalyst;

In some embodiments, this step is carried out at a suitable temperature, and the temperature is 20°C, 25°C, 50°C, 60°C, 100°C, preferably 60°C;

In some embodiments, this step is carried out in a suitable organic solvent, which may be selected from the group consisting of tetrahydrofuran, dichloromethane, N,N-dimethylformamide, N-methylpyrrolidone, dimethyl sulfoxide, n-heptane, n-hexane, ethyl acetate, preferably ethyl acetate.

### Step 3

The compound of Formula (II)-IM3 is obtained through a substitution reaction of the compound of Formula (II)-IM2,

In some embodiments, this step is carried out at a suitable temperature, and the temperature is 20°C, 25°C, 50°C, 60°C, 100°C, preferably 20°C;

In some embodiments, this step is carried out in a suitable organic solvent, which may be selected from the group consisting of tetrahydrofuran, dichloromethane, N,N-dimethylformamide, N-methylpyrrolidone, dimethyl sulfoxide, n-heptane, n-hexane, ethyl acetate, preferably ethyl acetate;

In some embodiments, this step is carried out under basic conditions, reagents providing the basic conditions include organic bases and inorganic bases, the organic base includes but is not limited to triethylamine, pyridine, N,N-diisopropylethylamine, n-butyllithium, lithium diisopropylamide, lithium bistrimethylsilylamide, sodium bistrimethylsilylamide; the inorganic base includes but is not limited to potassium carbonate, sodium carbonate, sodium bicarbonate, potassium tert-butoxide, sodium hydride, sodium hydroxide, potassium hydroxide, preferably triethylamine.

### Step 4

The compound of Formula (II)-IM4 is obtained through a coupling reaction of the compound of Formula (II)-IM3;

In some embodiments, this step is carried out at a suitable temperature, and the temperature is 20°C, 25°C, 50°C, 60°C, 70°C, 100°C, preferably 70°C;

In some embodiments, this step is carried out in a suitable organic solvent, which may be selected from the group consisting of tetrahydrofuran, dichloromethane, N,N-dimethylformamide, N-methylpyrrolidone, dimethyl sulfoxide, n-heptane, n-hexane, ethyl acetate, water, preferably a mixed solvent of tetrahydrofuran and water;

In some embodiments, this step is carried out under basic conditions, reagents providing the basic conditions include organic bases and inorganic bases, the organic base includes but is not limited to triethylamine, pyridine, N,N-diisopropylethylamine, n-butyllithium, lithium diisopropylamide, lithium bistrimethylsilylamide, sodium bistrimethylsilylamide; the inorganic base includes but is not limited to potassium carbonate, sodium carbonate, sodium bicarbonate, potassium tert-butoxide, sodium hydride, sodium hydroxide, potassium hydroxide, preferably N,N-diisopropylethylamine.

### Step 5

The compound of Formula (II)-IM5 is obtained through a reduction reaction of the compound of Formula (II)-IM4;

In some embodiments, this step is carried out in the presence of a suitable reducing agent, which may be selected from the group consisting of palladium catalyst, platinum catalyst, rhodium catalyst, preferably platinum catalyst;

In some embodiments, this step is carried out at a suitable temperature, and the temperature is 20°C, 25°C, 40°C, 50°C, 60°C, 100°C, preferably 40°C;

In some embodiments, this step is carried out in a suitable organic solvent, which may be selected from the group consisting of tetrahydrofuran, dichloromethane, N,N-dimethylformamide, N-methylpyrrolidone, dimethyl sulfoxide, n-heptane, n-hexane, ethyl acetate, preferably tetrahydrofuran.

### Step 6

The compound of Formula (II)-IM6 is obtained through a ring-closing reaction of the compound of Formula (II)-IM5;

In some embodiments, this step is carried out at a suitable temperature, and the temperature is 5°C, 20°C, 25°C, 40°C, 50°C, 60°C, 100°C, preferably 5°C;

In some embodiments, this step is carried out in a suitable organic solvent, which may be selected from the group consisting of trifluoroacetic acid, tetrahydrofuran, dichloromethane, N,N-dimethylformamide, N-methylpyrrolidone, dimethyl sulfoxide, n-heptane, n-hexane, ethyl acetate, tert-butanol, preferably a mixed solvent of tetrahydrofuran and tert-butanol.

### Step 7

The compound of Formula (II)-IM7 is obtained through a substitution reaction of the compound of Formula (II)-IM6;

In some embodiments, this step is carried out at a suitable temperature, and the temperature is 5°C, 20°C, 25°C, 40°C, 50°C, 60°C, 100°C, preferably 5°C;

In some embodiments, this step is carried out in a suitable organic solvent, which may be selected from the group consisting of trifluoroacetic acid, tetrahydrofuran, dichloromethane, N,N-dimethylformamide, N-methylpyrrolidone, dimethyl sulfoxide, n-heptane, n-hexane, ethyl acetate, preferably trifluoroacetic acid;

In some embodiments, this step is carried out under basic conditions, and reagents for providing the basic conditions include organic bases and inorganic bases, and the organic bases include but are not limited to triethylamine, pyridine, N,N-diisopropylethylamine, n-butyllithium, lithium diisopropylamide, lithium bistrimethylsilylamide, sodium bistrimethylsilylamide; the inorganic bases includes but are not limited to potassium carbonate, sodium carbonate, sodium bicarbonate, potassium tert-butoxide, sodium hydride, sodium hydroxide, potassium hydroxide, preferably potassium tert-butoxide.

### Step 8

The compound of Formula (II)-IM8 is obtained through a reduction reaction of the compound of Formula (II)-IM7;

In some embodiments, this step is carried out in the presence of a suitable reducing agent, which may be selected from the group consisting of palladium catalyst, platinum catalyst, rhodium catalyst, preferably palladium catalyst;

In some embodiments, this step is carried out at a suitable temperature, and the temperature is 20°C, 25°C, 40°C, 50°C, 60°C, 100°C, preferably 20°C;

In some embodiments, this step is carried out in a suitable organic solvent, which may be selected from the group consisting of methanol, tetrahydrofuran, dichloromethane, N,N-dimethylformamide, N-methylpyrrolidone, dimethyl sulfoxide, n-heptane, n-hexane, ethyl acetate, preferably methanol.

### Step 9

The compound of Formula (II)-IM9 is obtained through a substitution reaction of the compound of Formula (II)-IM8;

In some embodiments, this step is carried out at a suitable temperature, and the temperature is 5°C, 20°C, 25°C, 40°C, 50°C, 60°C, 100°C, preferably 20°C;

In some embodiments, this step is carried out under basic conditions, and reagents for providing the basic conditions include organic bases and inorganic bases, and the organic bases include but are not limited to triethylamine, pyridine, N,N-diisopropylethylamine, n-butyllithium, lithium diisopropylamide, lithium bistrimethylsilylamide, sodium bistrimethylsilylamide; the inorganic bases include but are not limited to potassium carbonate, sodium carbonate, sodium bicarbonate, potassium tert-butoxide, sodium hydride, sodium hydroxide, potassium hydroxide, preferably pyridine.

### Step 10

The compound of Formula (II)-IM10 is obtained through a hydrolysis reaction of the compound of Formula (II)-IM9;

In some embodiments, this step is carried out at a suitable temperature, and the temperature is 20°C, 25°C, 40°C, 50°C, 60°C, 100°C, preferably 60°C;

In some embodiments, this step is carried out in a suitable organic solvent, which may be selected from the group consisting of methanol, tetrahydrofuran, dichloromethane, N,N-dimethylformamide, N-methylpyrrolidone, dimethyl sulfoxide, n-heptane, n-hexane, ethyl acetate, preferably methanol.

In some embodiments, the reaction is carried out under acidic conditions, and reagents for providing the acidic conditions include hydrochloric acid, trifluoroacetic acid, formic acid, sulfuric acid, methanesulfonic acid, preferably hydrochloric acid.

### Step 11

The compound of Formula (II)-IM11 is obtained through ring-closing reaction of the compound of Formula (II)-IM10 and (S)-4-ethyl-4-hydroxy-7,8-dihydro-1H-pyrano[3,4-F]indolizine-3,6,10 (4H)-trione;

In some embodiments, this step is carried out at a suitable temperature, and the temperature is 20°C, 25°C, 40°C, 50°C, 60°C, 100°C, 140°C, preferably 140°C;

In some embodiments, this step is carried out in a suitable organic solvent, which may be selected from the group consisting of toluene, methanol, tetrahydrofuran, dichloromethane, N,N-dimethylformamide, N-methylpyrrolidone, dimethyl sulfoxide, n-heptane, n-hexane, ethyl acetate, preferably toluene.

In some embodiments, the reaction is carried out under acidic conditions, and reagents for providing the acidic conditions include p-toluenesulfonic acid, hydrochloric acid, trifluoroacetic acid, formic acid, sulfuric acid, methanesulfonic acid, preferably p-toluenesulfonic acid.

### Step 12

The compound of Formula (II)-IM12 is obtained through a hydrolysis reaction of the compound of Formula (II)-IM11;

In some embodiments, this step is carried out at a suitable temperature, and the temperature is 20°C, 25°C, 40°C, 50°C, 60°C, 100°C, 140°C, preferably 100°C;

In some embodiments, the reaction is carried out under acidic conditions, and a reagent for providing the acidic conditions includes p-toluenesulfonic acid, hydrochloric acid, trifluoroacetic acid, formic acid, sulfuric acid, methanesulfonic acid, preferably hydrochloric acid.

### Step 13

The compound of Formula (II)-IM13 is obtained through a substitution reaction of the compound of Formula (II)-IM12 and the compound of Formula (II)-SM2.

In some embodiments, this step is carried out at a suitable temperature, and the temperature is 20°C, 25°C, 40°C, 50°C, 60°C, 100°C, 140°C, preferably 50°C;

In some embodiments, this step is carried out in a suitable organic solvent selected from the group consisting of halogenated hydrocarbons (e.g., dichloromethane (DCM), chloroform (TCM), 1,2-dichloroethane (1,2-DCE), etc.), nitriles (e.g., acetonitrile (AN), etc.), N-methylpyrrolidone (NMP), N,N-dimethylformamide (DMF), N,N-dimethylacetamide (DMA), tetrahydrofuran (THF), 1,4-dioxane (Diox), dimethyl sulfoxide (DMSO) and any combination thereof, preferably acetonitrile.

In some embodiments, this step is carried out in the presence of a suitable base, which includes an organic base or an inorganic base, and the organic base may be selected from the group consisting of N,N-diisopropylethylamine (DIPEA), triethylamine (TEA), potassium tert-butoxide (t-BuOK) and pyridine (Py), the inorganic base may be selected from the group consisting of potassium phosphate (K₃PO₄), sodium hydride (NaH), potassium carbonate (K₂CO₃), sodium carbonate (Na₂CO₃), sodium bicarbonate (NaHCO₃), cesium carbonate (Cs₂CO₃) and NaOH, preferably Na₂CO₃ or NaHCO₃;

### Step 14

The compound of Formula (II)-IM14 is obtained through a condensation reaction of the compound of Formula (II)-IM13 and the compound of Formula (II)-SM3;

In some embodiments, this step is carried out in the presence of a suitable condensation reagent selected from the group consisting of HATU, HBTU, EDCI, DCC and HOBT, preferably HATU;

In some embodiments, this step is carried out at a suitable temperature, and the temperature is 20°C, 25°C, 40°C, 50°C, 60°C, 100°C, preferably 25°C;

In some embodiments, this step is carried out in a suitable organic solvent, which may be selected from the group consisting of methanol, tetrahydrofuran, dichloromethane, N,N-dimethylformamide, N-methylpyrrolidone, dimethyl sulfoxide, n-heptane, n-hexane, ethyl acetate, preferably N,N-dimethylformamide.

In some embodiments, this step is carried out in the presence of a suitable base, the base includes an organic base and an inorganic base, the organic base may be selected from the group consisting of DIPEA, TEA, t-BuOK and Py, and the inorganic base may be selected from the group consisting of K₃PO₄, NaH, K₂CO₃, Na₂CO₃, Cs₂CO₃ and NaOH, preferably DIPEA.

### Step 15

The compound of Formula (II)-1 is obtained through an acid hydrolysis reaction of the compound of Formula (II)-IM14;

In some embodiments, this step is carried out at a suitable temperature, and the temperature is 20°C, 25°C, 40°C, 50°C, 60°C, 100°C, 140°C, preferably 25°C;

In some embodiments, this step is carried out in a suitable organic solvent, which may be selected from the group consisting of methanol, tetrahydrofuran, dichloromethane, N,N-dimethylformamide, N-methylpyrrolidone, dimethyl sulfoxide, n-heptane, n-hexane, ethyl acetate, and a mixed solvent thereof, preferably a mixed solution of dichloromethane and methanol (volume ratio 2:1).

In some embodiments, the reaction is carried out under acidic conditions, and reagents for providing the acidic conditions include p-toluenesulfonic acid, hydrochloric acid, trifluoroacetic acid, formic acid, sulfuric acid, methanesulfonic acid, preferably hydrochloric acid.

The synthetic method of Formula (II)-SM3 is as follows: When PG is

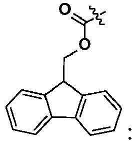

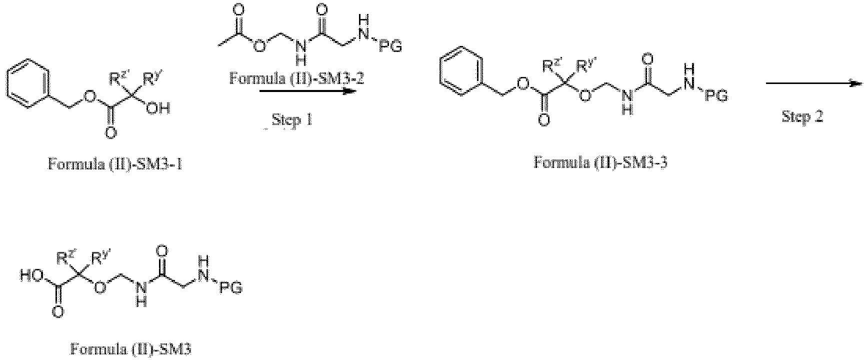

### Step 1

The compound of Formula (II)-SM3-3 is obtained through a substitution reaction of the compound of Formula (II)-SM3-1 and the compound of Formula (II)-SM3-2;

In some embodiments, this step is carried out at a suitable temperature, and the temperature is 20°C, 25°C, 40°C, 50°C, 60°C, 100°C, 140°C, preferably 0-25°C;

In some embodiments, this step is carried out in a suitable organic solvent, which may be selected from the group consisting of methanol, tetrahydrofuran, dichloromethane, N,N-dimethylformamide, N-methylpyrrolidone, dimethyl sulfoxide, n-heptane, n-hexane, ethyl acetate and mixed solvent thereof, preferably tetrahydrofuran.

In some embodiments, this step is carried out in the presence of a suitable base, the base includes an organic base and an inorganic base, the organic base may be selected from the group consisting of DIPEA, TEA, t-BuOK and Py, and the inorganic base may be selected from the group consisting of K₃PO₄, NaH, K₂CO₃, Na₂CO₃, Cs₂CO₃ and NaOH, preferably K₂CO₃.

### Step 2

The compound of Formula (II)-SM3 is obtained through a hydrogenation reaction of the compound of Formula (II)-SM3-3;

In some embodiments, this step is carried out at a suitable temperature, and the temperature is 20°C, 25°C, 40°C, 50°C, 60°C, 100°C, 140°C, preferably 25°C;

In some embodiments, this step is carried out in a suitable organic solvent, which may be selected from the group consisting of methanol, tetrahydrofuran, dichloromethane, N,N-dimethylformamide, N-methylpyrrolidone, dimethyl sulfoxide, n-heptane, n-hexane, ethyl acetate and mixed solvent thereof, preferably methanol.

In some embodiments, this step is carried out in the presence of a suitable reducing agent, which may be selected from the group consisting of palladium catalyst, platinum catalyst, rhodium catalyst, preferably palladium catalyst; when PG is 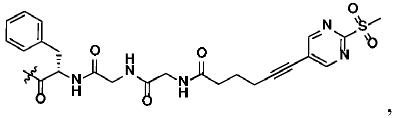 R^{y'} and R^{z'} are hydrogen:

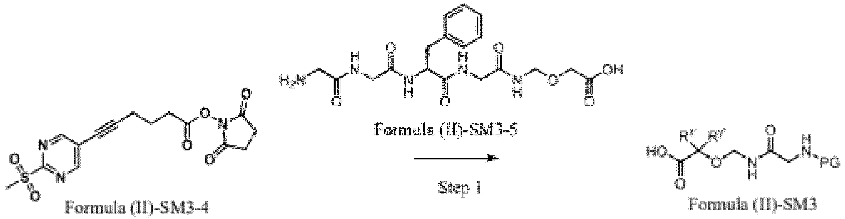

### Step 1

The compound of Formula (II)-SM3 is obtained through a condensation reaction of the compound of Formula (II)-SM3-4 and the compound of Formula (II)-SM3-5;

In some embodiments, this step is carried out at a suitable temperature, and the temperature is 20°C, 25°C, 40°C, 50°C, 60°C, 100°C, 140°C, preferably 25°C;

In some embodiments, this step is carried out in a suitable organic solvent, which may be selected from the group consisting of methanol, tetrahydrofuran, dichloromethane, N,N-dimethylformamide, N-methylpyrrolidone, dimethyl sulfoxide, n-heptane, n-hexane, ethyl acetate and mixed solvent thereof, preferably N,N-dimethylformamide.

Alternatively, the compound of Formula (II)-1 can be synthesized by the following synthetic route:

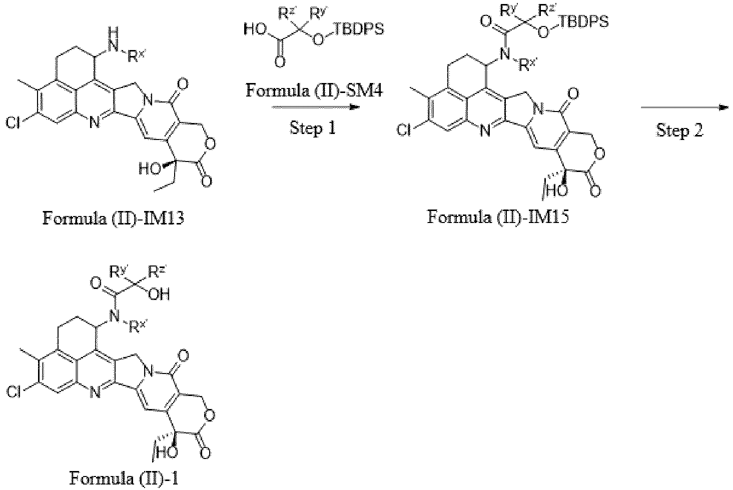

### Step 1

The compound of Formula (II)-IM15 is obtained through a condensation reaction of the compound of Formula (II)-IM13 and the compound of Formula (II)-SM4;

In some embodiments, this step is carried out in the presence of a suitable condensation reagent selected from the group consisting of HATU, HBTU, EDCI, DCC and HOBT, preferably HATU;

In some embodiments, this step is carried out at a suitable temperature, and the temperature is 20°C, 25°C, 40°C, 50°C, 60°C, 100°C, preferably 25°C;

In some embodiments, this step is carried out in a suitable organic solvent, which may be selected from the group consisting of methanol, tetrahydrofuran, dichloromethane, N,N-dimethylformamide, N-methylpyrrolidone, dimethyl sulfoxide, n-heptane, n-hexane, ethyl acetate, preferably N,N-dimethylformamide.

In some embodiments, this step is carried out in the presence of a suitable base, the base includes an organic base or an inorganic base, the organic base may be selected from the group consisting of DIPEA, TEA, t-BuOK and Py, and the inorganic base may be selected from the group consisting of K₃PO₄, NaH, K₂CO₃, Na₂CO₃, Cs₂CO₃ and NaOH, preferably DIPEA.

### Step 2

The compound of Formula (II)-1 is obtained by removing the silicon protecting group of the compound of Formula (II)-IM15;
alternatively, the compound of Formula (II) may be synthesized by the following synthetic route:

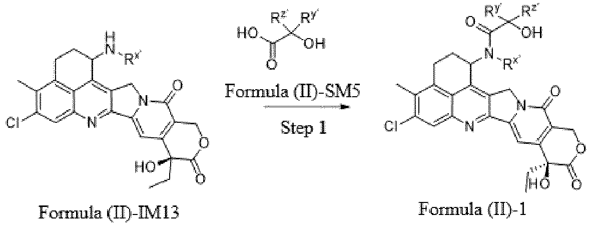

### Step 1

The compound of Formula (II) is obtained through a condensation reaction of the compound of Formula (II)-IM13 and the compound of Formula (II)-SM5;

In some embodiments, this step is carried out in the presence of a suitable condensation reagent selected from the group consisting of HATU, HBTU, EDCI, DCC and HOBT, preferably HATU;

In some embodiments, this step is carried out at a suitable temperature, and the temperature is 20°C, 25°C, 40°C, 50°C, 60°C, 100°C, preferably 25°C;

In some embodiments, this step is carried out in a suitable organic solvent, which may be selected from the group consisting of methanol, tetrahydrofuran, dichloromethane, N,N-dimethylformamide, N-methylpyrrolidone, dimethyl sulfoxide, n-heptane, n-hexane, ethyl acetate, preferably N,N-dimethylformamide.

In some embodiments, this step is carried out in the presence of a suitable base, the base includes an organic base or an inorganic base, the organic base may be selected from the group consisting of DIPEA, TEA, t-BuOK and Py, and the inorganic base may be selected from the group consisting of K₃PO₄, NaH, K₂CO₃, Na₂CO₃, Cs₂CO₃ and NaOH, preferably DIPEA.

The compound of Formula (III)-1 in the present invention can be synthesized by the following synthetic route. 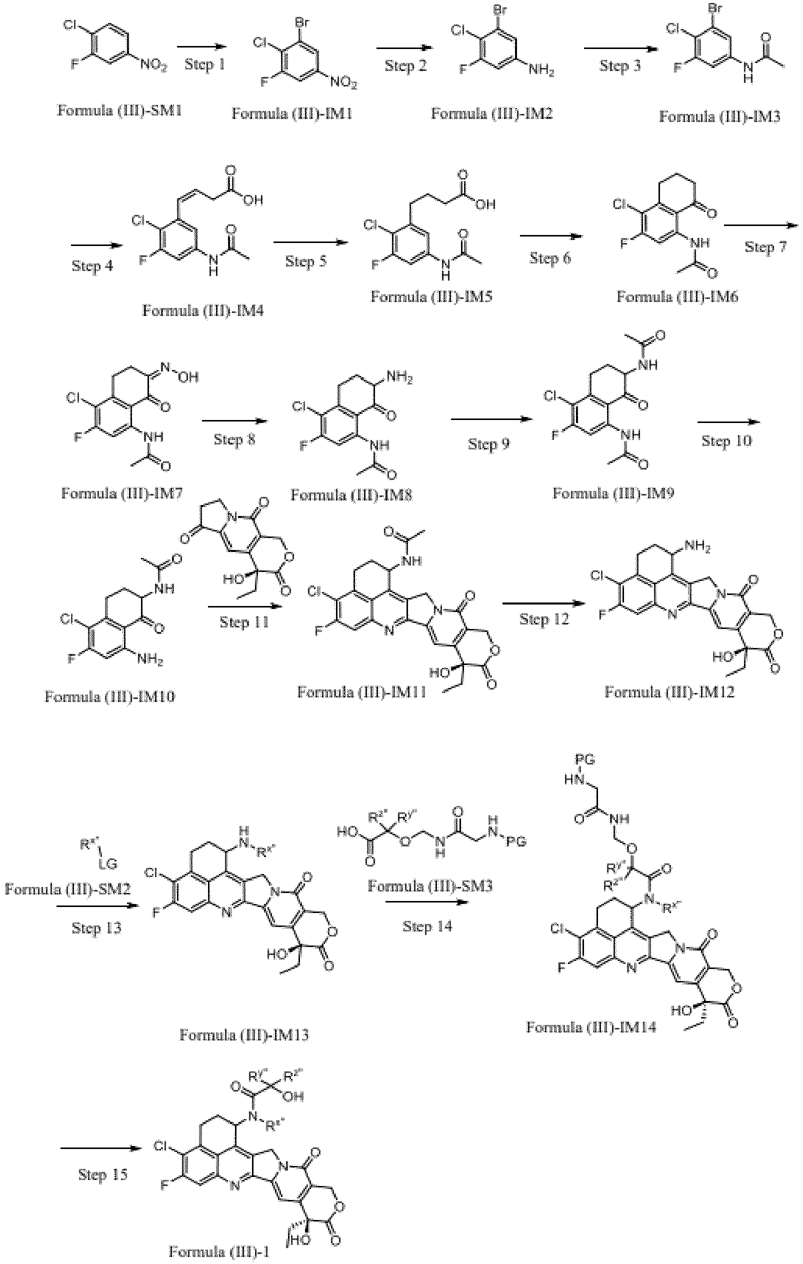 wherein, the meanings of R^{x"}, R^{y"} and R^{z"} are as described above; LG is a leaving group selected from the group consisting of methylsulfonyl, trifluoromethylsulfonyloxy and halogen, preferably trifluoromethylsulfonyloxy or chlorine; PG is a protecting group, selected from the group consisting of

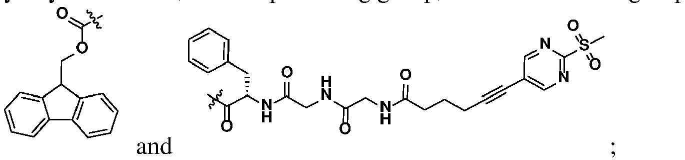

### Step 1

The compound of Formula (III)-IM1 is obtained through a substitution reaction of the compound of Formula (III)-SM1.

In some embodiments, this step is carried out at a suitable temperature, and the temperature is 20°C, 25°C, 50°C, 60°C, 100°C, preferably 50°C;

In some embodiments, this step is carried out in a suitable organic solvent, which may be selected from the group consisting of tetrahydrofuran, dichloromethane, N,N-dimethylformamide, N-methylpyrrolidone, dimethyl sulfoxide, n-heptane, n-hexane, ethyl acetate, preferably n-heptane.

### Step 2

The compound of Formula (III)-IM2 is obtained by a reduction reaction of the compound of Formula (III)-IM1;

In some embodiments, this step is carried out in the presence of a suitable reducing agent, which may be selected from the group consisting of palladium catalyst, platinum catalyst, rhodium catalyst, preferably platinum catalyst;

In some embodiments, this step is carried out at a suitable temperature, and the temperature is 20°C, 25°C, 50°C, 60°C, 100°C, preferably 60°C;

In some embodiments, this step is carried out in a suitable organic solvent, which may be selected from the group consisting of tetrahydrofuran, dichloromethane, N,N-dimethylformamide, N-methylpyrrolidone, dimethyl sulfoxide, n-heptane, n-hexane, ethyl acetate, preferably ethyl acetate.

### Step 3

The compound of Formula (III)-IM3 is obtained through a substitution reaction of the compound of Formula (III)-IM2,

In some embodiments, this step is carried out at a suitable temperature, and the temperature is 20°C, 25°C, 50°C, 60°C, 100°C, preferably 20°C;

In some embodiments, this step is carried out in a suitable organic solvent, which may be selected from the group consisting of tetrahydrofuran, dichloromethane, N,N-dimethylformamide, N-methylpyrrolidone, dimethyl sulfoxide, n-heptane, n-hexane, ethyl acetate, preferably ethyl acetate;

In some embodiments, this step is carried out under basic conditions, and reagents for providing the basic conditions include organic bases and inorganic bases, and the organic bases include but are not limited to triethylamine, pyridine, N,N-diisopropylethylamine, n-butyllithium, lithium diisopropylamide, lithium bistrimethylsilylamide, sodium bistrimethylsilylamide; the inorganic bases include but are not limited to potassium carbonate, sodium carbonate, sodium bicarbonate, potassium tert-butoxide, sodium hydride, sodium hydroxide, potassium hydroxide, preferably triethylamine.

### Step 4

The compound of Formula (III)-IM4 is obtained through a coupling reaction of the compound of Formula (III)-IM3;

In some embodiments, this step is carried out at a suitable temperature, and the temperature is 20°C, 25°C, 50°C, 60°C, 70°C, 100°C, preferably 70°C;

In some embodiments, this step is carried out in a suitable organic solvent, which may be selected from the group consisting of tetrahydrofuran, dichloromethane, N,N-dimethylformamide, N-methylpyrrolidone, dimethyl sulfoxide, n-heptane, n-hexane, ethyl acetate, water, preferably a mixed solvent of tetrahydrofuran and water;

In some embodiments, this step is carried out under basic conditions, reagents for providing the basic conditions include organic bases and inorganic bases, the organic bases include but are not limited to triethylamine, pyridine, N,N-diisopropylethylamine, n-butyllithium, lithium diisopropylamide, lithium bistrimethylsilylamide, sodium bistrimethylsilylamide; the inorganic bases include but are not limited to potassium carbonate, sodium carbonate, sodium bicarbonate, potassium tert-butoxide, sodium hydride, sodium hydroxide, potassium hydroxide, preferably N,N-diisopropylethylamine.

### Step 5

The compound of Formula (III)-IM5 is obtained by a reduction reaction of the compound of Formula (III)-IM4;

In some embodiments, this step is carried out in the presence of a suitable reducing agent, which may be selected from the group consisting of palladium catalyst, platinum catalyst, rhodium catalyst, preferably platinum catalyst;

In some embodiments, this step is carried out at a suitable temperature, and the temperature is 20°C, 25°C, 40°C, 50°C, 60°C, 100°C, preferably 40°C;

In some embodiments, this step is carried out in a suitable organic solvent, which may be selected from the group consisting of tetrahydrofuran, dichloromethane, N,N-dimethylformamide, N-methylpyrrolidone, dimethyl sulfoxide, n-heptane, n-hexane, ethyl acetate, preferably tetrahydrofuran.

### Step 6

The compound of Formula (III)-IM6 is obtained through a ring-closing reaction of the compound of Formula (III)-IM5;

In some embodiments, this step is carried out at a suitable temperature, and the temperature is 5°C, 20°C, 25°C, 40°C, 50°C, 60°C, 100°C, preferably 5°C;

In some embodiments, this step is carried out in a suitable organic solvent, which may be selected from the group consisting of trifluoroacetic acid, tetrahydrofuran, dichloromethane, N,N-dimethylformamide, N-methylpyrrolidone, dimethyl sulfoxide, n-heptane, n-hexane, ethyl acetate, tert-butanol, preferably a mixed solvent of tetrahydrofuran and tert-butanol.

### Step 7

The compound of Formula (III)-IM7 is obtained through a substitution reaction of the compound of Formula (III)-IM6;

In some embodiments, this step is carried out at a suitable temperature, and the temperature is 5°C, 20°C, 25°C, 40°C, 50°C, 60°C, 100°C, preferably 5°C;

In some embodiments, this step is carried out in a suitable organic solvent, which may be selected from the group consisting of trifluoroacetic acid, tetrahydrofuran, dichloromethane, N,N-dimethylformamide, N-methylpyrrolidone, dimethyl sulfoxide, n-heptane, n-hexane, ethyl acetate, preferably trifluoroacetic acid;

In some embodiments, this step is carried out under basic conditions, and reagents for providing the basic conditions include organic bases and inorganic bases, and the organic bases include but are not limited to triethylamine, pyridine, N,N-diisopropylethylamine, n-butyllithium, lithium diisopropylamide, lithium bistrimethylsilylamide, sodium bistrimethylsilylamide; the inorganic bases include but are not limited to potassium carbonate, sodium carbonate, sodium bicarbonate, potassium tert-butoxide, sodium hydride, sodium hydroxide, potassium hydroxide, preferably potassium tert-butoxide.

### Step 8

The compound of Formula (III)-IM8 is obtained by a reduction reaction of the compound of Formula (III)-IM7;

In some embodiments, this step is carried out in the presence of a suitable reducing agent, which may be selected from the group consisting of palladium catalyst, platinum catalyst, rhodium catalyst, preferably palladium catalyst;

In some embodiments, this step is carried out at a suitable temperature, and the temperature is 20°C, 25°C, 40°C, 50°C, 60°C, 100°C, preferably 20°C;

In some embodiments, this step is carried out in a suitable organic solvent, which may be selected from the group consisting of methanol, tetrahydrofuran, dichloromethane, N,N-dimethylformamide, N-methylpyrrolidone, dimethyl sulfoxide, n-heptane, n-hexane, ethyl acetate, preferably methanol.

### Step 9

The compound of Formula (III)-IM9 is obtained through a substitution reaction of the compound of Formula (III)-IM8;

In some embodiments, this step is carried out at a suitable temperature, and the temperature is 5°C, 20°C, 25°C, 40°C, 50°C, 60°C, 100°C, preferably 20°C;

In some embodiments, this step is carried out under basic conditions, reagents for providing the basic conditions include organic bases and inorganic bases, the organic bases include but are not limited to triethylamine, pyridine, N,N-diisopropylethylamine, n-butyllithium, lithium diisopropylamide, lithium bistrimethylsilylamide, sodium bistrimethylsilylamide; and the inorganic bases include but are not limited to potassium carbonate, sodium carbonate, sodium bicarbonate, potassium tert-butoxide, sodium hydride, sodium hydroxide, potassium hydroxide, preferably pyridine.

### Step 10

The compound of Formula (III)-IM10 is obtained through a hydrolysis reaction of the compound of Formula (III)-IM9;

In some embodiments, this step is carried out at a suitable temperature, and the temperature is 20°C, 25°C, 40°C, 50°C, 60°C, 100°C, preferably 60°C;

In some embodiments, this step is carried out in a suitable organic solvent, which may be selected from the group consisting of methanol, tetrahydrofuran, dichloromethane, N,N-dimethylformamide, N-methylpyrrolidone, dimethyl sulfoxide, n-heptane, n-hexane, ethyl acetate, preferably methanol.

In some embodiments, the reaction is carried out under acidic conditions, and reagents for providing the acidic conditions include hydrochloric acid, trifluoroacetic acid, formic acid, sulfuric acid, methanesulfonic acid, preferably hydrochloric acid.

### Step 11

The compound of Formula (III)-IM11 is obtained through a ring-closing reaction of the compound of Formula (III)-IM10 and (S)-4-ethyl-4-hydroxy-7,8-dihydro-1H-pyrano[3,4-F]indolizine-3,6,10(4H)-trione;

In some embodiments, this step is carried out at a suitable temperature, and the temperature is 20°C, 25°C, 40°C, 50°C, 60°C, 100°C, 140°C, preferably 140°C;

In some embodiments, this step is carried out in a suitable organic solvent, which may be selected from the group consisting of toluene, methanol, tetrahydrofuran, dichloromethane, N,N-dimethylformamide, N-methylpyrrolidone, dimethyl sulfoxide, n-heptane, n-hexane, ethyl acetate, preferably toluene.

In some embodiments, the reaction is carried out under acidic conditions, and reagents for providing the acidic conditions include p-toluenesulfonic acid, hydrochloric acid, trifluoroacetic acid, formic acid, sulfuric acid, methanesulfonic acid, preferably p-toluenesulfonic acid.

### Step 12

The compound of Formula (III)-IM12 is obtained through a hydrolysis reaction of the compound of Formula (III)-IM11;

In some embodiments, this step is carried out at a suitable temperature, and the temperature is 20°C, 25°C, 40°C, 50°C, 60°C, 100°C, 140°C, preferably 100°C;

In some embodiments, the reaction is carried out under acidic conditions, and reagents for providing the acidic conditions include p-toluenesulfonic acid, hydrochloric acid, trifluoroacetic acid, formic acid, sulfuric acid, methanesulfonic acid, preferably hydrochloric acid.

### Step 13

The compound of Formula (III)-IM13 is obtained by a substitution reaction of the compound of Formula (III)-IM12 and the compound of Formula (III)-SM2.

The substitution reaction is carried out to obtain the compound of Formula (II)-IM13.

In some embodiments, this step is carried out at a suitable temperature, and the temperature is 20°C, 25°C, 40°C, 50°C, 60°C, 100°C, 140°C, preferably 50°C;

In some embodiments, this step is carried out in a suitable organic solvent selected from the group consisting of halogenated hydrocarbons (e.g., dichloromethane (DCM), chloroform (TCM), 1,2-dichloroethane (1,2-DCE), etc.), nitrile (e.g., acetonitrile (AN), etc.), N-methylpyrrolidone (NMP), N,N-dimethylformamide (DMF), N,N-dimethylacetamide (DMA), tetrahydrofuran (THF), 1,4-dioxane (Dioxane), dimethyl sulfoxide (DMSO) and any combination thereof, preferably acetonitrile.

In some embodiments, this step is carried out in the presence of a suitable base, which includes an organic base or an inorganic base, the organic base may be selected from the group consisting of N,N-diisopropylethylamine (DIPEA), triethylamine (TEA), potassium tert-butoxide (t-BuOK) and pyridine (Py), and the inorganic base may be selected from the group consisting of potassium phosphate (K₃PO₄), sodium hydride (NaH), potassium carbonate (K₂CO₃), sodium carbonate (Na₂CO₃), sodium bicarbonate (NaHCO₃), cesium carbonate (Cs₂CO₃) and NaOH, preferably Na₂CO₃ or NaHCO₃;

### Step 14

The compound of Formula (III)-IM14 is obtained through a condensation reaction of the compound of Formula (III)-IM13 and the compound of Formula (III)-SM3;

In some embodiments, this step is carried out in the presence of a suitable condensation reagent selected from the group consisting of HATU, HBTU, EDCI, DCC and HOBT, preferably HATU;

In some embodiments, this step is carried out at a suitable temperature, and the temperature is 20°C, 25°C, 40°C, 50°C, 60°C, 100°C, preferably 25°C;

In some embodiments, this step is carried out in a suitable organic solvent, which may be selected from the group consisting of methanol, tetrahydrofuran, dichloromethane, N,N-dimethylformamide, N-methylpyrrolidone, dimethyl sulfoxide, n-heptane, n-hexane, ethyl acetate, preferably N,N-dimethylformamide.

In some embodiments, this step is carried out in the presence of a suitable base, the base includes an organic base or an inorganic base, the organic base may be selected from the group consisting of DIPEA, TEA, t-BuOK and Py, and the inorganic base may be selected from the group consisting of K₃PO₄, NaH, K₂CO₃, Na₂CO₃, Cs₂CO₃ and NaOH, preferably DIPEA.

### Step 15

The compound of Formula (III)-1 is obtained through an acid hydrolysis reaction of the compound of Formula (III)-IM14;

In some embodiments, this step is carried out at a suitable temperature, and the temperature is 20°C, 25°C, 40°C, 50°C, 60°C, 100°C, 140°C, preferably 25°C;

In some embodiments, this step is carried out in a suitable organic solvent, which may be selected from the group consisting of methanol, tetrahydrofuran, dichloromethane, N,N-dimethylformamide, N-methylpyrrolidone, dimethyl sulfoxide, n-heptane, n-hexane, ethyl acetate and mixed solvent thereof, preferably a mixed solution of dichloromethane and methanol (volume ratio 2:1).

In some embodiments, the reaction is carried out under acidic conditions, and reagents for providing the acidic conditions include p-toluenesulfonic acid, hydrochloric acid, trifluoroacetic acid, formic acid, sulfuric acid, methanesulfonic acid, preferably hydrochloric acid.

The synthetic method of Formula (III)-SM3 is as follows:
When PG is

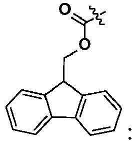

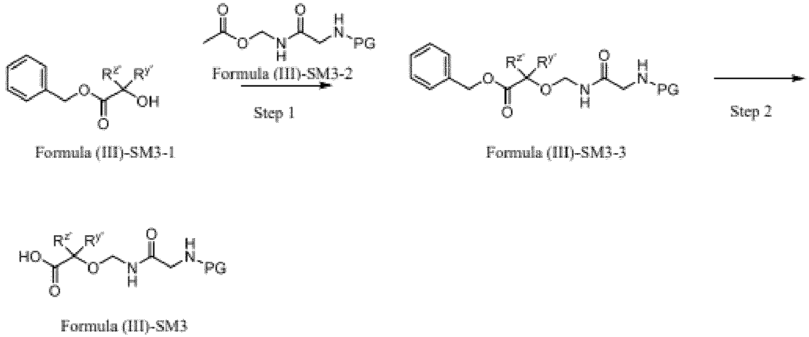

### Step 1:

The compound of Formula (III)-SM3-3 is obtained through a substitution reaction of the compound of Formula (III)-SM3-1 and the compound of Formula (II)-SM3-2;

In some embodiments, this step is carried out at a suitable temperature, and the temperature is 20°C, 25°C, 40°C, 50°C, 60°C, 100°C, 140°C, preferably 0-25°C;

In some embodiments, this step is carried out in a suitable organic solvent, which may be selected from the group consisting of methanol, tetrahydrofuran, dichloromethane, N,N-dimethylformamide, N-methylpyrrolidone, dimethyl sulfoxide, n-heptane, n-hexane, ethyl acetate and mixed solvent thereof, preferably tetrahydrofuran.

In some embodiments, this step is carried out in the presence of a suitable base, the base includes an organic base or an inorganic base, the organic base may be selected from the group consisting of DIPEA, TEA, t-BuOK and Py, and the inorganic base may be selected from the group consisting of K₃PO₄, NaH, K₂CO₃, Na₂CO₃, Cs₂CO₃ and NaOH, preferably K₂CO₃.

### Step 2:

The compound of Formula (III)-SM3 is obtained through a hydrogenation reaction of the compound of Formula (III)-SM3-3;

In some embodiments, this step is carried out at a suitable temperature, and the temperature is 20°C, 25°C, 40°C, 50°C, 60°C, 100°C, 140°C, preferably 25°C;

In some embodiments, this step is carried out in a suitable organic solvent, which may be selected from the group consisting of methanol, tetrahydrofuran, dichloromethane, N,N-dimethylformamide, N-methylpyrrolidone, dimethyl sulfoxide, n-heptane, n-hexane, ethyl acetate and mixed solvent thereof, preferably methanol.

In some embodiments, this step is carried out in the presence of a suitable reducing agent, which may be selected from the group consisting of palladium catalyst, platinum catalyst, rhodium catalyst, preferably palladium catalyst;
when PG is 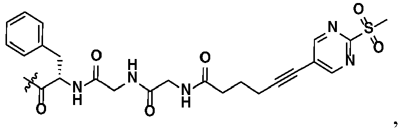 R^{y'} and R^{z'} are hydrogen:

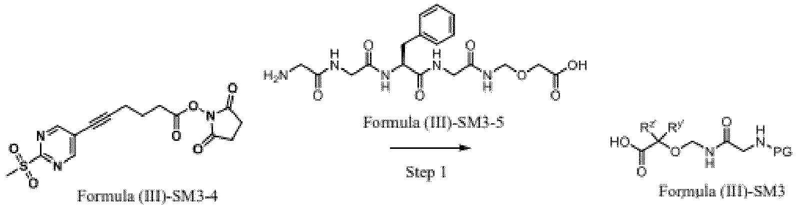

### Step 1:

The compound of Formula (III)-SM3 is obtained through a condensation reaction of the compound of Formula (III)-SM3-4 and the compound of Formula (III)-SM3-5;

In some embodiments, this step is carried out at a suitable temperature, and the temperature is 20°C, 25°C, 40°C, 50°C, 60°C, 100°C, 140°C, preferably 25°C;

In some embodiments, this step is carried out in a suitable organic solvent, which may be selected from the group consisting of methanol, tetrahydrofuran, dichloromethane, N,N-dimethylformamide, N-methylpyrrolidone, dimethyl sulfoxide, n-heptane, n-hexane, ethyl acetate and mixed solvent thereof, preferably N,N-dimethylformamide.

Alternatively, the compound of Formula (III)-1 may be synthesized by the following synthetic route:

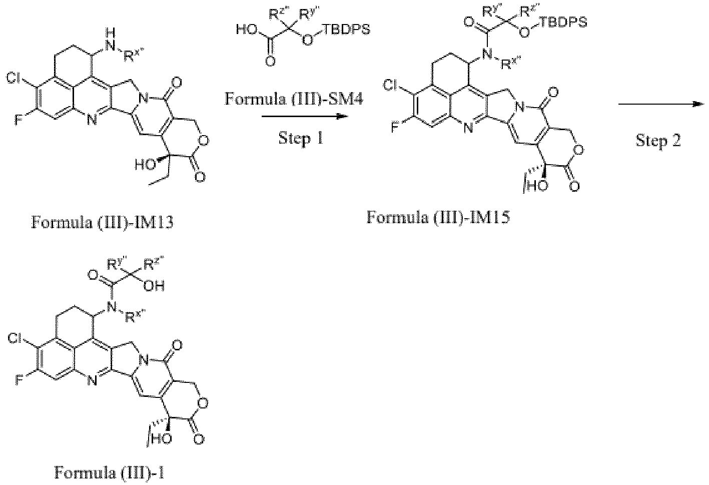

### Step 1:

The compound of Formula (III)-IM15 is obtained through a condensation reaction of the compound of Formula (III)-IM13 and the compound of Formula (III)-SM4;

In some embodiments, this step is carried out in the presence of a suitable condensation reagent selected from the group consisting of HATU, HBTU, EDCI, DCC and HOBT, preferably HATU;

In some embodiments, this step is carried out at a suitable temperature, and the temperature is 20°C, 25°C, 40°C, 50°C, 60°C, 100°C, preferably 25°C;

In some embodiments, this step is carried out in a suitable organic solvent, which may be selected from the group consisting of methanol, tetrahydrofuran, dichloromethane, N,N-dimethylformamide, N-methylpyrrolidone, dimethyl sulfoxide, n-heptane, n-hexane, ethyl acetate, preferably N,N-dimethylformamide.

In some embodiments, this step is carried out in the presence of a suitable base, the base includes an organic base or an inorganic base, the organic base may be selected from the group consisting of DIPEA, TEA, t-BuOK and Py, and the inorganic base may be selected from the group consisting of K₃PO₄, NaH, K₂CO₃, Na₂CO₃, Cs₂CO₃ and NaOH, preferably DIPEA.

### Step 2:

The compound of Formula (III)-1 is obtained by removing the silicon protecting group of the compound of Formula (III)-IM15;
alternatively, the compound of Formula (III)-1 can be synthesized by the following synthetic route:

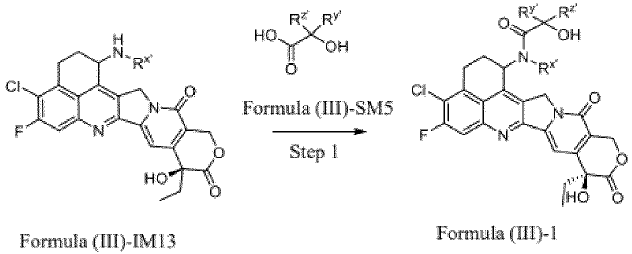

### Step 1:

The compound of Formula (III)-1 is obtained through a condensation reaction of the compound of Formula (III)-IM13 and the compound of Formula (III)-SM5;

In some embodiments, this step is carried out in the presence of a suitable condensation reagent selected from the group consisting of HATU, HBTU, EDCI, DCC and HOBT, preferably HATU;

In some embodiments, this step is carried out at a suitable temperature, and the temperature is 20°C, 25°C, 40°C, 50°C, 60°C, 100°C, preferably 25°C;

In some embodiments, this step is carried out in a suitable organic solvent, which may be selected from the group consisting of methanol, tetrahydrofuran, dichloromethane, N,N-dimethylformamide, N-methylpyrrolidone, dimethyl sulfoxide, n-heptane, n-hexane, ethyl acetate, preferably N,N-dimethylformamide.

In some embodiments, this step is carried out in the presence of a suitable base, the base includes an organic base or an inorganic base, the organic base may be selected from the group consisting of DIPEA, TEA, t-BuOK and Py, and the inorganic base may be selected from the group consisting of K₃PO₄, NaH, K₂CO₃, Na₂CO₃, Cs₂CO₃ and NaOH, preferably DIPEA.

The compound of Formula (IV) in the present invention can be synthesized and prepared by following synthetic route:
wherein, the meanings of R^{a}, R^{b}, R^{c}, R^{d} and R^{e} are as described above;
when q=1,

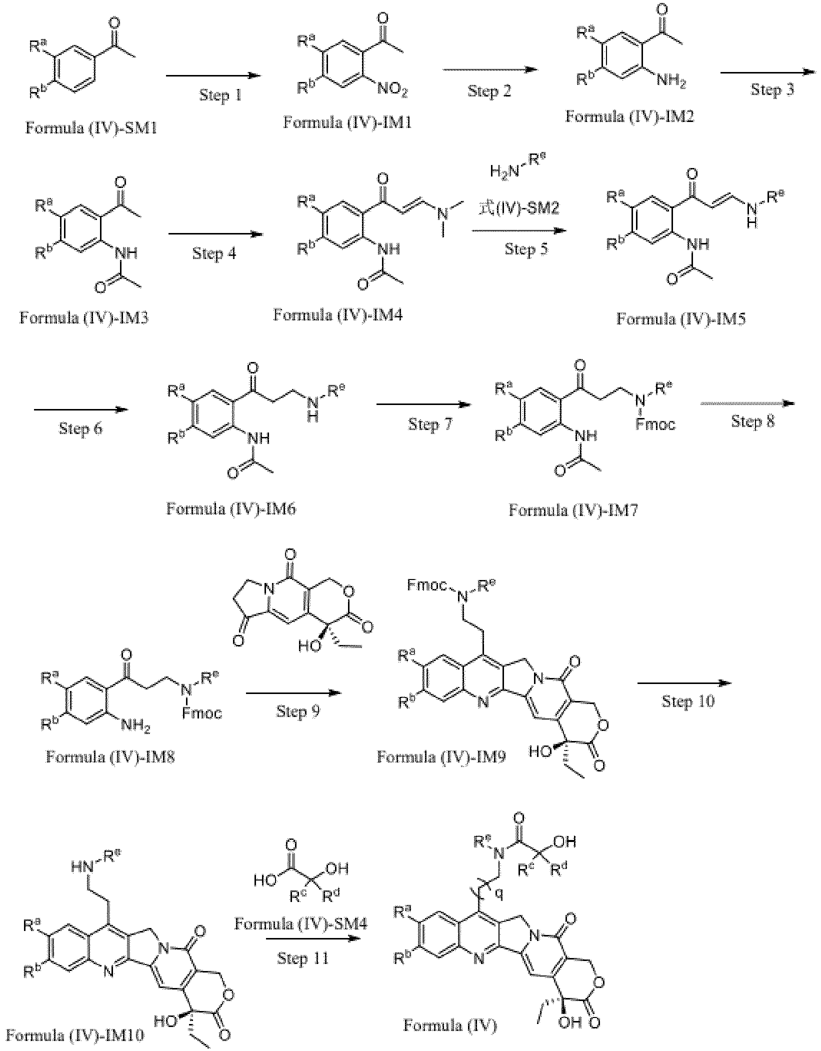

### Step 1

The compound of Formula (IV)-IM1 is obtained through a nitration reaction of the compound of Formula (IV)-SM1;

In some embodiments, this step is carried out at a suitable temperature, which is 5°C, 20°C, 25°C, 40°C, 50°C, 60°C, 100°C, preferably 25°C.

### Step 2

The compound of Formula (IV)-IM2 is obtained through a hydrogenation reaction of the compound of Formula (IV)-IM1;

In some embodiments, this step is carried out in the presence of a suitable reducing agent, which may be selected from the group consisting of palladium catalyst, platinum catalyst, rhodium catalyst, preferably palladium catalyst;

In some embodiments, this step is carried out at a suitable temperature, the temperature is 20°C, 25°C, 40°C, 50°C, 60°C, 100°C, preferably 25°C;

In some embodiments, this step is carried out in a suitable organic solvent, which may be selected from the group consisting of methanol, tetrahydrofuran, dichloromethane, N,N-dimethylformamide, N-methylpyrrolidone, dimethyl sulfoxide, n-heptane, n-hexane, ethyl acetate, preferably ethyl acetate.

### Step 3

The compound of Formula (IV)-IM3 is obtained through an acylation reaction of the compound of Formula (IV)-IM2,

In some embodiments, this step is carried out at a suitable temperature, and the temperature is 20°C, 25°C, 50°C, 60°C, 100°C, preferably 25°C;

### Step 4

The compound of Formula (IV)-IM4 is obtained through a reaction of the compound of Formula (IV)-IM3 and DMF-DMA;

In some embodiments, this step is carried out at a suitable temperature, and the temperature is 20°C, 25°C, 40°C, 50°C, 60°C, 100°C, 120°C, preferably 120°C;

### Step 5

The compound of Formula (IV)-IM5 is obtained through a substitution reaction of the compound of Formula (IV)-IM4 and the compound of Formula (IV)-SM2;

In some embodiments, this step is carried out at a suitable temperature, and the temperature is 20°C, 25°C, 40°C, 50°C, 60°C, 100°C, 140°C, preferably 50°C;

In some embodiments, this step is carried out in a suitable organic solvent, which may be selected from the group consisting of methanol, ethanol, N-methylpyrrolidone, dimethyl sulfoxide, preferably ethanol;

### Step 6

The compound of Formula (IV)-IM6 is obtained through a reduction reaction of the compound of Formula (IV)-IM5;

In some embodiments, this step is carried out in the presence of a suitable reducing agent, which is preferably sodium borohydride;

In some embodiments, this step is carried out at a suitable temperature, and the temperature is 20°C, 25°C, 40°C, 50°C, 60°C, 100°C, preferably 0-25°C;

In some embodiments, this step is carried out in a suitable organic solvent, which may be selected from the group consisting of tetrahydrofuran, glacial acetic acid, methanol and mixed solution thereof, preferably glacial acetic acid.

### Step 7

The compound of Formula (IV)-IM7 is obtained by protecting the amino group of the compound of Formula (IV)-IM6 with Fmoc;

### Step 8

The compound of Formula (IV)-IM8 is obtained by removing the acetyl protecting group of the amino group of the compound of Formula (IV)-IM7;

### Step 9

The compound of Formula (IV)-IM9 is obtained through a ring-closing reaction of the compound of Formula (IV)-IM8 under acidic conditions;

In some embodiments, this step is carried out at a suitable temperature, and the temperature is 20°C, 25°C, 40°C, 50°C, 60°C, 100°C, 120°C, preferably 120°C;

In some embodiments, this step is carried out in a suitable organic solvent, which may be selected from the group consisting of toluene, xylene, N,N-dimethylformamide, N-methylpyrrolidone, dimethyl sulfoxide, preferably toluene and xylene;

In some embodiments, this step is carried out under acidic conditions; and reagents for providing the acidic conditions include p-toluenesulfonic acid, hydrochloric acid, trifluoroacetic acid, formic acid, sulfuric acid, methanesulfonic acid, preferably p-toluenesulfonic acid.

### Step 10

The compound of Formula (IV)-IM10 is obtained by removing the Fmoc protecting group of the compound of Formula (IV)-IM9;

### Step 11

The compound of Formula (IV) is obtained through a condensation reaction of the compound of Formula (IV)-IM10 and the compound of Formula (IV)-SM4;

In some embodiments, this step is carried out in the presence of a suitable condensation reagent selected from the group consisting of HATU, HBTU, EDCI, DCC and HOBT, preferably HBTU;

In some embodiments, this step is carried out at a suitable temperature, and the temperature is 20°C, 25°C, 40°C, 50°C, 60°C, 100°C, preferably 25°C;

In some embodiments, this step is carried out in a suitable organic solvent, which may be selected from the group consisting of methanol, tetrahydrofuran, dichloromethane, N,N-dimethylformamide, N-methylpyrrolidone, dimethyl sulfoxide, n-heptane, n-hexane, ethyl acetate, preferably N,N-dimethylformamide.

In some embodiments, this step is carried out in the presence of a suitable base, the base includes an organic base or an inorganic base, the organic base may be selected from the group consisting of DIPEA, TEA, t-BuOK and Py, and the inorganic base may be selected from the group consisting of K₃PO₄, NaH, K₂CO₃, Na₂CO₃, Cs₂CO₃ and NaOH, preferably DIPEA.

Alternatively, when q=0,

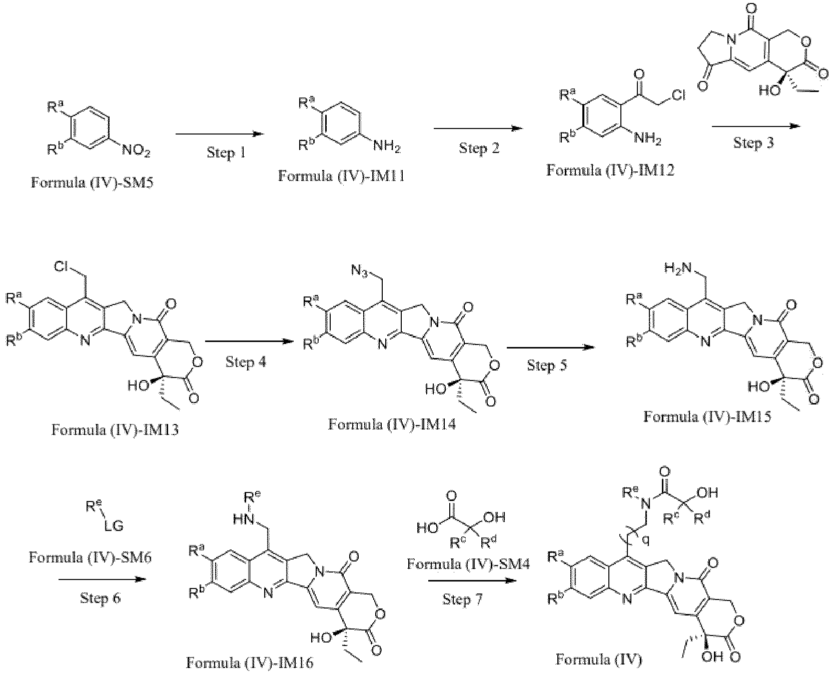

LG is a leaving group selected from the group consisting of methylsulfonyl, trifluoromethylsulfonyloxy and halogen, preferably trifluoromethylsulfonyloxy or iodine;

### Step 1

The compound of Formula (IV)-IM11 is obtained through a reduction reaction of the compound by Formula (IV)-SM5,

In some embodiments, this step is carried out in the presence of a suitable reducing agent, which may be selected from the group consisting of palladium catalyst, platinum catalyst, rhodium catalyst, preferably platinum catalyst;

In some embodiments, this step is carried out at a suitable temperature, and the temperature is 20°C, 25°C, 50°C, 60°C, 100°C, preferably 25°C;

In some embodiments, this step is carried out in a suitable organic solvent, which may be selected from the group consisting of tetrahydrofuran, dichloromethane, N,N-dimethylformamide, N-methylpyrrolidone, dimethyl sulfoxide, n-heptane, n-hexane, ethyl acetate, preferably ethyl acetate and tetrahydrofuran.

### Step 2

The compound of Formula (IV)-IM12 is obtained through a Friedel-Crafts acylation reaction of the compound of Formula (IV)-IM11;

In some embodiments, this step is carried out at a suitable temperature, which is 5°C, 20°C, 25°C, 40°C, 50°C, 60°C, 100°C, preferably 25°C.

### Step 3

The compound of Formula (IV)-IM13 is obtained through a ring-closing reaction of Formula (IV)-IM12 under acidic conditions;

In some embodiments, this step is carried out at a suitable temperature, and the temperature is 20°C, 25°C, 40°C, 50°C, 60°C, 100°C, 120°C, preferably 120°C;

In some embodiments, this step is carried out in a suitable organic solvent, which may be selected from the group consisting of toluene, xylene, N,N-dimethylformamide, N-methylpyrrolidone, dimethyl sulfoxide, preferably, toluene and xylene;

In some embodiments, this step is carried out under acidic conditions; and reagents for providing the acidic conditions include p-toluenesulfonic acid, hydrochloric acid, trifluoroacetic acid, formic acid, sulfuric acid, methanesulfonic acid, preferably p-toluenesulfonic acid.

### Step 4

The compound of Formula (IV)-IM14 is obtained through a substitution reaction of Formula (IV)-IM13;

In some embodiments, this step is carried out at a suitable temperature, and the temperature is 20°C, 25°C, 40°C, 50°C, 60°C, 100°C, 140°C, preferably 25°C;

In some embodiments, this step is carried out in a suitable organic solvent, which may be selected from the group consisting of methanol, ethanol, N, N-methylpyrrolidone, dimethyl sulfoxide, preferably dimethyl sulfoxide;

### Step 5

The compound of Formula (IV)-IM15 is obtained by a reduction reaction of the compound of Formula (IV)-IM14,

In some embodiments, this step is carried out in the presence of a suitable reducing agent selected from the group consisting of palladium catalyst, platinum catalyst, rhodium catalyst, triphenylphosphine, triethyl phosphite, preferably triethyl phosphite;

In some embodiments, this step is carried out at a suitable temperature, and the temperature is 20°C, 25°C, 50°C, 60°C, 80°C, 100°C, preferably 80°C;

In some embodiments, this step is carried out in a suitable organic solvent, which may be selected from the group consisting of methanol, tetrahydrofuran, dichloromethane, N,N-dimethylformamide, N-methylpyrrolidone, dimethyl sulfoxide, n-heptane, n-hexane, ethyl acetate, toluene, and a mixed solution thereof, preferably a mixed solution of methanol and toluene.

### Step 6

The compound of Formula (IV)-IM16 is obtained through a substitution reaction of the compound of Formula (IV)-IM15 and the compound of Formula (IV)-SM6.

In some embodiments, this step is carried out at a suitable temperature, and the temperature is 20°C, 25°C, 40°C, 50°C, 60°C, 100°C, 140°C, preferably 50°C;

In some embodiments, this step is carried out in a suitable organic solvent selected from the group consisting of halogenated hydrocarbons (e.g., dichloromethane (DCM), chloroform (TCM), 1,2-dichloroethane (1,2-DCE), etc.), nitriles (e.g., acetonitrile (AN), etc.), N-methylpyrrolidone (NMP), N,N-dimethylformamide (DMF), N,N-dimethylacetamide (DMA), tetrahydrofuran (THF), 1,4-dioxane (Dioxane), dimethyl sulfoxide (DMSO) and any combination thereof, preferably acetonitrile.

In some embodiments, this step is carried out in the presence of a suitable base, which includes an organic base or an inorganic base, the organic base may be selected from the group consisting of N,N-diisopropylethylamine (DIPEA), triethylamine (TEA), potassium tert-butoxide (t-BuOK) and pyridine (Py), and the inorganic base may be selected from the group consisting of potassium phosphate (K₃PO₄), sodium hydride (NaH), potassium carbonate (K₂CO₃), sodium carbonate (Na₂CO₃), sodium bicarbonate (NaHCO₃), cesium carbonate (Cs₂CO₃) and NaOH, preferably Na₂CO₃ or NaHCO₃;

### Step 7

The compound of Formula (IV) is obtained through a condensation reaction of the compound of Formula (IV)-IM16 and the compound of Formula (IV)-SM4;

In some embodiments, this step is carried out in the presence of a suitable condensation reagent, which may be selected from the group consisting of HATU, HBTU, EDCI, DCC and HOBT, preferably HBTU;

In some embodiments, this step is carried out at a suitable temperature, and the temperature is 20°C, 25°C, 40°C, 50°C, 60°C, 100°C, preferably 25°C;

In some embodiments, this step is carried out in a suitable organic solvent, which may be selected from the group consisting of methanol, tetrahydrofuran, dichloromethane, N,N-dimethylformamide, N-methylpyrrolidone, dimethyl sulfoxide, n-heptane, n-hexane, ethyl acetate, preferably N,N-dimethylformamide.

In some embodiments, this step is carried out in the presence of a suitable base, the base includes an organic base or an inorganic base, the organic base may be selected from the group consisting of DIPEA, TEA, t-BuOK and Py, and the inorganic base may be selected from the group consisting of K₃PO₄, NaH, K₂CO₃, Na₂CO₃, Cs₂CO₃ and NaOH, preferably DIPEA.

The compound of Formula (V)-1 in the present invention may be synthesized and prepared through a similar route as that of Formula (III) using the starting material

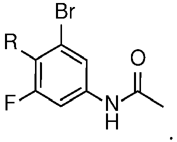

### Beneficial Effects of the present invention

The present invention provides the camptothecin compounds represented by Formula (I) to Formula (IV), and pharmaceutical compositions, preparation methods and applications thereof. This kind of compounds has good antitumor activity, has the potential to address drug resistance, and can be used for treating diseases related to abnormal cell proliferation, and the diseases include but are not limited to advanced solid tumors.

### Specific Models for Carrying Out the Invention

The present invention is further illustrated through the description of specific embodiments, but this is not a limitation to the present invention. Those skilled in the art can make various modifications or improvements according to the teaching of the present invention without departing from the basic idea and scope of the present invention.

Abbreviations in the present invention have the following meanings:

| Abbreviation of the reagents | Full name of the reagents | Abbreviation of the reagents | Full name of the reagents |
|---|---|---|---|
| NBS | N-Bromosuccinimide | HATU | N,N,N',N'-Tetramethyl- O-(7-azabenzotriazol-1-yl)urea hexafluorophosphate |
| DMF-DMA | N,N-Dimethylformamide dimethylacetal | DIPEA | Diisopropylethylamine |
| THF | Tetrahydrofuran | DMSO | Dimethyl sulfoxide |
| DMF | N,N-Dimethylformamide | Pd/C | Palladium on carbon |
| LCMS | Combination of liquid chromatography and mass spectrometry | Py | Pyridine |
| FBS | Fetal bovine serum | CCK8 | 2-(2-Methoxy-4-nitrophenyl)-3-(4-nitrophenyl)-5-(2,4-disulfobenzene)- 2H-tetrazole monosodium salt |

The structures of the compounds described in the following Examples were confirmed by nuclear magnetic resonance (¹H NMR) or mass spectrometry (MS).

The instrument for nuclear magnetic resonance (¹H NMR) was Bruker 400 MHz nuclear magnetic resonance instrument; hexadeuteriodimethylsulfoxide (DMSO-d₆); the internal standard substance was tetramethylsilane (TMS).

Abbreviations in nuclear magnetic resonance (NMR) spectra in Examples were shown below.
s: singlet, d: doublet, t: triplet, q: quartet, m: multiplet, br: broad, J: coupling constant, Hz: Hertz, DMSO-d₆: deuterated dimethylsulfoxide. The δ values were expressed in ppm.

The instrument for mass spectrometer (MS) was Agilent (ESI) mass spectrometer, Agilent 6120B.

Example 1: (R)-N-((1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl)-2 -hydroxypent-3-yneamide and (S)-N-((1S,9S)-9-ethyl-5-fluoro-9-hydroxy-4-methyl- 10,13-dioxo-2,3,9,10,13,15-hexahydro-1H,12H-benzo[de]pyrano[3',4':6,7]indolizino[1,2-b]quinolin-1-yl)-2-hydroxypent-3-yneamide

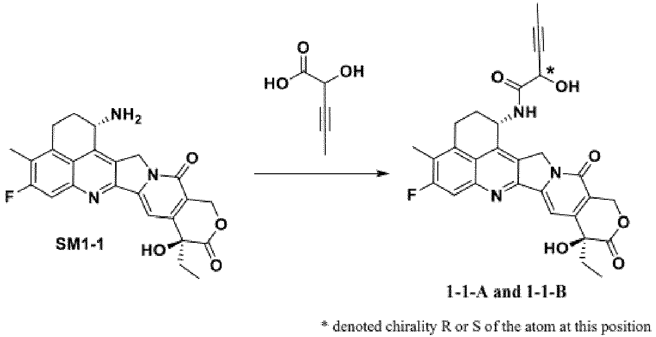

The compound 2-hydroxypent-3-ynoic acid (4.29 mg, 37.63 µmol) was dissolved in DMF (1 mL), added with HATU (21.46 mg, 56.44 µmol), SM1-1 (10.00 mg, 22.94 µmol) and DIPEA (7.29 mg, 56.44 µmol), and reacted at 25°C for 2 hours. The reaction solution was concentrated under reduced pressure, and the concentrate was directly purified by preparative high performance liquid chromatography (conditions as follows) to obtain the title compounds **1-1-A** (3.24 mg) and **1-1-B** (3.98 mg).
Chromatographic column: SunFire Prep C18 OBD 19 mm×150 mm×5.0 µm
Mobile phase A: acetonitrile; mobile phase B: water (0.05% formic acid)

| Time [min] | Mobile phase A [%] | Mobile phase B [%] | Flow rate [mL/min] |
|---|---|---|---|
| 0 | 30 | 70 | 28 |
| 18 | 90 | 10 | 28 |

Retention time: **1-1-A:** 10.8 min; **1-1-B:** 11.1 min.

The structural characterization data of **1-1-A** were as follows:
¹H NMR (400 MHz, DMSO-d₆) δ 8.58 (d, *J* = 8.4 Hz, 1H), 7.78 (d, *J=* 11.2 Hz,1H), 7.31 (s, 1H), 6.54 (s, 1H), 6.17 (d, *J* = 6.0 Hz, 1H), 5.58-5.47 (m, 1H), 5.42 (s, 2H), 5.23 (s, 2H), 4.73-4.64 (m, 1H), 3.25-3.06 (m, 2H), 2.39 (s, 3H), 2.27-2.05 (m, 2H), 1.96-1.77 (m, 5H), 0.87 (t, *J* = 7.2Hz, 3H).
ESI-MS (m/z): 532.2 [M+H]⁺.

The structural characterization data of **1-1-B** were as follows:
¹H NMR (400 MHz, DMSO-d₆) δ 8.62 (d, *J* = 8.8 Hz, 1H), 7.78 (d, *J* = 10.8 Hz, 1H), 7.31 (s, 1H), 6.54 (s, 1H), 6.19 (d, *J* = 6.0 Hz, 1H), 5.58-5.47 (m, 1H), 5.42 (s, 2H), 5.21 (d, *J* = 4.8 Hz, 2H), 4.73-4.65 (m, 1H), 3.27- 3.06 (m, 2H), 2.39 (s, 3H), 2.27-2.05 (m, 2H), 1.93-1.80 (m, 2H), 1.80 (d, *J* = 2.0 Hz, 3H), 0.87 (t, *J* = 7.2Hz, 3H).
ESI-MS (m/z): 532.2 [M+H]⁺.

Example 2: (S)-N-((1S,9S)-9-ethyl-5-fluoro-9-hydroxy-4-methyl-10,13-dioxo-2,3,9,10,13, 15-hexahydro-1H,12H-benzo[de]pyrano[3',4]:6,7]indolizino[1,2-b]quinolin-1-yl)-2-hydroxy-3-enamide and (R)-N-((1S,9S)-9-ethyl-5-fluoro-9-hydroxy-4-methyl-10,13-dioxo-2,3,9,10,13, 15-hexahydro-1H,12H-benzo[de]pyrano[3',4]:6,7]indolizino[1,2-b]quinolin-1-yl)-2-hydroxy-3-enamide

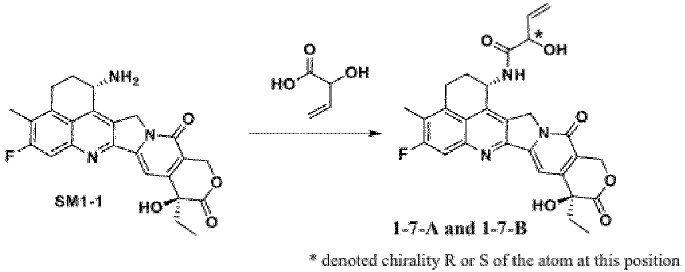

The compound ethylene glycolic acid (9.61 mg, 94.07 µmol) was dissolved in DMF (2 mL), added with HATU (44.70 mg, 117.58 µmol), compound **SM1-1** (25.00 mg, 0.047 mmol) and DIPEA (24.30 mg, 188.13 µmol), and reacted at 25°C for 2 hours. The reaction solution was concentrated under reduced pressure, and the concentrate was directly purified by preparative high performance liquid chromatography to obtain the title compounds **1-7-A** (4.00 mg) and **1-7-B** (1.38 mg).
Chromatographic column: SunFire Prep C18 OBD 19 mm×150 mm×5.0 µm
Mobile phase A: acetonitrile; mobile phase B: water (0.05% formic acid)

| Time [min] | Mobile phase A [%] | Mobile phase B [%] | Flow rate [mL/min] |
|---|---|---|---|
| 0 | 30 | 70 | 28 |
| 2 | 30 | 70 | 28 |
| 18 | 90 | 10 | 28 |

Retention time: **1-7-A:** 8.7 min; **1-7-B:** 9.1 min.

The structural characterization data of **1-7-A** were as follows:
¹H NMR (400 MHz, DMSO-d₆) δ 8.51 (s, *J* = 8.8 Hz, 1H), 7.76 (s, *J* = 10.8 Hz, 1H), 7.29 (s, 1H), 6.52 (s, 1H), 6.15 -6.04 (m, 1H), 5.58-5.49 (m, 1H), 5.42 (s, 2H), 5.39 (s, 1H), 5.24-5.01 (m, 3H), 4.54 (s, *J* = 4.9 Hz, 1H), 3.24-3.05 (m, 2H), 2.37 (s, 3H), 2.16 (s, 2H), 1.91-1.79 (m, 2H), 0.87 (t, *J* = 7.2 Hz, 3H).
ESI-MS (m/z): 520.1 [M+H]⁺.

The structural characterization data of **1-7-B** were as follows:
¹H NMR (400 MHz, DMSO-d₆) δ 8.48 (s, 1H), 7.78 (s, 1H), 7.30 (s, 1H), 6.12-5.92 (m, 1H), 5.54-5.47 (m, 1H), 5.42 (s, 2H), 5.37 (dt, *J* = 1.7 Hz, 1H), 5.18 (s, 2H), 5.16-5.14 (m, 1H), 4.5-4.52 (m, 1H), 3.22-3.08 (m, 2H), 2.38 (s, 3H), 2.25-2.16 (m, 1H), 2.16-2.06 (m, 1H), 1.93-1.79 (m, 2H), 0.87 (t, *J* = 7.2 Hz, 3H).
ESI-MS (m/z): 520.1 [M+H]⁺.

Example 3: N-((1S,9S)-5-chloro-9-ethyl-9-hydroxy-4-methyl-10,13-dioxo-2,3,9,10,13,15-hexahydro-1H,12H-benzo[de]pyrano[3',4':6,7]indolizino[1,2-b]quinolin-1-yl)-2-hydroxyacetamide and N-((1R,9S)-5-chloro-9-ethyl-9-hydroxy-4-methyl-10,13-dioxo-2,3,9,10,13,15- hexahydro-1H,12H-benzo[de]pyrano[3',4':6,7]indolizino[1,2-b]quinolin-1-yl)-2-hydroxyacetamide

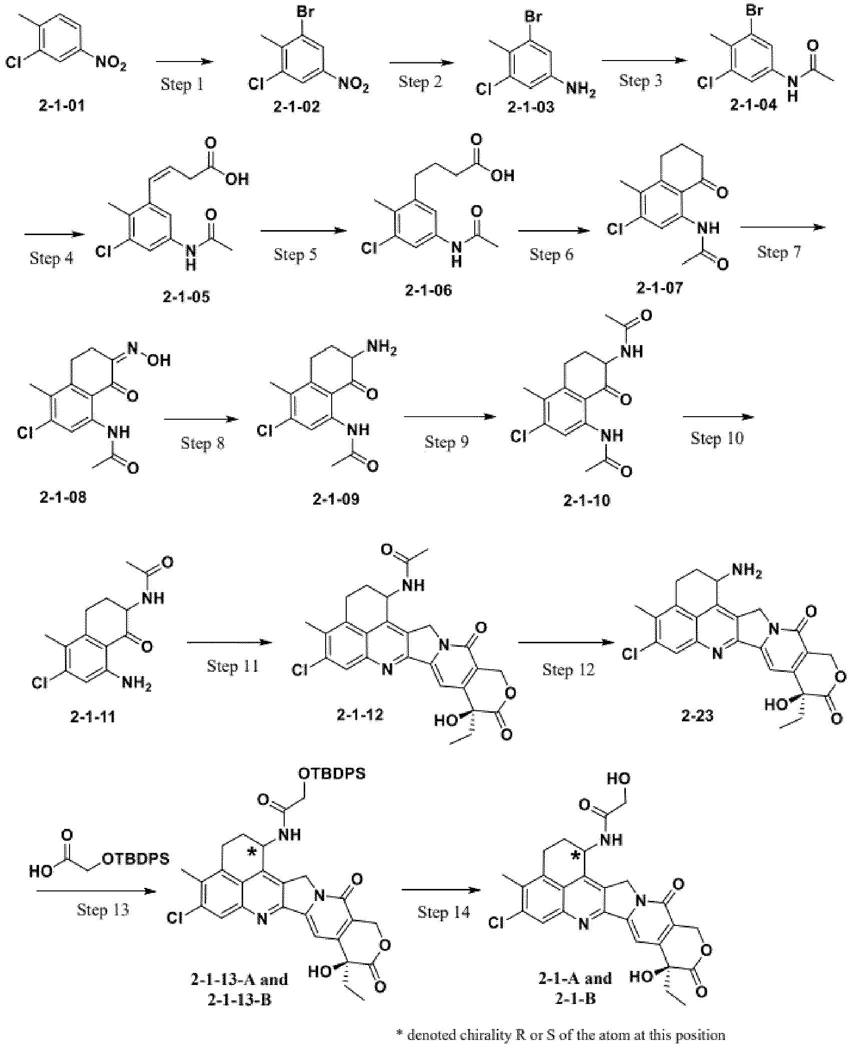

### Step 1: Synthesis of 1-chloro-3-bromo-2-methyl-5-nitrobenzene

Compound **2-1-01** (5.00 g, 29.14 mmol) was dissolved in n-heptane (25 mL) at 25°C, added with concentrated sulfuric acid (25 mL), heated to 50°C, added with NBS in batches at 50°C (6.22 g, 34.97 mmol), reacted at 50°C for 2 hours, the reaction was monitored by thin layer chromatography (ethyl acetate:petroleum ether=1:10), the reaction solution was cooled to room temperature and added into ice water in dropwise manner, extracted with toluene, the organic phases were combined, washed with sodium sulfite solution, water, and saturated brine, dried over anhydrous sodium sulfate, concentrated under reduced pressure, the crude product was purified by preparative high performance liquid chromatography, and the fractions was lyophilized to obtain 4.88 g of the title compound.
Chromatographic column: C18 ODS 45 mm×450 mm×8.0 µm
Mobile phase A: acetonitrile; Mobile phase B: water (0.05% formic acid)

| Time [min] | Mobile phase A [%] | Mobile phase B [%] | Flow rate [mL/min] |
|---|---|---|---|
| 0 | 60 | 40 | 60 |
| 10 | 60 | 40 | 60 |
| 40 | 100 | 0 | 60 |

### Step 2: Synthesis of 3-chloro-5-bromo-4-methylaniline

At 25°C, compound **2-1-02** (4.88 g, 19.48 mmol) was dissolved in ethyl acetate (100 mL), added with platinum on carbon (2.00 g, 19.48 mmol, content 5%), subjected to hydrogen replacement, and reacted under the protection of hydrogen balloon at 60°C for 4 hours, and the reaction was monitored by LCMS. The reaction solution was filtered, and the filtrate was concentrated to obtain 3.68 g of the crude title compound, which was directly used in the next reaction without further purification.

### Step 3: Synthesis of N-(3-chloro-5-bromo-4-methylphenyl)acetamide

At 20°C, compound **2-1-03** (3.63 g, 14.82 mmol) was dissolved in ethyl acetate (70 mL), added with triethylamine (4.50 g, 44.45 mmol) and acetic anhydride (2.27 g, 22.23 mmol), reacted at 20°C for 20 hours, and the reaction was monitored by LCMS. The reaction solution was added with water, extracted with ethyl acetate, the organic phases were combined, dried over anhydrous sodium sulfate, and concentrated under reduced pressure to obtain a crude product, which was slurried with a mixed solvent of ethyl acetate:petroleum ether=1:5 to obtain 2.86 g of the title compound.

### Step 4: Synthesis of (Z)-4-(5-acetamido-3-chloro-2-methylphenyl)but-3-enoic acid

At 20°C, compound **2-1-04** (1.80 g, 6.86 mmol) was dissolved in THF (20 mL) and water (5 mL), added with vinylacetic acid (708.31 mg, 8.23 mmol), DIPEA (1.95 g, 15.08 mmol), and tris(o-methylphenyl)phosphorus (62.60 mg, 0.20 mmol), the reaction system was subjected to nitrogen replacement, then heated to 70°C and reacted for 5 hours, and the reaction was monitored by LCMS. The reaction solution was added with 1N sodium hydroxide solution to adjust pH to 8, and ethyl acetate was added for extraction. The aqueous phase was adjusted to pH=3 with 1N hydrochloric acid, extracted with ethyl acetate, and the organic phases were combined, dried over anhydrous sodium sulfate, and concentrated under reduced pressure to obtain 0.82 g of the title compound, which was directly used in the next reaction.

### Step 5: Synthesis of 4-(5-acetamido-3-chloro-2-methylphenyl)butanoic acid

At 20°C, compound **2-1-05** (2.60 g, 9.71 mmol) was dissolved in THF (50 mL), added with Pd/C (0.52 g, content 10%), the system was subjected to hydrogen replacement, then reacted under the protection of hydrogen balloon at 40°C for 2 hours, and the reaction was monitored by LCMS. The reaction solution was filtered, and the filtrate was concentrated under reduced pressure to obtain 2.43 g of the title compound, which was directly used in the next reaction without further purification.

### Step 6: Synthesis of N-(3-chloro-4-methyl-8-oxo-5,6,7,8-tetrahydronaphthalen- 1-yl)acetamide

Compound **2-1-06** (2.43 g, 9.01 mmol) was dissolved in trifluoroacetic acid (10 mL), cooled to 5°C, added dropwise with trifluoroacetic anhydride (3.78 g, 18.02 mmol, 2.50 mL), reacted at 5°C for 4 hours, and the reaction was monitored by LCMS. The reaction solution was added to water, adjusted with 10N sodium hydroxide to pH = 9, extracted with ethyl acetate, the organic phases were combined, dried over anhydrous sodium sulfate, concentrated under reduced pressure, and purified by flash silica gel column (ethyl acetate: petroleum ether=0-20%) to obtain 1.53 g of the title compound.

### Step 7: Synthesis of (Z)-N-(3-chloro-7-(hydroxyimino)-4-methyl-8-oxo-5,6,7,8-tetrahydronaphthalen-1 -yl)acetamide

At 5°C, potassium tert-butoxide (1.50 g, 13.37 mmol) was dissolved in THF (16 mL) and tert-butanol (4 mL), added dropwise with compound **2-1-07** (1.53 g, 6.08 mmol) in THF solution (16 mL), then added dropwise with amyl nitrite (1.14 g, 9.73 mmol) after 10 minutes, and reacted at 5°C for 1 hour, the reaction was monitored by LCMS. The reaction solution was adjusted to pH=5 with 1N hydrochloric acid, extracted with ethyl acetate, the combined organic phase was dried over anhydrous sodium sulfate, concentrated under reduced pressure, and the concentrate was slurried with methyl tert-butyl ether to obtain 1.20 g of the title compound.

### Step 8: N-(7-Amino-3-chloro-4-methyl-8-oxo-5,6,7,8-tetrahydronaphthalen-1-yl)acetamide

At 20°C, compound **2-1-08** (0.50 g, 1.78 mmol) was dissolved in methanol (8 mL) and 2N hydrochloric acid (8 mL), added with Pd/C (0.15 g, content 10%), the system was subjected to hydrogen replacement, and reacted at 5°C for 2 hours under the protection of a hydrogen balloon, and the reaction was monitored by LCMS. The reaction solution was filtered, and the filtrate was concentrated under reduced pressure to obtain 0.52 g of the hydrochloride salt of the title compound, which was directly used in the next reaction without further purification.

### Step 9: Synthesis of N,N'-(3-chloro-4-methyl-8-oxo-5,6,7,8-tetrahydronaphthalen- 1,7-diyl)diacetamide

At 20°C, compound **2-1-09** (0.52 g, 1.70 mmol) was dissolved in pyridine (5 mL), added with acetic anhydride (2 mL), reacted at 20°C for 2 hours, and the reaction was monitored by LCMS. The reaction solution was added to water, extracted with ethyl acetate, the organic phases were washed with water, combined, dried over anhydrous sodium sulfate, concentrated under reduced pressure, and the concentrate was purified by flash silica gel column (ethyl acetate:petroleum ether=0-30%) to obtain 0.22 g of the title compound.

### Step 10: Synthesis of N-(8-amino-6-chloro-5-methyl-1-oxo-1,2,3,4-tetrahydronaphthalen- 2-yl)acetamide

At 20°C, compound **2-1-10** (0.45 g, 1.46 mmol) was dissolved in methanol (16 mL), added with 2N hydrochloric acid (16 mL), heated to 60°C and reacted for 2 hours, and the reaction was monitored by LCMS. The reaction solution was cooled, added with saturated sodium bicarbonate solution to adjust pH = 8, extracted with ethyl acetate, the organic phases were combined, dried over anhydrous sodium sulfate, and concentrated under reduced pressure to obtain 0.23 g of the title compound, which was used directly in the next step without further purification.

### Step 11: Synthesis of N-((9S)-5-chloro-9-ethyl-9-hydroxy-4-methyl-10,13-dioxo-2,3,9,10, 13,15-hexahydro-1H,12H-benzopyrano[3',4':6,7]indolizino[1,2-b]quinolin-1-yl)acetamide

Compound **2-1-11** (0.23 g, 0.78 mmol) was dissolved in toluene (10 mL), added with (S)-4-ethyl-4-hydroxy-7,8-dihydro-1H-pyrano[3,4-f]indolizine-3,6,10(4H)-trione (0.23 g, 0.87 mmol), p-toluenesulfonic acid (26.73 mg, 0.16 mmol), heated to 140°C and reacted for 5 hours, and the reaction was monitored by LCMS. The reaction solution was concentrated, and the crude product was purified by flash silica gel column (methanol:dichloromethane=0-10%) to obtain 0.15 g of the title compound.

### Step 12: Synthesis of (9S)-1-amino-5-chloro-9-ethyl-9-hydroxy-4-methyl-1,2,3,9,12,15-hexahydro-10H,13H-benzene pyrano[3',4':6,7]indolizino[1,2-b]quinoline-10,13-dione

Compound **2-1-12** (40.00 mg, 0.08 mmol) was added into concentrated hydrochloric acid (1 mL), heated to 100°C and reacted for 5 hours, and the reaction was monitored by LCMS. The reaction solution was filtered, the filtrate was purified by preparative high performance liquid chromatography, and the fractions were lyophilized to obtain 12.00 mg of the trifluoroacetic acid salt of the title compound **2-23.**
Chromatographic column: SunFire Prep C18 OBD 19 mm×150 mm×5.0 µm
Mobile phase A: acetonitrile; mobile phase B: water (0.05% trifluoroacetic acid)

| Time [min] | Mobile phase A [%] | Mobile phase B [%] | Flow rate [mL/min] |
|---|---|---|---|
| 0 | 5 | 95 | 28 |
| 2 | 5 | 95 | 28 |
| 18 | 50 | 50 | 28 |

The structural characterization data were as follows:
ESI-MS (m/z):452.1[M+H]⁺.

### Step 13: Synthesis of 2-((tert-butyldiphenylsilyl)oxy)-N-((1S,9S)-5-chloro-9-ethyl-9-hydroxy-4-methyl-10,13-dioxo-2,3,9,10,13,15-hexahydro-1H,12H-benzo[de]pyrano[3',4':6,7]indolizino[1,2-b]quinolin-1-yl)acetamide and 2-((tert-butyldiphenylsilyl)oxy)-N-((1R,9S)-5- chloro-9-ethyl-9-hydroxy-4-methyl-10,13-dioxo-2,3,9,10,13,15-hexahydro-1H,12H-benzo[de]pyrano[3',4':6,7]indolizino[1,2-b]quinolin-1-yl)acetamide

At 25°C, the trifluoroacetic acid salt of compound **2-23** (40.00 mg, 81.91 µmol) was dissolved in N,N-dimethylformamide (1 mL), added sequentially with 2-((tert-butyldiphenylsilyl)oxy)acetic acid (30.91 mg, 98.29 µmol), HATU (62.25 mg, 163.81 µmol) and N,N-diisopropylethylamine (42.34 mg, 327.63 µmol), reacted at 25°C for 0.5 hours, the reaction was monitored by LCMS. When the reaction was completed, the reaction solution was added with water, extracted with dichloromethane/methanol (v/v=10/1), the organic phases were combined, dried over anhydrous sodium sulfate, and concentrated under reduced pressure, and the crude product was purified and separated by preparative thin-layer chromatography (dichloromethane:methanol=20:1) to obtain two isomers, according to the Rf values, the two isomers were named **2-1-13-A** (15.00 mg, Rf value was 0.3) and **2-1-13-B** (12.00 mg, Rf value was 0.35).

### Step 14: Synthesis of N-((1S,9S)-5-chloro-9-ethyl-9-hydroxy-4-methyl-10,13-dioxo-2,3,9,10,13,15-hexahydro-1H,12H-benzo[de]pyrano[3',4':6,7]indolizino[1,2-b]quinolin-1-yl)-2-hydroxyacetamide and N-((1R,9S)-5-chloro-9-ethyl-9-hydroxy-4-methyl-10,13-dioxo-2,3,9,10, 13,15-hexahydro-1H,12H-benzo[de]pyrano[3',4':6,7]indolizino[1,2-b]quinolin-1-yl)-2-hydroxy acetamide

At 25°C, **2-1-13-A** (15.00 mg) and **2-1-13-B** (12.00 mg) were dissolved in tetrahydrofuran (1 mL) in two reaction flasks, respectively, added dropwise with a mixed solution of tetrabutyl ammonium fluoride (1M in tetrahydrofuran)/glacial acetic acid (v/v=13/1) (50 uL), reacted at 25°C for 0.5 hours, and the reaction was monitored by LCMS. When the reaction was completed, the reaction solution was purified by preparative high performance liquid chromatography, and the fractions were lyophilized to obtain the title compounds **2-1-A** (6.94 mg) and **2-1-B** (4.00 mg).
Chromatographic column: SunFire Prep C18 OBD 19 mm×150 mm×5.0 µm
Mobile phase A: acetonitrile; mobile phase B: water (0.05% formic acid)

| Time [min] | Mobile phase A [%] | Mobile phase B [%] | Flow rate [mL/min] |
|---|---|---|---|
| 0 | 20 | 80 | 28 |
| 3 | 20 | 80 | 28 |
| 18 | 90 | 10 | 28 |

The structural characterization data of **2-1-A** were as follows:
¹H NMR (400 MHz, DMSO-d₆) δ 8.43 (d, *J=* 8.8 Hz, 1H), 8.16 (s, 1H), 7.31 (s, 1H), 6.55 (s, 1H), 5.65-5.36 (m, 4H), 5.21 (q, *J* = 19.0 Hz, 2H), 3.95 (d, *J* = 5.7 Hz, 2H), 3.26-3.11 (m, 2H), 2.53 (s, 3H), 2.30-2.08 (m, 2H), 1.94-1.79 (m, 2H), 0.87 (t, *J* = 7.3 Hz, 3H).
ESI-MS (m/z): 510.1[M+H]⁺.

The structural characterization data of **2-1-B** were as follows:
¹H NMR (400 MHz, DMSO-d₆) δ 8.45 (d, *J=* 8.9 Hz, 1H), 8.15 (s, 1H), 7.31 (s, 1H), 6.54 (s, 1H), 5.64-5.35 (m, 4H), 5.19 (q, *J* = 19.0 Hz, 2H), 3.97 (d, *J* = 5.2 Hz, 2H), 3.27-3.10 (m, 2H), 2.51 (s, 3H), 2.27-2.10 (m, 2H), 1.93-1.80 (m, 2H), 0.88 (t, *J* = 7.3 Hz, 3H).
ESI-MS (m/z):510.1[M+H]⁺.

### Example 4: (2S)-N-((1S,9S)-5-chloro-9-ethyl-9-hydroxy-4-methyl-10,13-dioxo-2,3,9,10, 13,15-hexahydro-1H,12H-benzo[de]pyrano[3',4':6,7]indolizino[1,2-b]quinolin-1-yl)-2-hydroxypropylamine and (2S)-N-((1R,9S)-5-chloro-9-ethyl-9-hydroxy-4-methyl-10,13-dioxo-2,3,9,10,13, 15-hexahydro-1H,12H-benzo[de]pyrano[3',4':6,7]indolizino[1,2-b]quinolin-1-yl)-2-hydroxypropylamine

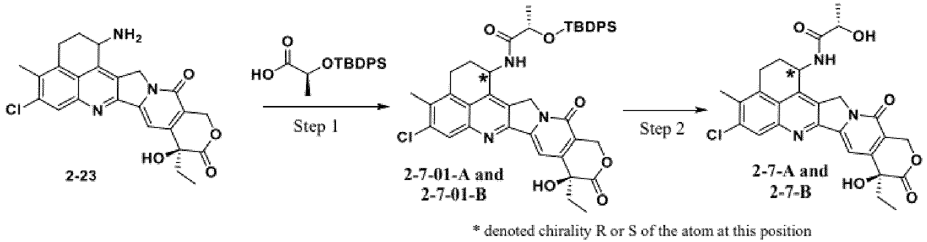

### Step 1: Synthesis of (2S)-2-((tert-butyldiphenylsilyl)oxy)-N-((1S,9S)-5-chloro-9-ethyl-9-hydroxy-4-methyl-10,13-dioxo-2,3,9,10,13,15-hexahydro- 1H, 12H-benzo[de]pyrano[3',4':6,7]indolizino[1,2-b]quinolin-1-yl)propylamine and (2S)-2-((tert-butyldiphenylsilyl)oxy)-N-((1R,9S)- 5-chloro-9-ethyl-9-hydroxy-4-methyl-10,13-dioxo-2,3,9,10,13,15-hexahydro-1H,12H-benzo[de]pyrano[3',4':6,7]indolizino[1,2-b]quinolin-1-yl)propylamine

At 25°C, the hydrochloride salt of **2-23** (30.00 mg, 61.43 µmol) was dissolved in N,N-dimethylformamide (1 mL), added sequentially with (S)-2-((tert-butyldiphenylsilyl) oxy)propionic acid (24.21 mg, 73.72 µmol), HATU (35.01 mg, 92.14 µmol) and N,N-diisopropylethylamine (23.82 mg, 184.29 µmol), reacted at 25°C for 1 hour, and the reaction was monitored by LCMS. When the reaction was completed, the reaction solution was added with water, extracted with dichloromethane/methanol (v/v=10/1), the organic phases were combined, dried over anhydrous sodium sulfate, and concentrated under reduced pressure, and the crude product was purified and separated by preparative thin-layer chromatography (dichloromethane:methanol=15:1) to obtain two isomers, and according to the Rf values, the two isomers were named **2-7-01-A** (6.00 mg, Rf value was 0.35) and **2-7-01-B** (6.00 mg, Rf value was 0.40).

### Step 2: Synthesis of (2S)-N-((1S,9S)-5-chloro-9-ethyl-9-hydroxy-4-methyl-10,13-dioxo-2,3,9,10,13,15-hexahydro-1H,12H-benzo[de]pyrano[3',4':6,7]indolizino[1,2-b]quinolin-1-yl)-2-hydroxypropylamine and (2S)-N-((1R,9S)-5-chloro-9-ethyl-9-hydroxy-4-methyl-10,13-dioxo-2,3,9,10,13,15-hexahydro-1H,12H-benzo[de]pyrano[3',4':6,7]indolizino[1,2-b]quinolin-1-yl)-2-hydroxypropylamine

At 25°C, **2-7-01-A** (6.00 mg, 7.87 µmol) and **2-7-01-B** (6.00 mg, 7.87 µmol) were dissolved in anhydrous THF (1 mL) in two reaction flasks, respectively, added dropwise with a mixed solution of tetrabutylammonium fluoride (1M in tetrahydrofuran)/glacial acetic acid (v/v=13/1) (50 µL), reacted at 25°C for 0.5 hours, and the reaction was monitored with LCMS. When the reaction was completed, the reaction solution was purified by preparative high performance liquid chromatography, and the fractions were lyophilized to obtain the title compounds 2-7-A (2.50 mg) and **2-7-B** (3.00 mg).
Chromatographic column: SunFire Prep C18 OBD 19 mm×150 mm×5.0 µm
Mobile phase A: acetonitrile; mobile phase B: water (0.05% formic acid)

| Time [min] | Mobile phase A [%] | Mobile phase B [%] | Flow rate [mL/min] |
|---|---|---|---|
| 0 | 15 | 85 | 28 |
| 16 | 90 | 10 | 28 |

The structural characterization data of **2-7-A** were as follows:
¹H NMR (400 MHz, DMSO-d₆) δ 8.38 (d, *J* = 8.8 Hz, 1H), 8.15 (s, 1H), 7.31 (s, 1H), 6.55 (s, 1H), 5.56 - 5.47 (m, 2H), 5.42 (s, 2H), 5.26 - 5.11 (m, 2H), 4.17 - 4.06 (m, 1H), 3.27 - 3.10 (m, 2H), 2.52 (s, 3H), 2.27 - 2.08 (m, 2H), 1.86 (tt, *J* = 14.1, 7.3 Hz, 2H), 1.30 (d, *J* = 6.7 Hz, 3H), 0.87 (t, *J* = 7.3 Hz, 3H).
ESI-MS (m/z): 524.2 [M+H]⁺.

The structural characterization data of **2-7-B** were as follows:
¹H NMR (400 MHz, DMSO-d₆) δ 8.48 (d, *J* = 9.1 Hz, 1H), 8.12 (s, 1H), 7.30 (s, 1H), 6.54 (s, 1H), 5.67 (d, *J* = 4.8 Hz, 1H), 5.55 (dd, *J* = 14.6, 7.3 Hz, 1H), 5.43 (s, 2H), 5.24 (d, *J* = 19.0 Hz, 1H), 5.03 (d, *J* = 19.0 Hz, 1H), 4.21-4.08 (m, 1H), 3.28 -3.08 (m, 2H), 2.51 (s, 3H), 2.16 (d, *J* = 6.2 Hz, 2H), 1.94-1.82 (m, 2H), 1.42 (d, *J* = 6.8 Hz, 3H), 0.88 (t, *J* = 7.3 Hz, 3H).
ESI-MS (m/z): 524.2 [M+H]⁺.

### Example 5: (2S)-N-((1S,9S)-5-chloro-9-ethyl-9-hydroxy-4-methyl-10,13-dioxo-2,3,9,10, 13,15-hexahydro-1H,12H-benzo[de]pyrano[3',4':6,7]indolizino[1,2-b]quinolin-1-yl)-2-cyclopropyl-2-hydroxyacetamide and (2S)-N-((1R,9S)-5-chloro-9-ethyl-9-hydroxy-4-methyl-10,13- dioxo-2,3,9,10,13,15-hexahydro-1H,12H-benzo[de]pyrano[3',4':6,7]indolizino[1,2-b]quinolin-1-yl)-2-cyclopropyl-2-hydroxyacetamide and (2R)-N-((1S,9S)-5-chloro-9-ethyl-9-hydroxy-4- methyl-10,13-dioxo-2,3,9,10,13,15-hexahydro-1H,12H-benzo[de]pyrano[3',4':6,7]indolizino[1,2-b]quinolin-1-yl)-2-cyclopropyl-2-hydroxyacetamide and (2R)-N-((1R,9S)-5-chloro-9-ethyl-9- hydroxy-4-methyl-10,13-dioxo-2,3,9,10,13,15-hexahydro-1H,12H-benzo[de]pyrano[3',4':6,7]indolizino[1,2-b]quinolin-1-yl)-2-cyclopropyl-2-hydroxy acetamide

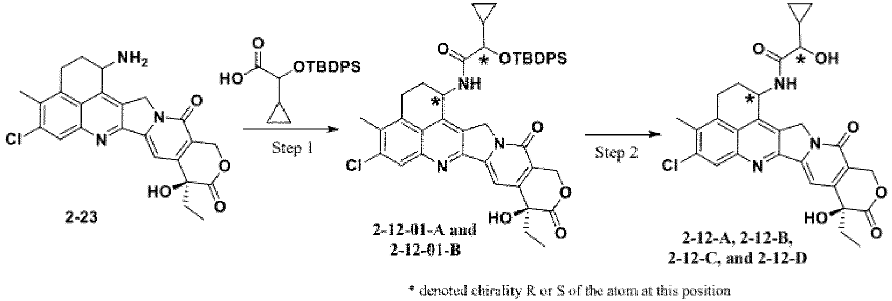

### Step 1: Synthesis of (2S)-2-((tert-butyldiphenylsilyl)oxy)-N-((1S,9S)-5-chloro-9-ethyl-9-hydroxy-4-methyl-10,13-dioxo-2,3,9,10,13,15-hexahydro-1H,12H-benzo[de]pyrano[3',4':6,7]indolizino[1,2-b]quinolin-1-yl)-2-cyclopropylacetamide and (2S)-2-((tert-butyldiphenylsilyl)oxy)- N-((1R,9S)-5-chloro-9-ethyl-9-hydroxy-4-methyl-10,13-dioxo-2,3,9,10,13,15-hexahydro-1H,12H-benzo[de]pyrano[3',4':6,7]indolizino[1,2-b]quinolin-1-yl)-2-cyclopropylacetamide and (2R)-2-((tert-butyldiphenylsilyl)oxy)-N-((1S,9S)-5-chloro-9-ethyl-9-hydroxy-4-methyl-10,13-dioxo-2,3,9,10,13,15-hexahydro- 1H, 12H-benzo[de]pyrano[3',4':6,7]indolizino[1,2-b]quinolin-1-yl)-2-cyclopropylacetamide and (2R)-2-((tert-butyldiphenylsilyl)oxy)-N-((1R,9S)-5-chloro-9- ethyl-9-hydroxy-4-methyl-10,13-dioxo-2,3,9,10,13,15-hexahydro-1H,12H-benzo[de]pyrano[3',4':6,7]indolizino[1,2-b]quinolin-1-yl)-2-cyclopropylacetamide

At 25°C, the hydrochloride salt of **2-23** (30.00 mg, 61.43 µmol) was dissolved in N,N-dimethylformamide (1 mL), added sequentially with 2-((tert-butyldiphenylsilyl)oxy)- 2-cyclopropylacetic acid (26.13 mg, 73.72 µmol), HATU (35.01 mg, 92.14 µmol) and N,N-diisopropylethylamine (23.82 mg, 184.29 µmol), reacted at 25°C for 1 hour, the reaction was monitored by LCMS. When the reaction was completed, the reaction solution was added with water, extracted with dichloromethane/methanol (v/v=10/1), the organic phases were combined, dried over anhydrous sodium sulfate, and concentrated under reduced pressure, and the crude product was purified and separated by preparative thin-layer chromatography (dichloromethane:methanol=15:1) to obtain two isomers, according to the Rf values, the two isomers were named **2-12-01-A** (8.00 mg, Rf value was 0.35) and **2-12-01-B** (10.00 mg, Rf value was 0.40).

### Step 2: Synthesis of (2S)-N-((1S,9S)-5-chloro-9-ethyl-9-hydroxy-4-methyl-10,13-dioxo-2,3,9,10,13,15-hexahydro-1H,12H-benzo[de]pyrano[3',4':6,7]indolizino[1,2-b]quinolin-1-yl)-2-cyclopropyl-2-hydroxyacetamide and (2S)-N-((1R,9S)-5-chloro-9-ethyl-9-hydroxy-4-methyl-10,13-dioxo-2,3,9,10,13,15-hexahydro-1H,12H-benzo[de]pyrano[3',4':6,7]indolizino[1,2-b]quinolin-1-yl)-2-cyclopropyl-2-hydroxyacetamide and (2R)-N-((1S,9S)-5-chloro-9-ethyl-9-hydroxy- 4-methyl-10,13-dioxo-2,3,9,10,13,15-hexahydro-1H,12H-benzo[de]pyrano[3',4':6,7]indolizino[1,2-b]quinolin-1-yl)-2-cyclopropyl-2-hydroxyacetamide and (2R)-N-((1R,9S)-5-chloro-9-ethyl-9- hydroxy-4-methyl-10,13-dioxo-2,3,9,10,13,15-hexahydro-1H,12H-benzo[de]pyrano[3',4':6,7]indolizino[1,2-b]quinolin-1-yl)-2-cyclopropyl-2-hydroxy acetamide

At 25°C, **2-12-01-A** (8.00 mg, 10.15 µmol) and **2-12-01-B** (10.00 mg, 12.68 µmol) were dissolved in anhydrous THF (1 mL) in two reaction flasks, respectively, added dropwise with a mixed solution of tetrabutylammonium fluoride (1M in tetrahydrofuran)/glacial acetic acid (v/v=13/1) (50 µL), reacted at 25°C for 0.5 hours, and the reaction was monitored by LCMS. When the reaction was completed, the reaction solution was purified by preparative high-performance liquid chromatography. The reaction using **2-12-01-A** as the raw material gave two isomeric products after the separation, and the corresponding fractions were lyophilized respectively to obtain compound **2-12-A** (0.77 mg), and **2-12-B** (1.03 mg); while the reaction using **2-12-01-B** as the raw material gave two isomeric products after the separation, and the corresponding fractions were lyophilized respectively to obtain compound **2-12-C** (2.50 mg), and **2-12-D** (1.00 mg). The purification conditions for **2-12-A/2-12-B** were as follows:
Chromatographic column: SunFire Prep C18 OBD 19 mm×150 mm×5.0 µm
Mobile phase A: acetonitrile; mobile phase B: water (0.05% formic acid)

| Time [min] | Mobile phase A [%] | Mobile phase B [%] | Flow rate [mL/min] |
|---|---|---|---|
| 0 | 20 | 80 | 28 |
| 2 | 20 | 80 | 28 |
| 18 | 80 | 20 | 28 |

Peak retention time: **2-12-A:** 10.0-11.0 min, **2-12-B:** 11.0-12.5 min

The purification conditions for **2-12-C/2-12-D** were as follows:
Chromatographic column: SunFire Prep C18 OBD 19 mm×150 mm×5.0 µm
Mobile phase A: acetonitrile; mobile phase B: water (0.05% formic acid)

| Time [min] | Mobile phase A [%] | Mobile phase B [%] | Flow rate [mL/min] |
|---|---|---|---|
| 0 | 20 | 80 | 28 |
| 2 | 20 | 80 | 28 |
| 18 | 80 | 20 | 28 |

Peak retention time: **2-12-C:** 10.6-11.4 min, **2-12-D:** 11.4-12.5 min

The structural characterization data of **2-12-A** were as follows:
¹H NMR (400 MHz, DMSO-d₆) δ 8.38 (d, *J=* 8.9 Hz, 1H), 8.15 (s, 1H), 7.30 (s, 1H), 6.54 (s, 1H), 5.63-5.53 (m, 1H), 5.51 (d, *J* = 5.1 Hz, 1H), 5.42 (s, 2H), 5.28 (d, *J* = 19.2 Hz, 1H), 5.16 (d, *J* = 19.1 Hz, 1H), 3.60 (t, *J* = 5.6 Hz, 1H), 3.28-3.11 (m, 2H), 2.52 (s, 3H), 2.22-2.10 (m, 2H), 1.86 (tt, *J* = 14.1, 7.3 Hz, 2H), 1.23 (d, *J* = 4.9 Hz, 1H), 0.87 (t, *J* = 7.2 Hz, 3H), 0.56-0.36 (m, 4H).
ESI-MS (m/z):550.2 [M+H]⁺.

The structural characterization data of **2-12-B** were as follows:
¹H NMR (400 MHz, DMSO-d₆) δ 8.36 (d, *J* = 8.7 Hz, 1H), 8.16 (s, 1H), 7.31 (s, 1H), 6.55 (s, 1H), 5.57-5.48 (m, 1H), 5.42 (s, 2H), 5.40 (d, *J* = 5.4 Hz, 1H), 5.26 (d, *J* = 19.3 Hz, 1H), 5.18 (d, *J* = 19.0 Hz, 1H), 3.65-3.60 (m, 1H), 3.26-3.12 (m, 2H), 2.52 (s, 3H), 2.26-2.09 (m, 2H), 1.86 (tt, *J* = 14.1, 7.2 Hz, 2H), 1.19-1.08 (m, 1H), 0.87 (t, *J* = 7.3 Hz, 3H), 0.51-0.27 (m, 4H).
ESI-MS (m/z): 550.2 [M+H]⁺.

The structural characterization data of **2-12-C** were as follows:
¹H NMR (400 MHz, DMSO-d₆) δ 8.42 (d, *J* = 9.0 Hz, 1H), 8.15 (s, 1H), 7.31 (s, 1H), 6.54 (s, 1H), 5.56 (dd, *J* = 14.8, 6.8 Hz, 1H), 5.52 (d, *J* = 5.2 Hz, 1H), 5.42 (s, 2H), 5.29 (d, *J* = 19.2 Hz, 1H), 5.16 (d, *J* = 19.1 Hz, 1H), 3.62-3.58 (m, 1H), 3.27 -3.08 (m, 2H), 2.51 (s, 3H), 2.27-2.08 (m, 2H), 1.87 (tt, *J* = 14.0, 7.2 Hz, 2H), 1.25 (dd, *J* = 13.2, 6.8 Hz, 1H), 0.87 (t, *J* = 7.3 Hz, 3H), 0.64-0.28 (m, 4H).
ESI-MS (m/z):550.1 [M+H]⁺.

The structural characterization data of **2-12-D** were as follows:
¹H NMR (400 MHz, DMSO-d₆) δ 8.38 (d, *J* = 8.7 Hz, 1H), 8.16 (s, 1H), 7.31 (s, 1H), 6.54 (s, 1H), 5.59-5.49 (m, 1H), 5.43 (d, *J =* 5.3 Hz, 1H), 5.43 (s, 2H), 5.26 (d, *J* = 19.1 Hz, 1H), 5.17 (d, *J* = 19.0 Hz, 1H), 3.64 (t, *J* = 5.8 Hz, 1H), 3.17 (dd, *J* = 15.6, 8.3 Hz, 2H), 2.27-2.07 (m, 2H), 1.94-1.79 (m, 2H), 1.19-1.06 (m, 1H), 0.87 (t, *J* = 7.3 Hz, 3H), 0.49-0.28 (m, 4H).
ESI-MS (m/z):550.1 [M+H]⁺.

### Example 6: N-((1S,9S)-5-chloro-9-ethyl-9-hydroxy-4-methyl-10,13-dioxo-2,3,9,10,13,15-hexahydro-1H,12H-benzo[de]pyrano[3',4':6,7]indolizino[1,2-b]quinolin-1-yl)-2-hydroxy-2-methylpropylamine and N-((1R,9S)-5-chloro-9-ethyl-9-hydroxy-4-methyl-10,13-dioxo-2,3,9,10,13, 15-hexahydro-1H,12H-benzo[de]pyrano[3',4':6,7]indolizino[1,2-b]quinolin-1-yl)-2-hydroxy-2-methylpropylamine

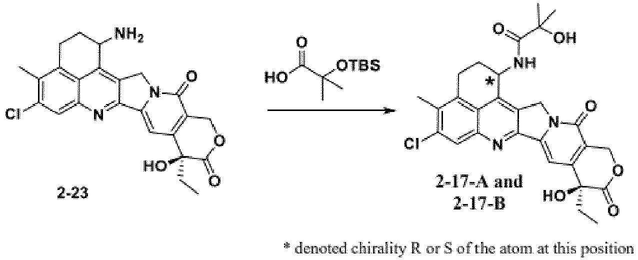

At 25°C, the hydrochloride salt of **2-23** (30.00 mg, 61.43 µmol) was dissolved in N,N-dimethylformamide (1 mL), added sequentially with 2-((tert-butyldimethylsilyl)oxy)-2-methylpropionic acid (16.10 mg, 73.72 µmol), HATU (35.01 mg, 92.14 µmol) and N,N-diisopropylethylamine (23.82 mg, 184.29 µmol), reacted at 25°C for 1 hour, the reaction was monitored by LCMS. When the reaction was completed, the reaction solution was concentrated, and the crude product was purified and separated by preparative high-performance liquid chromatography to obtain two isomers. The fractions were lyophilized respectively, and the two isomers were named **2-17-A** (2.65 mg) and **2-17-B** (2.69 mg).
Chromatographic column: SunFire Prep C18 OBD 19 mm×150 mm×5.0 µm
Mobile phase A: acetonitrile; mobile phase B: water (0.05% formic acid)

| Time [min] | Mobile phase A [%] | Mobile phase B [%] | Flow rate [mL/min] |
|---|---|---|---|
| 0 | 20 | 80 | 28 |
| 2 | 20 | 80 | 28 |
| 18 | 80 | 20 | 28 |

The peak retention time: **2-17-A:** 9.5-10.2 min, and **2-17-B:** 10.4-10.6 min.

The structural characterization data of **2-17-A** were as follows:
¹H NMR (400 MHz, DMSO-d₆) δ 8.36 (d, *J* = 9.1 Hz, 1H), 8.13 (s, 1H), 7.30 (s, 1H), 6.54 (s, 1H), 5.55-5.45 (m, 2H), 5.42 (s, 2H), 5.28 (d, *J* = 19.0 Hz, 1H), 5.05 (d, *J* = 19.0 Hz, 1H), 3.27-3.11 (m, 2H), 2.51 (s, 1H), 2.24-2.10 (m, 2H), 1.86 (tt, *J* = 14.0, 7.2 Hz, 2H), 1.46 (s, 3H), 1.35 (s, 3H), 0.87 (t, *J* = 7.3 Hz, 3H).
MS m/z (ESI): 538.2 [M+H]⁺.

The structural characterization data of **2-17-B** were as follows:
¹H NMR (400 MHz, DMSO-d₆) δ 8.40 (d, *J* = 9.2 Hz, 1H), 8.13 (s, 1H), 7.30 (s, 1H), 6.53 (s, 1H), 5.60-5.46 (m, 2H), 5.42 (s, 2H), 5.29 (d, *J* = 19.0 Hz, 1H), 5.02 (d, *J* = 19.0 Hz, 1H), 3.28-3.08 (m, 2H), 2.50 (s, 3H), 2.22-2.10 (m, 2H), 1.87 (tt, *J* = 14.2, 7.2 Hz, 2H), 1.47 (s, 3H), 1.35 (s, 3H), 0.88 (t, *J* = 7.3 Hz, 3H).
MS m/z (ESI): 538.2 [M+H]⁺.

### Example 7: N-((1S,9S)-5-chloro-9-ethyl-9-hydroxy-4-methyl-10,13-dioxo-2,3,9,10,13,15-hexahydro-1H,12H-benzo[de]pyrano[3',4':6,7]indolizino[1,2-b]quinolin-1-yl)-1-hydroxycyclopropane-1-carboxamide and N-((1R,9S)-5-chloro-9-ethyl-9-hydroxy-4-methyl-10,13-dioxo-2,3, 9,10,13,15-hexahydro-1H,12H-benzo[de]pyrano[3',4':6,7]indolizino[1,2-b]quinolin- 1-yl)-1-hydroxycyclopropane-1-carboxamide

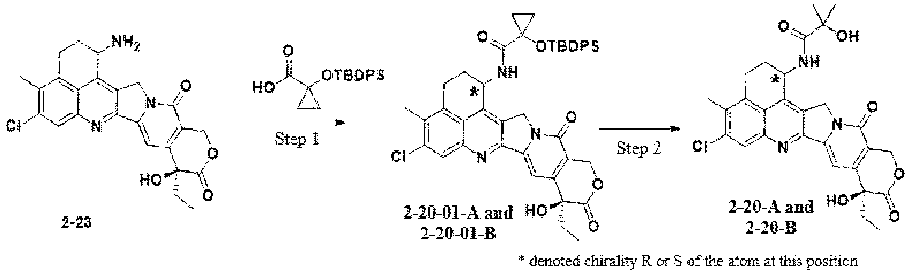

### Step 1: Synthesis of 1-((tert-butyldiphenylsilyl)oxy)-N-((1S,9S)-5-chloro-9-ethyl-9-hydroxy-4-methyl-10,13-dioxo-2,3,9,10,13,15-hexahydro- 1H, 12H-benzo[de]pyrano[3',4':6,7]indolizino[1,2-b]quinolin-1-yl)cyclopropane-1-carboxamide and 1-((tert-butyldiphenylsilyl)oxy)- N-((1R,9S)-5-chloro-9-ethyl-9-hydroxy-4-methyl-10,13-dioxo-2,3,9,10,13,15-hexahydro-1H,12H-benzo[de]pyrano[3',4':6,7]indolizino[1,2-b]quinolin-1-yl)cyclopropane-1 -carboxamide

At 25°C, the hydrochloride salt of **2-23** (30.00 mg, 61.43 µmol) was dissolved in N,N-dimethylformamide (1 mL), added sequentially with 1-((tert-butyldiphenylsilyl)oxy) cyclopropane-1-carboxylic acid (25.10 mg, 73.72 µmol), HATU (35.01 mg, 92.14 µmol) and N,N-diisopropylethylamine (23.82 mg, 184.29 µmol), reacted at 25°C for 1 hour, the reaction was monitored by LCMS. When the reaction was completed, the reaction solution was added with water, extracted with dichloromethane/methanol (v/v=10/1), the organic phases were combined, dried over anhydrous sodium sulfate, and concentrated under reduced pressure, and the crude product was purified and separated by preparative thin-layer chromatography (dichloromethane:methanol=15:1) to obtain two isomers, according to the Rf values, the two isomers were named **2-20-01-A** (4.00 mg, Rf value was 0.30) and **2-20-01-B** (4.00 mg, Rf value was 0.35).

### Step 2: Synthesis of N-((1S,9S)-5-chloro-9-ethyl-9-hydroxy-4-methyl-10,13-dioxo-2,3,9,10, 13,15-hexahydro-1H,12H-benzo[de]pyrano[3',4':6,7]indolizino[1,2-b]quinolin-1-yl)-1-hydroxycyclopropane-1-carboxamide and N-((1R,9S)-5-chloro-9-ethyl-9-hydroxy-4-methyl-10,13-dioxo- 2,3,9,10,13,15-hexahydro-1H,12H-benzo[de]pyrano[3',4':6,7]indolizino[1,2-b]quinolin- 1-yl)-1-hydroxycyclopropane-1-carboxamide

At 25°C, **2-20-01-A** (4.00 mg, 5.17 µmol) and **2-20-01-B** (4.00 mg, 5.17 µmol) were dissolved in anhydrous THF (1 mL) in two reaction flasks respectively, added dropwise with a mixed solution of tetrabutylammonium fluoride (1M in tetrahydrofuran)/glacial acetic acid (v/v=13/1) (50 µL), reacted at 25°C for 0.5 hours, and the reaction was monitored by LCMS. When the reaction was completed, the reaction solution was purified by preparative high-performance liquid chromatography, and the fractions were lyophilized to obtain the title compounds **2-20-A** (0.71 mg) and **2-20-B** (1.05 mg).

The purification conditions for **2-20-A** were as follows:
Chromatographic column: SunFire Prep C18 OBD 19 mm×150 mm×5.0 µm
Mobile phase A: acetonitrile; mobile phase B: water (0.05% formic acid)

| Time [min] | Mobile phase A [%] | Mobile phase B [%] | Flow rate [mL/min] |
|---|---|---|---|
| 0 | 15 | 85 | 28 |
| 16 | 90 | 10 | 28 |

The purification conditions for **2-20-B** were as follows:
Chromatographic column: SunFire Prep C18 OBD 19 mm×150 mm×5.0 µm
Mobile phase A: acetonitrile; mobile phase B: water (0.05% formic acid)

| Time [min] | Mobile phase A [%] | Mobile phase B [%] | Flow rate [mL/min] |
|---|---|---|---|
| 0 | 20 | 80 | 28 |
| 2 | 20 | 20 | 28 |
| 18 | 80 | 20 | 28 |

The structural characterization data of **2-20-A** were as follows:
¹H NMR (400 MHz, DMSO-d₆) δ 8.58 (d, *J* = 9.0 Hz, 1H), 8.15 (s, 1H), 7.31 (s, 1H), 6.55 (s, 1H), 6.30 (s, 1H), 5.55 (dd, *J* = 13.2, 8.2 Hz, 1H), 5.43 (s, 2H), 5.26 (d, *J* = 19.0 Hz, 1H), 5.10 (d, *J* = 19.0 Hz, 1H), 3.29-3.09 (m, 2H), 2.52 (s, 3H), 2.31-2.15 (m, 2H), 1.93-1.80 (m, 2H), 1.25-1.14 (m, 2H), 0.98-0.90 (m, 2H), 0.87 (t, *J* = 7.3 Hz, 3H).
ESI-MS (m/z):536.2 [M+H]⁺.

The structural characterization data of **2-20-B** were as follows:
¹H NMR (400 MHz, DMSO-d₆) δ 8.63 (d, *J* = 9.0 Hz, 1H), 8.15 (s, 1H), 7.31 (s, 1H), 6.54 (s, 1H), 6.35 (s, 1H), 5.55 (dd, *J* = 13.5, 8.6 Hz, 1H), 5.43 (s, 2H), 5.29 (d, *J* = 19.1 Hz, 1H), 5.08 (d, *J =* 19.1 Hz, 1H), 3.28-3.10 (m, 2H), 2.51 (s, 3H), 2.30- 2.14 (m, 2H), 1.93-1.81 (m, 2H), 1.26-1.14 (m, 2H), 1.02-0.90 (m, 2H), 0.89 (d, *J* = 10.9 Hz, 3H).
ESI-MS (m/z):536.2 [M+H]⁺.

### Example 8: (1S,9S)-1-amino-4-chloro-9-ethyl-5-fluoro-9-hydroxy-1,2,3,9,12,15- hexahydro-10H,13H-benzo[de]pyrano[3',4':6,7]indolizino[1,2-b]quinoline-10,13-dione and (1R,9S)-1-amino-4-chloro-9-ethyl-5-fluoro-9-hydroxy-1,2,3,9,12,15-hexahydro-10H,13H-benzo[de]pyrano[3',4': 6,7]indolizino[1,2-b]quinoline-10,13-dione

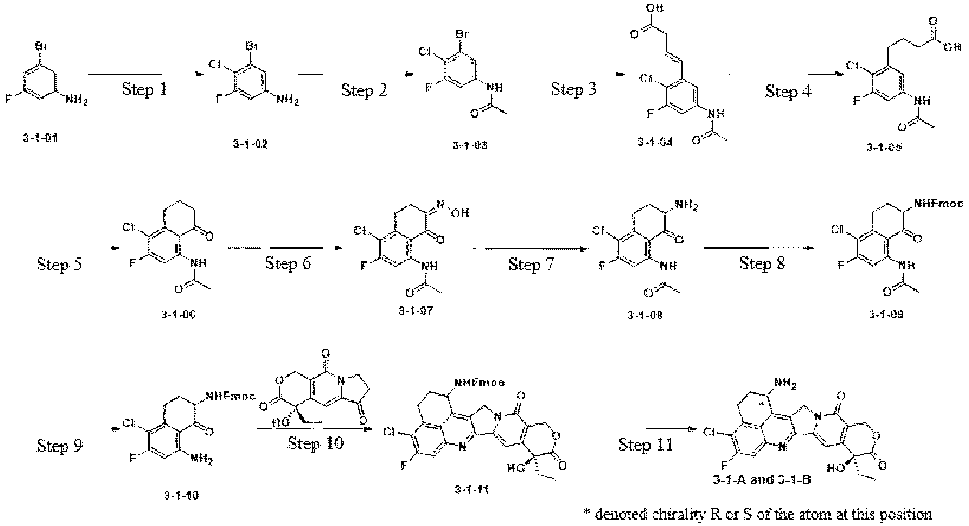

### Step 1: Synthesis of 3-bromo-4-chloro-5-fluoroaniline

Compound **3-1-01** (2.00 g, 10.53 mmol) was dissolved in N,N-dimethylformamide (30 mL), then slowly added with N-chlorosuccinimide (1.69 g, 12.63 mmol); after the addition, the reaction was carried out at room temperature for 16 hours and monitored by LCMS. The reaction solution was concentrated under reduced pressure to obtain a crude product, which was purified by flash silica gel column (ethyl acetate:petroleum ether=0-25%) to obtain 0.95 g of the title compound.

The structural characterization data were as follows:
¹H NMR (400 MHz, DMSO-d₆) δ 6.77 (dd, *J =* 2.5, 1.4 Hz, 1H), 6.51 (dd, *J* = 11.7, 2.5 Hz, 1H), 5.84 (s, 2H).

### Step 2: Synthesis of N-(3-bromo-4-chloro-5-fluorophenyl)acetamide

Compound **3-1-02** (0.95 g, 4.23 mmol) was dissolved in ethyl acetate (20 mL), and added with acetic anhydride (648.13 mg, 6.35 mmol) under nitrogen protection; after the addition, the temperature was raised to 50°C, the reaction was carried out for 15 hours and monitored by LCMS. The reaction solution was quenched with methanol (5 mL), and directly evaporated to dryness under reduced pressure to obtain a crude product, which was purified by flash silica gel column (ethyl acetate:petroleum ether=0-40%) to obtain 1.01 g of the title compound.

The structural characterization data were as follows:
ESI-MS (m/z): 265.9[M+H]⁺.

### Step 3: Synthesis of (E)-4-(5-acetylamino-2-chloro-3-fluorophenyl)-3-butenoic acid

Compound **3-1-03** and 3-butenoic acid (387.65 mg, 4.50 mmol) were dissolved in a mixed solvent of 1,4-dioxane (24 mL) and water (8 mL), then added with N,N-diisopropylethylamine (1.45 g, 11.26 mmol), tris(o-methylphenyl)phosphine (114.21 mg, 375.24 µmol) and palladium acetate (42.12 mg, 187.62 µmol); after the addition, the reaction system was subjected to nitrogen replacement three times, and heated up to 100°C to conduct the reaction for 16 hours under nitrogen atmosphere, and the reaction was monitored by LCMS. After the reaction solution was cooled to room temperature, 1N aqueous sodium hydroxide solution (60 mL) and ethyl acetate (50 mL) were added, shaken and layered. After the lower aqueous phase was separated, the pH was adjusted to about 3 with 4 mol/L hydrochloric acid aqueous solution, then subjected to extraction with ethyl acetate, the organic phases were combined, washed with saturated brine, dried over anhydrous sodium sulfate, filtered, and the filtrate was evaporated to dryness under reduced pressure to obtain 1.00 g of a crude product of the title compound.

The structural characterization data were as follows:
ESI-MS (m/z):272.0[M+H]⁺.

### Step 4: Synthesis of 4-(5-acetylamino-2-chloro-3-fluorophenyl)butanoic acid

The crude product of compound **3-1-04** (1.00 g, 3.68 mmol) was dissolved in tetrahydrofuran (15 mL), then added with 10% palladium on carbon (0.10 g), after the addition, the reaction system was subjected to replacement three times with hydrogen balloon, the reaction was carried out under hydrogen atmosphere for 4 hours and monitored by LCMS. The reaction solution was filtered, and the filtrate was concentrated to dryness under reduced pressure to obtain 1.00 g of a crude product of the title compound.

The structural characterization data were as follows:
ESI-MS (m/z):274.0[M+H]⁺.

### Step 5: Synthesis of N-(4-chloro-3-fluoro-8-oxo-5,6,7,8-tetrahydronaphthalen- 1-yl)acetamide

The crude product of compound **3-1-05** (1.00 g, 3.65 mmol) was dissolved in trifluoroacetic acid (5 mL), cooled to 5°C, then slowly added with trifluoroacetic anhydride (3.84 g, 18.27 mmol, 2.54 mL); after the addition, the reaction was carried out at 5°C for 2 hours and monitored by LCMS. The reaction solution was slowly poured into water, then extracted with ethyl acetate, the organic phases were combined, washed with saturated brine, dried over anhydrous sodium sulfate, and then filtered, and the filtrate was evaporated to dryness under reduced pressure to obtain a crude product, which was purified by flash silica gel column, to obtain 0.43 g of the title compound.

The structural characterization data were as follows:
ESI-MS (m/z):256.1[M+H]⁺.

### Step 6: Synthesis of N-(4-chloro-3-fluoro-7-(hydroxyimino)-8-oxo-5,6,7,8-tetrahydronaphthalen-1-yl)acetamide

Tetrahydrofuran (16 mL) and tert-butanol (4 mL) were added to a reaction flask, cooled to 5°C in an ice bath, added with potassium tert-butoxide (415.18 mg, 3.70 mmol), then compound **3-1-06** (0.43 mg, 1.68 mmol) was dissolved in tetrahydrofuran (1 mL) and added slowly thereto, followed by an addition of isoamyl nitrite (315.24 mg, 2.69 mmol) after 10 minutes, and after the addition, the reaction was carried out at 5°C for 1 hour, the reaction was monitored by LCMS. After the reaction solution was quenched with saturated ammonium chloride aqueous solution, it was extracted with ethyl acetate, the organic phases were combined, washed with saturated brine, dried over anhydrous sodium sulfate, and then filtered, and the filtrate was concentrated under reduced pressure to obtain 455.00 mg of a crude product of the title compound.

The structural characterization data were as follows:
ESI-MS (m/z):285.0[M+H]⁺.

### Step 7: Synthesis of N-(7-amino-4-chloro-3-fluoro-8-oxo-5,6,7,8-tetrahydronaphthalen- 1-yl)acetamide

The crude product of compound **3-1-07** (0.40 g, 1.41 mmol) was dissolved in methanol (10 mL), then added with 3 mol/L aqueous hydrochloric acid (1 mL) and 10% palladium on carbon (40.00 mg), after the addition, the reaction system was subjected to replacement three times with a hydrogen balloon, the reaction was carried out at room temperature under hydrogen atmosphere for 1 hour and monitored by LCMS. The reaction solution was filtered, and the filtrate was concentrated to dryness under reduced pressure to obtain 0.43 g of a crude hydrochloride of the title compound.

The structural characterization data were as follows:
ESI-MS (m/z):271.0[M+H]⁺.

### Step 8: Synthesis of (9H-fluoren-9-yl)methyl(8-acetamide-5-chloro-6-fluoro-1-oxo- 1,2,3,4-tetrahydronaphthalen-2-yl)carbamate

The crude hydrochloride of compound **3-1-08** (0.43 g, 1.19 mmol) was dissolved in 1,4-dioxane (15 mL), then added with sodium bicarbonate (400.35 mg, 4.77 mmol), water (5 mL) and 9-fluorenylmethyl-N-succinimidyl carbonate (481.81 mg, 1.43 mmol), after the addition, the reaction was carried out under stirring at room temperature for 2 hours and monitored by LCMS. The reaction solution was poured into water, then extracted with ethyl acetate, the organic phases were combined, washed with saturated brine, dried over anhydrous sodium sulfate, filtered, and the filtrate was concentrated under reduced pressure to obtain a crude product. The crude product was purified by a C18 reverse-phase column (acetonitrile: 0.05% formic acid in water = 20%-100%) to obtain 301.00 mg of the title compound.

The structural characterization data were as follows:
ESI-MS (m/z):493.2[M+H]⁺.

### Step 9: Synthesis of (9H-fluoren-9-yl)methyl(8-amino-5-chloro-6-fluoro-1-oxo- 1,2,3,4-tetrahydronaphthalen-2-yl)carbamate

Compound **3-1-09** (300.00 mg, 608.61 µmol) was dissolved in dioxane (5 mL), added with 12 mol/L concentrated hydrochloric acid (1 mL), after the addition, the temperature was raised to 60°C, the reaction was carried out for 2 hours and monitored by LCMS. The reaction solution was poured into water, then extracted with ethyl acetate, the organic phases were combined, washed with saturated brine, dried over anhydrous sodium sulfate, filtered, and the filtrate was concentrated under reduced pressure to obtain a crude product. The crude product was purified by flash silica gel column (ethyl acetate:petroleum ether=0-50%) to obtain 198.00 mg of the title compound.

The structural characterization data were as follows:
ESI-MS (m/z):451.1[M+H]⁺.

### Step 10: Synthesis of (9H-fluoren-9-yl)methyl((9S)-4-chloro-9-ethyl-5-fluoro- 9-hydroxy-10,13-dioxo-2,3,9,10,13,15-hexahydro-1H,12H-benzo[de]pyrano[3',4:6,7]indolizino[1,2-b]quinolin-1-yl)carbamate

(S)-4-Ethyl-4-hydroxy-7,8-dihydro-1H-pyrano[3,4-f]indolizine-3,6,10(4H)-trione (138.72 mg, 526.96 µmol) and compound **3-1-10** (198.00 mg, 439.13 µmol) were added in toluene (10 mL), and then added with p-toluenesulfonic acid (75.53 mg, 439.13 µmol), after the addition, the temperature was raised to 140°C, the reaction was carried out for 4 hours, the reaction solution was directly evaporated to dryness at 140°C under reduced pressure to obtain a crude product, and the crude product was purified by flash silica gel column (methanol:dichloromethane=0-5%) to obtain 256.00 mg of the title compound.

The structural characterization data were as follows:
ESI-MS (m/z):678.1[M+H]⁺.

### Step 11: Synthesis of (1S,9S)-1-amino-4-chloro-9-ethyl-5-fluoro-9-hydroxy-1,2,3,9,12,15-hexahydro-10H,13H-benzo[de]pyrano[3',4':6,7]indolizino[1,2-b]quinoline-10,13-dione and (1R,9S)-1-amino-4-chloro-9-ethyl-5-fluoro-9-hydroxy-1,2,3,9,12,15-hexahydro-10H,13H-benzo [de]pyrano [3',4':6,7]indolizino [1,2-b]quinoline-10,13-dione

Compound **3-1-11** (201.18 mg, 296.67 µmol) was dissolved in N,N-dimethylformamide (4 mL), then added with diethylamine (108.49 mg, 1.48 mmol), after the addition, the reaction was carried out at room temperature for 0.5 hours, and monitored by LCMS. After the reaction solution was distilled under reduced pressure to remove ethylenediamine, the pH was adjusted to 2-3 with 1 mol/L hydrochloric acid aqueous solution, and the reaction solution was directly purified by preparative high performance liquid chromatography to obtain the title compounds **3-1-A** (44.00 mg), **3-1-B** (43.00 mg).
Chromatographic column: SunFire Prep C18 OBD 19 mm×150 mm×5.0 µm
Mobile phase A: acetonitrile; Mobile phase B: water (0.05% formic acid)

| Time [min] | Mobile phase A [%] | Mobile phase B [%] | Flow rate [mL/min] |
|---|---|---|---|
| 0 | 10 | 90 | 28 |
| 3 | 10 | 90 | 28 |
| 18 | 70 | 30 | 28 |

**3-1-A** (6 min LCMS, the earlier peak with retention time of 1.276 min)

The structural characterization data were as follows:
¹H NMR (400 MHz, DMSO-d₆) δ 8.00 (d, *J =* 10.3 Hz, 1H), 7.33 (s, 1H), 6.54 (s, 1H), 5.62 (d, *J* = 19.3 Hz, 1H), 5.44 (s, 2H), 5.38 (d, *J* = 19.3 Hz, 1H), 4.43-4.38 (m, 1H), 3.28-3.10 (m, 2H), 2.22-2.12 (m, 1H), 2.12-2.02 (m, 1H), 1.93-1.80 (m, 2H), 0.87 (t, *J* = 7.3 Hz, 3H).
ESI-MS (m/z):456.1[M+H]⁺.

### 3-1-B (6 min LCMS, the later peak with a retention time of 1.300 min)

The structural characterization data thereof were as follows:
¹H NMR (400 MHz, DMSO-d₆) δ7.98 (d, *J* = 10.3 Hz, 1H), 7.32 (s, 1H), 5.61 (d, *J* = 19.4 Hz, 1H), 5.44 (s, 2H), 5.32 (d, *J* = 19.4 Hz, 1H), 4.44-4.36 (m, 1H), 3.33-3.25 (m, 1H), 3.22-3.11 (m, 1H), 2.23 - 2.13 (m, 1H), 2.11- 2.03 (m, 1H), 1.96 -1.82 (m, 2H), 0.89 (t, *J* = 7.3 Hz, 3H).
ESI-MS (m/z):456.1[M+H]⁺.
6 min LCMS conditions:
Chromatographic column: Waters SunFire C18 OBD 4.6 mm×50 mm×5.0 µm
Mobile phase A: 0.05% acetonitrile; mobile phase B: water (0.05% formic acid)

| Time [min] | Mobile phase A [%] | Mobile phase B [%] | Flow rate [mL/min] |
|---|---|---|---|
| 0 | 90 | 10 | 2 |
| 4.2 | 10 | 90 | 2 |
| 5.7 | 10 | 90 | 2 |
| 5.71 | 90 | 10 | 2 |
| 6.70 | 90 | 10 | 2 |

### Example 9: N-((1S,9S)-4-Chloro-9-ethyl-5-fluoro-9-hydroxy-10,13-dioxo-2,3,9,10,13,15-hexahydro-1H,12H-benzo[de]pyrano[3',4:6,7]indolizino[1,2-b]quinolin-1-yl)-2-hydroxyacetamide and N-((1R,9S)-4-Chloro-9-ethy1-5-fluoro-9-hydroxy-10,13-dioxo-2,3,9,10,13,15-hexahydro- 1H,12H-benzo[de]pyrano[3',4:6,7]indolizino[1,2-b]quinolin-1-yl)-2-hydroxyacetamide

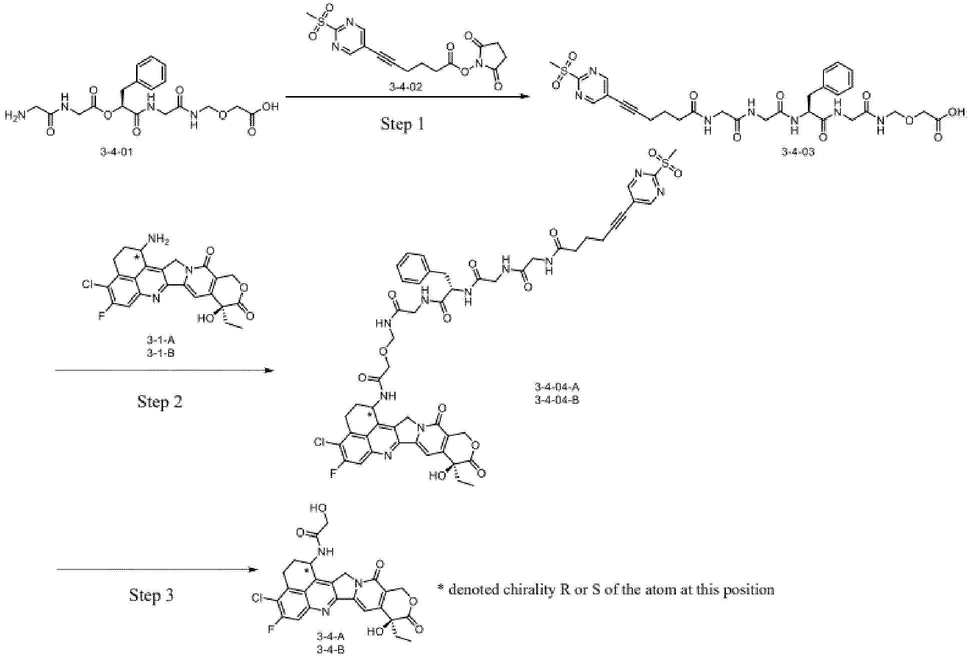

### Step 1: Synthesis of (S)-10-benzyl-23-(2-(methylsulfonyl)pyrimidin-5-yl)-6,9,12,15,18-pentoxo-3-oxo-5,8,11,14,17-pentaazoctadecane-22-yne-carboxylic acid

Compound **3-4-01** (30.00 mg, 70.00 µmol) was dissolved in N,N-dimethylformamide (1 mL), added with 2,5-dioxopyrrolidin-1-yl 6-(2-(methylsulfonyl)pyrimidin-5-yl)hex-5-ynyl-amide (28.00 mg, 77.00 µmol), and reacted at room temperature for 1 hour, and the reaction was monitored by LCMS. The reaction solution was directly purified by preparative high performance liquid chromatography, and the fractions was lyophilized to obtain the title compound **3-4-03** (20.00 mg).
Chromatographic column: SunFire Prep C18 OBD 19 mm×150 mm×5.0 µm
Mobile phase A: acetonitrile; Mobile phase B: water (0.05% formic acid)

| Time [min] | Mobile phase A [%] | Mobile phase B [%] | Flow rate [mL/min] |
|---|---|---|---|
| 0.00 | 10 | 90 | 28 |
| 2.00 | 10 | 90 | 28 |
| 18.00 | 90 | 10 | 28 |

The structural characterization data were as follows:
ESI-MS (m/z):691.0[M+18]+.

### Step 2: Synthesis of N-((S)-10-benzyl-1-((1S,9S)-4-chloro-9-ethyl-5-fluoro-9-hydroxy-10,13-dioxo-2,3,9,10,13,15-hexahydro-1H,12H-benzo[de]pyrano[3',4':6,7]indolizino[1,2-b]quinolin-1-yl)amino)-1,6,9,12,15-pentaoxo-3-oxa-5,8,11,14-tetraazahexadecan-16-yl)-6-(2-(methylsulfonyl)pyrimidin-5-yl)hex-5-ynyl-amide and N-((S)-10-benzyl-1-((1R,9S)-4-chloro-9-ethyl-5- fluoro-9-hydroxy-10,13-dioxo-2,3,9,10,13,15-hexahydro-1H,12H-benzo[de]pyrano[3',4':6,7]indolizino[1,2-b]quinolin-1-yl)amino)-1,6,9,12,15-pentoxo-3-oxa-5, 8,11,14-tetraazahexadecan-16-yl)-6-(2-(methylsulfonyl)pyrimidin-5-yl)hex-5-ynyl-amide

Compound **3-1-A** (36.00 mg, 79.70 µmol) and compound **3-4-03** (64.43 mg, 95.64 µmol) in single configuration were dissolved in N,N-dimethylformamide (2 mL), then added with 4-(4,6-dimethoxytriazin-2-yl)-4-methylmorpholine hydrochloride (46.98 mg, 159.40 µmol) and triethylamine (24.19 mg, 239.10 µmol), after the addition, the reaction was carried out at room temperature for 1 hour and monitored by LCMS. The reaction solution was directly purified by high performance liquid chromatography to obtain the title compound **3-4-04-A** (51.00 mg) in single configuration.
Chromatographic column: SunFire Prep C18 OBD 19 mm×150 mm×5.0 µm
Mobile phase A: acetonitrile; Mobile phase B: water (0.05% formic acid)

| Time [min] | Mobile phase A [%] | Mobile phase B [%] | Flow rate [mL/min] |
|---|---|---|---|
| 0 | 30 | 70 | 28 |
| 3 | 30 | 70 | 28 |
| 18 | 90 | 10 | 28 |

The structural characterization data were as follows:
ESI-MS (m/z):1111.0[M+H]⁺.

Compound **3-1-B** (36.00 mg, 79.70 µmol) and compound **3-4-03** (64.43 mg, 95.64 µmol) in single configuration were dissolved in N,N-dimethylformamide (2 mL), then added with 4-(4,6-dimethoxytriazin-2-yl)-4-methylmorpholine hydrochloride (46.98 mg, 159.40 µmol) and triethylamine (24.19 mg, 239.10 µmol), after the addition, the reaction was carried out at room temperature for 1 hour and monitored by LCMS. The reaction solution was directly purified by high performance liquid chromatography to obtain the title compound **3-4-04-B** (52.00 mg) in single configuration.
Chromatographic column: SunFire Prep C18 OBD 19 mm×150 mm×5.0 µm
Mobile phase A: acetonitrile; Mobile phase B: water (0.05% formic acid)

| Time [min] | Mobile phase A [%] | Mobile phase B [%] | Flow rate [mL/min] |
|---|---|---|---|
| 0 | 30 | 70 | 28 |
| 3 | 30 | 70 | 28 |
| 18 | 90 | 10 | 28 |

The structural characterization data were as follows:
ESI-MS (m/z):1111.0[M+H]⁺.

### Step 3: Synthesis of N-((1S,9S)-4-chloro-9-ethyl-5-fluoro-9-hydroxy-10,13-dioxo-2,3,9,10, 13,15-hexahydro-1H,12H-benzo[de]pyrano[3',4:6,7]indolizino[1,2-b]quinolin-1-yl)-2-hydroxyacetamide and N-((1R,9S)-4-chloro-9-ethy1-5-fluoro-9-hydroxy-10,13-dioxo-2,3,9,10,13,15- hexahydro-1H,12H-benzo[de]pyrano[3',4:6,7]indolizino[1,2-b]quinolin-1-yl)-2-hydroxy acetamide

Compound **3-4-04-A** (40.00 mg, 35.99 µmol) was weighed and dissolved in a mixed solvent of dichloromethane (2 mL) and methanol (1 mL), then added with 4 mol/L ethyl acetate hydrochloride (1 mL), after the addition, the reaction was carried out at room temperature for 0.5 hour and monitored by LCMS. The reaction solution was directly concentrated to dryness under reduced pressure to obtain a crude product, and the crude product was purified by high performance liquid chromatography to obtain the title compound **3-4-A** (4.75 mg) in single configuration.
Chromatographic column: SunFire Prep C18 OBD 19 mm×150 mm×5.0 µm
Mobile phase A: acetonitrile; Mobile phase B: water (0.05% formic acid)

| Time [min] | Mobile phase A [%] | Mobile phase B [%] | Flow rate [mL/min] |
|---|---|---|---|
| 0 | 15 | 85 | 28 |
| 3 | 15 | 85 | 28 |
| 18 | 90 | 10 | 28 |

The structural characterization data were as follows:
¹H NMR (400 MHz, DMSO-d₆) δ 8.50 (d, *J* = 8.9 Hz, 1H), 8.05 (d, *J* = 10.3 Hz, 1H), 7.33 (s, 1H), 6.55 (s, 1H), 5.67 -5.60 (m, 1H), 5.49 (t, *J* = 5.8 Hz, 1H), 5.43 (s, 2H), 5.21 (s, 2H), 3.96 (d, *J* = 5.8 Hz, 2H), 3.32-3.22 (m, 2H), 2.28-2.15 (m, 2H), 1.93-1.80 (m, 2H), 0.87 (t, *J* = 7.3 Hz, 3H).
ESI-MS (m/z):514.0[M+H]⁺.

Compound **3-4-04-B** (40.00 mg, 35.99 µmol) was weighed and dissolved in a mixed solvent of dichloromethane (2 mL) and methanol (1 mL), then added with 4 mol/L ethyl acetate hydrochloride (1 mL), after the addition, the reaction was carried out at room temperature for 0.5 hour and monitored by LCMS. The reaction solution was directly concentrated to dryness under reduced pressure to obtain a crude product, and the crude product was purified by high performance liquid chromatography to obtain the title compound 3-4-B (8.24 mg) in single configuration.
Chromatographic column: SunFire Prep C18 OBD 19 mm×150 mm×5.0 µm
Mobile phase A: acetonitrile; Mobile phase B: water (0.05% formic acid)

| Time [min] | Mobile phase A [%] | Mobile phase B [%] | Flow rate [mL/min] |
|---|---|---|---|
| 0 | 15 | 85 | 28 |
| 3 | 15 | 85 | 28 |
| 18 | 90 | 10 | 28 |

The structural characterization data were as follows:
¹H NMR (400 MHz, DMSO-d₆) δ 8.52 (d, *J=* 9.0 Hz, 1H), 8.05 (d, *J=* 10.3 Hz, 1H), 7.34 (s, 1H), 6.55 (s, 1H), 5.68 -5.58 (m, 1H), 5.53 (t, *J =* 5.8 Hz, 1H), 5.43 (d, *J =* 2.9 Hz, 2H), 5.20 (d, *J* = 7.3 Hz, 2H), 3.97 (d, *J* = 5.7 Hz, 2H), 3.31-3.21 (m, 2H), 2.26-2.15 (m, 2H), 1.92-1.82 (m, 2H), 0.87 (t, *J* = 7.3 Hz, 3H).
ESI-MS (m/z):514.0[M+H]⁺.

### Example 10: (S)-N-(2-(4-ethyl-8-fluoro-4-hydroxy-9-methyl-3,14-dioxo-3,4,12,14-tetrahydro-1H-pyrano[3',4':6,7]indolizino[1,2-b]quinolin-11-yl)ethyl)-2-hydroxy-N-isopropylacetamide

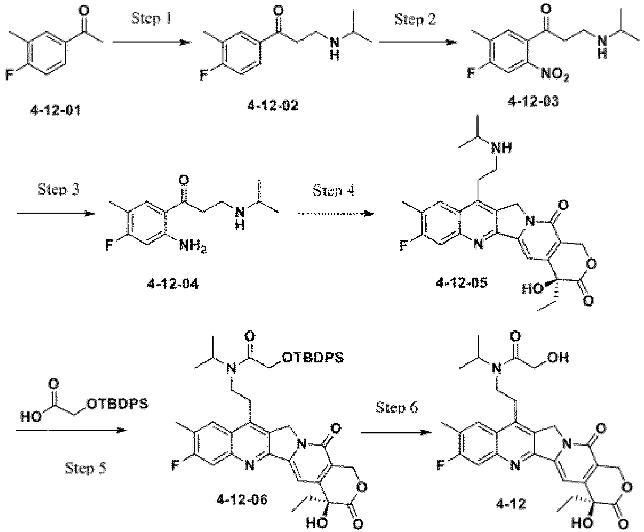

### Step 1: Synthesis of 1-(4-fluoro-3-methylphenyl)-3-(isopropylamino)propan-1-one

At 20°C, compound **4-12-01** (500.00 mg, 3.29 mmol), formaldehyde solution (2.5 mL, 37%) and isopropylamine (388.46 mg, 6.57 mmol) were dissolved in isopropanol (5 mL), added dropwise with concentrated hydrochloric acid (2.5 mL) at 0°C, the reaction solution was stirred at 100°C for 16 hours and monitored by LCMS. The reaction solution was concentrated under reduced pressure to obtain a crude product, which was purified by preparative high performance liquid chromatography, and the fractions were lyophilized to obtain 200.00 mg of the title compound.
Chromatographic column: SunFire Prep C18 OBD 19 mm×150 mm×5.0 µm
Mobile phase A: acetonitrile; Mobile phase B: water (0.05% formic acid)

| Time [min] | Mobile phase A [%] | Mobile phase B [%] | Flow rate [mL/min] |
|---|---|---|---|
| 0.00 | 10 | 90 | 28 |
| 2.00 | 10 | 90 | 28 |
| 18.00 | 90 | 10 | 28 |

The structural characterization data were as follows:
ESI-MS (m/z): 224.1 [M+H]⁺.

### Step 2: Synthesis of 1-(4-fluoro-5-methyl-2-nitrophenyl)-3-(isopropylamino)propan-1-one

At 0°C, compound **4-12-02** (100.00 mg, 0.49 mmol) was added into concentrated sulfuric acid (0.5 mL), then added with potassium nitrate (54.34 mg, 0.54 mmol), and the reaction was reacted at 0°C for 1 hour and monitored by LCMS. The reaction solution was poured into ice water, and purified by reverse phase column (acetonitrile: 0.05% formic acid water = 0-30%) to obtain 90.00 mg of the title compound.

The structural characterization data were as follows:
ESI-MS (m/z): 269.0 [M+H]⁺.

### Step 3: Synthesis of 1-(2-amino-4-fluoro-5-methylphenyl)-3-(isopropylamino)propan-1-one

At 25°C, compound **4-12-03** (200.00 mg, 0.75mmol) was added into methanol (20.0 mL), then added with 10% palladium on carbon (10.00 mg), the reaction solution was subjected to hydrogen replacement, reacted at 20°C for 16 hours under hydrogen atmosphere, the reaction was monitored by LCMS. The reaction solution was filtered and concentrated under reduced pressure to obtain 183.00 mg of the title compound.

The structural characterization data were as follows:
ESI-MS (m/z): 239.1 [M+H]⁺.

### Step 4: Synthesis of (S)-4-ethyl-8-fluoro-4-hydroxy-11-(2-(isopropylamino)ethyl)- 9-methyl-1,12-dihydro-14H-pyrano[3',4':6,7]indolizino[1,2-b]quinoline-3,14(4H)-dione

At 25°C, compound **4-12-04** (50.00 mg, 0.21 mmol) and (S)-4-ethyl-4-hydroxy- 7,8-dihydro-1H-pyrano[3,4-f]indolizine-3,6,10(4H)-trione (55.23 mg, 0.21 mmol) were added into toluene (3 mL), then added with p-toluenesulfonic acid (3.61 mg, 0.02 mmol), and the reaction solution was reacted at 130°C for 4 hours and monitored by LCMS. The reaction solution was concentrated under reduced pressure, the crude product was purified by preparative high-performance liquid chromatography, and the fractions were lyophilized to obtain 2.00 mg of a trifluoroacetic acid salt of the title compound.
Chromatographic column: SunFire Prep C18 OBD 19 mm×150 mm×5.0 µm
Mobile phase A: acetonitrile; mobile phase B: water (0.05% trifluoroacetic acid)

| Time [min] | Mobile phase A [%] | Mobile phase B [%] | Flow rate [mL/min] |
|---|---|---|---|
| 0.00 | 8 | 92 | 28 |
| 2.00 | 8 | 92 | 28 |
| 18.00 | 60 | 40 | 28 |

The structural characterization data were as follows:
¹H NMR (400 MHz, DMSO-d₆) δ 8.58 (s, 2H), 8.22 (d, *J* = 8.1 Hz, 1H), 7.97 (d, *J* = 10.7 Hz, 1H), 7.35 (s, 1H), 6.59 (s, 1H), 5.47 (s, 2H), 5.41 (s, 2H), 3.58-3.45 (m, 3H), 3.31-3.23 (m, 2H), 2.56 (s, 3H), 1.98-1.80 (m, 2H), 1.26 (d, *J* = 6.3 Hz, 6H), 0.89 (t, *J* = 7.3 Hz, 3H).
ESI-MS (m/z): 466.2 [M+H]⁺

### Step 5: Synthesis of (S)-2-((tert-butyldiphenylsilyl)oxy)-N-(2-(4-ethyl-8-fluoro-4-hydroxy-9-methyl-3,14-dioxo-3,4,12,14-tetrahydro-1H-pyrano[3',4':6,7]indolizino[1,2-b]quinolin-11-yl)ethyl)-N-isopropylacetamide

Compound **4-12-05** (22.00 mg, 47.26 µmol) and 2-((tert-butyldiphenylsilyl)oxy)acetic acid (16.35 mg, 51.99 µmol) were dissolved in N,N-dimethylformamide (1 mL), then added with HATU (21.55 mg, 56.71 µmol) and N,N-diisopropylethylamine (18.32 mg, 141.78 µmol), after the addition, the reaction was carried out at room temperature for 0.5 hours and monitored by LCMS. The reaction solution was directly purified by a C18 reverse-phase column (acetonitrile: 0.05% aqueous formic acid = 30%-100%) to obtain 18.00 mg of the title compound.

The structural characterization data were as follows:
ESI-MS (m/z):762.3[M+H]⁺.

### Step 6: Synthesis of (S)-N-(2-(4-ethyl-8-fluoro-4-hydroxy-9-methyl-3,14-dioxo-3,4,12,14-tetrahydro-1H-pyrano[3',4':6,7]indolizino[1,2-b]quinolin-11-yl)ethyl)-2-hydroxy-N-isopropylacetamide

Compound **4-12-06** (18.00 mg, 23.62 µmol) was dissolved in N,N-dimethylformamide (1 mL), then added with potassium fluoride (6.86 mg, 118.12 µmol), then heated to 50°C and reacted for 1 hour, and the reaction was monitored by LCMS. The reaction solution was directly purified by high performance liquid chromatography to obtain 1.53 mg of the title compound.
Chromatographic column: SunFire Prep C18 OBD 19 mm×150 mm×5.0 µm
Mobile phase A: acetonitrile; Mobile phase B: water (0.05% formic acid)

| Time [min] | Mobile phase A [%] | Mobile phase B [%] | Flow rate [mL/min] |
|---|---|---|---|
| 0.00 | 15 | 85 | 28 |
| 18.00 | 90 | 10 | 28 |

The structural characterization data were as follows:
¹H NMR (400 MHz, DMSO-d₆) δ 8.53 (d, *J* = 8.2 Hz, 1H), 7.91 (d, *J* = 10.8 Hz, 1H), 7.32 (s, 1H), 6.55 (s, 1H), 5.44 (d, *J* = 13.8 Hz, 4H), 4.72 (t, *J* = 5.5 Hz, 1H), 4.21 (d, *J* = 5.5 Hz, 2H), 3.99-3.90 (m, 1H), 3.54-3.38 (m, 4H), 2.54 (s, 3H), 1.92-1.83 (m, 2H), 1.17 (dd, *J* = 6.6, 3.1 Hz, 6H), 0.87 (t, *J* = 7.3 Hz, 3H).
ESI-MS (m/z):524.2[M+H]⁺.

### Example 11: (S)-N-((4-ethyl-8-fluoro-4-hydroxy-9-methyl-3,14-dioxo-3,4,12,14- tetrahydro-1H-pyrano[3',4':6,7]indolizino[1,2-b]quinolin-11-yl)methyl)-1-hydroxycyclopropane-carboxamide

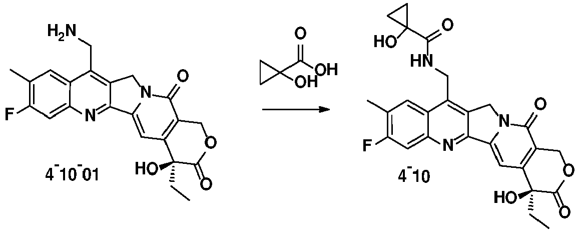

Raw material (S)-11-(aminomethyl)-4-ethyl-8-fluoro-4-hydroxy-9-methyl-1H-pyrano[3',4':6,7]indolizino[1,2-b]quinolin-3,14(4H,12H)-dione (**4-10-01**, prepared according to the method disclosed in WO2020219287, 30.00 mg, 67.00 µmol), and 1-hydroxycyclopropane-carboxylic acid (7.56 mg, 0.074 mmol) were dissolved in DMF (1 mL), added with HBTU (34.30 mg, 0.14 mmol) and diisopropylethylamine (26.09 mg, 0.20 mmol) under stirring, and reacted at room temperature for 4 hours. Water and ethyl acetate were added and stirred, and allowed to stand to separate the layers, and the organic phase was washed with saturated brine and concentrated under reduced pressure. The concentrate was purified by preparative thin-layer chromatography (dichloromethane:methanol=20:1), and then purified by preparative high-performance liquid chromatography to obtain 1.20 mg of solid.
Chromatographic column: SunFire Prep C18 OBD 19 mm×150 mm×5.0 µm
Mobile phase A: acetonitrile; Mobile phase B: water (0.05% formic acid)

| Time [min] | Mobile phase A [%] | Mobile phase B [%] | Flow rate [mL/min] |
|---|---|---|---|
| 0.00 | 20 | 80 | 28 |
| 2.00 | 20 | 80 | 28 |
| 18.00 | 80 | 20 | 28 |

The structural characterization data were as follows:
¹H NMR (400 MHz, DMSO-d₆) δ 8.96 (t, *J* = 6.0 Hz, 1H), 8.51 (d, *J* = 8.0 Hz, 1H), 7.90 (d, *J* = 10.8 Hz, 1H), 7.31 (s, 1H), 6.53 (s, 1H), 6.30 (s, 2H), 6.30 (s, 1H), 5.52 (s, 2H), 5.44 (s, 2H), 4.84 (d, *J* = 6.0 Hz, 2H),2.51(s,3H), 1.91-1.81 (m, 2H), 1.01 (dd, *J* = 7.2, 4.1 Hz, 2H), 0.87 (t, *J*= 7.3 Hz, 3H), 0.83 (t, *J=* 3.6 Hz, 2H).
ESI-MS (m/z):494.1[M+1]+.

### Example 12: (1S,9S)-1-Amino-9-ethyl-5-fluoro-9-hydroxy-1,4-dimethyl-1,2,3,9,12,15-hexahydro-10H,13H-benzo[de]pyrano[3',4':6,7]indolizino[1,2-b]quinolin-10,13-dione and (1R,9S)-1-amino-9-ethyl-5-fluoro-9-hydroxy-1,4-dimethyl-1,2,3,9,12,15-hexahydro-10H,13H-benzo[de]pyrano[3',4':6,7]indolizino[1,2-b]quinolin-10,13-dione

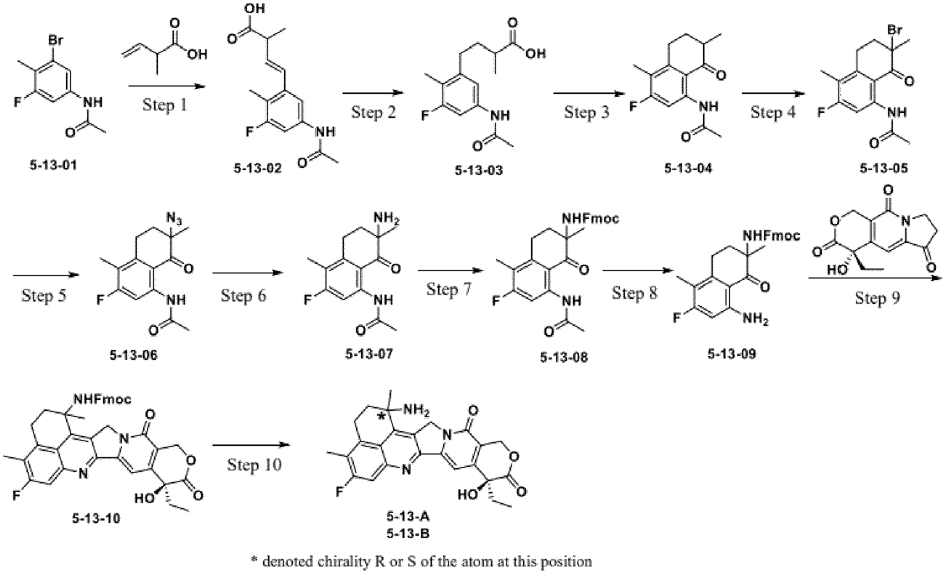

### Step 1: Synthesis of (E)-4-(5-acetylamino-3-fluoro-2-methylphenyl)-2-methyl-3-butenoic acid

N-(3-Bromo-5-fluoro-4-methylphenyl)acetamide (2.00 g, 8.13 mmol) and 2-methyl-3-butenoic acid (976.44 mg, 9.75 mmol) were weighed and dissolved in a mixed solvent of 1,4-dioxane (15 mL) and water (5 mL), then added with tris(o- methylphenyl)phosphine (247.37 mg, 812.76 µmol), palladium acetate (91.24 mg, 406.38 µmol) and N,N-diisopropylethylamine (2.31 g, 17.88 mmol), after the addition, the reaction system was subjected to nitrogen replacement three times, the temperature was raised to 80°C and the reaction was carried out for 3 hours under nitrogen atmosphere and monitored by LCMS. After the reaction solution was cooled to room temperature, 1 mol/L sodium hydroxide aqueous solution (60 mL) and ethyl acetate (50 mL) were added and shaken to separated layers. After separating the lower layer of aqueous phase, the pH was adjusted to about 3 with 4 mol/L hydrochloric acid aqueous solution, then extraction was carried out with ethyl acetate, the organic phases were combined and washed with saturated brine, dried over anhydrous sodium sulfate, filtered, and the filtrate was concentrated under reduced pressure to obtain 1.90 g of the title compound.

The structural characterization data were as follows:
ESI-MS (m/z):266.1[M+H]⁺.

### Step 2: Synthesis of 4-(5-acetylamino-3-fluoro-2-methylphenyl)-2-methylbutanoic acid

(E)-4-(5-Acetylamino-3-fluoro-2-methylphenyl)-2-methyl-3-butenoic acid (1.90 g, 7.16 mmol) was dissolved in methanol (40 mL), added with 10% palladium on carbon (0.15 g) under the protection of nitrogen, then the reaction system was subjected to replacement three times with a hydrogen balloon, the reaction was carried out under hydrogen atmosphere for 2 hours and monitored by LCMS. The reaction solution was filtered, and the filtrate was concentrated to dryness under reduced pressure to obtain 1.51 g of the title compound.

The structural characterization data were as follows:
ESI-MS (m/z):268.1[M+H]⁺.

### Step 3: Synthesis of N-(3-fluoro-4,7-dimethyl-8-oxo-5,6,7,8- tetrahydronaphthalen-1-yl)acetamide

4-(5-Acetylamino-3-fluoro-2-methylphenyl)-2-methylbutanoic acid (1.50 g, 5.61 mmol) was weighed and dissolved in trifluoroacetic acid (20 mL), cooled to 5°C, then added dropwise with trifluoroacetic anhydride (2.36 g, 11.22 mmol). After the addition, the reaction was carried out at 5°C for 2 hours and monitored by LCMS. The reaction solution was slowly poured into saturated aqueous sodium bicarbonate solution, then extracted with ethyl acetate, the organic phases were combined, washed with saturated brine, dried over anhydrous sodium sulfate, filtered, and the filtrate was evaporated to dryness under reduced pressure to obtain a crude product. The crude product was purified by flash silica gel column (ethyl acetate:petroleum ether=0-30%) to obtain 1.05 g of the title compound.

The structural characterization data were as follows:
ESI-MS (m/z):250.1[M+H]⁺.

### Step 4: Synthesis of N-(7-bromo-3-fluoro-4,7-dimethyl-8-oxo-5,6,7,8- tetrahydronaphthalen-1-yl)acetamide

N-(3-Fluoro-4,7-dimethyl-8-oxo-5,6,7,8-tetrahydronaphthalen-1-yl)acetamide (0.55 g, 2.21 mmol) was weighed and dissolved in acetic acid (8 mL), then added with bromine (387.85 mg, 2.43 mmol), after the addition, the temperature was raised to 50°C, the reaction was carried out for 15 hours and monitored by LCMS. The reaction solution was directly evaporated to dryness under reduced pressure to obtain a crude product, which was purified by flash silica gel column (ethyl acetate:petroleum ether=0-30%) to obtain 461.00 mg of the title compound.

The structural characterization data were as follows:
ESI-MS (m/z):328.0[M+H]⁺.

### Step 5: Synthesis of N-(7-azido-3-fluoro-4,7-dimethyl-8-oxo-5,6,7,8- tetrahydronaphthalen-1-yl)acetamide

N-(7-Bromo-3-fluoro-4,7-dimethyl-8-oxo-5,6,7,8-tetrahydronaphthalen-1-yl)acetamide (460.00 mg, 1.40 mmol) was weighed and dissolved in N,N-dimethylformamide (10 mL), then added with sodium azide (273.37 mg, 4.21 mmol), after the addition, the reaction was carried out at room temperature for 1 hour and monitored by LCMS. The reaction solution was slowly poured into water, then extracted with ethyl acetate, the organic phases were combined, washed with saturated brine, dried over anhydrous sodium sulfate, and filtered, and the filtrate was evaporated to dryness under reduced pressure to obtain a crude product, which was purified by flash silica gel column (ethyl acetate:petroleum ether=0-50%) to obtain 347.00 mg of the title compound.

The structural characterization data were as follows:
ESI-MS (m/z):291.1[M+H]⁺.

### Step 6: Synthesis of N-(7-amino-3-fluoro-4,7-dimethyl-8-oxo-5,6,7,8- tetrahydronaphthalen-1-yl)acetamide

N-(7-Azido-3-fluoro-4,7-dimethyl-8-oxo-5,6,7,8-tetrahydronaphthalen-1-yl)acetamide (347.00 mg, 1.20 mmol) was weighed and dissolved in tetrahydrofuran (10 mL), added with 10% palladium on carbon (30.00 mg) under nitrogen protection, then the reaction system was subjected to replacement three times with a hydrogen balloon, and the reaction was carried out for 2 hours under hydrogen atmosphere and monitored by LCMS. The reaction solution was filtered, and the filtrate was concentrated to dryness under reduced pressure to obtain a crude product, which was purified by a C18 reverse-phase column (acetonitrile: 0.05% formic acid aqueous solution = 0%-30%) to obtain 205.00 mg of the title compound.

The structural characterization data were as follows:
ESI-MS (m/z):265.1[M+H]⁺.

### Step 7: Synthesis of (9H-fluoren-9-yl)methyl(8-acetylamino-6-fluoro-2,5-dimethyl-1- oxo-1,2,3,4-tetrahydronaphthalen-2-yl)carbamate

N-(7-Amino-3-fluoro-4,7-dimethyl-8-oxo-5,6,7,8-tetrahydronaphthalen-1-yl)acetamide (200.00 mg, 756.73 µmol) was weighed and dissolved in a mixed solvent of 1,4-dioxane (6 mL) and water (3 mL), then added with sodium bicarbonate (254.28 mg, 3.03 mmol) and 9-fluorenylmethyl-N-succinimide carbonate (650.55 mg, 1.14 mmol), after the addition, the reaction was carried out under stirring at room temperature for 2 hours and monitored by LCMS. The reaction solution was slowly poured into water, then extracted with ethyl acetate, the organic phases were combined, washed with saturated brine, dried over anhydrous sodium sulfate, filtered, and the filtrate was evaporated to dryness under reduced pressure to obtain a crude product, which was purified by a C18 reverse phase column (acetonitrile: 0.05% aqueous formic acid = 20%-80%) to obtain 301.00 mg of the title compound.

The structural characterization data were as follows:
ESI-MS (m/z):487.0[M+H]⁺.

### Step 8: Synthesis of (9H-fluoren-9-yl)methyl(8-amino-6-fluoro-2,5-dimethyl-1- oxo-1,2,3,4-tetrahydronaphthalen-2-yl)carbamate

(9H-Fluoren-9-yl)methyl(8-acetylamino-6-fluoro-2,5-dimethyl-1-oxo-1,2,3,4-tetrahydronaphthalen-2-yl)carbamate (101.00 mg, 207.59 µmol) was dissolved in 1,4-dioxane (5 mL), then added with 3 mol/L hydrochloric acid aqueous solution (5 mL), the temperature was raised to 50°C, the reaction was carried out for 15 hours and monitored by LCMS. The reaction solution was slowly poured into saturated aqueous sodium bicarbonate solution, then extracted with ethyl acetate, the organic phases were combined and washed with saturated brine, dried over anhydrous sodium sulfate, filtered, and the filtrate was evaporated to dryness under reduced pressure to obtain a crude product, which was purified by flash silica gel column (methanol:dichloromethane=0%-5%) to obtain 71.00 mg of the title compound.

The structural characterization data were as follows:
ESI-MS (m/z):445.2[M+H]⁺.

### Step 9: Synthesis of (9H-fluoren-9-yl)methyl((9S)-9-ethyl-5-fluoro-9-hydroxy-1,4- dimethyl-10,13-dioxo-2,3,9,10,13,15-hexahydro-1H,12H-benzo[de]pyrano[3',4':6,7]indolizino[1,2-b]quinolin-1-yl)carbamate

(9H-Fluoren-9-yl)methyl(8-amino-6-fluoro-2,5-dimethyl-1-oxo-1,2,3,4-tetrahydronaphthalen-2-yl)carbamate (35.00 mg, 132.96 µmol) and (S)-4-ethyl-4-hydroxy-7,8-dihydro-1H- pyrano[3,4-f]indolazin-3,6,10(4H)-trione (49.25 mg, 110.80 µmol) were added into toluene (3 mL), then added with p-toluenesulfonic acid (19.08 mg, 110.80 µmol), the temperature was raised to 140°C and the reaction was carried out for 4 hours. The liquid reaction solution was directly evaporated to dryness under reduced pressure at 140°C to obtain a crude product. The crude product was purified by a C18 reverse phase column (acetonitrile: 0.05% aqueous formic acid = 20%-80%) to obtain 21.00 mg of the title compound.

The structural characterization data were as follows:
ESI-MS (m/z):672.2[M+H]⁺.

### Step 10: Synthesis of (1S,9S)-1-amino-9-ethyl-5-fluoro-9-hydroxy-1,4-dimethyl-1,2,3,9,12,15-hexahydro-10H,13H-benzo[de]pyrano[3',4':6,7]indolizino[1,2-b]quinolin-10,13-dione and (1R,9S)-1-amino-9-ethyl-5-fluoro-9-hydroxy-1,4-dimethyl-1,2,3,9,12,15-hexahydro-10H,13H-benzo[de]pyrano[3',4':6,7]indolizino[1,2-b]quinolin-10,13-dione

(9H-Fluoren-9-yl)methyl ((9S)-9-ethyl-5-fluoro-9-hydroxy-1,4-dimethyl-10,13-dioxo-2,3,9,10,13,15-hexahydro-1H,12H-benzo[de]pyrano[3',4':6,7]indolizino[1,2-b]quinolin-1-yl) carbamate (21.00 mg, 31.26 µmol) was dissolved in N,N-dimethylformamide (1 mL), then added with diethylamine (0.2 mL), the reaction was carried out at room temperature for 0.5 hours and monitored by LCMS. After the reaction solution was distilled under reduced pressure to remove ethylenediamine, the pH was adjusted to 2-3 with 1 mol/L hydrochloric acid aqueous solution, and the reaction solution was directly purified by preparative high performance liquid chromatography to obtain two isomers **5-13-A** (1.30 mg) and **5-13-B** (1.68 mg).
Chromatographic column: SunFire Prep C18 OBD 19 mm×150 mm×5.0 µm
Mobile phase A: acetonitrile; Mobile phase B: water (0.05% formic acid)

| Time [min] | Mobile phase A [%] | Mobile phase B [%] | Flow rate [mL/min] |
|---|---|---|---|
| 0.00 | 10 | 90 | 28 |
| 3.00 | 10 | 90 | 28 |
| 18.00 | 90 | 10 | 28 |

The structural characterization data of **5-13-A** (6 min LCMS, the earlier peak with retention time of 1.373 min) were as follows:
¹H NMR (400 MHz, DMSO-d₆) δ 7.88 (d, *J* = 10.6 Hz, 1H), 7.36 (s, 1H), 6.58 (s, 1H), 5.60 (d, *J* = 3.5 Hz, 2H), 5.46 (d, *J =* 2.5 Hz, 2H), 3.25-3.17 (m, 2H), 2.41 (s, 3H), 2.38-2.28 (m, 2H), 1.91-1.84 (m, 2H), 1.79 (s, 3H)), 0.88 (t, *J=* 7.3 Hz, 3H).
ESI-MS (m/z):450.2[M+H]⁺.

The structural characterization data of **5-13-B** (6 min LCMS, the later peak with a retention time of 1.523 min) were as follows:
¹H NMR (400 MHz, DMSO-d₆) δ 7.76 (d, *J* = 10.8 Hz, 1H), 7.30 (s, 1H), 6.52 (s, 1H), 5.73 (d, *J* = 19.8 Hz, 1H), 5.50 -5.40 (m, 3H), 3.26-3.17 (m, 1H), 3.08-2.96 (m, 1H), 2.38 (s, 3H), 2.19-2.11 (m, 1H), 2.04 (td, *J =* 13.0, 5.1 Hz, 1H), 1.91-1.79 (m, 2H), 1.34 (s, 3H), 0.87 (t, *J* = 7.3 Hz, 3H).
ESI-MS (m/z):450.2[M+H]⁺.
6 min LCMS conditions:
Chromatographic column: Waters SunFire C18 OBD 4.6 mm×50 mm×5.0 µm
Mobile phase A: 0.05% acetonitrile; mobile phase B: water (0.05% formic acid)

| Time [min] | Mobile phase A [%] | Mobile phase B [%] | Flow rate [mL/min] |
|---|---|---|---|
| 0 | 90 | 10 | 2 |
| 4.2 | 10 | 90 | 2 |
| 5.7 | 10 | 90 | 2 |
| 5.71 | 90 | 10 | 2 |
| 6.70 | 90 | 10 | 2 |

### Example 13: (1S,9S)-1-(Aminomethyl)-9-ethyl-5-fluoro-9-hydroxy-4-methyl-1,2,3,9, 12,15-hexahydro-10H,13H-benzo[de]pyrano[3',4':6,7]indolizino[1,2-b]quinolin-10,13-dione and (1R,9S)-1-(aminomethyl)-9-ethyl-5-fluoro-9-hydroxy-4-methyl-1,2,3,9,12,15-hexahydro-10H,13H-benzo[de]pyrano[3',4':6,7]indolizino[l,2-b]quinolin-10,13-dione

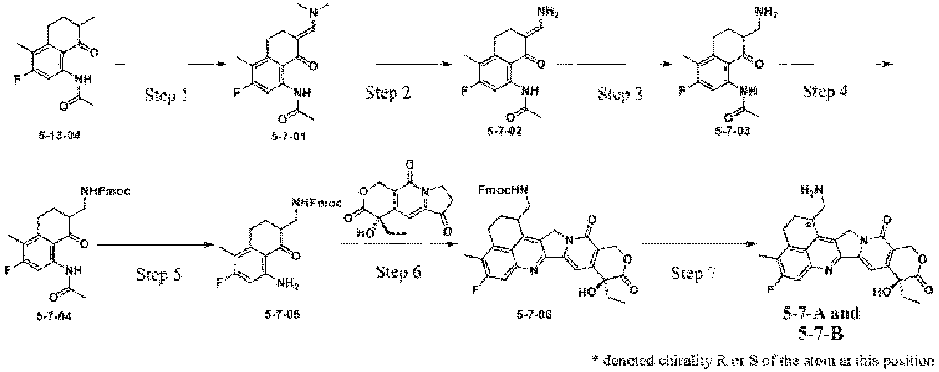

### Step 1: Synthesis of N-(7-((dimethylamino)methylene)-3-fluoro-4-methyl-8-oxo-5,6,7,8-tetrahydronaphthalen-1-yl)acetamide

Compound **5-13-04** (1.00 g, 4.25 mmol) was dissolved in N,N-dimethylformamide dimethyl acetal (10 mL), then heated to 120 °C and reacted for 3 hours, and the reaction was monitored by LCMS. After the reaction solution was cooled to room temperature, it was directly evaporated to dryness under reduced pressure to obtain a crude product, which was purified by flash silica gel column (ethyl acetate:petroleum ether=20%-100%) to obtain 891.00 mg of the title compound.

The structural characterization data were as follows:
ESI-MS (m/z):291.1[M+H]⁺.

### Step 2: Synthesis of N-(7-(aminomethylene)-3-fluoro-4-methyl-8-oxo-5,6,7,8-tetrahydronaphthalen-1 -yl)acetamide

Compound **5-7-01** (0.89 g, 3.07 mmol) was dissolved in ethanol (25 mL), then added with ammonium acetate (2.36 g, 30.65 mmol), after the addition, the reaction was carried out at room temperature for 16 hours and monitored by LCMS. The reaction solution was evaporated to dryness under reduced pressure, then added with dichloromethane (30 mL) and water (20 mL), stirred and allowed to stand to separate the organic phase, dried over anhydrous sodium sulfate, filtered, and the filtrate was evaporated to dryness under reduced pressure to obtain 785.00 mg of the title compound.

The structural characterization data were as follows:
ESI-MS (m/z):263.1[M+H]⁺.

### Step 3: Synthesis of N-(7-(aminomethyl)-3-fluoro-4-methyl-8-oxo-5,6,7,8-tetrahydronaphthalen-1 -yl)acetamide

Compound **5-7-02** (0.80 g, 3.05 mmol) was dissolved in ethanol (200 mL), then added with 10% palladium on carbon (0.40 mg) and concentrated hydrochloric acid (0.2 mL), and the reaction system was subjected to the replacement three times with a hydrogen balloon, and then the reaction was carried out at room temperature under hydrogen atmosphere for 3 hours and monitored by LCMS. The reaction solution was directly filtered, and the filtrate was evaporated to dryness under reduced pressure to obtain 905.00 mg of a hydrochloride of the title compound.

The structural characterization data were as follows:
ESI-MS (m/z):265.1[M+H]⁺.

### Step 4: Synthesis of (9H-fluoren-9-yl)methyl ((8-acetamido-6-fluoro-5-methyl-1-oxo-1,2,3,4-tetrahydronaphthalen-2-yl)methyl)carbamate

The hydrochloride salt of compound **5-7-03** (0.90 g, 2.99 mmol) was dissolved in 1,4-dioxane (20 mL), then added with sodium bicarbonate (1.01 g, 11.97 mmol), water (10 mL) and 9-fluorenylmethyl-N-succinimidyl carbonate (1.21 g, 3.59 mmol), after the addition, the reaction was carried out under stirring at room temperature for 1 hour and monitored by LCMS. The reaction solution was poured into water, then extracted with ethyl acetate, the organic phases were combined, washed with saturated brine, dried over anhydrous sodium sulfate, filtered, and the filtrate was dried under reduced pressure to obtain a crude product. The crude product was purified by flash silica gel column (ethyl acetate:petroleum ether=0-50%) to obtain 1.30 g of the title compound.

The structural characterization data were as follows:
ESI-MS (m/z):487.1[M+H]⁺.

### Step 5: Synthesis of (9H-fluoren-9-yl)methyl ((8-amino-6-fluoro-5-methyl-1-oxo-1,2,3,4-tetrahydronaphthalen-2-yl)methyl)carbamate

Compound **5-7-04** (0.80 g, 1.64 mmol) was dissolved in 1,4-dioxane (20 mL), and added with 3 mol/L hydrochloric acid aqueous solution (20 mL) under nitrogen protection. After addition, the temperature was raised to 60°C, the reaction was carried out for 15 hours and monitored by LCMS. The reaction solution was slowly poured into water, then extracted with ethyl acetate, the organic phases were combined and washed with saturated brine, dried over anhydrous sodium sulfate, filtered, and the filtrate was evaporated to dryness under reduced pressure to obtain a crude product. The crude product was purified by flash silica gel column (ethyl acetate:petroleum ether=0-40%) to obtain 561.00 mg of the title compound.

The structural characterization data were as follows:
ESI-MS (m/z):445.1[M+H]⁺.

### Step 6: Synthesis of (9H-fluoren-9-yl)methyl (((9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl)methyl)carbamate

(S)-4-Ethyl-4-hydroxy-7,8-dihydro-1H-pyrano[3,4-f]indolizin-3,6,10(4H)-trione (597.00 mg, 2.27 mmol) and compound **5-7-05** (840.00 mg, 1.89 mmol) were added to toluene (60 mL), then added with p-toluenesulfonic acid (325.00 mg, 1.89 mmol). After the addition, the temperature was raised to 140°C and the reaction was carried out for 4 hours, then the reaction solution was directly evaporated to dryness under reduced pressure at 140° C to obtain a crude product. The crude product was purified by flash silica gel column (methanol:dichloromethane=0-5%) to obtain 563.00 mg of the title compound.

The structural characterization data were as follows:
ESI-MS (m/z):672.2[M+H]⁺.

### Step 7: Synthesis of (1S,9S)-1-(aminomethyl)-9-ethyl-5-fluoro-9-hydroxy-4-methyl-1,2,3,9, 12,15-hexahydro-10H,13H-benzo[de]pyrano[3',4':6,7]indolizino[1,2-b]quinolin-10,13-dione and (1R,9S)-1-(aminomethyl)-9-ethyl-5-fluoro-9-hydroxy-4-methyl-1,2,3,9,12,15-hexahydro-10H,13H-benzo[de]pyrano[3',4':6,7]indolizino[1,2-b]quinolin-10,13-dione

Compound **5-7-06** (454.00 mg, 675.89 µmol) was dissolved in N,N-dimethylformamide (5 mL), then added with diethylamine (1 mL), and reacted at room temperature for 0.5 hours. The reaction was monitored by LCMS. After ethylenediamine was evaporated from the reaction solution under reduced pressure, the pH was adjusted to 2-3 with formic acid, and the reaction solution was directly purified by preparative high-performance liquid chromatography, and the fractions were lyophilized to obtain the title compounds **5-7-A** (32.00 mg) and **5-7-B** (56.00 mg).
Chromatographic column: SunFire Prep C18 OBD 19 mm×150 mm×5.0 µm
Mobile phase A: acetonitrile; Mobile phase B: water (0.05% formic acid)

| Time [min] | Mobile phase A [%] | Mobile phase B [%] | Flow rate [mL/min] |
|---|---|---|---|
| 0 | 10 | 90 | 28 |
| 3 | 10 | 90 | 28 |
| 18 | 90 | 10 | 28 |

The structural characterization data of **5-7-A** (6 min LCMS, the earlier peak with retention time of 1.488 min) were as follows:
¹H NMR (400 MHz, DMSO-d₆) δ 7.88 (d, *J* = 10.6 Hz, 1H), 7.36 (s, 1H), 6.58 (s, 1H), 5.60 (d, *J* = 3.5 Hz, 2H), 5.46 (d, *J* = 2.5 Hz, 2H), 3.25-3.17 (m, 2H), 2.41 (s, 3H), 2.38-2.28 (m, 2H), 1.91-1.84 (m, 2H), 1.79 (s, 3H)), 0.88 (t, *J* = 7.3 Hz, 3H).
ESI-MS (m/z):450.2[M+H]⁺.

The structural characterization data of **5-7-B** (6 min LCMS, the later peak with a retention time of 1.596 min) are as follows:
¹H NMR (400 MHz, DMSO-d₆) δ 7.76 (d, *J* = 10.8 Hz, 1H), 7.30 (s, 1H), 6.52 (s, 1H), 5.73 (d, *J* = 19.8 Hz, 1H), 5.50 -5.40 (m, 3H), 3.26-3.17 (m, 1H), 3.08-2.96 (m, 1H), 2.38 (s, 3H), 2.19-2.11 (m, 1H), 2.04 (td, *J* = 13.0, 5.1 Hz, 1H), 1.91- 1.79 (m, 2H), 1.34 (s, 3H), 0.87 (t, *J* = 7.3 Hz, 3H).
ESI-MS (m/z):450.2[M+H]⁺.
6 min LCMS conditions:
Chromatographic column: Waters SunFire C18 OBD 4.6 mm×50 mm×5.0 µm
Mobile phase A: 0.05% acetonitrile; mobile phase B: water (0.05% formic acid)

| Time [min] | Mobile phase A [%] | Mobile phase B [%] | Flow rate [mL/min] |
|---|---|---|---|
| 0 | 90 | 10 | 2 |
| 4.2 | 10 | 90 | 2 |
| 5.7 | 10 | 90 | 2 |
| 5.71 | 90 | 10 | 2 |
| 6.70 | 90 | 10 | 2 |

### Example 14: N-((1S,9S)-9-ethyl-5-fluoro-9-hydroxy-1,4-dimethyl-10, 13-dioxo-2,3,9, 10, 13,15-hexahydro-1H,12H-benzo[de]pyrano[3',4':6,7]indolizino[1,2-b]quinolin-1-yl)-1-hydroxycyclopropane-1-carboxamide or N-((1R,9S)-9-ethyl-5-fluoro-9-hydroxy-1,4-dimethyl-10,13- dioxo-2,3,9,10,13,15-hexahydro-1H,12H-benzo[de]pyrano[3',4':6,7]indolizino[1,2-b]quinolin-1-yl)-1-hydroxycyclopropane-1-carboxamide

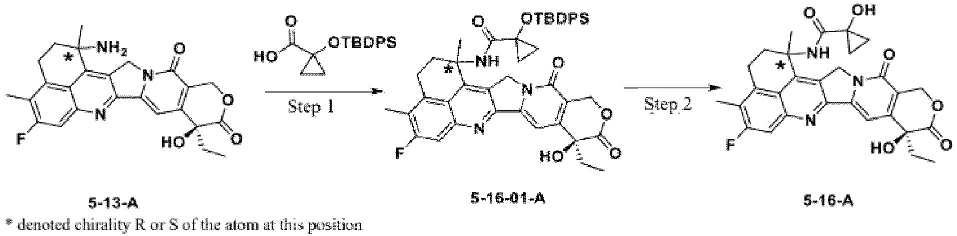

### Step 1: Synthesis of 1-((tert-butyldiphenylsilyl)oxy)-N-((1S,9S)-9-ethyl-5-fluoro-9-hydroxy-1,4-dimethyl-10,13-dioxo-2,3,9,10,13,15-hexahydro-1H,12H-benzo[de]pyrano[3',4':6,7]indolizino[1,2-b]quinolin-1-yl)cyclopropane-1-carboxamide or 1-((tert-butyldiphenylsilyl)oxy)- N-((1R,9S)-9-ethyl-5-fluoro-9-hydroxy-1,4-dimethyl-10,13-dioxo-2,3,9,10,13,15-hexahydro-1H,12H-benzo[de]pyrano[3',4':6,7]indolizino[1,2-b]quinolin-1-yl)cyclopropane-1 -carboxamide

Compound **5-13-A** (10.00 mg, 22.25 µmol) in single configuration and 1-((tert-butyldiphenylsilyl)oxy)cyclopropane-1-carboxylic acid (11.36 mg, 33.37 µmol) were dissolved in N,N-dimethylformamide (1 mL), then added with HATU (12.68 mg, 33.37 µmol) and N,N-diisopropylethylamine (8.63 mg, 66.74 µmol), after the addition, the reaction was carried out at room temperature for 0.5 hour and monitored by LCMS. The reaction solution was directly purified by C18 reverse phase column (acetonitrile: 0.05% formic acid aqueous solution = 30%-100%) to obtain the title compound **5-16-01-A** (7 mg) in single configuration.

The structural characterization data were as follows:
ESI-MS (m/z):772.3[M+H]⁺.

### Step 2: Synthesis of N-((1S,9S)-9-ethyl-5-fluoro-9-hydroxy-1,4-dimethyl-10,13-dioxo-2,3,9,10,13,15-hexahydro-1H,12H-benzo[de]pyrano[3',4':6,7]indolizino[1,2-b]quinolin-1-yl)-1-hydroxycyclopropane-1-carboxamide or N-((1R,9S)-9-ethyl-5-fluoro-9-hydroxy-1,4-dimethyl-10,13-dioxo-2,3,9,10,13,15-hexahydro-1H,12H-benzo[de]pyrano[3',4':6,7]indolizino[1,2-b]quinolin-1-yl)-1-hydroxycyclopropane-1-carboxamide

Compound **5-16-01-A** (7.00 mg, 9.07 µmol) was dissolved in N,N-dimethylformamide (1 mL), then added with potassium fluoride (2.63 mg, 45.34 µmol), and then the temperature was raised to 50°C, the reaction was carried out for 1 hour and monitored by LCMS. The reaction solution was directly purified by high performance liquid chromatography to obtain a single stereoisomer of the title compound **5-16-A** (1.73 mg).
Chromatographic column: SunFire Prep C18 OBD 19 mm×150 mm×5.0 µm
Mobile phase A: acetonitrile; Mobile phase B: water (0.05% formic acid)

| Time [min] | Mobile phase A [%] | Mobile phase B [%] | Flow rate [mL/min] |
|---|---|---|---|
| 0.00 | 10 | 90 | 28 |
| 18.00 | 90 | 10 | 28 |

The structural characterization data were as follows:
¹H NMR (400 MHz, DMSO-d₆) δ 8.26 (s, 1H), 7.78 (d, *J* = 10.8 Hz, 1H), 7.30 (s, 1H), 6.56 (s, 1H), 6.52 (s, 1H), 5.52 (d, *J* = 19.3 Hz, 1H), 5.43 (d, *J* = 4.4 Hz, 2H), 4.94 (d, *J* = 19.2 Hz, 1H), 3.30-3.24(m, 1H), 3.11-3.00 (m, 1H), 2.95-2.84 (m, 1H), 2.39 (s, 3H), 1.98-1.80 (m, 3H), 1.62 (s, 3H), 0.87 (t, *J* = 7.9 Hz, 3H).
ESI-MS (m/z):534.2[M+H]⁺.

### Example 15: N-((10S)-10-Benzyl-1-(((1S,9S)-5-chloro-9-ethyl-9-hydroxy-4-methyl-10,13-dioxo-2,3,9,10,13,15-hexahydro-1H,12H-benzo[de]pyrano[3',4';6,7]indolizino[1,2-b]quinolin-1-yl)amino)-1,6,9,12,15-pentaoxo-3-oxa-5,8,11,14-tetraazahexadecan-16-yl)-6-(2-(methylsulfonyl)pyrimidin-5-yl)hexadecanamide or N-((10S)-10-benzyl-1-(((1R,9S)-5-chloro-9-ethyl-9-hydroxy- 4-methyl-10,13-dioxo-2,3,9,10,13,15-hexahydro-1H,12H-benzo[de]pyrano[3',4';6,7]indolizino[1,2-b]quinolin-1-yl)amino)-1,6,9,12,15-pentoxo-3-oxa-5,8,11, 14-tetraazahexadecan-16-yl)-6-(2-(methylsulfonyl)pyrimidin-5-yl)hexadecanamide

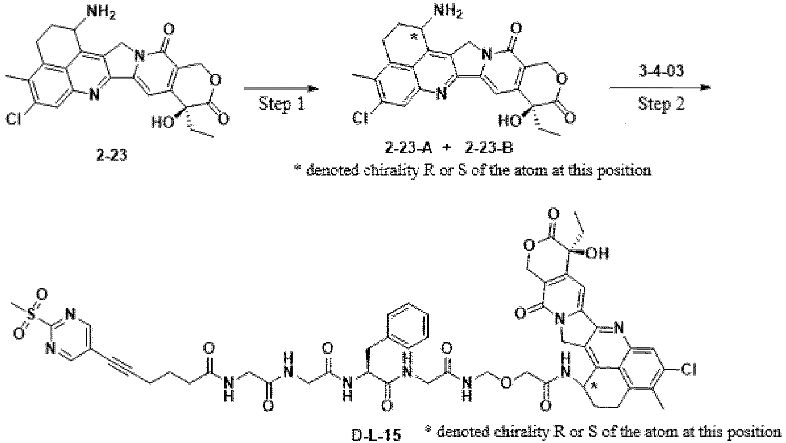

### Step 1: Separation and Purification of (9S)-1-amino-5-chloro-9-ethyl-9-hydroxy-4-methyl-1,2,3,9,12,15-hexahydro-10H,13H-benzopyrano[3',4':6,7]indolizino[1,2-b]quinolin-10,13-dione

Compound **2-23** (16.00 mg) was purified by preparative high-performance liquid chromatography, and two diastereoisomers were separated under the following purification conditions to obtain 5.10 mg of a trifluoroacetic acid salt of **2-23-A** (retention time 9.85 min) and 7.12 mg of a trifluoroacetic acid salt of **2-23-B** (retention time 10.62 min).
Chromatographic column: SunFire Prep C18 OBD 19 mm×150 mm×5.0 µm
Mobile phase A: acetonitrile; mobile phase B: water (0.05% trifluoroacetic acid)

| Time [min] | Mobile phase A [%] | Mobile phase B [%] | Flow rate [mL/min] |
|---|---|---|---|
| 0 | 5 | 95 | 28 |
| 2 | 5 | 95 | 28 |
| 18 | 50 | 50 | 28 |

The structural characterization data were as follows:
**2-23-A:**
   ¹H NMR (400 MHz, DMSO-d₆) δ 8.42 (s, 3H), 8.27 (s, 1H), 7.36 (s, 1H), 6.59 (s, 1H), 5.78-5.63 (m, 1H), 5.50- 5.36 (m, 3H), 5.10-5.06 (m, 1H), 3.20-3.04 (m, 2H), 2.56 (s, 3H), 2.26-2.13 (m, 2H), 1.93-1.79 (m, 2H), 0.88 (t, *J* = 7.2 Hz, 3H).
   ESI-MS (m/z):452.1[M+H]⁺.
**2-23-B:**
   ¹H NMR (400 MHz, DMSO-d₆) δ 8.42 (s, 3H), 8.27 (s, 1H), 7.36 (s, 1H), 6.58 (s, 1H), 5.78-5.63 (m, 1H), 5.50- 5.36 (m, 3H), 5.10-5.06 (m, 1H), 3.20-3.04 (m, 2H), 2.55 (s, 3H), 2.26-2.13 (m, 2H), 1.93-1.79 (m, 2H), 0.88 (t, *J* = 7.2 Hz, 3H).
   ESI-MS (m/z):452.0[M+H]⁺.

### Step 2: Synthesis of N-((10S)-10-benzyl-l-(((lS,9S)-5-chloro-9-ethyl-9-hydroxy-4-methyl-10,13-dioxo-2,3,9,10,13,15-hexahydro-1H,12H-benzo[de]pyrano[3',4';6,7]indolizino[1,2-b] quinolin-1-yl)amino)-1,6,9,12,15-pentaoxo-3-oxa-5,8,11,14-tetraazahexadecan-16-yl)-6-(2-(methylsulfonyl)pyrimidin-5-yl)hexadecanamide or N-((10S)-10-benzyl-1-(((1R,9S)-5-chloro-9-ethyl-9- hydroxy-4-methyl-10,13-dioxo-2,3,9,10,13,15-hexahydro-1H,12H-benzo[de]pyrano[3',4';6,7]indolizino[1,2-b]quinolin-1-yl)amino)-1,6,9,12,15-pentoxo-3-oxa-5, 8,11,14-tetraazahexadecan-16-yl)-6-(2-(methylsulfonyl)pyrimidin-5-yl)hexadecanamide

At 25°C, the trifluoroacetic acid salt of **2-23-A** (34.71 mg, 61.43 µmol) was dissolved in N,N-dimethylformamide (1 mL), added sequentially with **3-4-03** (49.66 mg, 73.72 µmol), HATU (35.01 mg, 92.14 µmol) and N,N-diisopropylethylamine (23.82 mg, 184.29 µmol), reacted at 25°C for 0.5 hours, and the reaction was monitored by LCMS, when the reaction was completed, the reaction solution was purified by preparative high-performance liquid chromatography (conditions were as follows), and the fractions were lyophilized to obtain 11.04 mg of the title compound **D-L-15** with a retention time of 7.5 min.
Chromatographic column: SunFire Prep C18 OBD 19 mm×150 mm×5.0 µm
Mobile phase A: acetonitrile; Mobile phase B: water (0.05% formic acid)

| Time [min] | Mobile phase A [%] | Mobile phase B [%] | Flow rate [mL/min] |
|---|---|---|---|
| 0 | 30 | 70 | 28 |
| 3 | 30 | 70 | 28 |
| 18 | 90 | 10 | 28 |

The structural characterization data were as follows:
**D-L-15:**
ESI-MS (m/z):1107.3[M+H]⁺.

### Example 16: N-((9S)-4-chloro-9-ethyl-5-fluoro-9-hydroxy-10,13-dioxo-2,3,9,10,13,15-hexahydro-1H,12H-benzo[de]pyrano[3',4':6,7]indolizino[1,2-b]quinolin-1-yl)-2-cyclopropyl-2-hydroxy acetamide

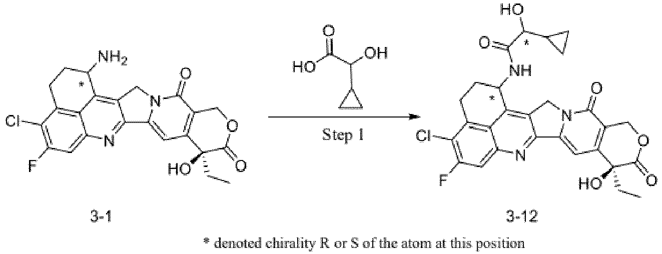

### Step 1: Synthesis of N-((9S)-4-chloro-9-ethyl-5-fluoro-9-hydroxy-10,13-dioxo-2,3,9,10, 13,15-hexahydro-1H,12H-benzo[de]pyrano[3',4':6,7]indolizino[1,2-b]quinolin-1-yl)-2-cyclopropyl-2-hydroxyacetamide

The formate salt of single stereoisomer compound **3-1-A** (50 mg, 109.68 µmol) and 2-cyclopropyl-2-hydroxyacetic acid (25.47 mg, 219.36 µmol) were dissolved in N,N-dimethylformamide (2 mL), then added with HATU (7.57 mg, 219.36 µmol) and N,N-diisopropylethylamine (42.53 mg, 329.04 µmol), after the addition, the reaction was carried out at room temperature for 0.5 hours and monitored by LCMS; the reaction solution was directly purified by preparative high-performance liquid chromatography to obtain two isomers of the title compound (**3-12-A**: 12.96 mg, **3-12-B:** 13.56 mg).
Chromatographic column: SunFire Prep C18 OBD 19mm×150mm×5.0µm
Mobile phase A: acetonitrile; Mobile phase B: water (0.05% formic acid)

| Time [min] | Mobile phase A [%] | Mobile phase B [%] | Flow rate [mL/min] |
|---|---|---|---|
| 0.00 | 30 | 90 | 28 |
| 3.00 | 30 | 90 | 28 |
| 18.00 | 90 | 10 | 28 |

The structural characterization data of **3-12-A** (6 min LCMS, the earlier peak) were as follows:
¹H NMR (400 MHz, DMSO-d₆) δ 8.44 (d, *J* = 9.0 Hz, 1H), 8.05 (d, *J* = 10.2 Hz, 1H), 7.33 (s, 1H), 6.54 (s, 1H), 5.62 (q, *J* = 6.7 Hz, 1H), 5.52 (d, *J=* 5.1 Hz, 1H), 5.42 (s, 2H), 5.24 (q, *J* = 19.2 Hz, 2H), 3.61 (dd, *J* = 6.2, 5.1 Hz, 1H), 3.32 - 3.21 (m, 2H), 2.19 (q, *J* = 6.5 Hz, 2H), 1.92 - 1.80 (m, 2H), 1.26 - 1.20 (m, 1H), 0.87 (t, *J* = 7.3 Hz, 3H), 0.57 - 0.34 (m, 4H).
ESI-MS (m/z):554.0[M+H]⁺.

The structural characterization data of **3-12-B** (6 min LCMS, the later peak) were as follows:
¹H NMR (400 MHz, DMSO-d₆) δ 8.43 (d, *J* = 8.7 Hz, 1H), 8.06 (d, *J* = 10.3 Hz, 1H), 7.33 (s, 1H), 5.57 (q, *J =* 6.7 Hz, 1H), 5.43 (s, 2H), 5.30 - 5.17 (m, 2H), 3.64 (d, *J* = 6.2 Hz, 1H), 3.29 (q, *J* = 6.7 Hz, 2H), 2.28 - 2.13 (m, 2H), 1.93 - 1.78 (m, 2H), 1.18 - 1.08 (m, 1H), 0.87 (t, *J* = 7.3 Hz, 3H), 0.50 - 0.29 (m, 4H).
ESI-MS (m/z):554.0[M+H]⁺.

The formate salt of another single stereoisomer compound **3-1-B** (50 mg, 109.68 µmol) and 2-cyclopropyl-2-hydroxyacetic acid (25.47 mg, 219.36 µmol) were dissolved in N,N-dimethylformamide (2 mL), then added with HATU (7.57 mg, 219.36 µmol) and N,N-diisopropylethylamine (42.53 mg, 329.04 µmol), after the addition, the reaction was carried out at room temperature for 0.5 hours and monitored by LCMS; the reaction solution was directly purified by preparative high performance liquid chromatography to obtain two isomers of the title compound (**3-12-C**: 20.19 mg, **3-12-D:** 18.33 mg).
Chromatographic column: SunFire Prep C18 OBD 19mm×150mm×5.0µm
Mobile phase A: acetonitrile; Mobile phase B: water (0.05% formic acid)

| Time [min] | Mobile phase A [%] | Mobile phase B [%] | Flow rate [mL/min] |
|---|---|---|---|
| 0.00 | 30 | 90 | 28 |
| 3.00 | 30 | 90 | 28 |
| 18.00 | 90 | 10 | 28 |

The structural characterization data of **3-12-C** (6 min LCMS, the earlier peak) were as follows:
¹H NMR (400 MHz, DMSO-d₆) δ 8.47 (d, *J* = 9.0 Hz, 1H), 8.06 (d, *J* = 10.3 Hz, 1H), 7.33 (s, 1H), 6.54 (s, 1H), 5.62 (q, *J* = 6.5 Hz, 1H), 5.53 (d, *J* = 5.1 Hz, 1H), 5.43 (s, 2H), 5.32 - 5.16 (m, 2H), 3.61 (dd, *J* = 6.3, 5.1 Hz, 1H), 3.32 - 3.22 (m, 2H), 2.19 (q, *J* = 6.5 Hz, 2H), 1.92 - 1.80 (m, 2H), 1.28 - 1.20 (m, 1H), 0.87 (t, *J* = 7.3 Hz, 3H), 0.54 - 0.35 (m, 4H).
ESI-MS (m/z):554.0[M+H]⁺.

The structural characterization data of **3-12-D** (6 min LCMS, the later peak) were as follows:
¹H NMR (400 MHz, DMSO-d₆) δ 8.44 (d, *J* = 8.8 Hz, 1H), 8.05 (d, *J* = 10.2 Hz, 1H), 7.34 (s, 1H), 6.55 (s, 1H), 5.58 (q, *J* = 6.7 Hz, 1H), 5.45 (d, *J* = 5.2 Hz, 1H), 5.43 (s, 2H), 5.31 - 5.14 (m, 2H), 3.65 (t, *J* = 5.7 Hz, 1H), 3.33 - 3.21 (m, 2H), 2.28 - 2.13 (m, 2H), 1.95 - 1.80 (m, 2H), 1.16 - 1.09 (m, 1H), 0.88 (t, *J* = 7.3 Hz, 3H), 0.46 - 0.31 (m, 4H).
ESI-MS (m/z):554.0[M+H]⁺.

### Example 17: Preparation of (S)-N-((1S,9S)-4-Chloro-9-ethyl-5-fluoro-9-hydroxy-10,13-dioxo-2,3,9,10,13,15-hexahydro-1H,12H-benzo[de]pyrano[3',4':6,7]indolizino[1,2-b]quinolin-1-yl)-2-hydroxypropylamine and (S)-N-((1R,9S)-4-chloro-9-ethyl-5-fluoro-9-hydroxy-10,13-dioxo-2,3,9,10,13,15-hexahydro-1H,12H-benzo[de]pyrano[3',4':6,7]indolizino[1,2-b]quinolin-1-yl)-2-hydroxypropylamine

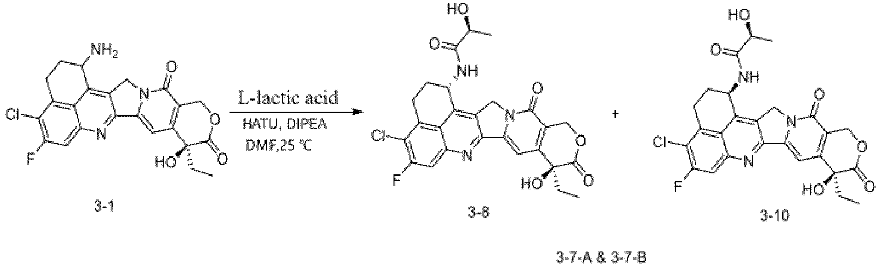

At 25°C, (9S)-1-amino-4-chloro-9-ethyl-5-fluoro-9-hydroxy-2,3,12,15-tetrahydrobenzo[de] pyrano[3',4':6,7]indolizino[1,2-b]quinolin-10,13(1H,9H)-dione (80.0 mg, 175.5 µmol) and L-lactic acid (31.6 mg, 351.0 µmol) were dissolved in DMF (3 mL), then added with HATU (121.1 mg, 351.0 µmol) and DIPEA (68.0 mg, 526.5 µmol), and reacted at room temperature for 2 hour; the reaction solution was directly purified by preparative high-performance liquid chromatography to obtain compound **3-7-A** (6.1 mg, yield 12%) and compound **3-7-B** (9.6 mg, yield 20%).
Chromatographic column: SunFire Prep C18 OBD 19mm×150mm×5.0µm
Mobile phase A: acetonitrile; Mobile phase B: water (0.05% formic acid)

| Time [min] | Mobile phase A [%] | Mobile phase B [%] | Flow rate [mL/min] |
|---|---|---|---|
| 0.00 | 30 | 70 | 24 |
| 2.00 | 30 | 70 | 24 |
| 18.00 | 90 | 10 | 24 |

The structural characterization data of compound **3-7-A** (6 min LC-MS, the earlier peak with a retention time of 2.49 min) were as follows:
MS m/z (ESI): 528.2 [M+H]⁺
¹H NMR (400 MHz, DMSO-d₆) δ 8.55 (d, *J* = 9.2 Hz, 1H), 8.06 (d, *J* = 10.4 Hz, 1H), 7.33 (s, 1H), 6.55 (s, 1H), 6.67 (d, *J* = 4.8 Hz, 1H), 5.65 - 5.59 (m, 1H), 5.43 (s, 2H), 5.29 - 5.21 (m, 1H), 5.14 - 5.10 (m, 1H), 4.15 - 4.10 (m,, 1H), 3.27 - 3.20 (m, 1H), 2.22 - 2.15 (m, 2H), 1.92 - 1.81 (m, 2H), 1.41 (d, *J* = 6.8 Hz, 3H), 1.30 - 1.23 (m, 1H), 0.89 - 0.85 (t, *J* = 7.2 Hz, 3H).

The structural characterization data of compound **3-7-B** (6 min LC-MS, the later peak with a retention time of 2.50 min) were as follows:
MS m/z (ESI): 528.2 [M+H]⁺
¹H NMR (400 MHz, DMSO-d₆) δ 8.46 (d, *J* = 8.8 Hz, 1H), 8.06 (d, *J* = 10.4 Hz, 1H), 7.33 (s, 1H), 6.56 (s, 1H), 5.60 - 5.53 (ms, 1H), 5.51 (d, *J* = 5.2 Hz, 1H), 5.43 (s, 2H), 5.27 - 5.14 (m, 2H), 4.16 - 4.08 (m, 1H), 3.28 - 3.22 (m, 1H), 2.22 - 2.19 (m, 2H), 1.92 - 1.81 (m, 2H), 1.49 - 1.39 (m, 1H), 1.29 (d, *J* = 6.8 Hz, 3H), 0.87 (t, *J* = 7.2 Hz, 3H).

### Example 18: Synthesis of N-((1S,9S)-4-chloro-9-ethyl-5-fluoro-9-hydroxy-10,13-dioxo-1,2,3,9,10,12,13,15-octahydrobenzo[de]pyrano[3',4':6,7]indolizino[1,2-b]quinolin-1-yl-1-hydroxycyclopropylcarboxamide and N-((1R,9S)-4-chloro-9-ethyl-5-fluoro-9-hydroxy- 10,13-dioxo-1,2,3,9,10,12,13,15-octahydrobenzo[de]pyrano[3',4':6,7]indolizino[1,2-b]quinolin-1-yl-1-hydroxycyclopropylcarboxamide

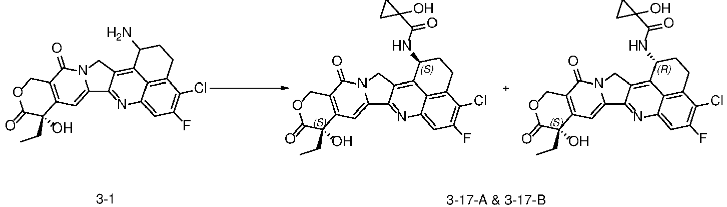

At 25°C, (9S)-1-amino-4-chloro-9-ethyl-5-fluoro-9-hydroxy-2,3,12,15-tetrahydrobenzo[de] pyrano[3',4':6,7]indolizino[1,2-b]quinolin-10,13(1H,9H)-dione (80 mg, 175.49 µmol) and 1-hydroxycyclopropanecarboxylic acid (35.83 mg, 350.98 µmol,) were dissolved in DMF (2 mL), then added with HATU (121.14 mg, 350.98 µmol) and DIPEA (68.04 mg, 526.47 µmol), after the addition, the reaction was carried out at room temperature for 0.5 hour and monitored by HPLC-MS; the reaction solution was concentrated to dryness and directly purified by preparative high-performance liquid chromatography to obtain the title compounds **3-17-A** (5.3 mg) and **3-17-B** (3.5 mg).
Chromatographic column: SunFire Prep C18 OBD 19mm×150mm×5.0µm
Mobile phase A: acetonitrile; Mobile phase B: water (0.05% formic acid)

| Time [min] | Mobile phase A [%] | Mobile phase B [%] | Flow rate [mL/min] |
|---|---|---|---|
| 0.00 | 15 | 85 | 28 |
| 2.00 | 15 | 85 | 28 |
| 18.00 | 90 | 10 | 28 |

The structural characterization data were as follows:
The structural characterization data of **3-17-A** (6 min LCMS, the earlier peak with a retention time of 2.657 min) were as follows:
ESI-MS (m/z):540.0[M+H]⁺.
¹H NMR (400 MHz, DMSO-d₆) δ 8.69 (d, *J* = 8.8 Hz, 1H), 8.06 (d, *J* = 10.2 Hz, 1H), 7.35 (s, 1H), 6.57 (s, 1H), 6.32 (s, 1H), 5.62 (s, 1H), 5.45 (s, 2H), 5.34 - 5.24 (m, 1H), 5.20 - 5.10(m, 1H), 2.26 (s, 2H), 2.00 (s, 1H), 1.88 (s, 2H), 1.47 - 1.12 (m, 8H), 1.01 - 0.80(m, 6H).

The structural characterization data of **3-17-A** (6 min LCMS, the later peak with a retention time of 2.724 min) were as follows:
ESI-MS (m/z):540.0[M+H]⁺.
¹H NMR (400 MHz, DMSO-d₆) δ 8.69 (d, *J* = 8.8 Hz, 1H), 8.06 (d, *J* = 10.2 Hz, 1H), 7.35 (s, 1H), 6.57 (s, 1H), 6.32 (s, 1H), 5.62 (s, 1H), 5.45 (s, 2H), 5.34 - 5.24 (m, 1H), 5.20 - 5.10(m, 1H), 2.26 (s, 2H), 2.00 (s, 1H), 1.88 (s, 2H), 1.47 - 1.12 (m, 8H), 1.01 - 0.80(m, 6H).

### Example 19: Synthesis of N-((1S,9S)-4-cyclopropyl-9-ethyl-5-fluoro-9-hydroxy-10,13-dioxo-1,2,3,9,10,12,13,15-tetrahydrobenzo[de]pyrano[3',4':6,7]indolizino[1,2-b]quinolin-1-yl)-2-hydroxy acetamide

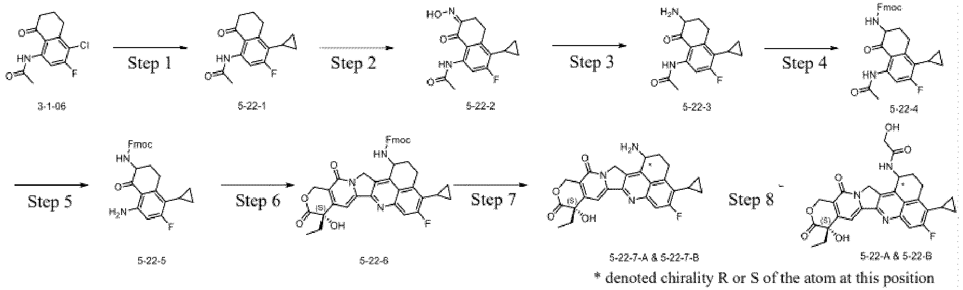

### Step 1: Synthesis of N-(4-chloro-3-fluoro-7-(hydroxyimino)-8-oxo-5,6,7,8-tetrahydronaphthalen-1-yl)acetamide

N-(4-Chloro-3-fluoro-8-oxo-5,6,7,8-tetrahydronaphthalen-1-yl)acetamide (570 mg, 2.23 mmol) and cyclopropylboronic acid (574.56 mg, 6.69 mmol) were dissolved in 1,4-dioxane, then added with dichlorodi-tert-butyl-(4-dimethylaminophenyl)phosphine palladium (II) (480 mg, 677.97 µmol) and cesium carbonate (2.17 g, 6.69 mmol); under nitrogen protection, the reaction was carried out in microwaves at 115 °C for 2 hours and monitored by LCMS; the reaction solution was diluted with ethyl acetate and filtered, the filtrate was extracted with ethyl acetate (30ml*3), the organic phases were combined, washed with saturated brine (50 mL), dried over anhydrous sodium sulfate, and filtered, the filtrate was evaporated to dryness under reduced pressure, and the resultant crude product was purified by column chromatography silica gel column (PE:EA=1:4) to obtain 550 mg of the title compound.

The structural characterization data were as follows:
ESI-MS (m/z):262.1[M+H]⁺.

### Step 2: Synthesis of N-(4-cyclopropyl-3-fluoro-7-(hydroxyimino)-8-oxo-5,6,7,8-tetrahydronaphthalen-1-yl)acetamide

Tetrahydrofuran (30 mL) and tert-butanol (10 mL) were added to a reaction flask, cooled to 5°C in an ice bath, then added with potassium tert-butoxide (945 mg, 8.42 mmol), then N-(4-chloro-3-fluoro-7-(hydroxyimino)-8-oxo-5,6,7,8- tetrahydronaphthalen-1-yl)acetamide (1.0 g, 3.83 mmol) was dissolved in tetrahydrofuran (1 mL) and slowly added thereto in dropwise manner, followed by an addition of isoamyl nitrite (718 mg, 6.12 mmol) after 10 minutes, after the addition, the reaction was carried out at 5°C for 1 hour and monitored by LCMS; the reaction solution was quenched with saturated ammonium chloride aqueous solution (50 mL), extracted with ethyl acetate (40ml*3), the organic phases were combined, washed with saturated brine (50 mL), dried over anhydrous sodium sulfate, and then filtered, and the filtrate was evaporated to dryness under reduced pressure to obtain 1.2 g of a crude product of the title compound.

The structural characterization data were as follows:
ESI-MS (m/z):291.1 [M+H]⁺.

### Step 3: Synthesis of N-(7-amino-4-cyclopropyl-3-fluoro-8-oxo-5,6,7,8-tetrahydronaphthalen-1-yl)acetamide hydrochloride

The crude product of N-(4-cyclopropyl-3-fluoro-7-(hydroxyimino)-8-oxo-5,6,7,8-tetrahydronaphthalen-1-yl)acetamide (1.2 g, 1.41 mmol) was dissolved in methanol (7.5 mL) and tetrahydrofuran (7.5 mL), then added with 1 mol/L hydrochloric acid aqueous solution (7.5 mL) and 10% palladium on carbon (450 mg), after the addition, the reaction system was subjected to replacement three times with a hydrogen balloon, and the reaction was carried out at room temperature under hydrogen atmosphere for 1 hour and monitored by LCMS; the reaction solution was filtered, and the filtrate was concentrated to dryness under reduced pressure to obtain 1.05 g of a crude product.

The structural characterization data were as follows:
ESI-MS (m/z):277.1[M+H]⁺.

### Step 4: Synthesis of (9H-fluoren-9-yl)methyl(8-acetamide-5-cyclopropyl-6-fluoro-1-oxo-1,2,3,4-tetrahydronaphthalen-2-yl)carbamate

Crude product of N-(7-amino-4-cyclopropyl-3-fluoro-8-oxo-5,6,7,8-tetrahydronaphthalen- 1-yl)acetamide hydrochloride (1.05 g, 3.80 mmol) was dissolved in 1,4-dioxane (10 mL), then added with sodium bicarbonate (1.3 g, 15.20 mmol), water (10 mL) and 9-fluorenylmethyl- N-succinimidyl carbonate (1.54 g, 4.56 mmol), after the addition, the reaction was carried out under stirring at room temperature for 2 hours and monitored by LCMS; the reaction solution was poured into water (50 mL), and then extracted with ethyl acetate (40 mL *3), the organic phases were combined, washed with saturated brine (50 mL), dried over anhydrous sodium sulfate, and filtered, and the filtrate was dried under reduced pressure to obtain a crude product. The crude product was purified by C18 reverse phase column to obtain 2.0 g of the title compound.

The structural characterization data were as follows:
ESI-MS (m/z):499.2[M+H]⁺.

### Step 5: Synthesis of (9H-fluoren-9-yl)methyl(8-amino-5-cyclopropyl-6-fluoro-1-oxo-1,2,3,4-tetrahydronaphthalen-2-yl)carbamate

(9H-Fluoren-9-yl)methyl(8-acetamido-5-cyclopropyl-6-fluoro-1-oxo-1,2,3,4-tetrahydronaphthalen-2-yl)carbamate (2.0 g, 3.21 mmol, 80%) was dissolved in dioxane (20 mL), then added with 12 mol/L concentrated hydrochloric acid (5 mL), after the addition, the temperature was raised to 70°C, the reaction was carried out for 2 hours and monitored by LCMS; the reaction solution was poured into water (40 mL), then extracted with ethyl acetate (30 mL*3), the organic phases were combined, washed with saturated brine (40 mL), dried over anhydrous sodium sulfate, and filtered, and the filtrate was dried under reduced pressure to obtain a crude product. The crude product was purified by column chromatography silica gel column (PE:EA=2:1) to obtain 740 mg of the title compound.

The structural characterization data were as follows:
ESI-MS (m/z):457.3[M+H]⁺.

### Step 6: Synthesis of (9H-fluoren-9-yl)methyl ((9S)-4-cyclopropyl-9-ethyl-5-fluoro-9-hydroxy-10,13-dioxo-2,3,9,10,13,15-hexahydro-1H,12H-benzo[de]pyran[3',4:6,7]indolizino[1,2-b]quinolin-1-yl)carbamate

(S)-4-Ethyl-4-hydroxyl-7,8-dihydro-1H-pyrano[3,4-f]indolizin-3,6,10(4H)-trione (442 mg, 1.68 mmol) and (9H-fluoren-9-yl)methyl(8-amino-5-cyclopropyl-6-fluoro-1-oxo-1,2,3,4-tetrahydronaphthalen-2-yl)carbamate (640 mg, 1.40 mmol) were added to toluene (30 mL), and then added with p-toluenesulfonic acid (242 mg, 1.40 mmol), after the addition, the temperature was raised to 135°C, the reaction was carried out for 2 hours. The reaction solution was directly evaporated to dryness at 140°C under reduced pressure to obtain a crude product; and the crude product was purified by column chromatography silica gel column (DCM:MeOH=33:1) to obtain 1.02 g of the title compound.

The structural characterization data were as follows:
ESI-MS (m/z):684.1[M+H]⁺.

### Step 7: Synthesis of (1S,9S)-1-amino-4-cyclopropyl-9-ethyl-5-fluoro-9-hydroxy-1,2,3,9, 12.15-hexahydro-10H,13H-benzo[de]pyrano[3',4':6,7]indolizino[1,2-b]quinolin-10,13-dione (Compound 5-29-1) and (1R,9S)-1-amino-4-cyclopropyl-9-ethyl-5-fluoro-9-hydroxy-1,2,3,9, 12,15-hexahydro-10H,13H-benzo[de ]pyrano[3',4':6,7]indolizino[1,2-b]quinolin-10,13-dione

(9H-Fluoren-9-yl)methyl ((9S)-4-cyclopropyl-9-ethyl-5-fluoro-9-hydroxy-10,13-dioxo-2, 3,9,10,13,15-hexahydro-1H,12H-benzo[de]pyran[3',4:6,7]indolizino[1,2-b]quinolin-1-yl)carbamate (1.02 g, 1.49 mmol) were dissolved in N,N-dimethylformamide (15 mL), then added with diethylamine (5 ml), after the addition, the reaction was carried out at room temperature for 0.5 hours and monitored by LCMS; the reaction solution was distilled under reduced pressure to remove ethylenediamine, then adjusted to pH=2-3 with 1 mol/L hydrochloric acid aqueous solution, the reaction solution was then directly purified by preparative high-performance liquid chromatography to obtain two isomers of the title compound (**5-22-7-A:** 60 mg; **5-22-7-B:** 55 mg).
Chromatographic column: SunFire Prep C18 OBD 19mm×150mm×5.0µm
Mobile phase A: acetonitrile; Mobile phase B: water (0.05% formic acid)

| Time [min] | Mobile phase A [%] | Mobile phase B [%] | Flow rate [mL/min] |
|---|---|---|---|
| 0.00 | 10 | 90 | 28 |
| 3.00 | 10 | 90 | 28 |
| 18.00 | 70 | 30 | 28 |

The structural characterization data of **5-22-7-A** (6 min LCMS, the earlier peak with a retention time of 2.28 min) were as follows:
ESI-MS (m/z):462.2[M+H]⁺.

The structural characterization data of **5-22-7-B** (6 min LCMS, the later peak with a retention time of 2.35 min) were as follows:
ESI-MS (m/z):462.2[M+H]⁺.

### Step 8: Synthesis of N-((1S,9S)-4-cyclopropyl-9-ethyl-5-fluoro-9-hydroxy-10,13-dioxo-1,2, 3,9,10,12,13,15-tetrahydrobenzo[de]pyrano[3',4':6,7]indolizino[1,2-b]quinolin-1-yl)-2-hydroxyacetamide and N-((1R,9S)-4-cyclopropyl-9-ethyl-5-fluoro-9-hydroxy-10,13-dioxo-1,2,3,9,10,12, 13,15-tetrahydrobenzo[de]pyrano[3',4':6,7]indolizino[1,2-b]quinolin-1-yl)-2-hydroxyacetamide

At 25°C, single stereoisomer compound **5-28-7-A** (40 mg, 86 µmol) and glycolic acid (8 mg, 104 µmol) were dissolved in DMF (2 mL), then added with HATU (40 mg, 104 µmol) and DIPEA (36 mg, 258 µmol), after the addition, the reaction was carried out at room temperature for 0.5 hr and monitored by LCMS; the reaction solution was directly purified by preparative high-performance liquid chromatography to obtain 12.5 mg of compound **5-22-A.**
Chromatographic column: SunFire Prep C18 OBD 19mm×150mm×5.0µm
Mobile phase A: acetonitrile; Mobile phase B: water (0.05% formic acid)

| Time [min] | Mobile phase A [%] | Mobile phase B [%] | Flow rate [mL/min] |
|---|---|---|---|
| 0.00 | 10 | 90 | 28 |
| 2.00 | 10 | 90 | 28 |
| 18.00 | 90 | 10 | 28 |

The structural characterization data were as follows:
The structural characterization data of **5-22-A** (6 min LCMS, the earlier peak with a retention time of 2.540 min) were as follows:
ESI-MS (m/z):520.0[M+H]⁺.
¹H NMR (400 MHz, DMSO-d₆) δ 8.46 (d, *J* = 8.9 Hz, 1H), 7.74 (d, *J* = 11.9 Hz, 1H), 7.30 (s, 1H), 6.53 (s, 1H), 5.64 - 5.56 (m, 1H), 5.49 (t, *J* = 5.8 Hz, 1H), 5.42 (s, 2H), 5.19 (s, 2H), 3.96 (d, *J =* 5.7 Hz, 2H), 2.25 - 2.10 (m, 2H), 2.04 - 1.79 (m, 4H), 1.23 (s, 2H), 1.15 - 1.05 (m, 2H), 0.87 (t, *J* = 7.2 Hz, 3H), 0.80 - 0.70 (m, 2H).

At 25°C, single stereoisomer compound **5-28-7-B** (30 mg, 65 µmol) and glycolic acid (6 mg, 78 µmol) were dissolved in DMF (2 mL), then added with HATU (40 mg, 104 µmol) and DIPEA (17 mg, 130 µmol), after the addition, the reaction was carried out at room temperature for 0.5 hr and monitored by LCMS; the reaction solution was concentrated to dryness and directly purified by preparative high-performance liquid chromatography to obtain 13.83 mg of compound **5-22-B.**
Chromatographic column: SunFire Prep C18 OBD 19mm×150mm×5.0µm
Mobile phase A: acetonitrile; Mobile phase B: water (0.05% formic acid)

| Time [min] | Mobile phase A [%] | Mobile phase B [%] | Flow rate [mL/min] |
|---|---|---|---|
| 0.00 | 10 | 90 | 28 |
| 2.00 | 10 | 90 | 28 |
| 18.00 | 90 | 10 | 28 |

The structural characterization data were as follows:
The structural characterization data of **5-22-B** (6 min LCMS, the earlier peak with a retention time of 2.612 min) were as follows:
ESI-MS (m/z):520.0[M+H]⁺.
¹H NMR (400 MHz, DMSO-d₆) δ 8.49 (d, *J* = 8.9 Hz, 1H), 7.74 (d, *J* = 11.9 Hz, 1H), 7.31 (s, 1H), 6.53 (s, 1H), 5.60 (s, 1H), 5.51 (t, *J* = 5.9 Hz, 1H), 5.43 (s, 2H), 5.25 - 5.13 (m, 2H), 3.97 (d, *J=* 5.8 Hz, 2H), 2.18 (s, 2H), 2.04 - 1.91 (m, 4H), 1.90 - 1.80 (m, 1H), 1.23 (s, 6H), 1.15 - 1.05(m, 2H), 0.87 (t, *J* = 7.2 Hz, 4H), 0.80 - 0.70 (m, 2H).

### Example 20: Synthesis of (R)-3-(dimethylamino)-N-((1S,9S)-9-ethyl-5-fluoro-9-hydroxy- 4-methyl-10,13-dioxo-1,2,3,9,10,12,13,15-octahydrobenzo[de]pyrano[3',4':6,7]indolizino[1,2-b]quinolin-1-yl)-2-hydroxypropionamide and (S)-3-(dimethylamino)-N-((1S,9S)-9-ethyl-5-fluoro- 9-hydroxy-4-methyl-10,13-dioxo-1,2,3,9,10,12,13,15-octahydrobenzo[de]pyrano[3',4':6,7]indolizino[1,2-b]quinolin-1-yl)-2-hydroxypropionamide

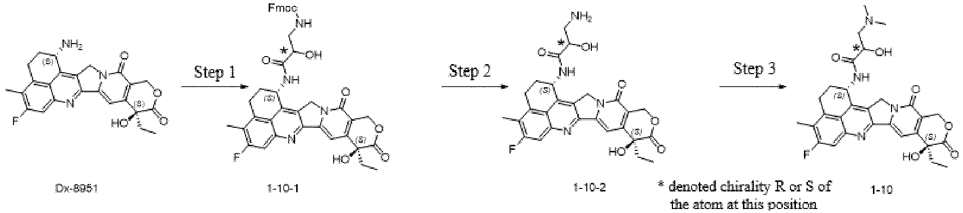

### Step 1: Synthesis of (9H-fluoro-9-yl)methyl ((S)-3-(((1S,9S)-9-ethyl-5-fluoro-9-hydroxy-4-methyl-10,13-dioxo-1,2,3,9,10,12,13,15-octahydrobenzo[de]pyrano[3',4':6,7]indolizino[1,2-b]quinolin-1-yl)amino)-2-hydroxy-3-oxopropyl)carbamate and (9H-fluoro-9-yl)methyl ((R)-3-(((1S,9S)-9-ethyl-5-fluoro-9-hydroxy-4-methyl-10,13-dioxo-1,2,3,9,10,12,13,15-octahydrobenzo[de]pyrano[3',4':6,7]indolizino[1,2-b]quinolin-1-yl)amino)-2-hydroxy-3-oxopropyl)carbamate

At 25°C, (1S,9S)-1-amino-9-ethyl-5-fluoro-9-hydroxy-4-methyl-2,3,12,15-benzo[de]pyrano [3',4':6,7]indolizino[1,2-b]quinolin-10,13(1H,9H)-dione mesylate (72 mg, 166 µmol) and 3-((((9H-fluoren-9-yl)methoxy)carbonyl)amino)-2-hydroxypropionic acid (65 mg, 199 µmol,) were dissolved in DMF (2 mL), then added with HATU (95 mg, 250 µmol) and DIPEA (65 mg, 498 µmol), after the addition, the reaction was carried out at room temperature for 0.5 hours and monitored by LCMS; the reaction solution was concentrated to dryness and directly purified by preparative high-performance liquid chromatography to obtain the title compounds (Compound **1-10-1-A,** 24 mg; and Compound **1-10-1-B,** 28 mg).
Chromatographic column: SunFire Prep C18 OBD 19mm×150mm×5.0µm
Mobile phase A: acetonitrile; Mobile phase B: water (0.05% formic acid)

| Time [min] | Mobile phase A [%] | Mobile phase B [%] | Flow rate [mL/min] |
|---|---|---|---|
| 0.00 | 15 | 85 | 28 |
| 2.00 | 15 | 85 | 28 |
| 18.00 | 90 | 10 | 28 |

The structural characterization data of compound **1-10-1-A** (6 min LCMS, the earlier peak with a retention time of 3.283 min) were as follows:
ESI-MS (m/z):745.4[M+H]⁺.

The structural characterization data of compound **1-10-1-B** (6 min LCMS, the later peak with a retention time of 3.465 min) were as follows:
ESI-MS (m/z):745.4[M+H]⁺.

### Step 2: Synthesis of (R)-3-amino-N-((1S,9S)-9-ethyl-5-fluoro-9-hydroxy-4-methyl-10,13-dioxo-1,2,3,9,10,12,13,15-octahydrobenzo[de]pyrano[3',4':6,7]indolizino[1,2-b]quinolin-1-yl)-2-hydroxypropionamide and (S)-3-amino-N-((1S,9S)-9-ethyl-5-fluoro-9-hydroxy-4-methyl-10,13-dioxo-1,2,3,9,10,12,13,15-octahydrobenzo[de]pyrano[3',4':6,7]indolizino[1,2-b]quinolin-1-yl)-2-hydroxypropionamide

At 25°C, compound **1-10-1-B** (28 mg, 37 µmol) was dissolved in DMF (2 mL), then added with diethylamine (1 mL), after the addition, the reaction was carried out at room temperature for 1.0 hour; the reaction solution was concentrated to dryness to obtain 28 mg of a crude product (compound **1-10-2-B**), which was directly used in the next reaction.

At 25°C, compound **1-10-1-A** (24 mg, 33 µmol) was dissolved in DMF (2 mL), then added with diethylamine (1 mL), after the addition, the reaction was carried out at room temperature for 1.0 hour; the reaction solution was concentrated to dryness to obtain 24 mg of a crude product (compound **1-10-2-A**), which was directly used in the next reaction.

The structural characterization data were as follows:
ESI-MS (m/z):523.2[M+H]⁺.

### Step 3: Synthesis of (R)-3-(dimethylamino)-N-((1S,9S)-9-ethyl-5-fluoro-9-hydroxy-4-methyl-10,13-dioxo-1,2,3,9,10,12,13,15-octahydrobenzo[de]pyrano[3',4':6,7]indolizino[1,2-b]quinolin-1-yl)-2-hydroxypropionamide and (S)-3-(dimethylamino)-N-((1S,9S)-9-ethyl-5-fluoro- 9-hydroxy-4-methyl-10,13-dioxo-1,2,3,9,10,12,13,15-octahydrobenzo[de]pyrano[3',4':6,7]indolizino[1,2-b]quinolin-1-yl)-2-hydroxypropionamide

At 25°C, compound **1-10-2-B** (28 mg, 37 µmol, 70%) was dissolved in methanol (2 mL), then added with formaldehyde solution (1 mL), after the addition, the reaction was carried out at room temperature for 16.0 hours, then sodium cyanoborohydride (7.07 mg, 96.45 µmol) was then added and reacted at room temperature for 1.0 hour; the reaction was monitored by LCMS; the reaction solution was concentrated to dryness, and directly purified by preparative high performance liquid chromatography to obtain 1.3 mg of the title compound (Compound **1-10B**).
Chromatographic column: SunFire Prep C18 OBD 19mm×150mm×5.0µm
Mobile phase A: methanol; mobile phase B: water (0.05% formic acid)

| Time [min] | Mobile phase A [%] | Mobile phase B [%] | Flow rate [mL/min] |
|---|---|---|---|
| 0.00 | 15 | 85 | 28 |
| 2.00 | 15 | 85 | 28 |
| 18.00 | 90 | 10 | 28 |

The structural characterization data of compound **1-10-B** (6 min LCMS, the earlier peak with a retention time of 1.937 min) were as follows:
ESI-MS (m/z):551.2[M+H]⁺.
¹H NMR (400 MHz, DMSO-d₆) δ 8.48 (d, *J* = 8.6 Hz, 1H), 8.31 (s, 2H), 7.80 (d, *J* = 11.0 Hz, 1H), 7.31 (s, 1H), 6.55 (s, 1H), 5.54 (s, 1H), 5.43 (s, 2H), 5.34 (d, *J* = 19.2 Hz, 1H), 5.19 (d, *J* = 19.1 Hz, 1H), 4.09 - 4.06 (m, 1H), 3.20 - 3.15 (m, 2H), 2.59 - 2.53 (m, 1H), 2.45 -2.42 (m, 1H), 2.42 - 2.38 (s, 3H), 2.23 - 2.19 (d, *J* = 7.0 Hz, 1H), 2.13 (s, 6H), 2.12 - 2.08 (m, 1H), 2.02 - 1.95 (m, 1H), 1.90 - 1.85 (m, 2H), 1.23 (s, 2H), 0.88 (d, *J* = 7.2 Hz, 3H).

At 25°C, compound **1-10-2-A** (24 mg, 33 µmol, 70%) was dissolved in methanol (2 mL), then added with formaldehyde solution (1 mL), after the addition, the reaction was carried out at room temperature for 16.0 hours, then sodium cyanoborohydride (6.06 mg, 96.45 µmol) was added and reacted at room temperature for 1.0 hour; the reaction was monitored by LCMS; the reaction solution was concentrated to dryness and directly purified by preparative high performance liquid chromatography to obtain 4.44 mg of the title compound (Compound **1-10-A**).
Chromatographic column: SunFire Prep C18 OBD 19mm×150mm×5.0µm
Mobile phase A: methanol; mobile phase B: water (0.05% formic acid)

| Time [min] | Mobile phase A [%] | Mobile phase B [%] | Flow rate [mL/min] |
|---|---|---|---|
| 0.00 | 15 | 85 | 28 |
| 2.00 | 15 | 85 | 28 |
| 18.00 | 90 | 10 | 28 |

The structural characterization data of compound **1-10-A** (6 min LCMS, the earlier peak with a retention time of 1.920 min) were as follows:
ESI-MS (m/z):551.2[M+H]⁺.
¹H NMR (400 MHz, DMSO-d₆) δ 8.58 (d, *J* = 9.0 Hz, 1H), 8.28 (s, 1H), 7.84 (d, *J* = 10.9 Hz, 1H), 7.37 (s, 1H), 6.61 (s, 1H), 5.66 - 5.59 (m, 1H), 5.49 (s, 2H), 5.35 (d, *J* = 19.1 Hz, 1H), 5.17 (d, *J* = 18.9 Hz, 1H), 4.23 - 4.16 (m, 1H), 3.23 (d, *J* = 7.8 Hz, 2H), 2.75 - 2.67 (m, 2H), 2.45 (s, 3H), 2.30 (s, 6H), 2.27 - 2.17 (m, 2H), 2.14 - 1.99 (m, 1H), 1.98 - 1.87 (m, 2H), 1.30 (s, 2H), 0.93 (t, *J* = 7.3 Hz, 3H).

### Example 21: (S)-14-(2-(Cyclopropylamino)ethyl)-7-ethyl-7-hydroxy-7H-[1,3]dioxolano[4, 5-g]pyrano[3',4':6,7]indolizino[1,2-b]quinolin-8,11(10H,13H)-dione (Compound 4-14)

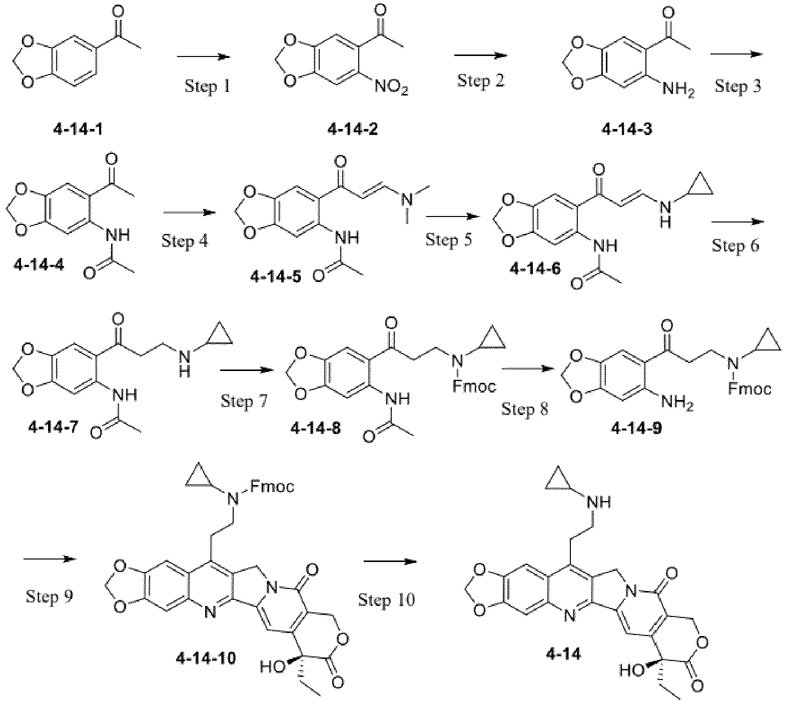

### Step 1: Synthesis of 2-nitro-4,5-methylenedioxyacetophenone

Compound **4-14-1** (10.0 g, 60.92 mmol) was dissolved in nitromethane (100 mL), then slowly added dropwise with concentrated nitric acid (26 mL) under stirring, and reacted at room temperature for 2 hours. The reaction was monitored by TLC, showing a small amount of starting material remained, and the product was obvious. The reaction solution was neutralized by slowly adding saturated aqueous sodium bicarbonate solution in dropwise manner, extraction was carried out three times by adding dichloromethane, the organic phases were combined, washed with saturated brine three times, then dried, and concentrated to obtain a crude product, which was purified by silica gel column (eluent: 0-20% ethyl acetate/petroleum ether) to obtain 9.8 g of the title compound.

### Step 2: Synthesis of 6-amino-3,4-methylenedioxyacetophenone

Compound **4-14-2** (2.0 g, 9.56 mmol) was dissolved in ethyl acetate (20 mL), then added with 10% palladium on carbon (0.2 g), subjected to hydrogen replacement and reacted under hydrogen protection with stirring for 4 hours. After filtration, the filtrate was concentrated under reduced pressure to obtain 1.7 g of a crude product of the title compound.

### Step 3: Synthesis of 6-acetamido-3,4-methylenedioxyacetophenone

Compound **4-14-3** (1.7 g, 9.49 mmol) was dissolved in acetic anhydride (17 mL), and reacted under stirring for 1 hour. After the solvent was evaporated under reduced pressure, water was added and stirred, the solid was filtered, washed with water, and dried in vacuum to obtain 2.08 g of a crude product of the title compound.

### Step 4: Synthesis of (E)-N-(6-(3-(dimethylamino)acryloyl)benzo[d][1,3]dioxol- 5-yl)acetamide

Compound **4-14-4** (1.88 g, 8.50 mmol) was dissolved in DMF-DMA (30 mL), heated to 120°C and reacted for 2 hours. The solvent was evaporated under reduced pressure to obtain 2.33 g of a crude product of the title compound.

The structural characterization data were as follows:
ESI-MS (m/z):277.2[M+1]+.

### Step 5: Synthesis of (E)-N-(6-(3-(cyclopropylamino)acryloyl)benzo[d][1,3]dioxol- 5-yl)acetamide

Compound **4-14-5** (200 mg, 0.72 mmol) was dissolved in ethanol (5 mL), then added dropwise with cyclopropylamine (413.3 mg, 7.24 mmol), heated to 50°C and reacted for 16 hours. The solvent was evaporated under reduced pressure to obtain 208 mg of a crude product of the title compound.

The structural characterization data were as follows:
ESI-MS (m/z):289.2[M+1]+.

### Step 6: Synthesis of N-(6-(3-(cyclopropylamino)propionyl)benzo[d][1,3]dioxol- 5-yl)acetamide

Compound **4-14-6** (208 mg, 0.72 mmol) was dissolved in glacial acetic acid (4 mL), then added with sodium borohydride (13.65 mg, 0.36 mmol) under stirring in an ice-water bath, then warmed to room temperature and reacted under stirring for 3 hours. The solvent was evaporated under reduced pressure to obtain 209 mg of a crude product of the title compound.

The structural characterization data were as follows:
ESI-MS (m/z):291.1[M+1]+.

### Step 7: Synthesis of (9H-fluoren-9-yl)methyl (3-(6-acetamidobenzo[d][1,3]dioxol- 5-yl)-3-oxopropyl)(cyclopropyl)carbamate

Compound **4-14-7** (200 mg, 0.69 mmol) was dissolved in 1,4-dioxane (20 mL) and water (20 mL), then added with 9-fluorenylmethyl-N-succinimido carbonate (395 mg, 0.68 mmol) and sodium bicarbonate (231.5 mg, 2.76 mmol) under stirring, and reacted at room temperature for 2 hours. Water and ethyl acetate were added under stirring, allowed to stand for liquid separation, the organic phase was washed with saturated brine, dried and concentrated, and purified by silica gel column (eluent: 30% ethyl acetate/petroleum ether) to obtain 350 mg of the title compound.

### Step 8: Synthesis of (9H-fluoren-9-yl)methyl (3-(6-aminobenzo[d][1,3]dioxol-5-yl)- 3-oxopropyl)(cyclopropyl)carbamate

Compound **4-14-8** (350 mg, 0.68 mmol) was dissolved in 1,4-dioxane (10 mL), then added dropwise with 3N aqueous hydrochloric acid (10 mL), heated to 60°C and reacted under stirring for 16 hours. Water and ethyl acetate were added and stirred, allowed to stand for liquid separation, the organic phase was washed with water, dried and concentrated, and purified by silica gel column (eluent: 33% ethyl acetate/petroleum ether) to obtain 218 mg of the title compound.

### Step 9: Synthesis of (S)-(9H-fluoren-9-yl)methylcyclopropyl (2-(7-ethyl-7-hydroxy-8,11-dioxo-8,10,11,13-tetrahydro-7H-[1,3]dioxolano[4,5-g]pyrano[3,4]:6,7]indolizino[1,2-b]quinolin-14-yl)ethyl)carbamate

Compound **4-14-9** (40 mg, 0.085 mmol) and (S)-4-ethyl-4-hydroxy-7,8-dihydro-1H-pyrano[3,4-f]indolizin-3,6,10(4H)-trione (24.62 mg, 0.094 mmol) were dissolved in toluene (1 mL), then added with p-toluenesulfonic acid (2.93 mg, 0.017 mmol), heated to 120°C and reacted for 4 hours. After concentration under reduced pressure, 59 mg of a crude product of the title compound was obtained.

The structural characterization data were as follows:
ESI-MS (m/z):698.1[M+1]+.

### Step 10: Synthesis of (S)-14-(2-(cyclopropylamino)ethyl)-7-ethyl-7-hydroxy-7H-[1,3] dioxolano[4,5-g]pyrano[3',4':6,7]indolizino[1,2-b]quinolin-8,11(10H,13H)-dione

Compound **4-14-10** (59 mg, 0.085 mmol) was dissolved in DMF (1 mL), then added dropwise with diethylamine (0.5 mL), and reacted under stirring for 1 hour. The reaction solution was distilled under reduced pressure to remove diethylamine, acidified by adding dropwise with 3N hydrochloric acid, purified by HPLC (purification conditions were as follows), and lyophilized to obtain 12.66 mg of a trifluoroacetic acid salt of the title compound.
Chromatographic column: SunFire Prep C18 OBD 19mm×150mm×5.0µm
Mobile phase A: acetonitrile; mobile phase B: water (0.05% trifluoroacetic acid)

| Time [min] | Mobile phase A [%] | Mobile phase B [%] | Flow rate [mL/min] |
|---|---|---|---|
| 0 | 20 | 80 | 28 |
| 2 | 20 | 80 | 28 |
| 18 | 80 | 20 | 28 |

The structural characterization data were as follows:
¹H NMR (400 MHz, DMSO-d₆) δ 8.73 (s, 2H), 7.67 (s, 1H), 7.57 (s, 1H), 7.26 (s, 1H), 6.54 (s, 1H), 6.33 (s, 2H), 5.44 (s, 2H), 5.34 (s, 2H), 3.40 (s, 4H), 2.82 (s, 1H), 1.91 - 1.81 (m, 2H), 0.87 (t, *J* = 7.2 Hz, 5H), 0.79 (d, *J* = 7.4 Hz, 2H).
ESI-MS (m/z):476.1[M+1]+.

### Example 22: (S)-7-Ethyl-7-hydroxy-14-(2-((2-methoxyethyl)amino)ethyl)-7H-[1,3] dioxolano[4,5-g]pyrano[3',4':6,7]indolizino[1,2-b]quinolin-8,11(10H,13H)-dione (Compound 4-15)

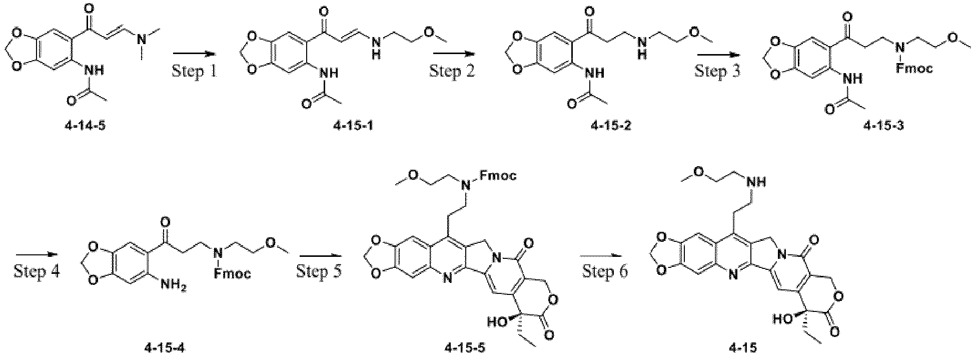

### Step 1: Synthesis of (E)-N-(6-(3-((2-methoxyethyl)amino)acryloyl)benzo[d][1,3]dioxol- 5-yl) acetamide

Compound **4-14-5** (200 mg, 0.72 mmol) was dissolved in ethanol (5 mL), then added dropwise with 2-methoxyethylamine (543.7 mg, 7.24 mmol), heated to 50°C and reacted for 16 hours. The reaction was monitored by LCMS, showing a small amount of starting material remained, and the product was obvious. The solvent was evaporated under reduced pressure to obtain 221 mg of the title compound, which was directly used in the next reaction.

The structural characterization data were as follows:
ESI-MS (m/z):307.1[M+1]+

### Step 2: Synthesis of N-(6-(3-((2-methoxyethyl)amino)propionyl)benzo[d][1,3]dioxol- 5-yl)acetamide

Compound **4-15-1** (200 mg, 0.65 mmol) was dissolved in glacial acetic acid (4 mL), then added with sodium borohydride (12.35 mg, 0.33 mmol) under stirring in an ice-water bath, warmed to room temperature and reacted under stirring for 3 hours. The reaction was monitored by LCMS, showing the starting material disappeared and the product was obvious. The solvent was evaporated under reduced pressure to obtain 200 mg of the title compound, which was directly used in the next reaction.
ESI-MS (m/z):309.1[M+1]+

### Step 3: Synthesis of (9H-fluoren-9-yl)methyl (3-(6-acetamidobenzo[d][1,3]dioxol-5-yl)- 3-oxopropyl)(2-methoxyethyl)carbamate

The crude product of compound **4-15-2** (200 mg, 0.65 mmol) was dissolved in 1,4-dioxane (20 mL) and water (20 mL), then added with 9-fluorenylmethyl-N-succinylimino carbonate (372 mg, 0.65 mmol) and sodium bicarbonate (231.5 mg, 2.76 mmol) under stirring, and reacted at room temperature for 2 hours. The reaction was monitored by TLC, showing the starting material disappeared, and the product was obvious. Water and ethyl acetate were added, stirred, allowed to stand for liquid separation, the organic phase was washed with saturated brine, dried and concentrated, and purified by silica gel column (eluent: 50% ethyl acetate/petroleum ether) to obtain 180 mg of the title compound.

### Step 4: Synthesis of ((9H-fluoren-9-yl)methyl (3-(6-aminobenzo[d][1,3]dioxol-5-yl)- 3-oxopropyl)(2-methoxyethyl)carbamate

Compound **4-15-3** (180 mg, 0.68 mmol) was dissolved in 1,4-dioxane (5 mL), added dropwise with 3N aqueous hydrochloric acid (5 mL), heated to 60°C and reacted under stirring for 16 hours. Water and ethyl acetate were added and stirred, allowed to stand for liquid separation, the organic phase was washed with water, dried and concentrated, and purified by silica gel column (eluent: 45% ethyl acetate/petroleum ether) to obtain 132 mg of the title compound.

### Step 5: Synthesis of (S)-(9H-fluoren-9-yl)methyl (2-(7-ethyl-7-hydroxy-8,11-dioxo-8,10,11,13-tetrahydro-7H-[1,3]dioxolano[4,5-g]pyrano[3',4:6,7]indolizino[1,2-b]quinolin-14-yl)ethyl)(2-methoxyethyl)carbamate

Compound **4-15-4** (130 mg, 0.266 mmol) and rac-(4S)-4-ethyl-4-hydroxy-7,8-dihydro-1H-pyrano[3,4-f]indolizin-3,6,10-trione (70.05 mg, 0.266 mmol) were dissolved in toluene (4 mL), then added with p-toluenesulfonic acid (9.16 mg, 0.053 mmol), heated to 120°C and reacted for 4 hours. The reaction was monitored by LCMS, showing the starting material disappeared and the product was obvious. After concentration under reduced pressure, 190 mg of a crude product of the title compound was obtained.

The structural characterization data were as follows:
ESI-MS (m/z):716.1[M+1]+

### Step 6: Synthesis of (S)-7-ethyl-7-hydroxy-14-(2-((2-methoxyethyl)amino)ethyl)- 7H-[1,3]dioxolano[4,5-g]pyrano[3',4':6,7]indolizino[1,2-b]quinolin-8,11(10H,13H)-dione

Compound **4-15-5** (190 mg, 0.265 mmol) was dissolved in DMF (3 mL), then added dropwise with diethylamine (2 mL), and reacted under stirring for 1 hour. The reaction was monitored by LCMS, showing the starting material disappeared and the product was obvious. The reaction solution was distilled under reduced pressure to remove diethylamine, acidified by adding dropwise with 3N hydrochloric acid, purified, and lyophilized to obtain 99.28 mg of the title compound.
Chromatographic column: SunFire Prep C18 OBD 19mm×150mm×5.0µm
Mobile phase A: acetonitrile; Mobile phase B: water (0.05% formic acid)

| Time [min] | Mobile phase A [%] | Mobile phase B [%] | Flow rate [mL/min] |
|---|---|---|---|
| 0 | 20 | 80 | 28 |
| 2 | 20 | 80 | 28 |
| 18 | 80 | 20 | 28 |

The structural characterization data were as follows:
¹H NMR (400 MHz, DMSO-d₆): δ 8.68 (s, 2H), 7.68 (s, 1H), 7.56 (s, 1H), 7.26 (s, 1H), 6.52 (s, 1H), 6.32 (s, 2H), 5.44 (s, 2H), 5.31 (s, 2H), 3.65 - 3.58 (m, 2H), 3.42 (d, *J* = 10.2 Hz, 2H), 3.36 (s, 3H), 3.22 (d, *J* = 4.0 Hz, 4H), 1.94 - 1.80 (m, 2H), 0.87 (t, *J* = 7.3 Hz, 3H).
ESI-MS (m/z):494.2[M+1]+

### Example 23: N-((1S,9S)-9-Ethyl-5-fluoro-9-hydroxy-4-methoxy-10,13-dioxo-2,3,9,10, 13,15-hexahydro-1H,12H-benzo[de]pyrano[3',4:6,7]indolizino[1,2-b]quinolin-1-yl)-2-hydroxy acetamide and N-((1R,9S)-9-ethyl-5-fluoro-9-hydroxy-4-methoxy-10,13-dioxo-2,3,9,10,13,15- hexahydro-1H,12H-benzo[de]pyrano[3',4:6,7]indolizino[1,2-b]quinolin-1-yl)-2-hydroxyacetamide

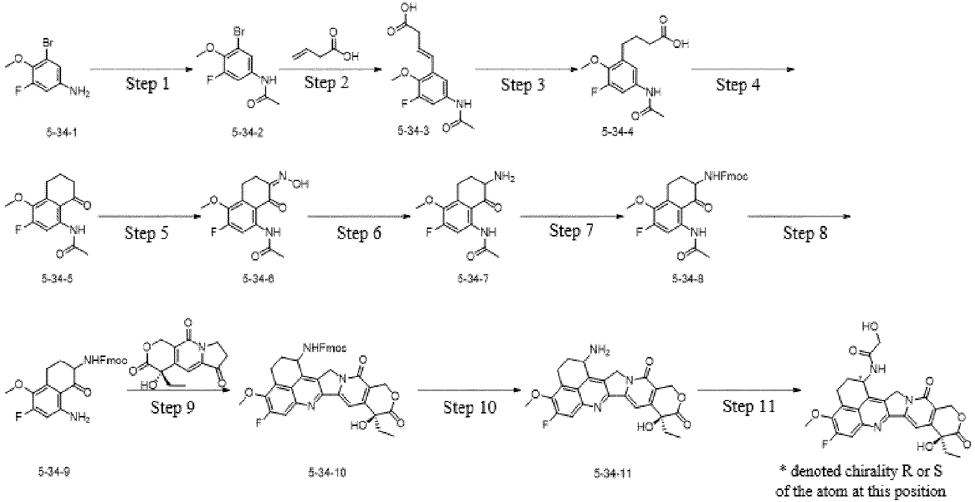

### Step 1: Synthesis of N-(3-bromo-5-fluoro-4-methoxyphenyl)acetamide

3-Bromo-5-fluoro-4-methoxy-aniline (1.7 g, 7.73 mmol) was dissolved in tetrahydrofuran (30 mL), then added with triethylamine (2.35 g, 23.18 mmol) and acetic anhydride (1.18 g, 11.59 mmol), after the addition, the temperature was raised to 50°C, the reaction was stirred for 4 hours and monitored by LCMS; after the reaction solution was cooled to room temperature, it was diluted with ethyl acetate (50 mL), then washed with water (30 mL) and saturated brine (30 mL) once each, the organic phase was separated and dried over anhydrous sodium sulfate, filtered, and the filtrate was evaporated to dryness under reduced pressure to obtain a crude product, which was slurried (with petroleum ether: ethyl acetate = 5:1) and purified to obtain 1.1 g of the title compound.

The structural characterization data were as follows:
ESI-MS (m/z):262.0[M+H]⁺.

### Step 2: Synthesis of (E)-4-(5-acetylamino-3-fluoro-2-methoxyphenyl)-3-butenoic acid

N-(3-Bromo-5-fluoro-4-methoxyphenyl)acetamide (1.1 g, 4.20 mmol) and 3-butenoic acid (397.47 mg, 4.62 mmol) were dissolved in 1,4-dioxahexane (20 mL) and water (5 mL), then added with triethylamine (1.27 g, 12.59 mmol), tris(o-methylphenyl)phosphine (127.75 mg, 419.73 µmol) and palladium acetate (47.12 mg, 209.89 µmol), after the addition, the reaction system was subjected to nitrogen replacement three times, heated to 100°C and reacted for 4 hours under nitrogen atmosphere; the reaction was monitored by LCMS; after the reaction solution was cooled to room temperature, 1 mol/L sodium hydroxide aqueous solution (50 mL) and ethyl acetate (50 mL) were added and shaken for liquid separation. The lower aqueous phase was separated and adjusted to pH of about 3 with 4 mol/L hydrochloric acid aqueous solution, and then extracted with ethyl acetate (40 mL*2), the organic phases were combined and washed with saturated brine (40 mL), dried over anhydrous sodium sulfate, and filtered, and the filtrate was evaporated to dryness under reduced pressure to obtain 1.1 g of a crude product of the title compound.

The structural characterization data were as follows:
ESI-MS (m/z):268.1[M+H]⁺.

### Step 3: Synthesis of 4-(5-acetylamino-3-fluoro-2-methoxyphenyl)butanoic acid

The crude product of (E)-4-(5-acetylamino-3-fluoro-2-methoxyphenyl)-3-butenoic acid (1.1 g, 4.12 mmol,) was dissolved in methanol (20 mL), then added with 10% palladium on carbon (100 mg), after the addition, the reaction system was subjected to hydrogen replacement three times with a hydrogen balloon, and the reaction was carried out for 4 hours under hydrogen atmosphere and monitored by LCMS; the reaction solution was filtered, the filtrate was concentrated under reduced pressure to dryness to obtain 1.05 g of a crude product of the title compound.

The structural characterization data were as follows:
ESI-MS (m/z):270.1[M+H]⁺.

### Step 4: Synthesis of N-(3-fluoro-4-methoxy-8-oxo-5,6,7,8-tetrahydronaphthalen- 1-yl)acetamide

The crude product of 4-(5-acetylamino-3-fluoro-2-methoxyphenyl)butanoic acid (1.1 g, 4.09 mmol) was dissolved in trifluoroacetic acid (10 mL), cooled to 5°C, then added slowly with trifluoroacetic anhydride (4.29 g, 20.43 mmol), after the addition, the reaction system was naturally warmed to room temperature and reacted for 2 hours; the reaction was monitored by LCMS; the reaction solution was slowly poured into water (60 mL), and then extracted with ethyl acetate (40 mL*3), the organic phases were combined, washed with saturated aqueous sodium bicarbonate until neutral, and then washed with saturated brine (40 mL), dried over anhydrous sodium sulfate, and filtered, and the filtrate was evaporated to dryness under reduced pressure to obtain a crude product, which was purified by flash silica gel column (ethyl acetate:petroleum ether=0-40%) to obtain 503 mg of the title compound.

The structural characterization data were as follows:
ESI-MS (m/z):252.1[M+H]⁺.

### Step 5: Synthesis of N-(3-fluoro-7-(hydroxyimino)-4-methoxy-8-oxo-5,6,7,8-tetrahydronaphthalen-1-yl)acetamide

Tetrahydrofuran (15 mL) and tert-butanol (4 mL) were added to a reaction flask, cooled to 5°C in an ice bath, added with potassium tert-butoxide (491.26 mg, 4.38 mmol), then N-(3-fluoro-4-methoxy-8-oxo-5,6,7,8-tetrahydronaphthalen- 1-yl)acetamide (500 mg, 1.99 mmol) was dissolved in tetrahydrofuran (5 mL) and slowly added dropwise thereto, followed by an addition of isoamyl nitrite (373.01 mg, 3.18 mmol) after 10 minutes, after the addition, the reaction was carried out at 5°C for 1 hour and monitored by LCMS; the reaction solution was quenched with saturated ammonium chloride aqueous solution (50 mL), extracted with ethyl acetate (40*2), the organic phases were combined, washed with saturated brine (40 mL), dried over anhydrous sodium sulfate, then filtered, and the filtrate was evaporated to dryness under reduced pressure to obtain 550 mg of a crude product of the title compound.

The structural characterization data were as follows:
ESI-MS (m/z):281.1 [M+H]⁺.

### Step 6: Synthesis of N-(7-amino-3-fluoro-4-methoxy-8-oxo-5,6,7,8-tetrahydronaphthalen- 1-yl)acetamide

The crude product of N-(3-fluoro-7-(hydroxyimino)-4-methoxy-8-oxo-5,6,7,8-tetrahydronaphthalen-1-yl)acetamide (520 mg, 1.86 mmol) was dissolved in a mixed solution of methanol (10 mL) and tetrahydrofuran (10 mL), then added with 1 mol/L aqueous hydrochloric acid (3.71 mL) and 10% palladium on carbon (50 mg), after the addition, the reaction system was subjected to hydrogen replacement three times with a hydrogen ball, and the reaction was carried out at room temperature under hydrogen atmosphere for 1 hour and monitored by LCMS; the reaction solution was filtered, and the filtrate was concentrated under reduced pressure to dryness to obtain 551 mg of a crude hydrochloride of the title compound.

The structural characterization data were as follows:
ESI-MS (m/z):267.1[M+H]⁺.

### Step 7: Synthesis of (9H-fluoren-9-yl)methyl (8-acetamide-6-fluoro-5-methoxy-1-oxo-1,2,3,4-tetrahydronaphthalen-2-yl)carbamate

The crude hydrochloride of N-(7-amino-3-fluoro-4-methoxy-8-oxo-5,6,7,8-tetrahydronaphthalen-1-yl)acetamide (550 mg, 1.64 mmol) was dissolved in 1,4-dioxane (15 mL), then added with sodium bicarbonate (549.45 mg, 6.54 mmol), water (5 mL) and 9-fluorenylmethyl-N-succinimido carbonate (1.12 g, 1.96 mmol), after the addition, the reaction was carried out under stirring at room temperature for 2 hours and monitored by LCMS; the reaction solution was poured into water (50 mL), and then extracted with ethyl acetate (40 mL*2), the organic phases were combined, washed with saturated brine (40 mL), dried over anhydrous sodium sulfate, then filtered, and the filtrate was dried under reduced pressure to obtain a crude product. The crude product was purified by C18 (acetonitrile/0.05% formic acid aqueous solution, 20% acetonitrile~100% acetonitrile) reverse phase column to obtain 410 mg of the title compound.

The structural characterization data were as follows:
ESI-MS (m/z):489.1[M+H]⁺.

### Step 8: Synthesis of (9H-fluoren-9-yl)methyl (8-amino-6-fluoro-5-methoxy-1-oxo- 1,2,3,4-tetrahydronaphthalen-2-yl)carbamate

(9H-Fluoren-9-yl)methyl (8-acetamide-6-fluoro-5-methoxy-1-oxo-1,2,3,4-tetrahydro naphthalen-2-yl)carbamate (410 mg, 839.29 µmol) was dissolved in dioxane (10 mL), and added with 12 mol/L concentrated hydrochloric acid (2 mL), after the addition, the temperature was raised to 70°C, the reaction was carried out for 2 hours and monitored by LCMS; the reaction solution was poured into water (30 mL), then extracted with ethyl acetate (30 mL*2), the organic phases were combined, washed with saturated brine (30 mL), dried over anhydrous sodium sulfate, then filtered, and the filtrate was dried under reduced pressure to obtain a crude product, which was purified by flash silica gel (ethyl acetate:petroleum ether=0-60%) to obtain 351 mg of the title compound.

The structural characterization data were as follows:
ESI-MS (m/z):447.1[M+H]⁺.

### Step 9: Synthesis of (9H-fluoren-9-yl)methyl ((9S)-9-ethyl-5-fluoro-9-hydroxy-4-methoxy-10,13-dioxo-2,3,9,10,13,15-hexahydro-1H,12H-benzo[de]pyrano[3',4:6,7]indolizino[1,2-b]quinolin- 1-yl)carbamate

(S)-4-Ethyl-4-hydroxy-7,8-dihydro-1H-pyrano[3,4-f]indolizin-3,6,10(4H)-trione (247.64 mg, 940.71 µmol) and (9H-fluoren-9-yl)methyl (8-amino-6-fluoro-5-methoxy-1-oxo-1,2,3,4-tetrahydronaphthalen-2-yl)carbamate (350 mg, 783.93 µmol) were added to toluene (15 mL), and then added with p-toluenesulfonic acid (134.84 mg, 783.93 µmol). After the addition, the temperature was raised to 135°C, the reaction was carried out for 4 hours. The reaction solution was directly evaporated to dryness at 135°C under reduced pressure to obtain a crude product; and the crude product was purified by flash silica gel (methanol:dichloromethane=0-6%) column to obtain 358 mg of the title compound.

The structural characterization data were as follows:
ESI-MS (m/z):674.2[M+H]⁺.

### Step 10: Synthesis of (9S)-1-amino-9-ethyl-5-fluoro-9-hydroxy-4-methoxy-1,2,3,9,12,15-hexahydro-10H,13H-benzo[de]pyrano[3',4':6,7]indolizino[1,2-b]quinolin-10,13-dione

(9H-Fluoren-9-yl)methyl ((9S)-9-ethyl-5-fluoro-9-hydroxy-4-methoxy-10,13-dioxo-2,3,9, 10,13,15-hexahydro-1H,12H-benzo[de]pyrano[3',4:6,7]indolizino[1,2-b]quinolin-1-yl)carbamate (358 mg, 531.41 µmol) was dissolved in N,N-dimethylformamide (4 mL), then added with diethylamine (0.4 mL), after the addition, the reaction was carried out at room temperature for 0.5 hours and monitored by LCMS; the reaction solution was evaporated to dryness under reduced pressure to obtain a crude product, which was slurried in ethyl acetate for purification to obtain 220 mg of the title compound.

The structural characterization data were as follows:
ESI-MS (m/z):452.1[M+H]⁺.

### Step 11: Synthesis of N-((1S,9S)-9-ethyl-5-fluoro-9-hydroxy-4-methoxy-10,13-dioxo-2,3,9,10,13,15-hexahydro-1H,12H-benzo[de]pyrano[3',4:6,7]indolizino[1,2-b]quinolin-1-yl)-2-hydroxyacetamide and N-((1R,9S)-9-ethyl-5-fluoro-9-hydroxy-4-methoxy-10,13-dioxo-2,3,9,10, 13,15-hexahydro-1H,12H-benzo[de]pyrano[3',4:6,7]indolizino[1,2-b]quinolin-1-yl)-2-hydroxy acetamide

(9S)-1-Amino-9-ethyl-5-fluoro-9-hydroxy-4-methoxy-1,2,3,9,12,15-hexahydro-10H,13H-benzo[de]pyrano[3',4':6,7]indolizino[1,2-b]quinolin-10,13-dione (50 mg, 110.76 µmol) and 2-hydroxyacetic acid (16.85 mg, 221.51 µmol) were dissolved in N,N-dimethylformamide (2 mL), then added with HATU (84.17 mg, 221.51 µmol) and N,N-diisopropylethylamine (42.94 mg, 332.27 µmol), after the addition, the reaction was carried out at room temperature for 0.5 hours and monitored by LCMS; the reaction solution was directly purified by preparative high performance liquid chromatography to obtain two isomers (**5-34-A**: 6.22 mg, **5-34-B:** 9.81 mg).
Chromatographic column: SunFire Prep C18 OBD 19mm×150mm×5.0µm
Mobile phase A: acetonitrile; Mobile phase B: water (0.05% formic acid)

| Time [min] | Mobile phase A [%] | Mobile phase B [%] | Flow rate [mL/min] |
|---|---|---|---|
| 0.00 | 10 | 90 | 28 |
| 2.00 | 10 | 90 | 28 |
| 18.00 | 90 | 10 | 28 |

The structural characterization data of **5-34-A** wre as follows:
¹H NMR (400 MHz, DMSO-d₆) δ 8.53 (d, *J* = 9.0 Hz, 1H), 7.89 (d, *J* = 12.3 Hz, 1H), 7.33 (s, 1H), 5.58 (q, *J* = 7.5, 7.0 Hz, 1H), 5.42 (d, *J* = 2.1 Hz, 2H), 5.25 - 5.09 (m, 2H), 3.98 (s, 2H), 3.96 (d, *J* = 1.1 Hz, 3H), 3.30 - 3.10 (m, 2H), 2.15 (q, *J* = 7.4 Hz, 2H), 1.93 - 1.80 (m, 2H), 0.87 (t, *J* = 7.3 Hz, 3H).
ESI-MS (m/z):510.2[M+H]⁺.

The structural characterization data of **5-34-B** were as follows:
¹H NMR (400 MHz, DMSO-d₆) δ 8.55 (d, *J* = 9.0 Hz, 1H), 7.90 (d, *J* = 12.3 Hz, 1H), 7.34 (s, 1H), 5.59 (q, *J* = 7.4, 6.8 Hz, 1H), 5.43 (s, 2H), 5.25 - 5.11 (m, 2H), 3.99 (s, 2H), 3.96 (d, *J* = 1.1 Hz, 3H), 3.30 - 3.13 (m, 2H), 2.15 (q, *J* = 6.4 Hz, 2H), 1.93 - 1.82 (m, 2H), 0.88 (t, *J* = 7.3 Hz, 3H).
ESI-MS (m/z):510.2[M+H]⁺.

### Example 24: (2R)-N-((1S,9S)-5-Chloro-9-ethyl-9-hydroxy-4-methyl-10,13-dioxo-2,3,9,10, 13,15-hexahydro-1H,12H-benzo[de]pyrano[3',4:6,7]indolizino[1,2-b]quinolin-1-yl)-2-hydroxypropylamine and (2R)-N-((1R,9S)-5-chloro-9-ethyl-9-hydroxy-4-methyl-10,13-dioxo-2,3,9,10, 13,15-hexahydro-1H,12H-benzo[de]pyrano[3',4:6,7]indolizino[1,2-b]quinolin-1-yl)-2-hydroxypropylamine

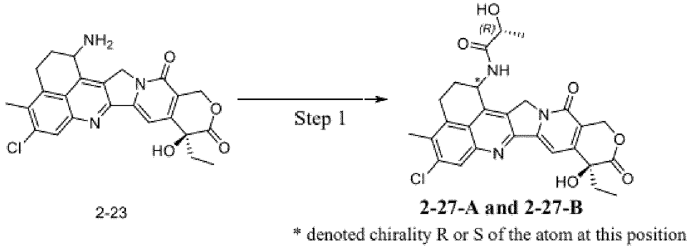

### Step 1: Synthesis of (2R)-N-((1S,9S)-5-chloro-9-ethyl-9-hydroxy-4-methyl-10,13-dioxo-2,3,9,10,13,15-hexahydro-1H,12H-benzo[de]pyrano[3',4:6,7]indolizino[1,2-b]quinolin-1-yl)-2-hydroxypropylamine and (2R)-N-((1R,9S)-5-chloro-9-ethyl-9-hydroxy-4-methyl-10,13-dioxo-2,3, 9,10,13,15-hexahydro-1H,12H-benzo[de]pyrano[3',4:6,7]indolizino[1,2-b]quinolin-1-yl)-2-hydroxypropylamine

(9S)-1-Amino-5-chloro-9-ethyl-9-hydroxy-4-methyl-1,2,3,9,12,15-hexahydro-10H,13H-benzo[de]pyrano[3',4':6,7]indolizino[1,2-b]quinolin-10,13-dione (50 mg, 110.64 µmol) and (2R)-2-hydroxypropane acid (19.93 mg, 221.29 µmol) were dissolved in N,N-dimethylformamide (2 mL), then added with HATU (84.09 mg, 221.29 µmol) and N,N-diisopropylethylamine (42.90 mg, 331.93 µmol), after the addition, the reaction was carried out at room temperature for 0.5 hours and monitored by LCMS; the reaction solution was directly purified by preparative high performance liquid chromatography to obtain two isomers of the title compound (**2-27-A**: 5.73 mg, **2-27-B**: 7.59 mg)
Chromatographic column: SunFire Prep C18 OBD 19mm×150mm×5.0µm
Mobile phase A: acetonitrile; mobile phase B: water (0.05% trifluoroacetic acid)

| Time [min] | Mobile phase A [%] | Mobile phase B [%] | Flow rate [mL/min] |
|---|---|---|---|
| 0.00 | 30 | 90 | 28 |
| 2.00 | 30 | 90 | 28 |
| 18.00 | 90 | 10 | 28 |

The structural characterization data of **2-27-A** were as follows:
¹H NMR (400 MHz, DMSO-d₆) δ 8.45 (d, *J* = 9.1 Hz, 1H), 8.14 (s, 1H), 7.30 (s, 1H), 6.54 (s, 1H), 5.63 (s, 1H), 5.56 (q, *J* = 8.0 Hz, 1H), 5.42 (s, 2H), 5.25 (d, *J* = 19.0 Hz, 1H), 5.08 (d, *J* = 19.0 Hz, 1H), 4.13 (q, *J* = 6.7 Hz, 1H), 3.27 - 3.12 (m, 2H), 2.51 (s, 3H), 2.23 - 2.13 (m, 2H), 1.92 - 1.80 (m, 2H), 1.41 (d, *J* = 6.8 Hz, 3H), 0.87 (t, *J* = 7.3 Hz, 3H).
ESI-MS (m/z):524.2[M+H]⁺.

The structural characterization data of **2-27-B** were as follows:
¹H NMR (400 MHz, DMSO-d₆) δ 8.40 (d, *J* = 8.9 Hz, 1H), 8.15 (s, 1H), 7.31 (s, 1H), 6.54 (s, 1H), 5.55 - 5.49 (m, 1H), 5.43 (d, *J* = 2.2 Hz, 2H), 5.18 (q, *J* = 19.0 Hz, 2H), 4.13 (q, *J* = 6.6 Hz, 1H), 3.24 - 3.12 (m, 2H), 2.51 (s, 3H), 2.22 - 2.10 (m, 2H), 1.92 - 1.82 (m, 2H), 1.30 (d, *J* = 6.7 Hz, 3H), 0.88 (t, *J* = 7.3 Hz, 3H).
ESI-MS (m/z):524.2[M+H]⁺.

### Example 25: Preparation of (R)-N-((1S,9S)-4-chloro-9-ethyl-5-fluoro-9-hydroxy-10,13-dioxo-2,3,9,10,13,15-hexahydro-1H,12H-benzo[de]pyrano[3',4':6,7]indolizino[1,2-b]quinolin-1-yl)-2-hydroxypropylamine and (R)-N-((1R,9S)-4-chloro-9-ethyl-5-fluoro-9-hydroxy-10,13-dioxo-2,3,9,10,13,15-hexahydro-1H,12H-benzo[de]pyrano[3',4':6,7]indolizino[1,2-b]quinolin-1-yl)-2-hydroxypropylamine

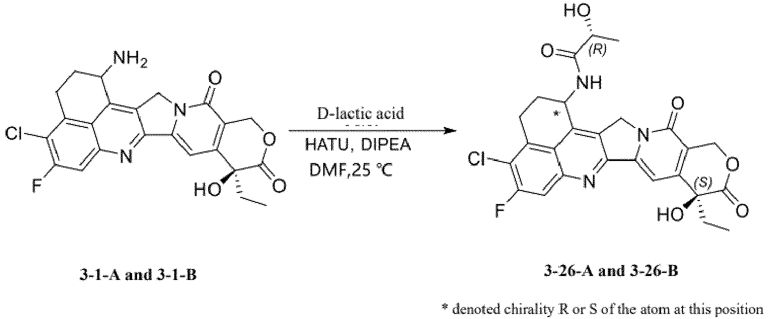

At 25°C, **3-1-A** (20.0 mg, 43.9 µmol) and D-lactic acid (7.90 mg, 87.8 µmol) were dissolved in DMF (1.0 mL), then added with HATU (33.4 mg, 87.8 µmol) and DIPEA (17.0 mg, 131.6 µmol), reacted at room temperature for 2 hours; the reaction solution was concentrated to remove most of the DMF, and the residue was purified by preparative high performance liquid chromatography to obtain compound **3-26-A** (15.4 mg, yield 64%).
Chromatographic column: SunFire Prep C18 OBD 19mm×150mm×5.0µm
Mobile phase A: acetonitrile; Mobile phase B: water (0.05% formic acid)
Retention time: 5.3~6.2 min

| Time [min] | Mobile phase A [%] | Mobile phase B [%] | Flow rate [mL/min] |
|---|---|---|---|
| 0.00 | 30 | 70 | 28 |
| 2.00 | 30 | 70 | 28 |
| 18.00 | 90 | 10 | 28 |

The structural characterization data were as follows:
MS m/z (ESI): 528.2 [M+H]⁺
¹H NMR (400 MHz, DMSO-d₆) δ 8.52 (d, *J* = 9.2 Hz, 1H), 8.05 (d, *J* = 10.0 Hz, 1H), 7.33 (s, 1H), 6.55 (s, 1H), 5.62 - 5.59 (m, 2H), 5.43 (s, 2H), 5.28 - 5.10 (m, 2H), 4.13 - 4.11 (m, 1H), 3.41 - 3.38 (m, 1H), 3.28 - 3.22 (m, 1H), 2.20 - 2.18 (m, 2H), 1.92 - 1.80 (m, 2H), 1.40 (d, *J* = 6.8 Hz, 3H), 0.87 (t, *J* = 7.2 Hz, 3H).

At 25°C, **3-1-B** (20.0 mg, 43.9 µmol) and D-lactic acid (7.90 mg, 87.8 µmol) were dissolved in DMF (1.0 mL), then added with HATU (33.4 mg, 87.8 µmol) and DIPEA (17.0 mg, 131.6 µmol), reacted at room temperature for 2 hours; the reaction solution was concentrated to remove most of the DMF, and the residue was purified by preparative high performance liquid chromatography to obtain compound **3-26-B** (4.8 mg, yield 20%).
Chromatographic column: SunFire Prep C18 OBD 19mm×150mm×5.0µm
Mobile phase A: acetonitrile; Mobile phase B: water (0.05% formic acid)
Retention time: 7.5~8.5 min

| Time [min] | Mobile phase A [%] | Mobile phase B [%] | Flow rate [mL/min] |
|---|---|---|---|
| 0.00 | 30 | 70 | 28 |
| 4.00 | 30 | 70 | 28 |
| 20.00 | 90 | 10 | 28 |

The structural characterization data were as follows:
MS m/z (ESI): 528.2 [M+H]⁺
¹H NMR (400 MHz, DMSO-d₆) δ 8.48 (d, *J* = 8.8 Hz, 1H), 8.06 (d, *J* = 10.4 Hz, 1H), 7.34 (s, 1H), 6.57 (br, 1H), 5.61 - 5.55 (m, 1H), 5.48 - 5.39 (m, 2H), 5.27 - 5.16 (m, 2H), 4.15 - 4.10 (m, 1H), 3.32 - 3.21 (m, 3H), 2.23 - 2.16 (m, 2H), 1.92 - 1.81 (m, 2H), 1.30 (d, *J* = 6.4 Hz, 3H), 0.87 (t, *J* = 7.2 Hz, 3H).

### Biological evaluation

### 1. Tumor cell proliferation inhibition test

### 1. Inhibitory effect of compounds on HT29 cell proliferation

(1) Cell plating: First, tumor cells HT29 were cultured in corresponding medium, then digested with trypsin, centrifuged, resuspended, and counted, and the cells were adjusted to an appropriate concentration for plating. The source of the tumor cells was listed in Table 1.

**Table 1. Source of tumor cells**

| Cell name | Tumor type | Source |
|---|---|---|
| HT29 | Human colon cancer cells | Cell Bank of Chinese Academy of Sciences |

Co-incubation of the compound of the present invention and tumor cells: after the cells adhered to the wall, the medium was removed, and the diluted bioactive molecule (the compound of the present invention) was added to the wells of the above plate, and incubated for 72 h.

In vitro cell viability detection: After the incubation, 50 µL of Cell Counting-Lite^{™} 2.0 reagent (Vazyme/Novazyme) was added to each well, shaken and mixed well in the dark, reacted for 10 minutes, and then the detection was carried out with a microplate reader (manufacturer: BMG, Model: PHERAStar-FS) for reading. The background RLU was obtained by using cell-free medium (with Cell Counting-Lite^{™}), and the vehicle RLU was obtained by using cell-containing medium (with Cell Counting-Lite^{™}). Cell inhibition rate = 1 - (sample RLU - sample RLU)/(solvent RLU - background RLU) × 100%. According to the four-parameter model fitting curve, the half maximal inhibitory concentration (IC50) of the compound was calculated, and the detection results were shown in Table 2.

### (2) Data results

**Table 2. Inhibitory activity on HT29 cell proliferation**

| Name | IC₅₀ (nM) |
|---|---|
| Comparative Compound 1 | 11.27 |
| Example 1 (1-1-A) | 1.28 |
| Example 1 (1-1-B) | 1.54 |
| Example 3 (2-1-A) | 5.03 |
| Example 3 (2-2-B) | 7.52 |
| Example 4 (2-7-A) | 1.56 |
| Example 4 (2-7-B) | 5.44 |
| Example 5 (2-12-A) | 3.01 |
| Example 5 (2-12-B) | 2.34 |
| Example 5 (2-12-C) | 1.49 |
| Example 5 (2-12-D) | 3.29 |
| Example 6 (2-17-A) | 1.24 |
| Example 6 (2-17-B) | 1.59 |
| Example 7 (2-20-A) | 1.29 |
| Example 7 (2-20-B) | 4.03 |
| Example 8 (3-1-A) | 0.16 |
| Example 8 (3-1-B) | 0.71 |
| Example 9 (3-4-A) | 5.55 |
| Example 9 (3-4-B) | 4.98 |
| Example 10 (4-12) | 4.79 |
| Example 11 (4-10) | 0.52 |
| Example 12 (5-13-A) | 0.88 |
| Example 13 (5-7-A) | 3.13 |
| Example 13 (5-7-B) | 4.66 |
| Example 14 (5-16-A) | 3.87 |
| Example 20 (1-10-A) | 61.60 |
| Example 20 (1-10-B) | 10.26 |
| Example 21 (4-14) | 0.56 |
| Example 22 (4-15) | 5.20 |

The detection results showed that the compounds of the present invention in Table 2 had potent inhibitory effect on the proliferation of HT29 colon cancer cells.

### 2. Inhibitory effect of compounds on A549 cell proliferation

(1) Cell plating: First, tumor cells A549 were cultured in the corresponding medium, then digested with trypsin, centrifuged and resuspended, and counted, and the cells were adjusted to an appropriate concentration for plating. The source of the tumor cells was listed in Table 3.

**Table 3. Source of tumor cells**

| Cell name | Tumor type | Source |
|---|---|---|
| A549 | Human lung cancer cells | Cell Bank of Chinese Academy of Sciences |

Co-incubation of the compound of the present invention and tumor cells: after the cells adhered to the wall, the medium was removed, and the diluted bioactive molecule (the compound of the present invention) was added to the wells of the above plate, and incubated for 72 h.

In vitro cell activity detection: After the incubation, 50 µL of Cell Counting-Lite^{™} 2.0 reagent (Vazyme/Novazyme) was added to each well, shaken and mixed well in the dark, reacted for 10 minutes, and then the detection was carried out with a microplate reader (manufacturer: BMG, Model: PHERAStar-FS) for reading. The background RLU was obtained by using cell-free medium (with Cell Counting-Lite^{™}), and the vehicle RLU was obtained by using cell-containing medium (with Cell Counting-Lite^{™}). Cell inhibition rate = 1 - (sample RLU - sample RLU)/(solvent RLU - background RLU) × 100%. According to the four-parameter model fitting curve, the half maximal inhibitory concentration (IC50) of the compound was calculated, and the detection results were shown in Table 4.

### (2) Data results

**Table 4. Inhibitory activity on A549 cell proliferation**

| Name | IC₅₀ (nM) |
|---|---|
| Comparative Compound 1 | 94.18 |
| Example 1 (1-1-A) | 66.56 |
| Example 4 (2-7-A) | 6.97 |
| Example 4 (2-7-B) | 16.39 |
| Example 5 (2-12-A) | 6.96 |
| Example 5 (2-12-B) | 5.52 |
| Example 5 (2-12-C) | 6.64 |
| Example 7 (2-20-A) | 8.14 |
| Example 7 (2-20-B) | 23.06 |
| Example 11 (4-10) | 68.48 |

The detection results showed that the compounds of the present invention in Table 4 had significant inhibitory effect on the proliferation of A549 human lung cancer cells.

### 3. Inhibitory effect of compounds on NCI-H1806 cell proliferation

(1) Cell plating: First, tumor cells HCC1806 were cultured in corresponding medium, then digested with trypsin, centrifuged, resuspended, and counted, and the cells were adjusted to an appropriate concentration for plating. The source of the tumor cells was listed in Table 5.

**Table 5. Source of tumor cells**

| Cell name | Tumor type | Source |
|---|---|---|
| HCC1806 | Human breast squamous carcinoma cells | Cell Bank of Chinese Academy of Sciences |

Co-incubation of the compound of the present invention and tumor cells: after the cells adhered to the wall, the medium was removed, and the diluted bioactive molecule (the compound of the present invention) was added to the wells of the above plate, and incubated for 72 h.

In vitro cell viability detection: After the incubation, 50 µL of Cell Counting-Lite^{™} 2.0 reagent (Vazyme/Novazyme) was added to each well, shaken and mixed well in the dark, reacted for 10 minutes, and then the detection was carried out with a microplate reader (manufacturer: BMG, Model: PHERAStar-FS) for reading. The background RLU was obtained by using cell-free medium (with Cell Counting-Lite^{™}), and the vehicle RLU was obtained by using cell-containing medium (with Cell Counting-Lite^{™}). Cell inhibition rate = 1 - (sample RLU - sample RLU)/(solvent RLU - background RLU) × 100%. According to the four-parameter model fitting curve, the half maximal inhibitory concentration (IC50) of the compound was calculated, and the detection results were shown in Table 6.

### (2) Data results

**Table 6. Inhibitory activity on HCC1806 cell proliferation**

| Name | IC₅₀ (nM) |
|---|---|
| Comparative Compound 1 | 3.03 |
| Example 3 (2-1-A) | 1.43 |
| Example 4 (2-7-A) | 1.25 |
| Example 5 (2-12-A) | 1.40 |
| Example 5 (2-12-B) | 1.07 |
| Example 5 (2-12-C) | 2.08 |
| Example 12 (5-13-A) | 0.96 |
| Example 16 (3-12-A) | 0.16 |
| Example 16 (3-12-B) | 1.76 |
| Example 16 (3-12-C) | 1.91 |
| Example 16 (3-12-D) | 1.53 |
| Example 17 (3-7-B) | 1.94 |
| Example 18 (3-17-A) | 1.70 |
| Example 18 (3-17-B) | 2.26 |
| Example 19 (5-22-A) | 2.57 |
| Example 19 (5-22-B) | 3.71 |
| Example 24 (2-27-A) | 4.55 |
| Example 24 (2-27-B) | 3.93 |
| Example 25 (3-26-A) | 1.27 |
| Example 25 (3-26-B) | 4.93 |

The detection results showed that the compounds of the present invention in Table 6 had significant inhibitory effect on the proliferation of HCC1806 human breast squamous carcinoma cells.

### 4. Inhibitory effect of compounds on SKOV-3 cell proliferation

(1) Cell plating: First, tumor cells SKOV-3 were cultured in corresponding medium, then digested with trypsin, centrifuged, resuspended, and counted, and the cells were adjusted to an appropriate concentration for plating. The source of the tumor cells was listed in Table 7.

**Table 7. Source of tumor cells**

| Cell name | Tumor type | Source |
|---|---|---|
| SKOV-3 | Human ovarian cancer cells | Nanjing Cobioer Biosciences |

Co-incubation of the compound of the present invention and tumor cells: after the cells adhered to the wall, the medium was removed, and the diluted bioactive molecule (the compound of the present invention) was added to the wells of the above plate, and incubated for 72 h.

In vitro cell viability detection: After the incubation, 50 µL of Cell Counting-Lite^{™} 2.0 reagent (Vazyme/Novazyme) was added to each well, shaken and mixed well in the dark, reacted for 10 minutes, and then the detection was carried out with a microplate reader (manufacturer: BMG, Model: PHERAStar-FS) for reading. The background RLU was obtained by using cell-free medium (with Cell Counting-Lite^{™}), and the vehicle RLU was obtained by using cell-containing medium (with Cell Counting-Lite^{™}). Cell inhibition rate = 1 - (sample RLU - sample RLU)/(solvent RLU - background RLU) × 100%. According to the four-parameter model fitting curve, the half maximal inhibitory concentration (IC50) of the compound was calculated, and the detection results were shown in Table 8.

### (2) Data results

**Table 8. Inhibitory activity on SKOV-3 cell proliferation**

| Name | IC₅₀ (nM) |
|---|---|
| Comparative Compound 1 | 17.14 |
| Example 1 (1-1-A) | 7.81 |
| Example 3 (2-1-A) | 7.12 |
| Example 4 (2-7-A) | 8.64 |
| Example 4 (2-7-B) | 9.47 |
| Example 5 (2-12-A) | 6.85 |
| Example 5 (2-12-B) | 4.99 |
| Example 5 (2-12-C) | 6.52 |
| Example 7 (2-20-A) | 6.79 |
| Example 12 (5-13-A) | 2.09 |
| Example 21 (4-14) | 2.16 |

The detection results showed that the compounds of the present invention in Table 8 had significant inhibitory effect on the proliferation of SKOV-3 human ovarian cancer cells.

### 5. Inhibitory effect of compounds on NCI-H358 cell proliferation

(1) Cell plating: First, tumor cells NCI-H358 were cultured in corresponding medium, then digested with trypsin, centrifuged, resuspended, and counted, and the cells were adjusted to an appropriate concentration for plating. The source of the tumor cells was listed in Table 9.

**Table 9. Source of tumor cells**

| Cell name | Tumor type | Source |
|---|---|---|
| NCI-H358 | Human non-small cell lung cancer cells | Nanjing Cobioer Biosciences |

Co-incubation of the compound of the present invention and tumor cells: after the cells adhered to the wall, the medium was removed, and the diluted bioactive molecule (the compound of the present invention) was added to the wells of the above plate, and incubated for 72 h.

In vitro cell viability detection: After the incubation, 50 µL of Cell Counting-Lite^{™} 2.0 reagent (Vazyme/Novazyme) was added to each well, shaken and mixed well in the dark, reacted for 10 minutes, and then the detection was carried out with a microplate reader (manufacturer: BMG, Model: PHERAStar-FS) for reading. The background RLU was obtained by using cell-free medium (with Cell Counting-Lite^{™}), and the vehicle RLU was obtained by using cell-containing medium (with Cell Counting-Lite^{™}). Cell inhibition rate = 1 - (sample RLU - sample RLU)/(solvent RLU - background RLU) × 100%. According to the four-parameter model fitting curve, the half maximal inhibitory concentration (IC50) of the compound was calculated, and the detection results were shown in Table 10.

### (2) Data results

**Table 10. Inhibitory activity on NCI-H358 cell proliferation**

| Name | IC₅₀ (nM) |
|---|---|
| Comparative Compound 1 | 58.84 |
| Example 1 (1-1-A) | 65.22 |
| Example 2 (1-7-A) | 11.38 |
| Example 2 (1-7-B) | 51.52 |
| Example 3 (2-1-A) | 68.62 |
| Example 4 (2-7-A) | 35.42 |
| Example 4 (2-7-B) | 51.06 |
| Example 5 (2-12-A) | 17.65 |
| Example 5 (2-12-B) | 15.76 |
| Example 5 (2-12-C) | 23.08 |
| Example 7 (2-20-A) | 18.56 |
| Example 12 (5-13-A) | 7.01 |
| Example 16 (3-12-A) | 15.45 |
| Example 16 (3-12-B) | 19.77 |
| Example 16 (3-12-C) | 28.29 |
| Example 16 (3-12-D) | 20.75 |
| Example 17 (3-7-A) | 49.38 |
| Example 17 (3-7-B) | 22.22 |
| Example 18 (3-17-A) | 13.34 |
| Example 18 (3-17-B) | 37.34 |
| Example 19 (5-22-A) | 29.94 |
| Example 19 (5-22-B) | 61.36 |
| Example 21 (4-14) | 11.98 |
| Example 22 (4-15) | 17.76 |
| Example 24 (2-27-A) | 56.46 |
| Example 24 (2-27-B) | 69.12 |
| Example 25 (3-26-A) | 31.64 |

The detection results showed that the compounds of the present invention in Table 10 had a significant inhibitory effect on the proliferation of NCI-H358 human non-small cell lung cancer cells.

### 6. Inhibitory effect of compounds on NCI-N87 cell proliferation

(1) Cell plating: First, tumor cells NCI-N87 were cultured in corresponding medium, then digested with trypsin, centrifuged, resuspended, and counted, and the cells were adjusted to an appropriate concentration for plating. The source of the tumor cells was listed in Table 11.

**Table 11. Source of tumor cells**

| Cell name | Tumor type | Source |
|---|---|---|
| NCI-N87 | Human gastric cancer cells | ATCC |

Co-incubation of the compound of the present invention and tumor cells: after the cells adhered to the wall, the medium was removed, and the diluted bioactive molecule (the compound of the present invention) was added to the wells of the above plate, and incubated for 72 h.

In vitro cell viability detection: After the incubation, 50 µL of Cell Counting-Lite^{™} 2.0 reagent (Vazyme/Novazyme) was added to each well, shaken and mixed well in the dark, reacted for 10 minutes, and then the detection was carried out with a microplate reader (manufacturer: BMG, Model: PHERAStar-FS) for reading. The background RLU was obtained by using cell-free medium (with Cell Counting-Lite^{™}), and the vehicle RLU was obtained by using cell-containing medium (with Cell Counting-Lite^{™}). Cell inhibition rate = 1 - (sample RLU - sample RLU)/(solvent RLU - background RLU) × 100%. According to the four-parameter model fitting curve, the half maximal inhibitory concentration (IC50) of the compound was calculated, and the detection results were shown in Table 12.

### (2) Data results

**Table 12. Inhibitory activity on NCI-N87 cell proliferation**

| Name | IC₅₀(nM) |
|---|---|
| Comparative Compound 1 | 7.18 |
| Example 1 (1-1-A) | 4.58 |
| Example 2 (1-7-A) | 2.70 |
| Example 2 (1-7-B) | 5.48 |
| Example 4 (2-7-A) | 3.76 |
| Example 5 (2-12-A) | 4.91 |
| Example 5 (2-12-B) | 4.97 |
| Example 5 (2-12-C) | 4.51 |
| Example 7 (2-20-A) | 4.54 |
| Example 8 (3-1-A) | 0.37 |
| Example 8 (3-1-B) | 1.34 |
| Example 9 (3-4-A) | 4.38 |
| Example 9 (3-4-B) | 6.51 |
| Example 11 (4-10) | 2.29 |
| Example 12 (5-13-A) | 2.33 |
| Example 13 (5-7-A) | 6.03 |
| Example 13 (5-7-B) | 7.31 |
| Example 16 (3-12-A) | 0.44 |
| Example 16 (3-12-B) | 3.45 |
| Example 16 (3-12-C) | 3.99 |
| Example 16 (3-12-D) | 3.03 |
| Example 17 (3-7-A) | 5.32 |
| Example 17 (3-7-B) | 4.54 |
| Example 18 (3-17-A) | 3.01 |
| Example 18 (3-17-B) | 4.19 |
| Example 19 (5-22-A) | 3.35 |
| Example 19 (5-22-B) | 5.51 |
| Example 24 (2-27-A) | 8.32 |
| Example 24 (2-27-B) | 14.67 |
| Example 25 (3-26-A) | 1.23 |
| Example 25 (3-26-B) | 25.34 |

The detection results showed that the compounds of the present invention in Table 12 had significant inhibitory effect on the proliferation of NCI-N87 human gastric cancer cells.

### 7. Inhibitory effect of compounds on Hela cell proliferation

(1) Cell plating: First, tumor cells Hela were cultured in corresponding medium, then digested with trypsin, centrifuged, resuspended, and counted, and the cells were adjusted to an appropriate concentration for plating. The source of the tumor cells was listed in Table 13.

**Table 13. Source of tumor cells**

| Cell name | Tumor type | Source |
|---|---|---|
| Hela | Human cervical cancer cells | Nanjing Cobioer Biosciences |

Co-incubation of the compound of the present invention and tumor cells: after the cells adhered to the wall, the medium was removed, and the diluted bioactive molecule (the compound of the present invention) was added to the wells of the above plate, and incubated for 72 h.

In vitro cell viability detection: After the incubation, 50 µL of Cell Counting-Lite^{™} 2.0 reagent (Vazyme/Novazyme) was added to each well, shaken and mixed well in the dark, reacted for 10 minutes, and then the detection was carried out with a microplate reader (manufacturer: BMG, Model: PHERAStar-FS) for reading. The background RLU was obtained by using cell-free medium (with Cell Counting-Lite^{™}), and the vehicle RLU was obtained by using cell-containing medium (with Cell Counting-Lite^{™}). Cell inhibition rate = 1 - (sample RLU - sample RLU)/(solvent RLU - background RLU) × 100%. According to the four-parameter model fitting curve, the half maximal inhibitory concentration (IC50) of the compound was calculated, and the detection results were shown in Table 14.

### (2) Data results

**Table 14. Inhibitory activity on Hela cell proliferation**

| Name | IC₅₀(nM) |
|---|---|
| Comparative Compound 1 | 17.57 |
| Example 1 (1-1-A) | 4.57 |
| Example 1 (1-1-B) | 3.65 |
| Example 3 (2-1-A) | 16.98 |
| Example 4 (2-7-A) | 13.77 |
| Example 5 (2-12-A) | 13.25 |
| Example 5 (2-12-B) | 16.68 |
| Example 7 (2-20-A) | 11.17 |
| Example 11 (4-10) | 3.04 |

The detection results showed that the compounds of the present invention in Table 14 had significant inhibitory effect on the proliferation of Hela human cervical cancer cells.

### 8. Inhibitory effect of compounds on HCC70 cell proliferation

(1) Cell plating: First, tumor cells HCC70 were cultured in corresponding medium, then digested with trypsin, centrifuged, resuspended, and counted, and the cells were adjusted to an appropriate concentration for plating. The source of the tumor cells was listed in Table 15.

**Table 15. Source of tumor cells**

| Cell name | Tumor type | Source |
|---|---|---|
| HCC70 | Human breast cancer cells | Nanjing Cobioer Biosciences |

Co-incubation of the compound of the present invention and tumor cells: after the cells adhered to the wall, the medium was removed, and the diluted bioactive molecule (the compound of the present invention) was added to the wells of the above plate, and incubated for 72 h.

In vitro cell viability detection: After the incubation, 50 µL of Cell Counting-Lite^{™} 2.0 reagent (Vazyme/Novazyme) was added to each well, shaken and mixed well in the dark, reacted for 10 minutes, and then the detection was carried out with a microplate reader (manufacturer: BMG, Model: PHERAStar-FS) for reading. The background RLU was obtained by using cell-free medium (with Cell Counting-Lite^{™}), and the vehicle RLU was obtained by using cell-containing medium (with Cell Counting-Lite^{™}). Cell inhibition rate = 1 - (sample RLU - sample RLU)/(solvent RLU - background RLU) × 100%. According to the four-parameter model fitting curve, the half maximal inhibitory concentration (IC50) of the compound was calculated, and the detection results were shown in Table 16.

### (2) Data results

**Table 16. Inhibitory activity on HCC70 cell proliferation**

| Name | IC₅₀(nM) |
|---|---|
| Comparative Compound 1 | 226.10 |
| Example 1 (1-1-A) | 224.85 |
| Example 4 (2-7-A) | 150.70 |
| Example 4 (2-7-B) | 192.20 |
| Example 5 (2-12-A) | 170.93 |
| Example 5 (2-12-B) | 176.16 |
| Example 5 (2-12-C) | 210.68 |
| Example 7 (2-20-A) | 104.99 |
| Example 12 (5-13-A) | 36.65 |

The detection results showed that the compounds of the present invention in Table 16 had significant inhibitory effect on the proliferation of HCC70 human breast cancer cells.

### 9. Inhibitory effect of compounds on MDA-MB-231 cell proliferation

(1) Cell plating: First, tumor cells MDA-MB-231 were cultured in corresponding medium, then digested with trypsin, centrifuged, resuspended, and counted, and the cells were adjusted to an appropriate concentration for plating. The source of the tumor cells was listed in Table 17.

**Table 17. Source of tumor cells**

| Cell name | Tumor type | Source |
|---|---|---|
| MDA-MB-231 | Human breast cancer cells | Nanjing Cobioer Biosciences |

Co-incubation of the compound of the present invention and tumor cells: after the cells adhered to the wall, the medium was removed, and the diluted bioactive molecule (the compound of the present invention) was added to the wells of the above plate, and incubated for 72 h.

In vitro cell viability detection: After the incubation, 50 µL of Cell Counting-Lite^{™} 2.0 reagent (Vazyme/Novazyme) was added to each well, shaken and mixed well in the dark, reacted for 10 minutes, and then the detection was carried out with a microplate reader (manufacturer: BMG, Model: PHERAStar-FS) for reading. The background RLU was obtained by using cell-free medium (with Cell Counting-Lite^{™}), and the vehicle RLU was obtained by using cell-containing medium (with Cell Counting-Lite^{™}). Cell inhibition rate = 1 - (sample RLU - sample RLU)/(solvent RLU - background RLU) × 100%. According to the four-parameter model fitting curve, the half maximal inhibitory concentration (IC50) of the compound was calculated, and the detection results were shown in Table 18.

### (2) Data results

**Table 18. Inhibitory activity on MDA-MB-231 cell proliferation**

| Name | IC₅₀(nM) |
|---|---|
| Comparative Compound 1 | 381.70 |
| Example 1 (1-1-A) | 363.60 |
| Example 3 (2-1-A) | 299.70 |
| Example 4 (2-7-A) | 123.50 |
| Example 5 (2-12-A) | 123.50 |
| Example 9 (3-4-A) | 284.80 |
| Example 12 (5-13-A) | 332.60 |
| Example 13 (5-7-B) | 172.40 |
| Example 20 (1-10-A) | 97.57 |
| Example 20 (1-10-B) | 27.24 |
| Example 22 (4-15) | 175.40 |

The detection results showed that the compounds of the present invention in Table 18 had significant inhibitory effect on the proliferation of MDA-MB-231 human breast cancer cells.

### 10. Inhibitory effect of compounds on Jeko-1 cell proliferation

(1) Cell plating: First, tumor cells Jeko-1 were cultured in corresponding medium, then digested with trypsin, centrifuged, resuspended, and counted, and the cells were adjusted to an appropriate concentration for plating. The source of the tumor cells was listed in Table 19.

**Table 19. Source of tumor cells**

| Cell name | Tumor type | Source |
|---|---|---|
| Jeko-1 | Human mantle cell lymphoma | ATCC |

Co-incubation of the compound of the present invention and tumor cells: after the cells adhered to the wall, the medium was removed, and the diluted bioactive molecule (the compound of the present invention) was added to the wells of the above plate, and incubated for 72 h.

In vitro cell viability detection: After the incubation, 50 µL of Cell Counting-Lite^{™} 2.0 reagent (Vazyme/Novazyme) was added to each well, shaken and mixed well in the dark, reacted for 10 minutes, and then the detection was carried out with a microplate reader (manufacturer: BMG, Model: PHERAStar-FS) for reading. The background RLU was obtained by using cell-free medium (with Cell Counting-Lite^{™}), and the vehicle RLU was obtained by using cell-containing medium (with Cell Counting-Lite^{™}). Cell inhibition rate = 1 - (sample RLU - sample RLU)/(solvent RLU - background RLU) × 100%. According to the four-parameter model fitting curve, the half maximal inhibitory concentration (IC50) of the compound was calculated, and the detection results were shown in Table 20.

### (2) Data results

**Table 20. Inhibitory activity on Jeko-1 cell proliferation**

| Name | IC₅₀(nM) |
|---|---|
| Comparative Compound 1 | 1.25 |
| Example 1 (1-1-A) | 1.33 |
| Example 2 (1-7-A) | 0.15 |
| Example 2 (1-7-B) | 0.66 |
| Example 4 (2-7-A) | 0.28 |
| Example 9 (3-4-A) | 0.27 |
| Example 9 (3-4-B) | 0.15 |
| Example 11 (4-10) | 0.45 |
| Example 13 (5-7-A) | 0.16 |
| Example 13 (5-7-B) | 0.18 |
| Example 21 (4-14) | 0.34 |
| Example 22 (4-15) | 0.11 |

The detection results showed that the compounds of the present invention in Table 20 had significant inhibitory effect on the proliferation of Jeko-1 human mantle cell lymphoma cells.

### 11. Inhibitory effect of compounds on MDA-MB-453 cell proliferation

(1) Cell plating: First, tumor cells MDA-MB-453 were cultured in corresponding medium, then digested with trypsin, centrifuged, resuspended, and counted, and the cells were adjusted to an appropriate concentration for plating. The source of the tumor cells was listed in Table 21.

**Table 21. Source of tumor cells**

| Cell name | Tumor type | Source |
|---|---|---|
| MDA-MB-453 | Human breast cancer cells | Concortis |

Co-incubation of the compound of the present invention and tumor cells: after the cells adhered to the wall, the medium was removed, and the diluted bioactive molecule (the compound of the present invention) was added to the wells of the above plate, and incubated for 72 h.

In vitro cell viability detection: After the incubation, 50 µL of Cell Counting-Lite^{™} 2.0 reagent (Vazyme/Novazyme) was added to each well, shaken and mixed well in the dark, reacted for 10 minutes, and then the detection was carried out with a microplate reader (manufacturer: BMG, Model: PHERAStar-FS) for reading. The background RLU was obtained by using cell-free medium (with Cell Counting-Lite^{™}), and the vehicle RLU was obtained by using cell-containing medium (with Cell Counting-Lite^{™}). Cell inhibition rate = 1 - (sample RLU - sample RLU)/(solvent RLU - background RLU) × 100%. According to the four-parameter model fitting curve, the half maximal inhibitory concentration (IC50) of the compound was calculated, and the detection results were shown in Table 22.

### (2) Data results

**Table 22. Inhibitory activity on MDA-MB-453 cell proliferation**

| Name | IC₅₀(nM) |
|---|---|
| Comparative Compound 1 | 6.99 |
| Example 16 (3-12-A) | 0.47 |
| Example 16 (3-12-B) | 4.32 |
| Example 16 (3-12-C) | 5.52 |
| Example 16 (3-12-D) | 3.98 |
| Example 17 (3-7-A) | 6.23 |
| Example 17 (3-7-B) | 4.87 |
| Example 18 (3-17-A) | 3.32 |
| Example 18 (3-17-B) | 6.16 |
| Example 19 (5-22-A) | 7.34 |
| Example 19 (5-22-B) | 5.67 |
| Example 24 (2-27-A) | 11.83 |
| Example 25 (3-26-A) | 3.95 |

The detection results showed that the compounds of the present invention in Table 22 had significant inhibitory effect on the proliferation of MDA-MB-453 human breast cancer cells.

The structures of Comparative Compound **1** and Comparative Compound **2** were as follows:

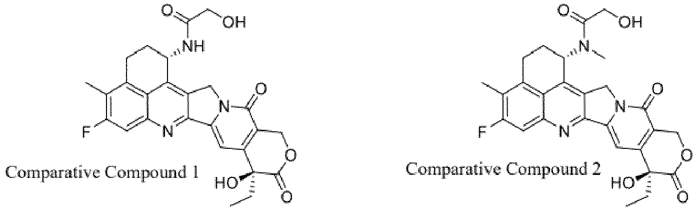

### 2. Antibody conjugation test

The preparation and conjugation of ADC D-L-15 sample was as follows:

1.036 mL of hIgG antibody (anti-chicken lysozyme antibody, 19.3 mg/mL) was diluted with 0.1M edetate disodium solution (pH 7.6), then adjusted with 1M Na₂HPO₄ solution to pH of 7.6, added with 10 mM TCEP (tris(2-carboxyethyl)phosphine) solution (pH 7.6) in an amount 2.4 times the amount of substance, mixed well and allowed to stand at room temperature for 90 minutes. The above solution system was added with Compound **D-L-15** dissolved in dimethyl sulfoxide in an amount 5 times the amount of substance, mixed well, allowed to stand at room temperature for 2 hours, and the buffer was replaced with 10 mM histidine buffer solution at pH 6.0 by using NAP-5 gel column (Cytiva), then sucrose and Tween-20 were added and mixed well to obtain the antibody drug conjugate ADC D-L-15 (1.77 mL, 8.60 mg/mL).

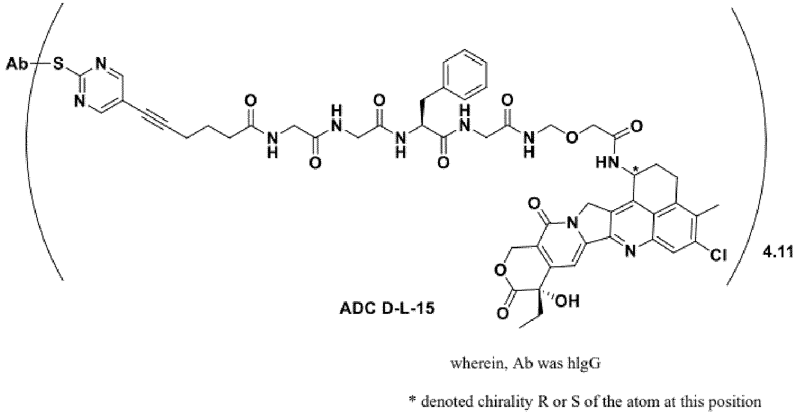

The molecular weight of ADC D-L-15 was determined by LC-MS, and the calculated drug to antibody ratio, i.e., DAR value, was 4.11, as shown in Table 23 and Table 24.

**Table 23: Measured molecular weight of ADC D-L-15**

| Peptide chain | | mAb | DAR1 | DAR2 | DAR3 | DAR4 |
|---|---|---|---|---|---|---|
| LC | Measured value | 23356.98 | 24384.43 | N/A | N/A | N/A |
| HC | Measured value | 50172.24 | 51199.41 | 52226.65 | 53253.93 | N/A |

**Table 24: DAR values for ADC D-L-15**

| Name | Maximum signal intensity | Ratio | DAR |
|---|---|---|---|
| LC-DAR0 | 3266.67 | 0.453 | 4.11 |
| LC-DAR1 | 3948.06 | 0.547 | |
| HC-DAR0 | 551.28 | 0.175 | |
| HC-DAR1 | 1141.65 | 0.362 | |
| HC-DAR2 | 774.39 | 0.246 | |
| HC-DAR3 | 685.97 | 0.218 | |
| HC-DAR4 | 0.00 | 0.000 | |

Chromatographic determination conditions:
Liquid chromatography column: Thermo MAbPac RP 3.0*100 mm;
Mobile phase A: 0.1 %FA/H₂O; Mobile phase B: 0.1%FA/ACN;
Flow rate: 0.25 mL/min; Sample chamber temperature: 8°C; Column temperature: 60°C; Injection volume: 2 µL;

| Time (min) | 2 | 20 | 22 | 25 | 26 | 30 |
|---|---|---|---|---|---|---|
| Mobile phase A (vol.%) | 75 | 60 | 5 | 5 | 75 | 75 |
| Mobile phase B (vol.%) | 25 | 40 | 95 | 95 | 25 | 25 |

Mass spectrometry conditions:
Mass spectrometer model: AB Sciex Triple TOF 5600+;
GS1 35; GS2 35; CUR 30; TEM 350; ISVF 5500; DP 250; CE 10; Accumulation time 0.5 s; m/z 600-4000; Time bins to sum 40.

The preparation and conjugation of ADC 3-4-04-A sample was as follows:

0.518 mL of hIgG antibody (anti-chicken lysozyme antibody, 19.3 mg/mL) was diluted with 0.1M edetate disodium solution (pH 7.6), then adjusted to pH7.6 with 1M Na₂HPO₄ solution, added with 10 mM TCEP (tris(2-carboxyethyl)phosphine) solution (pH 7.6) in an amount 5.5 times the amount of substance, mixed well and allowed to stand at room temperature for 90 minutes. The above solution system was added with Compound **3-4-04-A** dissolved in dimethyl sulfoxide in an amount 10 times the amount of substance, mixed well and allowed to stand at room temperature for 2 hours; the buffer was replaced with 10 mM histidine buffer solution at pH 6.0 by using NAP-5 gel column (Cytiva), then sucrose and Tween-20 were added and mixed well to obtain the antibody-drug conjugate ADC 3-4-04-A (1.50 mL, 5.60 mg/mL).

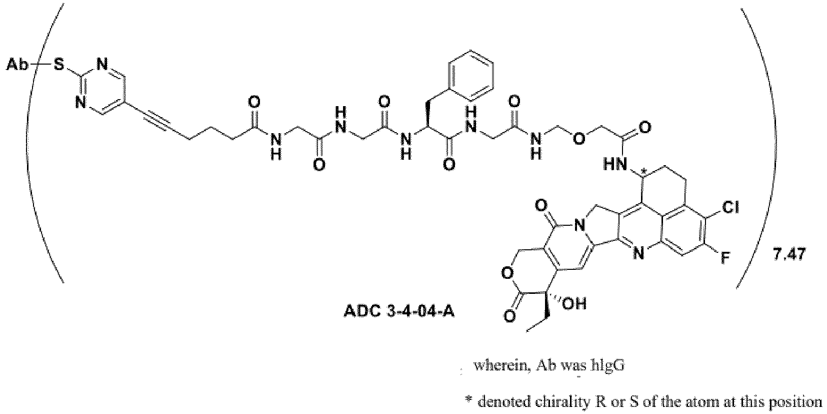

The molecular weight of ADC 3-4-04-A was determined by LC-MS, and the calculated drug to antibody ratio, i.e., DAR value, was 7.47, as shown in Table 25 and Table 26. The chromatographic determination conditions were the same as that of ADC D-L-15.

**Table 25: Measured molecular weight of ADC 3-4-04-A**

| Peptide chain | | mAb | DAR1 | DAR2 | DAR3 | DAR4 |
|---|---|---|---|---|---|---|
| LC | Measured value | 23356.79 | 24388.20 | N/A | N/A | N/A |
| HC | Measured value | 50171.57 | 51199.68 | 52233.48 | 53265.97 | 54297.82 |

**Table 26: DAR values for ADC 3-4-04-A**

| Name | Maximum signal intensity | Ratio | DAR |
|---|---|---|---|
| LC-DAR0 | 223.44 | 0.061 | 7.47 |
| LC-DAR1 | 3458.32 | 0.939 | |
| HC-DAR0 | 122.34 | 0.068 | |
| HC-DAR1 | 0.09 | 0.000 | |
| HC-DAR2 | 1.11 | 0.001 | |
| HC-DAR3 | 1682.87 | 0.932 | |
| HC-DAR4 | 0.18 | 0.000 | |

The preparation and conjugation of ADC 3-4-04-B sample was as follows:
0.518 mL of hIgG antibody (anti-chicken lysozyme antibody, 19.3 mg/mL) was diluted with 0.1M edetate disodium solution (pH 7.6), then adjusted to pH7.6 with 1M Na₂HPO₄ solution, added with 10 mM TCEP (tris(2-carboxyethyl)phosphine) solution (pH 7.6) in an amount 5.5 times the amount of substance, mixed well and allowed to stand at room temperature for 90 minutes. The above solution system was added with Compound **3-4-04-B** dissolved in dimethyl sulfoxide in an amount of 10 times the amount of substance, mixed well and allowed to stand at room temperature for 2 hours, and the buffer was replaced with 10 mM histidine buffer solution at pH 6.0 by using NAP-5 gel column (Cytiva), then sucrose and Tween-20 were added and mixed well to obtain the antibody-drug conjugate ADC 3-4-04-B (1.50 mL, 5.60 mg/mL).

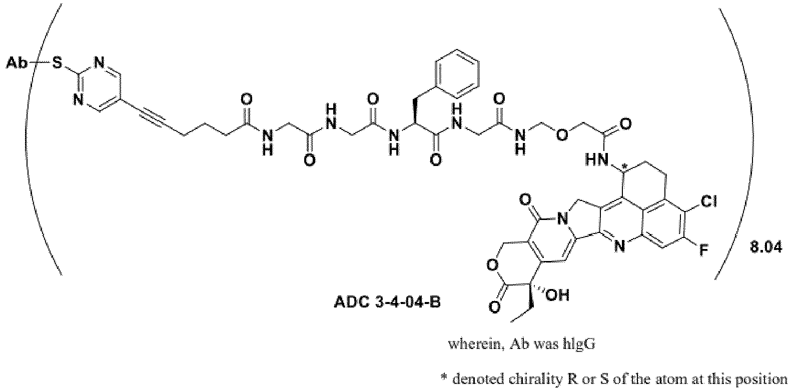

The molecular weight of ADC 3-4-04-B was determined by LC-MS, and the calculated drug to antibody ratio, i.e., DAR value, was 8.04, as shown in Table 27 and Table 28. The chromatographic determination conditions were the same as that of ADC D-L-15.

**Table 27: Measured molecular weight of ADC 3-4-04-B**

| Peptide chain | | mAb | DAR1 | DAR2 | DAR3 | DAR4 |
|---|---|---|---|---|---|---|
| LC | Measured value | 23356.32 | 24388.01 | N/A | N/A | N/A |
| HC | Measured value | 50166.19 | 51196.05 | 52232.67 | 53265.82 | 54296.20 |

**Table 28: DAR values for ADC 3-4-04-B**

| Name | Maximum signal intensity | Ratio | DAR |
|---|---|---|---|
| LC-DAR0 | 0.02 | 0.000 | 8.04 |
| LC-DAR1 | 3735.97 | 1.000 | |
| HC-DAR0 | 0.68 | 0.000 | |
| HC-DAR1 | 0.12 | 0.000 | |
| HC-DAR2 | 0.05 | 0.000 | |
| HC-DAR3 | 1867.41 | 0.980 | |
| HC-DAR4 | 36.69 | 0.019 | |

The above experiments prove that the cytotoxic drug-linker compound of the present invention could be successfully conjugated with an antibody to obtain an antibody drug conjugate.

Although the specific implementation of the present invention has been described in detail, those skilled in the art will understand that, according to all the teachings that have been disclosed, various modifications and substitutions can be made to those details, and these changes are all within the protection scope of the present invention. The full scope of the present invention is given by the appended claims and any equivalents thereof.

## Claims

1. A compound or a pharmaceutically acceptable salt, ester, stereoisomer, polymorph, solvate, nitrogen oxide, isotope-labeled product, metabolite or prodrug thereof, wherein the compound has the structure shown below: 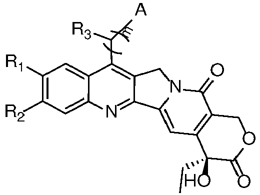 wherein,
R₁ and R₂ are each independently selected from the group consisting of hydrogen, halogen, C₁₋₆ alkyl, C₁₋₆ alkoxyl, C₁₋₆ haloalkyl, hydroxyl, cyano and C₃₋₆ cycloalkyl; or, R₁ and R₂ are connected with adjacent carbon atoms to form a 5- to 6-membered oxygen-containing heterocyclic ring;
R₃ is hydrogen or is connected with an ortho-carbon atom of R₁ to form a 6-membered carbocyclic ring;
A is selected from one of 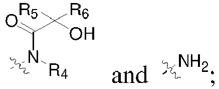
R₄ is selected from the group consisting of hydrogen, C₁₋₆ alkyl, C₁₋₆ haloalkyl, C₁₋₆ alkoxyalkyl, C₃₋₆ cycloalkyl and 3- to 6-membered heterocyclyl;
R₅ and R₆ are each independently selected from the group consisting of hydrogen, C₁₋₆ alkyl, C₁₋₆ haloalkyl, C₁₋₆ alkylaminoalkyl, C₁₋₆ alkoxyalkyl, C₂₋₆ alkenyl, C₂₋₆ alkynyl, C₃₋₆ cycloalkyl, 3- to 6-membered heterocyclyl, aryl and heteroaryl; or R₅ and R₆ are connected with adjacent carbon atoms to form a 3- to 6-membered carbocyclic or heterocyclic ring;
m=1 or 2.

2. The compound or a pharmaceutically acceptable salt, ester, stereoisomer, polymorph, solvate, nitrogen oxide, isotope-labeled product, metabolite or prodrug thereof according to claim 1, wherein the compound has the structure of Formula (I): 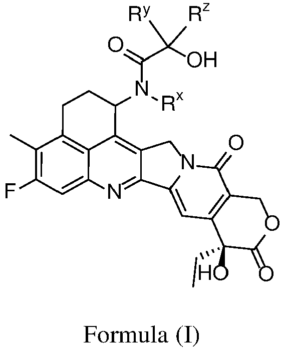
in the above Formula (I), R^{x} is selected from the group consisting of hydrogen, C₁₋₆ alkyl, C₁₋₆ haloalkyl, C₁₋₆ alkoxyalkyl, C₃₋₆ cycloalkyl and 3- to 6-membered heterocyclyl;
R^{y} and R^{z} are not hydrogen at the same time, and are independently selected from the group consisting of hydrogen, C₁₋₆ alkyl, C₁₋₆ haloalkyl, C₂₋₆ alkenyl, C₂₋₆ alkynyl, C₁₋₆ alkylaminoalkyl, C₁₋₆ alkoxyalkyl, 3- to 6-membered heterocyclylalkyl and 3- to 6-membered heterocyclyl;
preferably, R^{x} is selected from the group consisting of hydrogen and C₁₋₆ alkyl; preferably, R^{x} is hydrogen;
preferably, R^{y} and R^{z} are not hydrogen at the same time, and are independently selected from the group consisting of hydrogen 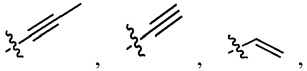 dimethylaminomethylene, morpholinomethylene and methoxymethylene;
preferably, R^{y} is hydrogen and R^{z} is selected from the group consisting of 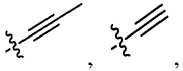 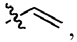 dimethylaminomethylene, morpholinomethylene and methoxymethylene.

3. The compound or a pharmaceutically acceptable salt, ester, stereoisomer, polymorph, solvate, nitrogen oxide, isotope-labeled product, metabolite or prodrug thereof according to claim 1, wherein the compound has the structure of Formula (II): 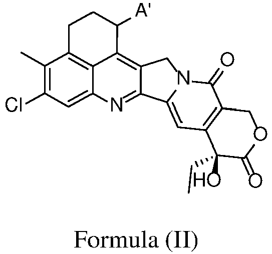
in the above Formula (II), A' is selected from one of 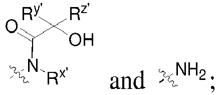
R^{x'} is selected from the group consisting of hydrogen, C₁₋₆ alkyl, C₁₋₆ haloalkyl, C₁₋₆ alkoxyalkyl, C₃₋₆ cycloalkyl and 3- to 6-membered heterocyclyl;
R^{y'} and R^{z'} are independently selected from the group consisting of hydrogen, C₁₋₆ alkyl, C₁₋₆ haloalkyl, C₁₋₆ alkoxyalkyl, C₁₋₆ alkylaminoalkyl, C₁₋₆ alkoxyalkyl, C₃₋₆ cycloalkyl, 3- to 6-membered heterocyclyl, 3- to 6-membered heterocyclylalkyl, C₂₋₆ alkenyl, C₂₋₆ alkynyl, aryl and heteroaryl, or R^{y'} and R^{z'} are connected with adjacent carbon atoms to form a 3- to 6-membered ring;
preferably, R^{x'} is selected from the group consisting of hydrogen and C₁₋₆ alkyl;
preferably, R^{y'} and R^{z'} are independently selected from the group consisting of hydrogen, C₁₋₆ alkyl, C₁₋₆ alkoxyalkyl, C₁₋₆ alkylaminoalkyl, C₃₋₆ cycloalkyl and C₂₋₆ alkenyl, or R^{y'} and R^{z'} are connected with adjacent carbon atoms to form a 3- to 6-membered cycloalkyl;
preferably, R^{y'} is selected from the group consisting of hydrogen and C₁₋₆ alkyl, R^{z'} is selected from the group consisting of hydrogen, C₁₋₆ alkyl and C₃₋₆ cycloalkyl, or R^{y} and R^{z'} are connected with adjacent carbon atoms to form a 3- to 6-membered cycloalkyl.

4. The compound or a pharmaceutically acceptable salt, ester, stereoisomer, polymorph, solvate, nitrogen oxide, isotope-labeled product, metabolite or prodrug thereof according to claim 1, wherein the compound has the structure of Formula (III): 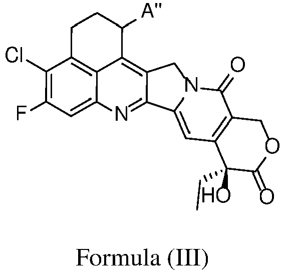
in the above Formula (III), A" is selected from one of 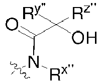 and 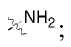
R^{x"} is selected from the group consisting of hydrogen, C₁₋₆ alkyl, C₁₋₆ haloalkyl, C₁₋₆ alkoxyalkyl, C₃₋₆ cycloalkyl and 3- to 6-membered heterocyclyl;
R^{y"} and R^{z"} are independently selected from the group consisting of hydrogen, C₁₋₆ alkyl, C₁₋₆ haloalkyl, C₁₋₆ alkoxyalkyl, C₁₋₆ alkylaminoalkyl, C₁₋₆ alkoxyalkyl, C₃₋₆ cycloalkyl, 3- to 6-membered heterocyclyl, 3- to 6-membered heterocyclylalkyl, 4- to 6-membered heterocyclyl, C₂₋₆ alkenyl, C₂₋₆ alkynyl, aryl and heteroaryl, or R^{y"} and R^{z"} are connected with adjacent carbon atoms to form a 3- to 6-membered ring;
preferably, R^{x"} is hydrogen;
preferably, R^{y"} and R^{z"} are each independently selected from the group consisting of hydrogen, C₁₋₆ alkyl, C₁₋₆ haloalkyl, C₁₋₆ alkoxyalkyl, C₁₋₆ alkylaminoalkyl, C₃₋₆ cycloalkyl and vinyl, or R^{y"} and R^{z"} are connected with adjacent carbon atoms to form a 3- to 6-membered ring;
preferably, R^{y"} is hydrogen, R^{z"} is selected from the group consisting of hydrogen, C₁₋₆ alkyl, C₃₋₆ cycloalkyl and vinyl, or R^{y"} and R^{z"} are connected with adjacent carbon atoms to form a 3- to 6-membered carbocyclic ring.

5. The compound or a pharmaceutically acceptable salt, ester, stereoisomer, polymorph, solvate, nitrogen oxide, isotope-labeled product, metabolite or prodrug thereof according to claim 1, wherein the compound has the structure of Formula (IV): 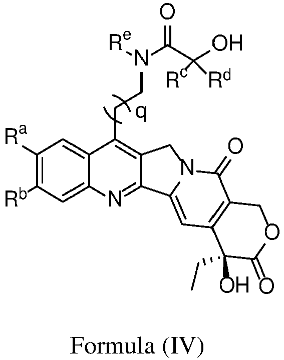 in the above Formula (IV),
R^{a} and R^{b} are independently selected from the group consisting of hydrogen, halogen, C₁₋₆ alkyl, C₁₋₆ hydroxyalkyl, C₁₋₆ alkoxyalkyl, C₁₋₆ alkoxy, C₁₋₆ haloalkyl, hydroxyl and cyano; or R^{a} and R^{b} are connected with adjacent carbon atoms to form a 5- to 6-membered oxygen-containing heterocyclic ring;
R^{c} and R^{d} are independently selected from the group consisting of hydrogen, C₁₋₆ alkyl, C₁₋₆ haloalkyl, C₁₋₆ alkoxyalkyl, C₁₋₆ alkylaminoalkyl, C₃₋₆ cycloalkyl, 3- to 6-membered heterocyclyl, 3- to 6-membered heterocyclylalkyl, C₂₋₆ alkenyl and C₂₋₆ alkynyl, or R^{c} and R^{d} are connected with adjacent carbon atoms to form a 3- to 6-membered carbocyclic or heterocyclic ring;
R^{e} is selected from the group consisting of hydrogen, C₁₋₆ alkyl, C₃₋₆ cycloalkyl, C₁₋₆ haloalkyl, C₁₋₆ alkoxyalkyl and C₂-C₅ heterocyclyl;
q=0 or 1;
when q=0, R^{c} and R^{d} are not hydrogen at the same time;
preferably, R^{a} and R^{b} are independently selected from the group consisting of hydrogen, halogen and C₁₋₆ alkyl, or R^{a} and R^{b} are connected with adjacent carbon atoms to form a 5- to 6-membered oxygen-containing heterocyclic ring;
preferably, R^{a} and R^{b} are independently selected from the group consisting of hydrogen, fluorine, chlorine and methyl, or R^{a} and R^{b} together with the benzene ring connected thereto form 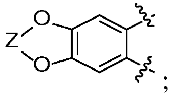
wherein Z is selected from the group consisting of -CH₂-, -CD₂-, -CH₂CH₂- and -CF₂-;
preferably, R^{a} is methyl, R^{b} is fluorine, or R^{a} and R^{b} together with the benzene ring connected thereto form 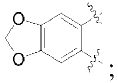
preferably, R^{c} and R^{d} are independently selected from the group consisting of hydrogen, 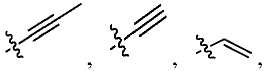 C₁₋₆ alkoxyalkyl and C₁₋₆ alkylaminoalkyl, or R^{c} and R^{d} are connected with adjacent carbon atoms to form a 3- to 6-membered carbocyclic ring;
preferably, R^{c} is hydrogen, R^{d} is selected from the group consisting of hydrogen, 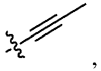 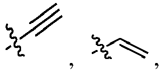 methoxyethyl and cyclopropyl, or R^{c} and R^{d} are connected with adjacent carbon atoms to form a 3- to 6-membered carbocyclic ring;
preferably, R^{e} is selected from the group consisting of hydrogen and C₁₋₆ alkyl.

6. The compound or a pharmaceutically acceptable salt, ester, stereoisomer, polymorph, solvate, nitrogen oxide, isotope-labeled product, metabolite or prodrug thereof according to claim 1, wherein the compound has the structure of Formula (V): 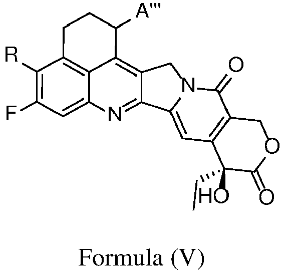
in the above Formula (V), R is selected from the group consisting of C₃₋₆ cycloalkyl and C₁₋₆ alkoxy;
A‴ is selected from one of 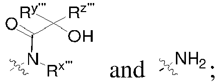
R^{x‴} is selected from the group consisting of hydrogen, C₁₋₆ alkyl, C₁₋₆ haloalkyl, C₁₋₆ alkoxyalkyl, C₃₋₆ cycloalkyl and 3- to 6-membered heterocyclyl (e.g., 3- to 6-membered carbocyclyl or 3- to 6-membered heterocyclyl);
R^{y‴} and R^{z‴} are independently selected from the group consisting of hydrogen, C₁₋₆ alkyl, C₁₋₆ haloalkyl, C₁₋₆ alkoxyalkyl, C₁₋₆ alkylaminoalkyl, C₃₋₆ cycloalkyl, 3- to 6-membered heterocyclyl, 3- to 6-membered heterocyclylalkyl, C₂₋₆ alkenyl, C₂₋₆ alkynyl, aryl and heteroaryl, or R^{y‴} and R^{z‴} are connected with adjacent carbon atoms to form a 3- to 6-membered ring;
preferably, R is selected from the group consisting of methyl, methoxy and cyclopropyl; preferably, R^{y‴} and R^{z‴} are each independently selected from the group consisting of hydrogen, C₁₋₆ alkyl, C₁₋₆ haloalkyl, C₁₋₆ alkoxyalkyl, C₁₋₆ alkylaminoalkyl, C₃₋₆ cycloalkyl and vinyl, or R^{y‴} and R^{z‴} are connected with adjacent carbon atoms to form a 3- to 6-membered carbocyclic ring;
preferably, both R^{y‴} and R^{z‴} are hydrogen, or R^{y‴} and R^{z‴} are connected with adjacent carbon atoms to form a 3- to 6-membered carbocyclic ring;
preferably, R^{x‴} is selected from the group consisting of hydrogen and C₁₋₆ alkyl;
preferably, R^{x‴} is hydrogen.

7. The compound or a pharmaceutically acceptable salt, ester, stereoisomer, polymorph, solvate, nitrogen oxide, isotope-labeled product, metabolite or prodrug thereof according to claim 1, wherein the compound has the structure as follows:

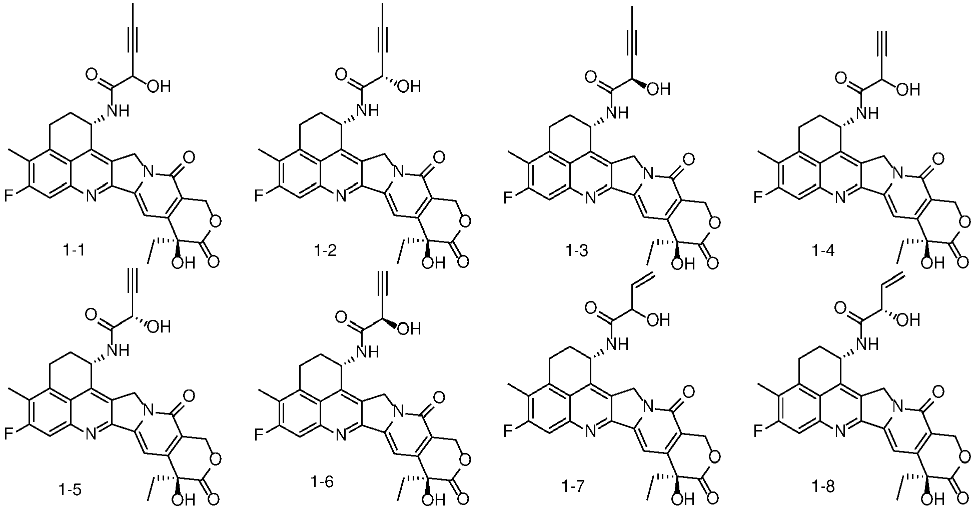

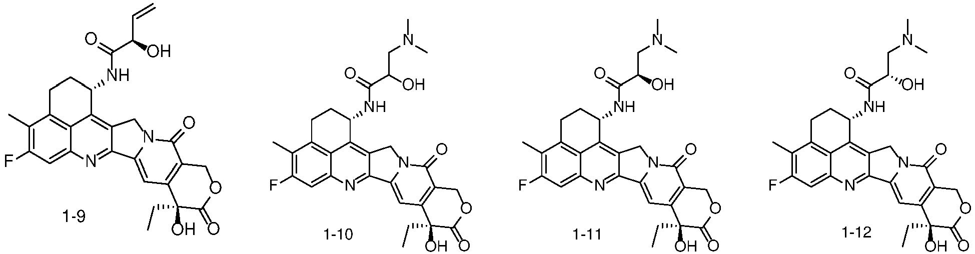

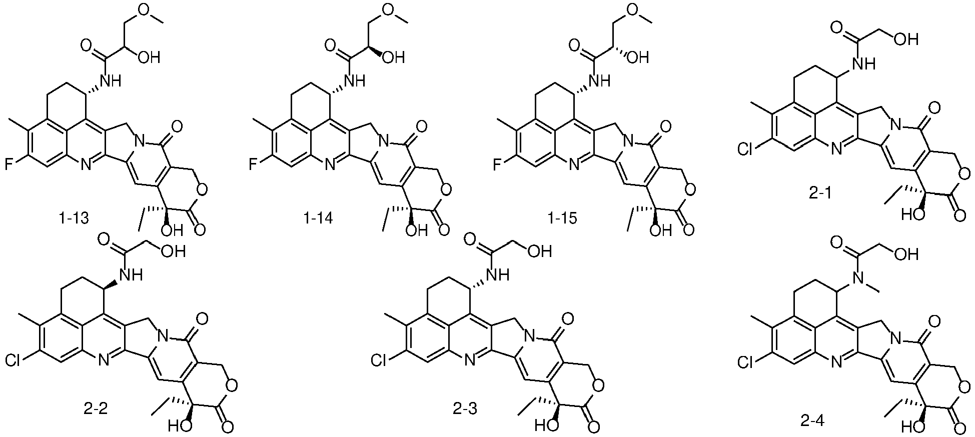

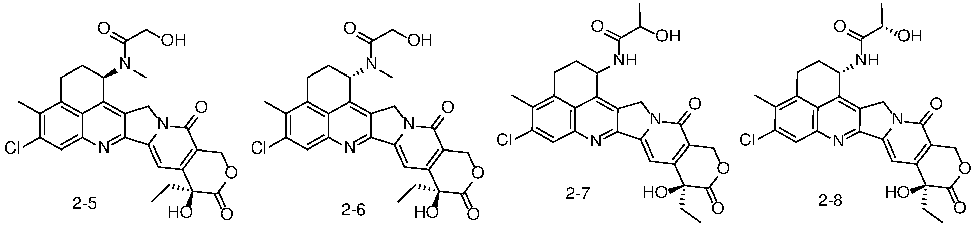

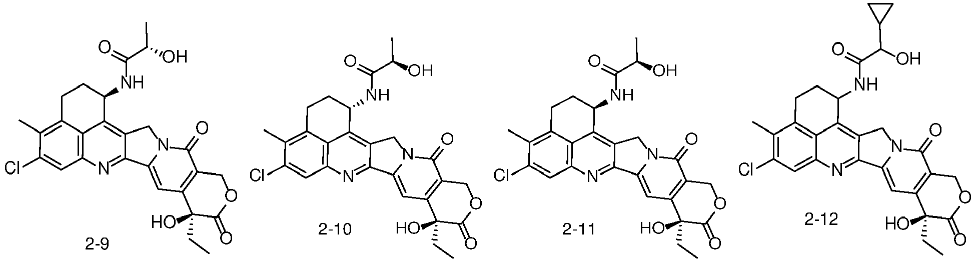

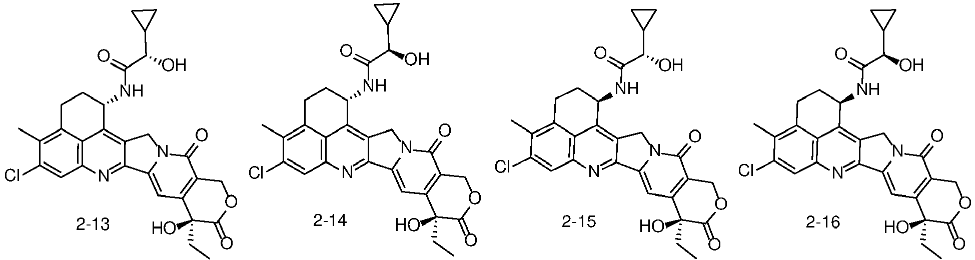

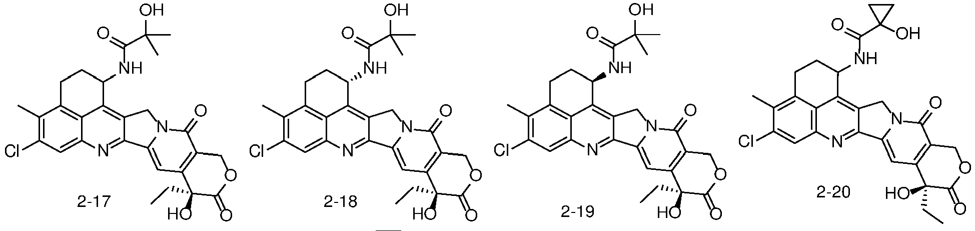

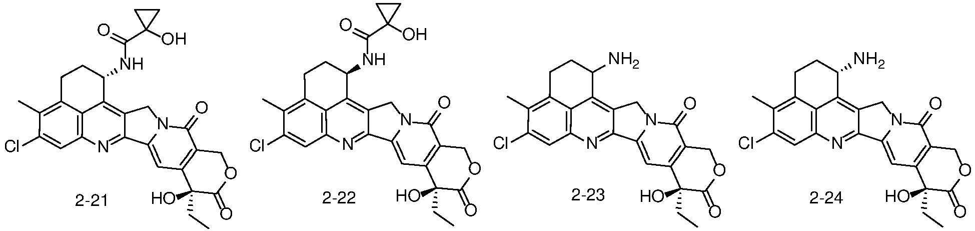

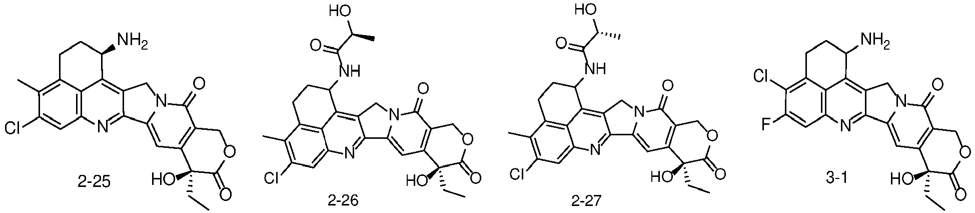

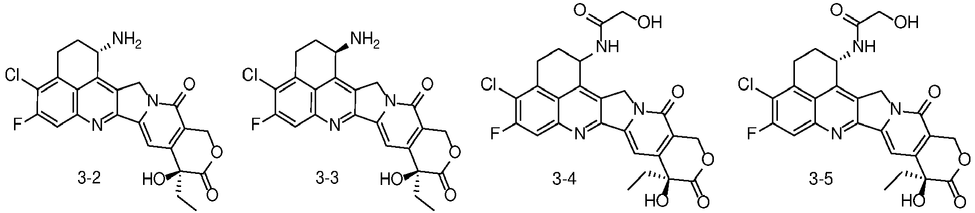

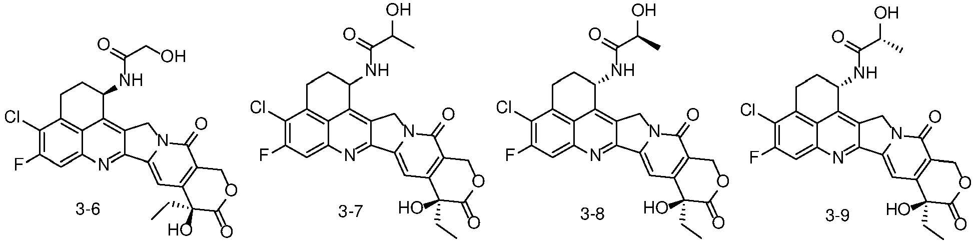

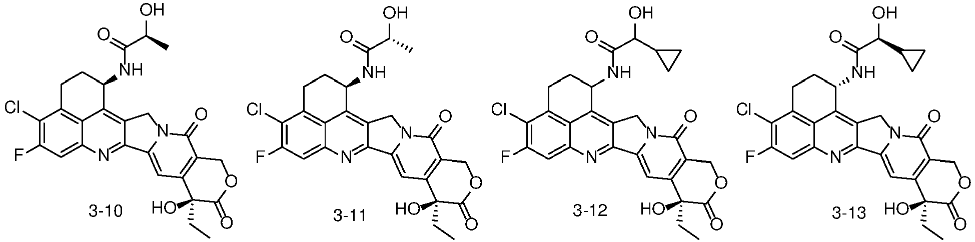

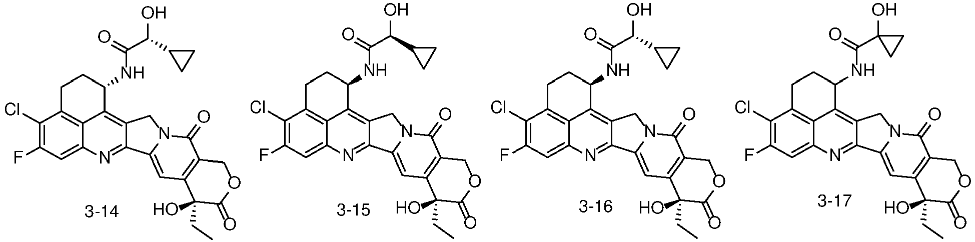

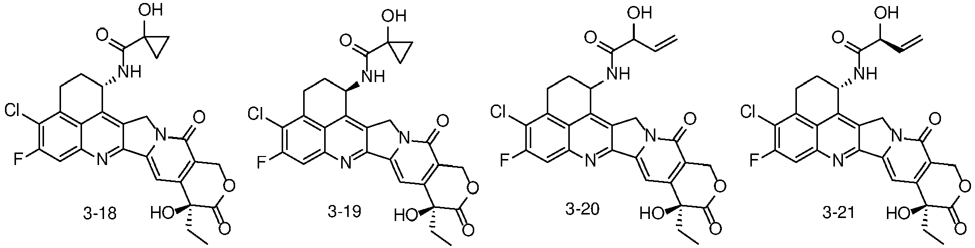

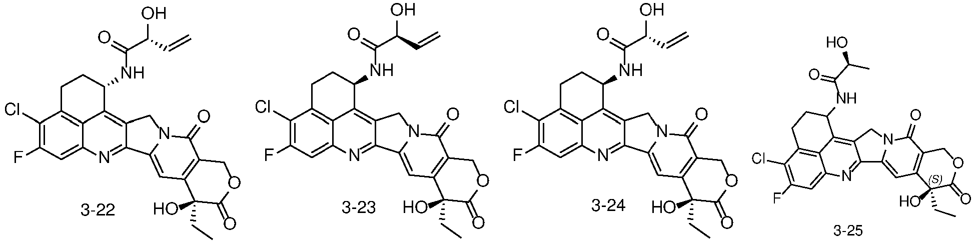

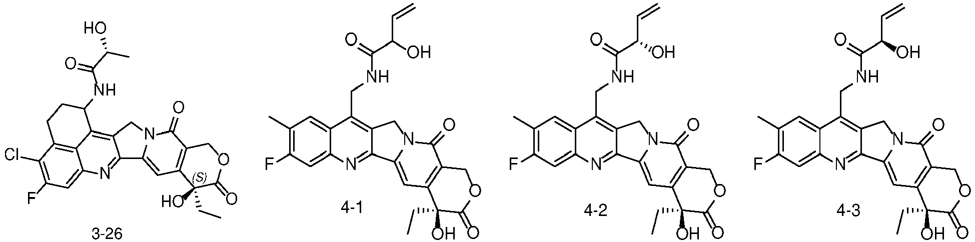

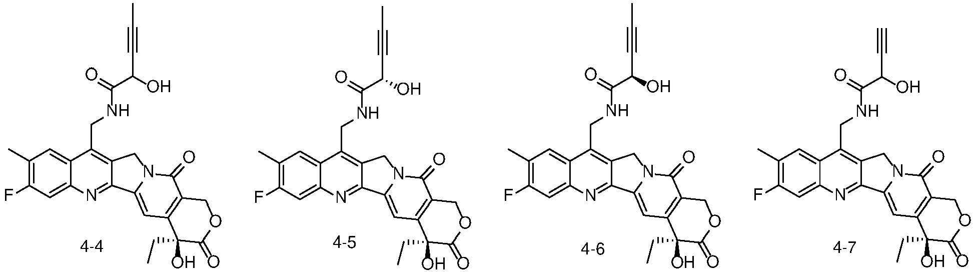

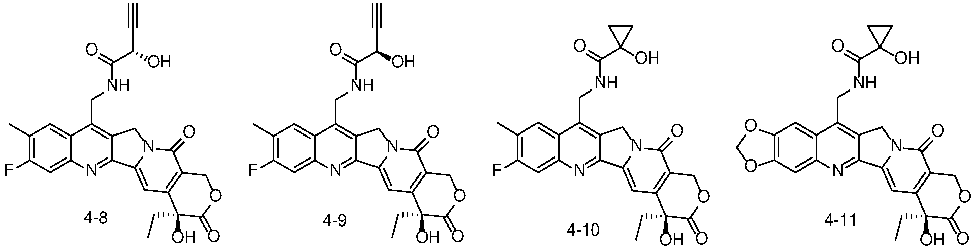

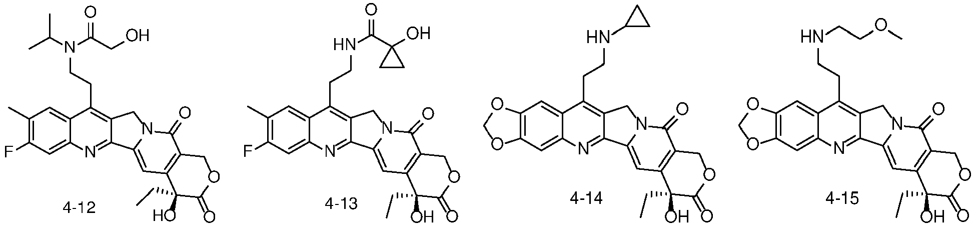

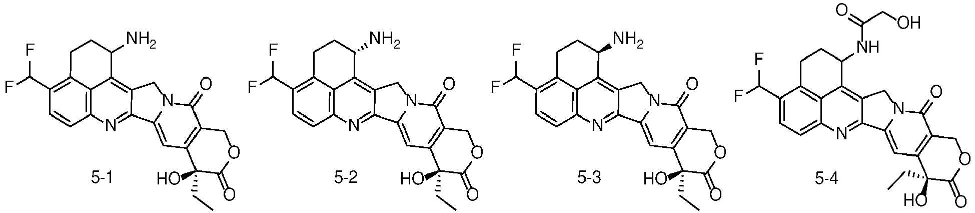

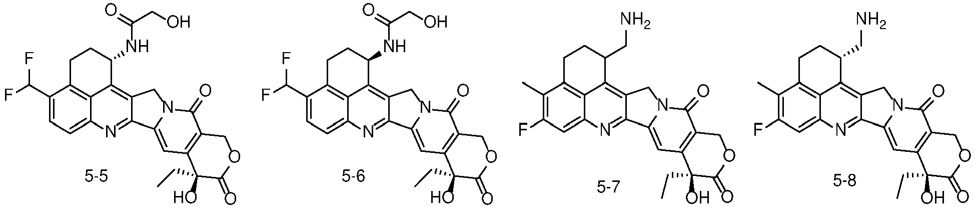

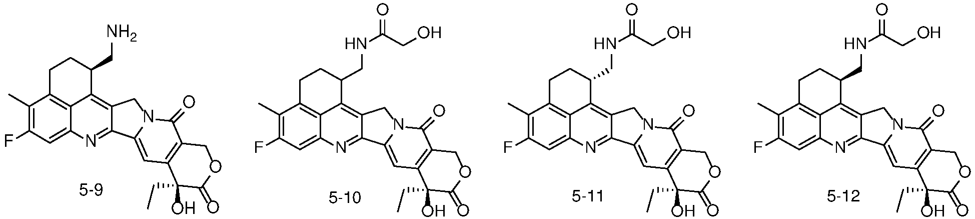

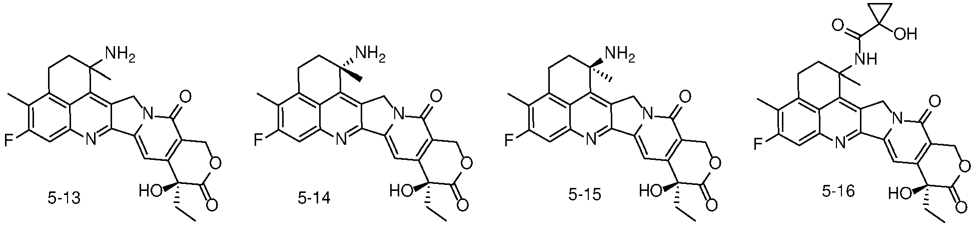

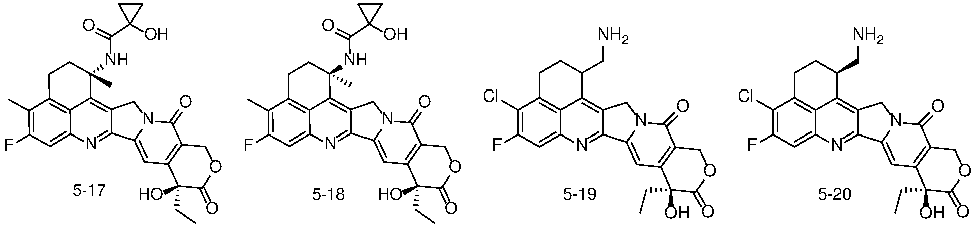

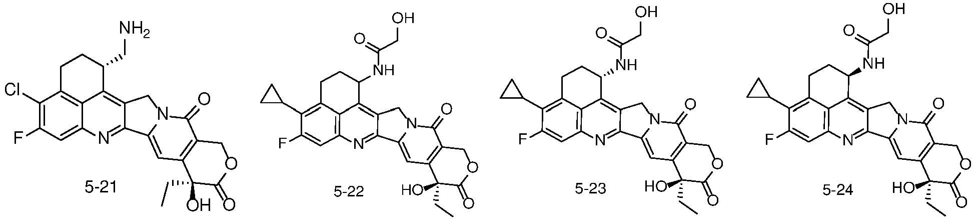

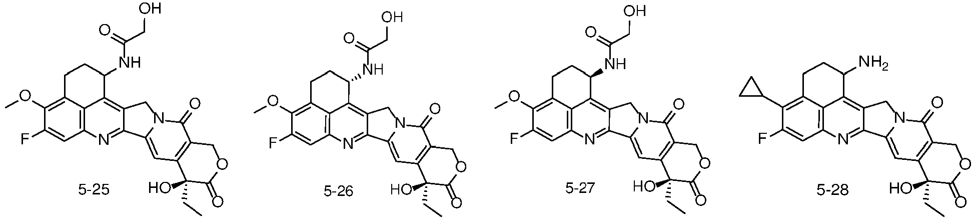

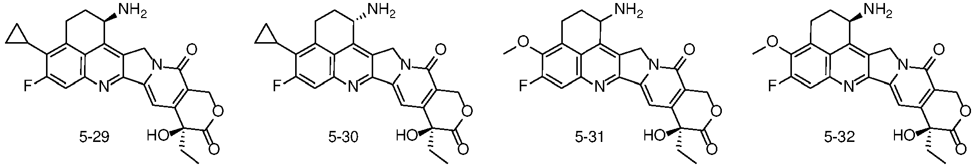

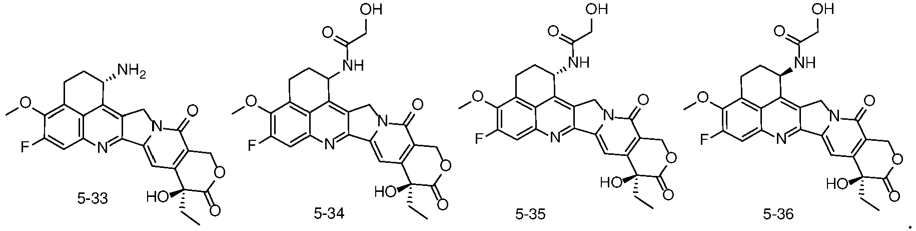

8. A compound represented by Formula (VI) or a pharmaceutically acceptable salt, ester, stereoisomer, polymorph, solvate, nitrogen oxide, isotope-labeled product, metabolite or prodrug thereof, wherein the compound has the structure as follows:
M-L-E-D Formula (VI)
wherein,
M is a linker moiety of an antibody or antigen-binding fragment thereof;
L is a linker connecting the linker moiety M and E;
E is a structural fragment connecting L and D;
D is a structural fragment of a cytotoxic drug;
preferably, M is selected from the group consisting of the following structures: 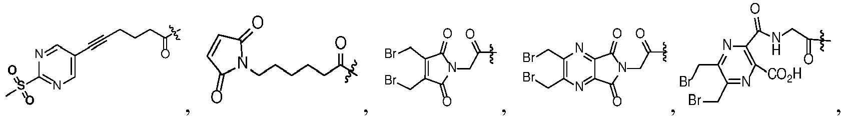 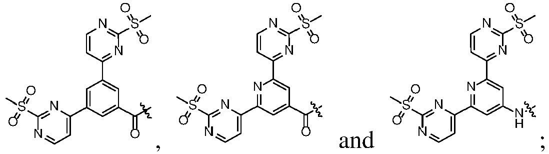
preferably, M is selected from the group consisting of the following structures: 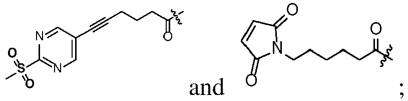
preferably, L is a divalent structure constituted of one or more selected from the group consisting of: C₁₋₆ alkylene, -N(R')-, carbonyl, -O-, Val, Cit, Phe, Lys, D-Val, Leu, Gly, Ala, Asn, Val-Cit, Val-Ala, Val-Lys, Val-Lys(Ac), Phe-Lys, Phe-Lys(Ac), D-Val-Leu-Lys, Gly- Gly-Arg, Ala-Ala-Asn, Ala-Ala-Ala, Val-Lys-Ala, Gly-Gly-Gly, Gly-Gly-Phe-Gly, Gly-Gly-Gly-Gly-Gly, 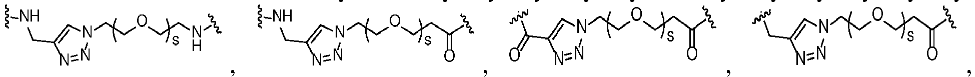 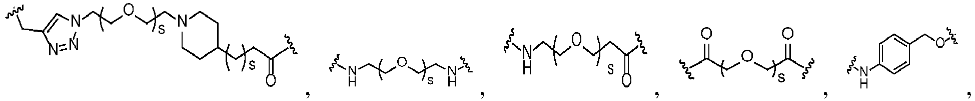 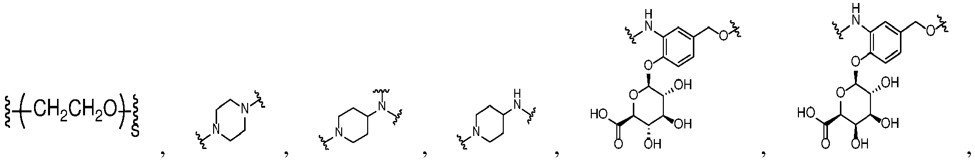 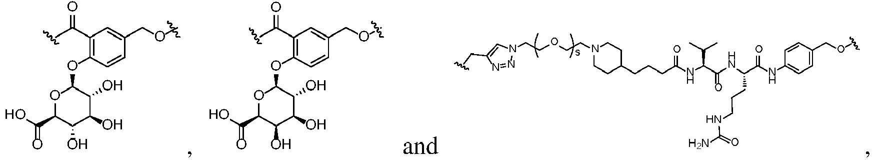
wherein R' represents hydrogen, C₁₋₆ alkyl or -(CH₂CH₂O)ᵣ-containing alkyl; r is an integer selected from 1 to 10; s is an integer selected from 1 to 10;
preferably, L is selected from the group consisting of the following structures: 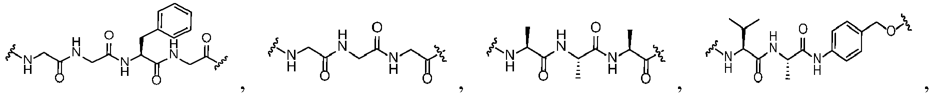 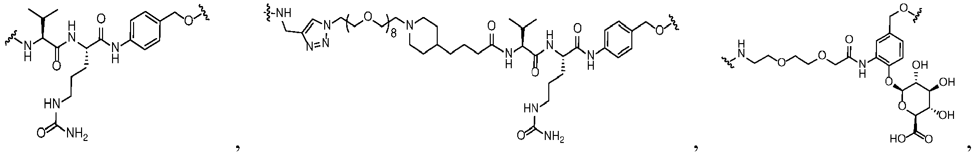 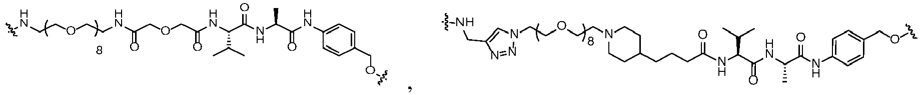 and 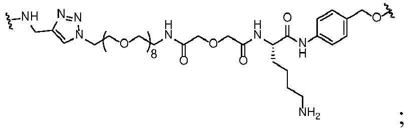
preferably, L is selected from the following structure: 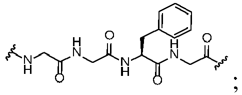
preferably, E is selected from the group consisting of single bond, -NH-CH₂-, 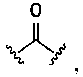 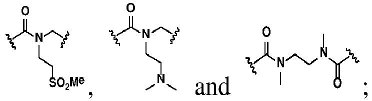
preferably, E is -NH-CH₂-;
preferably, the cytotoxic drug is selected from the compound according to any one of claims 1 to 6;
preferably, the cytotoxic drug is selected from the group consisting of the compounds 1-1 to 1-15; 2-1 to 2-27; 3-1 to 3-26; 4-1 to 4-15; or 5-1 to 5-36 according to claim 7;
preferably, D is selected from the structure formed by removing a hydrogen atom from the compound according to any one of claims 1-6;
preferably, D is selected from the structure form by removing a hydrogen atom from the compounds 1-1 to 1-15; 2-1 to 2-27; 3-1 to 3-26; 4-1 to 4-15; or 5-1 to 5-36 according to claim 7;
preferably, D is selected from the group consisting of the following structures: 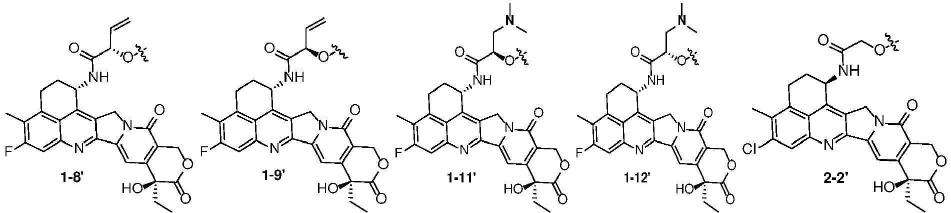 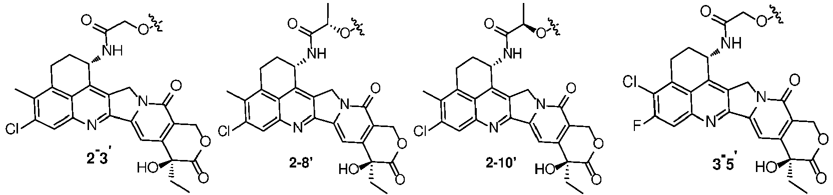 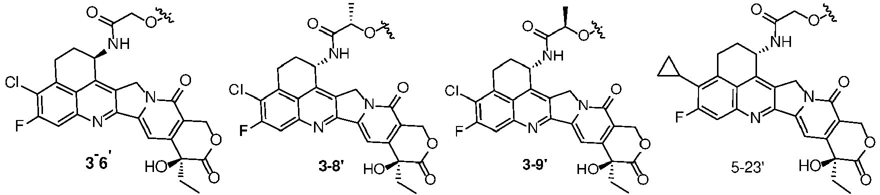 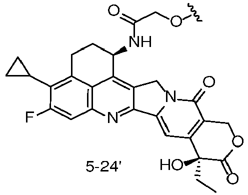
preferably, D is preferably selected from the group consisting of the following structures:

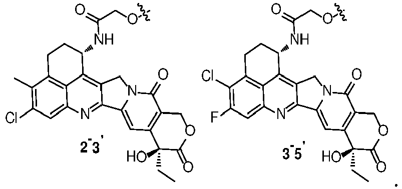

9. The compound or a pharmaceutically acceptable salt, ester, stereoisomer, polymorph, solvate, nitrogen oxide, isotope-labeled product, metabolite or prodrug thereof according to claim 8, wherein the compound has the structure as shown below: 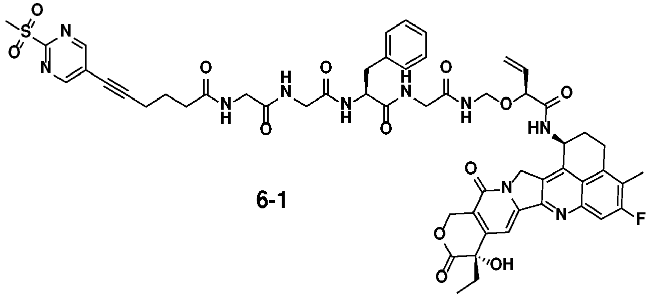 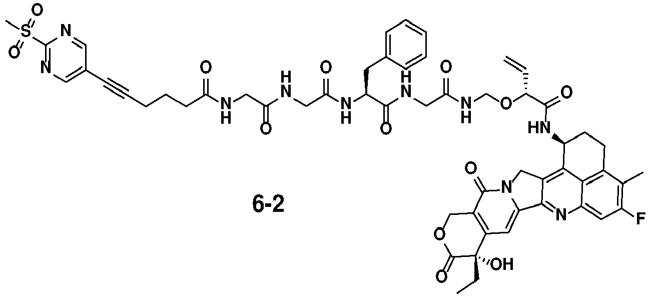 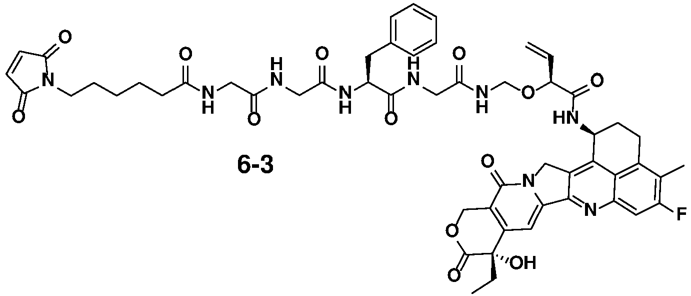 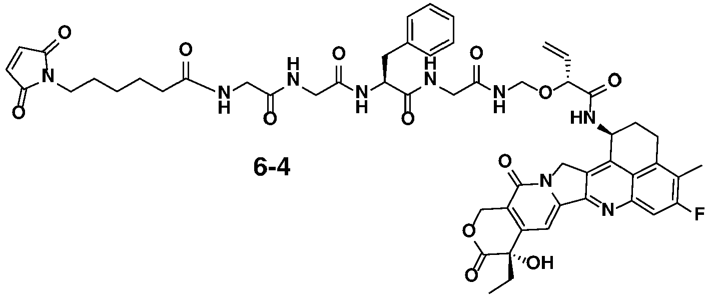 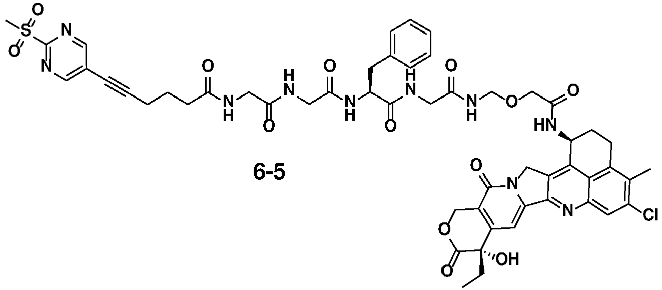 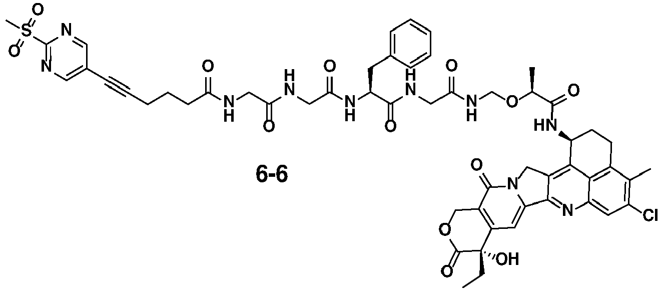 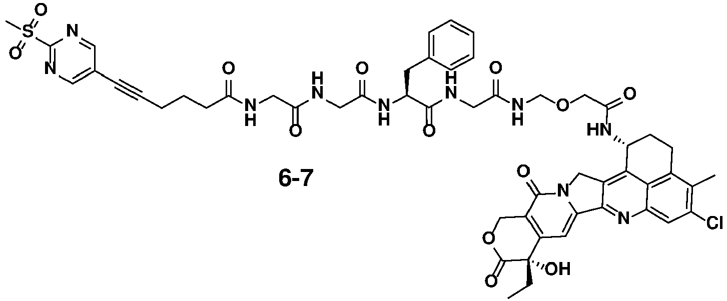 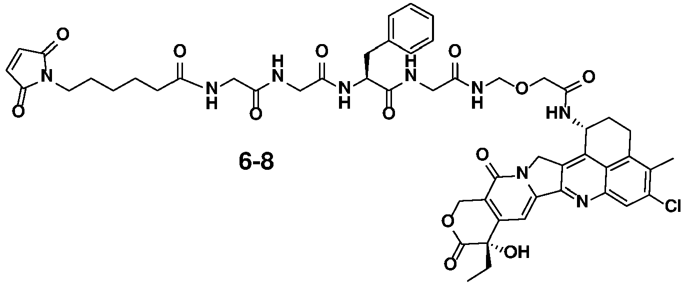 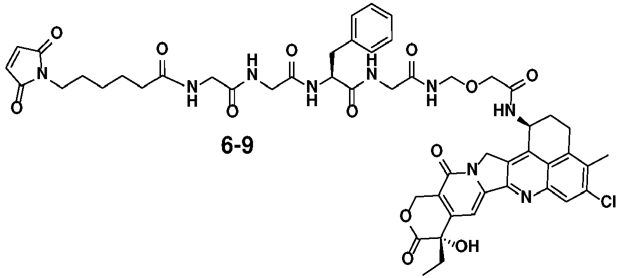 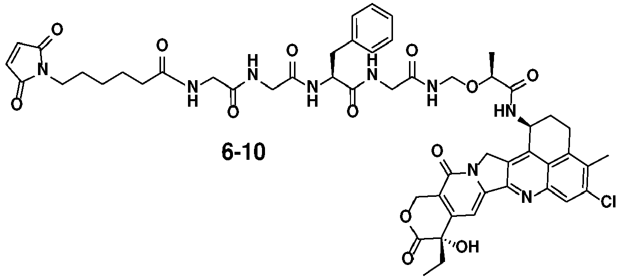 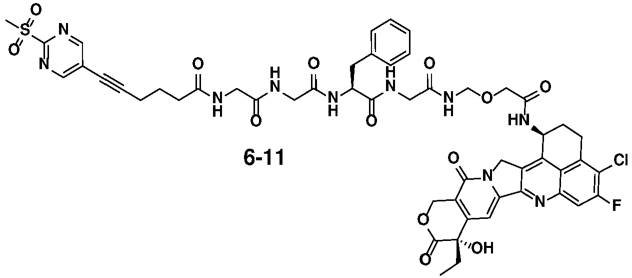 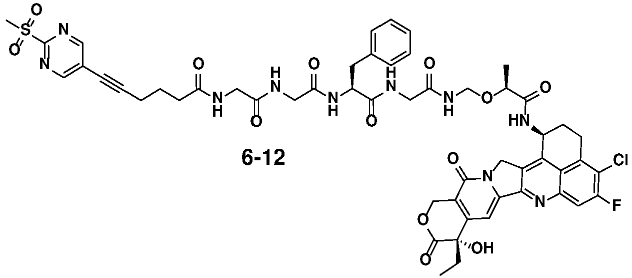 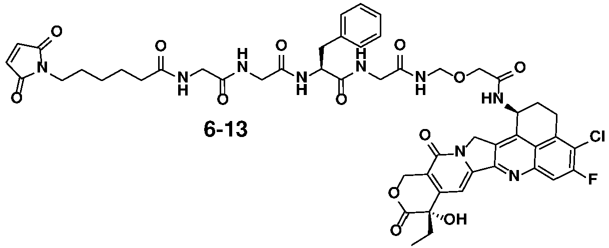 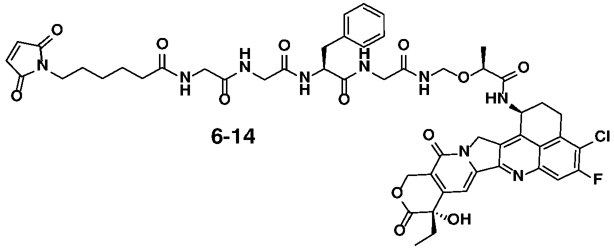 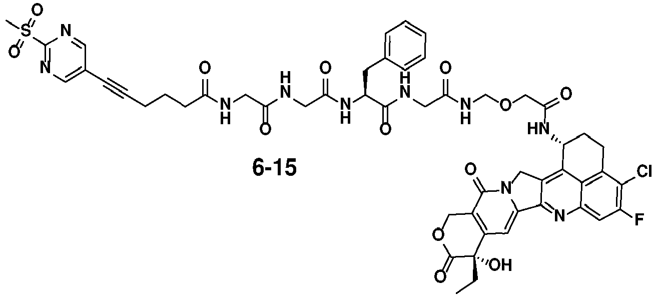 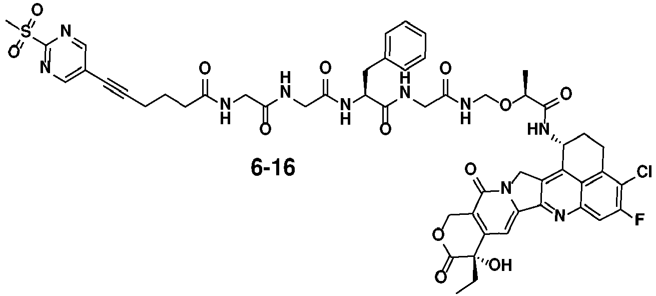 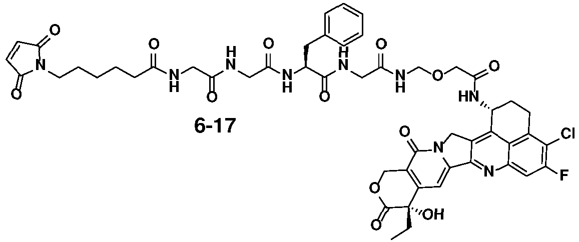 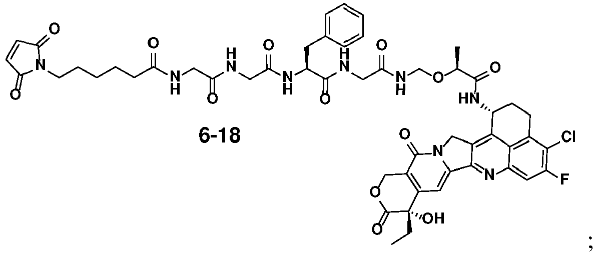 preferably, the compound is selected from:

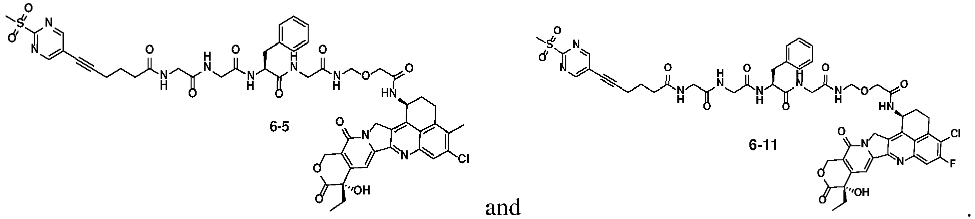

10. A pharmaceutical composition, which comprises the compound or a pharmaceutically acceptable salt, ester, stereoisomer, polymorph, solvate, nitrogen oxide, isotope-labeled product, metabolite or prodrug thereof according to any one of claims 1-9, and one or more pharmaceutically acceptable carriers.

11. A kit product, which comprises:
a) at least one of the compound or a pharmaceutically acceptable salt, ester, stereoisomer, polymorph, solvate, nitrogen oxide, isotope-labeled product, metabolite or prodrug thereof according to any one of claims 1-9, or the pharmaceutical composition according to claim 10, which is used as a first therapeutic agent;
b) optionally at least one additional therapeutic agent as a second therapeutic agent, or a pharmaceutical composition containing the additional therapeutic agent as a second pharmaceutical composition; and
c) optionally a packaging and/or instruction for use.

12. Use of the compound or a pharmaceutically acceptable salt, ester, stereoisomer, polymorph, solvate, nitrogen oxide, isotope-labeled product, metabolite or prodrug thereof according to any one of claims 1-9, the pharmaceutical composition according to claim 10 or the kit product according to claim 11 in the manufacture of a medicament for treating a disease related to abnormal cell proliferation.

13. A method for treating abnormal cell proliferation, comprising a step of administering to an individual in need thereof a therapeutically effective amount of the compound or a pharmaceutically acceptable salt, ester, stereoisomer, polymorph, solvate, nitrogen oxide, isotope-labeled product, metabolite or prodrug thereof according to any one of claims 1-9, or the pharmaceutical composition according to claim 10.

14. The compound or a pharmaceutically acceptable salt, ester, stereoisomer, polymorph, solvate, nitrogen oxide, isotope-labeled product, metabolite or prodrug thereof according to any one of claims 1-9, the pharmaceutical composition according to claim 10 or the kit product according to claim 11, for use in treating a disease related to abnormal cell proliferation.
